(19) Europäisches Patentamt / European Patent Office / Office européen des brevets

(11) **EP 3 194 959 B1**

(12) **EUROPEAN PATENT SPECIFICATION**

(45) Date of publication and mention of the grant of the patent:
**29.05.2019 Bulletin 2019/22**

(21) Application number: **15742293.2**

(22) Date of filing: **28.07.2015**

(51) Int Cl.:
***G01N 33/50*** *(2006.01)*

(86) International application number:
**PCT/EP2015/067301**

(87) International publication number:
**WO 2016/016258 (04.02.2016 Gazette 2016/05)**

(54) **MEANS AND METHODS FOR DIAGNOSING HEART FAILURE ON THE BASIS OF CHOLESTEROL PARAMETERS, SPHINGOMYELINS AND/OR TRIACYLGLYCEROLS**

MITTEL UND VERFAHREN ZUR DIAGNOSE VON HERZINFARKT AUF BASIS VON CHOLESTERINESTERN

MOYENS ET PROCÉDÉS PERMETTANT DE DIAGNOSTIQUER UNE INSUFFISANCE CARDIAQUE SUR LA BASE D'ESTERS DE CHOLESTÉRYLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.07.2014 EP 14178722**
**05.03.2015 US 201562128554 P**

(43) Date of publication of application:
**26.07.2017 Bulletin 2017/30**

(73) Proprietors:
• **Metanomics GmbH**
  **10589 Berlin (DE)**
• **Ruprecht-Karls-Universität Heidelberg**
  **69117 Heidelberg (DE)**

(72) Inventors:
• **SCHATZ, Philipp**
  **10435 Berlin (DE)**
• **WITT, Henning**
  **10435 Berlin (DE)**
• **PETER, Erik**
  **14473 Potsdam (DE)**
• **TERNES, Philipp**
  **14624 Dallgow (DE)**
• **MAPPES, Philipp**
  **13349 Berlin (DE)**
• **KATUS, Hugo, A.**
  **69120 Heidelberg (DE)**
• **WEIS, Tanja**
  **69257 Wiesenbach (DE)**
• **DUENGEN, Hans, Dirk**
  **13503 Berlin (DE)**
• **FREY, Norbert**
  **24119 Kronshagen (DE)**
• **TRIPPEL, Tobias, Daniel**
  **10435 Berlin (DE)**
• **TAHIROVIC, Elvis**
  **12053 Berlin (DE)**

(74) Representative: **Herzog, Fiesser & Partner Patentanwälte PartG mbB**
**Dudenstrasse 46**
**68167 Mannheim (DE)**

(56) References cited:
**WO-A1-2015/028671    WO-A2-2011/092285**
**WO-A2-2013/014286**

• **EVANS ET AL: "The use of three esterase kits to measure plasma cholesterol concentration in the rat and three other species", JOURNAL OF COMPARATIVE PATHOLOGY, ACADEMIC PRESS, LONDON, GB, vol. 96, no. 5, 1 September 1986 (1986-09-01), pages 551-556, XP022996704, ISSN: 0021-9975, DOI: 10.1016/0021-9975(86)90075-7 [retrieved on 1986-09-01]**

• HE X ET AL: "An Enzymatic Assay for Quantifying Sphingomyelin in Tissues and Plasma from Humans and Mice with Niemann-Pick Disease", ANALYTICAL BIOCHEMISTRY, ACADEMIC PRESS INC, NEW YORK, vol. 293, no. 2, 15 June 2001 (2001-06-15) , pages 204-211, XP027226815, ISSN: 0003-2697 [retrieved on 2001-06-15]
• Geoffrey S Ginsburg ET AL: "PHYSICAL PROPERTIES OF CHOLESTERYL ESTERS AND MOLECULAR MOTIONS CRYSTAL AND SMECTIC MESOPHASE STRUCTURE OF CHOLESTERYL ESTERS FROM X-P, AY POWDER PATTERNS SUMMARY REFERENCES", Prog. Lipid Res, 1 January 1984 (1984-01-01), pages 135-167, XP055155142, Retrieved from the Internet: URL:http://ac.els-cdn.com/016378278490002X /1-s2.0-016378278490002X-main.pdf?_tid=570 44d22-7552-11e4-9efa-00000aacb35f&acdnat=1 416995903_730d8b46ddb1fb55dc9d16fdc532ec3 c [retrieved on 2014-11-26]

**Description**

The present invention relates to diagnostic means and methods for heart failure. In particular, the invention relates to a method for diagnosing heart failure in a subject suspected to suffer therefrom comprising determining the amount of total sphingomyelins and total triacylglycerols in a sample of the said subject, and comparing the amount(s) determined in step (a) to a reference, whereby it is diagnosed whether the subject suffers from heart failure, or not. Moreover, the invention pertains to the use, in general, of enzymatic detection agents for at least one cholesterol parameter and/or total sphingomyelins and/or total triacylglycerols for diagnosing in a sample of a subject whether the said subject suffers from heart failure, or not. Finally, contemplated is a kit for diagnosing heart failure in a subject suspected to suffer therefrom.

The invention is defined by the appended claims.

Heart failure is a severe problem in modern medicine. The impaired function of the heart can give rise to life-threatening conditions and results in discomfort for the patients suffering from heart failure. Heart failure can affect the right or the left heart, respectively, and can vary in strength. A classification system was originally developed by the New York Heart association (NYHA). According to the classification system, the mild cases of heart failure are categorized as class I cases. These patients only show symptoms under extreme exercise. The intermediate cases show more pronounced symptoms already under less exercise (classes II and III) while class IV, shows already symptoms at rest (New York Heart Association. Diseases of the heart and blood vessels. Nomenclature and criteria for diagnosis, 6th ed. Boston: Little, Brown and co, 1964;114).

Patent document WO2013/014286 discloses diagnostic means and methods for diagnosing heart failure.

The prevalence of heart failure steadily increased in the population of the western developed countries over the last years. One reason for said increase can be seen in an increased average life expectation due to modern medicine. The mortality rate caused by heart failure, however, could be further reduced by improved diagnostic and therapeutic approaches. The so-called "Framingham" study reported a reduction of the 5 year mortality from 70% to 59% in men and from 57% to 45% in women when comparing a time window of 1950 to 1969 with 1990 to 1999 (Fox C.S. et al., Circulation 110, 522-527, 2004). The "Mayo" study shows a reduction from 65% to 50% for men for a time window of 1996 to 2000 compared to 1979 to 1984 and from 51% to 46% for women (Roger VL, Weston SA, Redfield MM, et al., JAMA-J. Am. Med. Assoc. 292, 344-350, 2004). Notwithstanding this reduction of the mortality rate, the overall mortality due to heart failure is still a major burden to societies. One-year mortality for NYHA class II to III patients under ACE inhibitor therapy is still between 9-12% (SOLVED study; The SOLVD Investigators, NEJM 325, 293-302, 1991; The SOLVD Investigators, New Engl. J. Med. 327, 685-691, 1992) and for NYHA class IV without ACE inhibitor therapy 52% (Consensus study; The Consensus Trial Study Group, New Engl. J. Med. 316, 1429-1435, 1987).

Zordoky discloses a metabolomics analysis of patients with heart failure with preserved ejection fraction (Zordoky et al. (2015) Metabolomic Fingerprint of Heart Failure with Preserved Ejection Fraction. PLoS ONE 10(5):e0124844. doi.10.1371/journal.pone.0124844).

Diagnostic techniques such as echocardiography are dependent on the experience of the individual investigator and, thus, not always reliable. Moreover, these techniques sometimes fail to diagnose the early onset of heart failure. Biochemical assays which are based on cardiac hormones such as brain natriuretic peptides (BNP) including amino-terminal pro-brain natriuretic peptide (NT-proBNP) are limited by their lack of sensitivity in early stages of heart failure (see Rodeheffer, R.J., J. Am. Coll. Cardiol. 44, 740-749, 2004) and are also influenced by other diseases and disorders such as renal insufficiency or depend on the overall physical condition of the patient (Balion C. et al. 2013. AHRQ Comparative Effectiveness Reviews. Agency for Healthcare Research and Quality (US), Rockville (MD)). This represents a huge medical problem since many patients progress through an asymptomatic phase of left ventricular systolic dysfunction (ALVSD) before the development of overt symptomatic disease (heart failure). Because pharmacotherapy in asymptomatic patients was shown to positively affect clinical outcome, a reliable diagnosis of treatable early stages and in consequence, guideline-driven treatment is expected to reduce morbidity and mortality and have a high health economic impact. Nevertheless, brain natriuretic peptides are the current gold standard for biochemically assessing heart failure. In groups of symptomatic patients, a diagnostic odds ratio of 27 for BNP compares with a sensitivity of 85% and specificity of 84% in detecting heart failure (Ewald 2008, Intern Med J 38 (2):101-13.).

However, it is a goal of modern medicine to reliably identify and treat patients with heart failure and, in particular, to identify them at the early onset of heart failure, i.e. at the early NYHA stages I to III and in particular at NYHA stage I. Accordingly, means and methods for reliably diagnosing heart failure are highly desired but not yet available.

The technical problem underlying the present invention can be seen as the provision of means and methods for complying with the aforementioned needs. The said technical problem is solved by the embodiments characterized in the claims and herein below.

The present invention relates to a method for diagnosing heart failure in a subject suspected to suffer therefrom comprising:

(a) determining the amount of total sphingomyelins and total triacylglycerols in a sample of the said subject; and

(b) comparing the amount(s) determined in step (a) to a reference, whereby it is diagnosed whether the subject suffers from heart failure, or not.

The method as referred to in accordance with the present invention includes a method which essentially consists of the aforementioned steps or a method which includes further steps.

However, it is to be understood that the method, in a preferred embodiment, is a method carried out ex vivo, i.e. not practised on the human or animal body. The method, preferably, can be assisted by automation.

The term "diagnosing" as used herein refers to assessing whether a subject suffers from the heart failure, or not. Thus, it is envisaged to diagnose the absence, and in particular, the presence of the disease. As will be understood by those skilled in the art, such an assessment, although preferred to be, may usually not be correct for 100% of the investigated subjects. The term, however, requires that a statistically significant portion of subjects can be correctly assessed and, thus, diagnosed. Whether a portion is statistically significant can be determined without further ado by the person skilled in the art using various well known statistic evaluation tools, e.g., determination of confidence intervals, p-value determination, Student's t-test, Mann-Whitney test, etc. Details are found in Dowdy and Wearden, Statistics for Research, John Wiley & Sons, New York 1983. Preferred confidence intervals are at least 50%, at least 60%, at least 70%, at least 80%, in particular at least 90% or at least 95%. Further preferred confidence intervals are at least 99% or 99.9%. The p-values are, preferably, 0.2, 0.1, or 0.05. Alternatively, the p-value may be 0.01.

The term includes individual diagnosis of heart failure or its symptoms as well as continuous monitoring of a patient. Monitoring, i.e. diagnosing the presence or absence of heart failure or the symptoms accompanying it at various time points, includes monitoring of patients known to suffer from heart failure as well as monitoring of subjects known to be at risk of developing heart failure, in particular, symptomatic heart failure. Furthermore, monitoring can also be used to determine whether a patient is treated successfully or whether at least symptoms of heart failure can be ameliorated over time by a certain therapy. Moreover, the term also includes classifying a subject according to the New York Heart Association (NYHA) classes for heart failure. According to this classification, heart failure can be subdivided into four classes. Subjects exhibiting class I show no limitation in activities except under strong physical exercise. Subjects exhibiting class II show slight, mild limitation of activity, while comfortable at rest or under mild exertion. Subjects exhibiting class III show marked limitation of any activity, while comfortable only at rest. Subjects exhibiting class IV show discomfort and symptoms even at rest. Preferably, heart failure to be determined in accordance with the present invention is asymptomatic heart failure, i.e. heart failure according to NYHA class I, or symptomatic heart failure, i.e. heart failure at least according to NYHA class II and/or III.

Another staging system is provided by the American Heart Association. Four stages of heart failure are subdivided: Stage A: Patients at high risk for developing HF in the future but no functional or structural heart disorder. Stage B: a structural heart disorder but no symptoms at any stage. Stage C: previous or current symptoms of heart failure in the context of an underlying structural heart problem, but managed with medical treatment. Stage D: advanced disease requiring hospital-based support, a heart transplant or palliative care. It will be understood that the method of the present invention can also be used for staging heart failure according to this system, preferably, the identified biomarkers shall allow to diagnose heart failure according to stages A to C and to discriminate between the asymptomatic stages A and B and the more severe stage C, i.e. symptomatic heart failure.

The term "heart failure" as used herein relates to an impaired function of the heart. Preferably, heart failure as referred to herein is congestive heart failure. The said impairment can be a systolic dysfunction resulting in a significantly reduced ejection fraction of blood from the heart and, thus, a reduced blood flow. Specifically, systolic heart failure is characterized by a significantly reduced left ventricular ejection fraction (LEVF), preferably, an ejection fraction of less than 55% and, most preferably less than 40-35%. Alternatively, the impairment can be a diastolic dysfunction, i.e. a failure of the ventricle to properly relax. The latter is usually accompanied by a stiffer ventricular wall. The diastolic dysfunction causes inadequate filling of the ventricle and, therefore, results in consequences for the blood flow, in general. Thus, diastolic dysfunction also results in elevated end-diastolic pressures, and the end result is comparable to the case of systolic dysfunction (pulmonary edema in left heart failure, peripheral edema in right heart failure.) Heart failure may, thus, affect the right heart (pulmonary circulation), the left heart (body circulation) or both. Techniques for measuring an impaired heart function and, thus, heart failure, are well known in the art and include echocardiography, electrophysiology, angiography, and the determination of peptide biomarkers, such as the Brain Natriuretic Peptide (BNP) or the N-terminal fragment of its propeptide (NT-proBNP), in the blood. It will be understood that the impaired function of the heart can occur permanently or only under certain stress or exercise conditions. Dependent on the strength of the symptoms, heart failure can be classified as set forth elsewhere herein. Typical symptoms of heart failure include dyspnea, chest pain, dizziness, confusion, pulmonary and/or peripheral edema.

It will be understood that the occurrence of the symptoms as well as their severity may depend on the severity of heart failure and the characteristics and causes of the heart failure, systolic or diastolic or restrictive i.e. right or left heart located heart failure. Further symptoms of heart failure are well known in the art and are described in the standard text books of medicine, such as Stedman or Brunnwald.

Heart failure is, preferably, the final common stage of many cardiovascular diseases and is defined as a clinical syndrome in which patients in the final stage show typical signs and symptoms of effort intolerance and/or fluid retention resulting from an abnormality of cardiac structure or function. As outlined further herein below, the term "heart failure" in the context of the present invention encompasses both symptomatic forms and asymptomatic forms of heart failure. Accordingly, e.g., asymptomatic left ventricular systolic dysfunction or asymptomatic diastolic dysfunction may be diagnosed.

Two subsets of heart failure with different pathophysiology are described based on a measurement of left ventricular ejection fraction (LVEF), which is the percentage of the total amount of blood in the left ventricle that is pushed out with each heartbeat: Heart failure with reduced left ventricular ejection fraction (HFrEF) and heart failure with preserved left ventricular ejection fraction (HFpEF).

Preferably, heart failure as used herein relates to systolic heart failure or heart failure with reduced ejection fraction (HFrEF).

HFrEF, also known as systolic heart failure, is characterized by reduced heart muscle contraction and emptying of the left ventricle. The expression "reduced left ventricular ejection fraction", preferably, relates to a left ventricular ejection fraction (LVEF) of lower than 50%. Moreover it is envisaged that the LVEF is lower than 40%, in particular lower than 35%. Also, the LVEF may be mildly reduced. Thus, the HFrEF may also be heart failure with a left ventricular ejection fraction of lower than 50% but larger than 35%.

Preferably, said HFrEF is associated with an ischemic cardiomyopathy (ICM) or a dilated cardiomyopathy (DCM). ICM results from coronary artery disease and/or prior infarction and happens when narrowed or blocked coronary arteries restrict the blood flow to the myocardium such that the tissue becomes damaged. DCM has non-ischemic causes, such as infection (myocarditis), hypertension, heart valve disease or idiopathic causes, and is characterized by an enlargement, dilatation or weakening of the left ventricle.

A subject who suffers from ICM, preferably, has a reduced LVEF and more than 50% coronary stenosis. A subject who suffers from DCM, preferably, has a reduced LVEF and less than 50% coronary stenosis. In particular, a subject who suffers from DCM, preferably, has a reduced LVEF, less than 50% coronary stenosis and a left ventricle wall thickness > 55 mm.Further, a subject who suffers from DCM may have a reduced LVEF, less than 50% coronary stenosis and a left ventricular end diastolic diameter of larger than 55 mm.

In the context of the studies underlying the present invention, it has been shown that the biomarkers and combinations of biomarkers as set forth herein were preferably used for diagnosing HFrEF, in particular ICM, with a LVEF of lower than 35%. Thus, the heart failure to be diagnosed is in an embodiment HFrEF, in particular ICM, with a LVEF of lower than 35%.

HFrEF in accordance with the method of the present invention may be symptomic or asymptomatic. Accordingly, the present invention, preferably, allows for the diagnosis of symptomic or asymptomatic systolic dysfunction.

As set forth above, the heart failure to be diagnosed may be heart failure with preserved left ventricular ejection fraction (HFpEF), also known as diastolic heart failure. The term "preserved left ventricular ejection fraction", preferably, refers to a LVEF of larger than 50%. Also envisaged is a LVEF of larger than 60%. Alternatively, the term "preserved left ventricular ejection fraction" preferably refers to a LVEF of larger than 55%. HFpEF is characterized by a disturbed relaxation and dilatation of the left ventricle. Diastolic left ventricular dysfunction results from LV hypertrophy and cardiac fibrosis resulting in increased myocardial stiffness. The muscle becomes stiff and loses some of its ability to relax, especially during diastole. As a result, the affected chamber has trouble filling with blood during diastole, leading to an elevated left ventricular filling pressure. HFpEF in accordance with the method of the present invention may be symptomic or asymptomatic. Accordingly, the present invention preferably allows for the the diagnosis of symptomic or asymptomatic diastolic dysfunction.

A subject suffering from HFpEF preferably has a cardium septum thickness of larger than 12 mm. Alternatively, the subject may have a cardium septum thickness of larger than 11 mm.

Moreover, heart failure as used herein relates to symptomatic or asymptomatic heart failure. Accordingly, the method of the present invention allows for diagnosing symptomatic heart failure and, in particular, asymptomatic heart failure. Thus, the present invention, in particular, allows for diagnosing symptomatic or asymptomic CHF, HFrEF, DCM, ICM and/or HFpEF.

Asymptomatic heart failure is preferably heart failure according to NHYA class I. Symptomatic heart failure is, preferably, heart failure according to NHYA class II, III and/or VI, in particular according to NHYA class II and/or III.

The term "sample" as used herein refers to samples from body fluids, preferably, blood, plasma, serum, saliva or urine, or samples derived, e.g., by biopsy, from cells, tissues or organs, in particular from the heart. More preferably, the sample is a blood, plasma or serum sample, most preferably, a plasma sample. The aforementioned samples can be derived from a subject as specified elsewhere herein. Techniques for obtaining the aforementioned different types of biological samples are well known in the art. For example, blood samples may be obtained by blood taking while tissue or organ samples are to be obtained, e.g., by biopsy.

In an embodiment of the present invention the sample is a fasting sample, in particular a fasting blood, plasma or serum sample. Thus, preferably, the sample is obtained from a fasting subject. A fasting subject, in particular, is a subject who refrained from food and beverages, except for water, prior to obtaining the sample to be tested. Preferably, a fasting subject refrained from food and beverages, except for water, for at least eight hours prior to obtaining the sample to be tested. More preferably, the sample has been obtained from the subject after an overnight fast. Preferably said fasting continued up to at least one hour before sample taking, more preferably up to at least 30 min before sample taking, still more preferable up to at least 15 min before sample taking, most preferably until the sample was taken.

The aforementioned samples are, preferably, pre-treated before they are used for the method of the present invention. As described in more detail below, said pre-treatment may include treatments required to release or separate the compounds or to remove excessive material or waste. Furthermore, pre-treatments may aim at sterilizing samples and/or removing contaminants such as undesired cells, bacteria or viruses. Suitable techniques comprise centrifugation, extraction, fractioning, e.g., by normal phase chromatography, gas chromatography, liquid chromatography or thin layer chromatography, ultrafiltration, protein precipitation followed by filtration and purification and/or enrichment of compounds. Moreover, other pre-treatments are carried out in order to provide the compounds in a form or concentration suitable for compound analysis. For example, if gas-chromatography coupled mass spectrometry is used in the method of the present invention, it will be required to derivatize the compounds prior to the said gas chromatography. For enzymatic determination, further sample pre-treatments may be required as discussed elsewhere herein or described in the accompanying Examples. Another kind of pre-treatment may be the storage of the samples under suitable storage conditions. Storage conditions as referred to herein include storage temperature, pressure, humidity, time as well as the treatment of the stored samples with preserving agents. Suitable and necessary pre-treatments also depend on the means used for carrying out the method of the invention and are well known to the person skilled in the art. Pre-treated samples as described before are also comprised by the term "sample" as used in accordance with the present invention.

In the case of the cholesterol parameters total cholesterol and total cholesteryl esters, it might be necessary to release the said cholesterol and cholesteryl esters from transport lipoproteins of the blood, e.g., apo-lipoproteins which form, e.g., high density, low density or very low density lipoproteins (HDL, LDL or VLDL). Moreover, the sample may be enriched for HDL, LDL and/or VLDL by, e.g., centrifugation and/or fractioning.

A sample as referred to in accordance with the present invention may, thus, comprise a subfraction of sphingomyelins obtained by any one of the aforementioned techniques which is representative for the amount of the total sphingomyelins. Similarly, subfractions of the cholesterol parameter or the total triacylglycerols may be used as samples. Also subfractions of the total cholesteryl esters may be used as samples. Moreover, the sample according to the present invention may also comprise a derivative derived from the sphingomyelins or the cholesterol parameter or the triacylglycerols which is representative for the amount of the said total sphingomyelins or the cholesterol parameter or the total triacylglycerols. Also moreover, the sample according to the present invention may comprise a derivative derived from the total cholesteryl esters which is representative for the amount of said total cholesteryl esters. As described, inter alia, in Example 3, below, typical derivatives of sphingomyelins may be molecules which can be obtained from all sphingomyelins in a stoichiometric manner such as O-methyl-sphingosine, threo-sphingosine, erythro-sphingosine and/or 1-hydroxy-2-amino-(cis,trans)-3,5-octadecadiene. Moreover, very long chain fatty acids (preferably saturated or monounsaturated), after derivatization, predominantly arise from sphingomyelins and, thus, may also be used as derivatives to be determined. As described, inter alia, in Example 5, below, cholesterol as a cholesterol parameter according to the invention may be obtained by hydrolysis of cholesteryl esters. Such hydrolysis may be performed enzymatically, using the enzyme esterase or an alternative enzyme capable of hydrolysing cholesteryl esters, or non-enzymatically. After such hydrolysis has been performed, the amount of cholesterol liberated from the cholesteryl esters is representative for the total amount of cholesteryl esters in the same sample.

The term "subject" as used herein relates to animals and, preferably, to mammals. More preferably, the subject is a primate and, most preferably, a human. The subject, preferably, is suspected to suffer from heart failure, more preferably, it may already show some or all of the symptoms associated with the disease or has been suggested to suffer from heart failure by other biomarkers such as natriuretic peptides and, in particular, NT-proBNP. However, also encompassed as subjects suspected to suffer from heart failure are those which belong into risk groups or subjects that are included in disease screening projects or measures. Thus, the subject to be investigated is, preferably, an adult subject, more preferably, a subject having an age of between 35 to 100 years, 40 to 90 years, 45 to 80 years, 45 to 75 years, 40 to 70 years or 50 to 70 years. More preferably, the subject is an asymptomatic subject exhibiting symptoms according to NYHA classes I or a symptomatic subject exhibiting symptoms according to NYHA class II and/or III. Moreover, the subject shall also preferably exhibit systolic heart failure due to contractile dysfunction such as dilated or ischemic cardiomyopathy. Preferably, the subject, however, is besides the aforementioned diseases and disorders apparently healthy. In particular, it shall, preferably, not exhibit symptoms according to NYHA class IV patients or suffer from stroke, myocardial infarction within the last 4 month before the sample has been taken or from acute or chronic inflammatory diseases and malignant tumors. Furthermore, the subject is preferably in stable medications within the last 4 weeks before the sample was taken.

In a preferred embodiment the subject is female. In another preferred embodiment, the subject is male.

Preferably, the subject may be overweight. A subject who is overweight, preferably, has a body mass index (BMI) of more than 28.0 kg/m$^2$, in particular of more than 30.0 kg/m$^2$.

Also preferably, the subject is not overweight. A subject who is not overweight, preferably, has a body mass index (BMI) of less than 28.0 kg/m$^2$, in particular of less than 25.0 kg/m$^2$.

The subject to be tested in accordance with the present invention, preferably, shows symptoms of heart failure. In this case, it is in particular diagnosed whether the subject suffers from symptomatic heart failure (or for example symptomatic HFrEF, DCM, ICM or HFpEF). More preferably, the subject does not show symptoms of heart failure. In this case asymptomatic heart failure is diagnosed. In this case, it is in particular diagnosed whether the subject suffers from asymptomatic heart failure (or asymptomatic HFrEF, DCM, ICM or HFpEF). However, it is also envisaged to diagnose the absence of the disease.

In aged and/or overweight subjects, NT-proBNP and BNP have been reported to be less reliable markers for heart failure. Therefore, the subject may be aged or overweight. In one embodiment, the subject is older than 60 years of age, in particular, older than 70 years of age. In yet another embodiment, the subject has a body mass index (BMI) of more than 28.0 kg/m$^2$, in particular of more than 30.0 kg/m$^2$.

In an embodiment, the subject to be tested has a body mass index of larger than 28 m$^2$/kg, preferably, larger than 30 m$^2$/kg. Even more preferably, the subject is also male. Advantageously, the use of the biomarkers as referred to herein allows for reducing the rate of false negative diagnostic tests in said subjects.

In another embodiment, the subject to be tested is female. Preferably, the subject also has a body mass index of lower than 30 m$^2$/kg, even more preferably, lower than 28 m$^2$/kg. Advantageously, the use of the biomarkers as referred to herein allows for reducing the rate of false positive diagnostic tests in said subjects.

The term "total amount of sphingomyelins" refers to the amount of the entirety of sphingomyelins present in the sample to be investigated. The entirety of sphingomyelines or the amount thereof is sometimes referred to herein as biomarker. As described elsewhere herein, representative subfractions or derivatives of total sphingomyelins may also serve as biomarkers indicative for the total amount of the sphingomyelins.

The term "cholesterol parameter" as used in accordance with the present invention refers to a parameter selected from the group consisting of: total cholesterol, total cholesteryl esters and the sum parameter of total cholesterol and total cholesteryl esters.

Under the term "at least one cholesterol parameter" it will be understood that in accordance with the method of the present invention, one or more of the aforementioned cholesterol parameters can be determined. For example, the amount of total cholesteryl esters and the amount of total cholesterol can be (separately) determined. Likewise, the amount of total cholesterol and the sum parameter of total cholesterol and total cholesteryl esters can be determined or the amount of total cholesteryl esters and the sum parameter of total cholesterol and total cholesteryl esters can be determined. Further, total cholesterol, total cholesteryl esters and the sum parameter of total cholesterol and total cholesteryl esters may be determined. Thus, at least one in the sense of the invention may be, preferably, one, two or three. Therefore, the determination of at least one cholesterol parameter relates to the determination of each combination of two of the aforementioned parameters. Also preferably, all three cholesterol parameters mentioned above may be determined.

More preferably, the at least one cholesterol parameter in accordance with the invention relates to the determination of the two cholesterol parameters total cholesteryl esters and total cholesterol. Also more preferably, the at least one cholesterol parameter may be either total cholesterol or total cholesteryl esters alone. In another embodiment the at least one cholesterol parameter in accordance with the invention relates to the determination of the two cholesterol parameters total cholesterol and the sum parameter of total cholesterol and total cholesteryl esters. Particular combinations of cholesterol parameters with the other biomarkers referred to herein to be determined in accordance with the present invention are disclosed, e.g., in Table 19, 20, or 21, below. In accordance with the claims, one or more of the following biomarkers or combinations of biomarkers are to be used in the method of the present invention (see, e.g., Examples 9, 10, and 11): combinations of biomarkers which are not covered by the scope of the claims, are descriptive examples.

- Total sphingomyelins, total cholesteryl esters, total cholesterol, and NT-proBNP (Such as panel 1 as described in the Examples section + NT-proBNP);
- Total sphingomyelins, total cholesteryl esters, and total cholesterol (Such as panel 1);
- Total sphingomyelins, total cholesteryl esters, and NT-proBNP (Such as panel 2 + NT-proBNP);
- Total sphingomyelins and total cholesteryl esters (Such as panel 2);
- Total sphingomyelins, total cholesterol, and NT-proBNP (Such as panel 3 + NT-proBNP);
- Total sphingomyelins, and total cholesterol (Such as panel 3);
- Total sphingomyelins, and NT-proBNP (Such as panel 4 + NT-proBNP);
- Total sphingomyelins (Such as panel 4);

- Total cholesteryl esters, total cholesterol, and NT-proBNP (Such as panel 5 + NT-proBNP);
- Total cholesteryl esters, and total cholesterol (Such as panel 5);
- Total cholesteryl esters, and NT-proBNP (Such as panel 6 + NT-proBNP);
- Total cholesteryl esters (Such as panel 6);
- Total cholesterol, and NT-proBNP (Such as panel 7 + NT-proBNP);
- Total cholesterol (Such as panel 7);
- Total sphingomyelins, total cholesteryl esters, total cholesterol, total triacylglcerols, and NT-proBNP (Such as panel 8 + NT-proBNP);
- Total sphingomyelins, total cholesteryl esters, total cholesterol, and total triacylglcerols (Such as panel 8);
- Total sphingomyelins, total cholesteryl esters, total triacylglcerols, and NT-proBNP (Such as panel 9 + NT-proBNP);
- Total sphingomyelins, total cholesteryl esters, and total triacylglcerols (Such as panel 9);
- Total sphingomyelins, total cholesterol, total triacylglcerols, and NT-proBNP (Such as panel 10 + NT-proBNP);
- Total sphingomyelins, total cholesterol, and total triacylglcerols (Such as panel 10).

In a further preferred embodiment it is envisaged to use the following combinations of biomarkers:

- Total sphingomyelins and total triacylglcerols (Such as panel 11)
- Total sphingomyelins, total triacylglcerols, and NT-proBNP (Such as panel 11 + NT-proBNP).
- Total sphingomyelins, sum parameter of total cholesteryl esters and total cholesterol, total cholesterol, total triacylglcerols, and NT-proBNP;
- Total sphingomyelins, sum parameter of total cholesteryl esters and total cholesterol, total cholesterol, total triacylglcerols;
- Total sphingomyelins, sum parameter of total cholesteryl esters and total cholesterol, total triacylglcerols, and NT-proBNP;
- Total sphingomyelins, sum parameter of total cholesteryl esters and total cholesterol, and total triacylglcerols);

In particular, the following combinations are preferred.

- Total sphingomyelins, total cholesteryl esters, total triacylglcerols (Such as panel 9)
- Total sphingomyelins, sum parameter of total cholesteryl esters and total cholesterol, total triacylglcerols;
- Total sphingomyelins, total cholesteryl esters, total triacylglcerols, and NT-proBNP (Such as panel 9 + NT-proBNP);
- Total sphingomyelins, sum parameter of total cholesteryl esters and total cholesterol, total triacylglcerols, and NT-proBNP;
- Total sphingomyelins, total cholesterol, total triacylglcerols (Such as panel 10)
- Total sphingomyelins, total cholesterol, total triacylglcerols, and NT-proBNP (Such as panel 10 + NT-proBNP);
- Total sphingomyelins, total cholesteryl esters, total cholesterol, total triacylglcerols (Such as panel 8)
- Total sphingomyelins, sum parameter of total cholesteryl esters and total cholesterol, total cholesterol, total triacylglcerols;
- Total sphingomyelins, total cholesteryl esters, total cholesterol, total triacylglcerols, and NT-proBNP (Such as panel 8 + NT-proBNP);
- Total sphingomyelins, sum parameter of total cholesteryl esters and total cholesterol, total cholesterol, total triacylglcerols, and NT-proBNP;
- Total sphingomyelins and total triacylglcerols (Such as panel 11).
- Total sphingomyelins, total triacylglcerols, and NT-proBNP (Such as panel 11 + NT-proBNP).

Using any panel selected from panels 8 + NT-proBNP, 9 + NT-proBNP, 10 + NT-proBNP, and 11 + NT-proBNP, highest positive likelihood ratios (LR+) could be observed for CHF subgroups with severely reduced LVEF (subgroup 'ICM LVEF < 35%', followed by 'HFrEF LVEF < 35%'). The present invention therefore in particular envisages the determination of the biomarkers of panels 8, 9, 10, or 11, preferably in combination with BNP or NT-proBNP, for the diagnosis HFrEF, in particular ICM, with a LVEF of lower than 35%. Preferably, the subject to be tested shows symptoms of heart failure. The use of these panels in these groups allows for high positive likelihood ratios.

With respect to mild or asymptomatic forms of CHF, highest LR+ could be observed for mild or asymptomatic forms of ICM (subgroups 'ICM asymptomatic' and 'ICM LVEF 35% to 50%'), followed by mild or asymptomatic forms of HFrEF (subgroups 'HFrEF asymptomatic' and 'HFrEF LVEF 35% to 50%'), using any panel selected from panels 8 + NT-proBNP, 9 + NT-proBNP, 10 + NT-proBNP, and 11 + NT-proBNP. Thus, these panels are preferably used for the diagnosis of ICM in a patient who is asymptomatic and/or has a LVEF of larger than 35% but lower than 50%. The use of these panels (in particular in combination with NT-proBNP) in these groups allows for high positive likelihood ratios.

Highest PPV based on the prevalence estimates given in Example 13 could be observed for CHF, using any panel selected from panels 8 + NT-proBNP, 9 + NT-proBNP, 10 + NT-proBNP, and 11 + NT-proBNP, combined with any of the cut-off values determined by one of the methods 'Match sensitivity' or 'Match sensitivity + 5%. Thus, these marker combinations are preferably used for the diagnosis of heart failure in general.

As compared to NT-proBNP alone, highest net reclassification index (NRI) could be observed in subgroup 'HFpEF asymptomatic' followed by 'ICM symptomatic', using any panel selected from the panels 8 + NT-proBNP, 9 + NT-proBNP, and 10 + NT-proBNP. Thus, these panels (in particular in combination with NT-proBNP) are preferably used for the diagnosis of HFpEP in asymptomatic patients, or for the diagnosis of ICM in patients showing symptoms of heart failure. The use of these panels allows for a high NRI in these groups.

With respect to HFpEF, highest NRI could be observed for the subgroup 'HFpEF asymptomatic' followed by 'HFpEF', using any panel selected from panels 8 + NT-proBNP, 9 + NT-proBNP, 10 + NT-proBNP, and 11 + NT-proBNP. Thus, the panels (in particular in combination with NT-proBNP) are preferably used for the diagnosis HFpEF in asymptomatic patients. The use of the these panels allows for a high NRI in these groups.

With respect to the HFpEF subgroups 'HFpEF, 'HFpEF asymptomatic', and 'HFpEF symptomatic', highest NRI was observed with panel 9 + NT-proBNP. Thus, the marker combination of panel 9(+NT-proBNP) is preferably used for the diagnosis of HFpEF, in particular asymptomatic or symptomatic HFpEF. Thus, these panels(in particular in combination with NT-proBNP) can be used for reliably diagnosing asymptomatic and symptomatic HFpEF. In particular, these panels allow for a high NRI.

As compared to NT-proBNP alone, highest improvements in LR+ could be observed in the subgroup 'ICM LVEF < 35%' using any panel selected from panels 8 + NT-proBNP, 9 + NT-proBNP, 10 + NT-proBNP, and 11 + NT-proBNP. Also, subgroup 'HFrEF LVEF < 35%' as well as subgroups showing symptoms of heart failure ('CHF symptomatic', 'HFrEF symptomatic', 'ICM symptomatic', and 'DCM symptomatic') show high improvements in LR+. Thus, these panels (in particular in combination with NT-proBNP) advantageously can be used for the diagnosis of HFrEF with of LVEF of lower than 35% as well as for diagnosis of CHF, HFrEF, ICM and DCM in symptomatic subjects. The use of these panels in the groups allows for high positive likelihood ratios

With respect to asymptomatic forms of HFrEF, highest improvements in LR+ could be observed in the subgroup 'HFpEF asymptomatic', followed by the subgroups 'CHF asymptomatic', 'HFrEF asymptomatic', and 'ICM asymptomatic', using any panel selected from panels 8 + NT-proBNP, 9 + NT-proBNP, 10 + NT-proBNP, and 11 + NT-proBNP. Thus, these panels(in particular in combination with NT-proBNP) advantageously can be used for the diagnosis of HFrEF with of LVEF of lower than 35%. as well as for diagnosis of CHF, HFpEF and ICM in asymptomatic subjects. The use of these panels in the groups allows for high positive likelihood ratios

Highest improvements in LR+ were observed with Panel 9 + NT-proBNP or Panel 11 + NT-proBNP in combination with a cut-off value determined according to the method 'Match sensitivity' in most CHF subgroups. This shows that these panels (in particular in combination with NT-proBNP) can be reliably used for the diagnosis of CHF when using a cut-off value determined according to the method 'Match sensitivity'.

Highest improvements in LR+ in combination with a cut-off value determined according to the method 'Match sensitivity + 5%' were observed with Panel 11 + NT-proBNP in most CHF subgroups. Thus, panel 11 (in particular in combination with NT-proBNP) in combination with a cut-off value determined according to the method 'Match sensitivity + 5%' can be reliably used for the diagnosis of heart failure.

As compared to NT-proBNP alone, highest improvements in PPV (positive predictive value) were observed for subgroup 'HFrEF', using any panel selected from panels 8 + NT-proBNP, 9 + NT-proBNP, 10 + NT-proBNP, and 11 + NT-proBNP. Thus, the use of these panels (in particular in combination with NT-proBNP) advantagenously improves the PPV.

Further preferred marker combinations that can be used for the diagnosis for certain forms of heart failure (such as ICM, DCM etc.) are described in Example 13 and 14.

The term "total cholesterol" as used herein refers to the entirety of cholesterol present in the sample to be investigated. Said cholesterol is, preferably, free or molecular cholesterol, i.e. it is not covalently linked to other molecules, such as fatty acids that are esterified to cholesterol in the case of cholesteryl esters. However, total cholesterol shall encompass cholesterol molecules which are present in lipoproteins, e.g., HDL, LDL or VLDL. Moreover, cholesterol molecules which are part of the total cholesterol in a sample may also be associated with other proteins such as albumins. The entirety of cholesterol or the amount thereof is also sometimes referred to herein as biomarker. As described elsewhere herein, representative subfractions or derivatives of total cholesterol may also serve as biomarkers indicative for the total amount of the cholesterol.

The term "total amount of cholesteryl esters" as used herein refers to the amount of the entirety of cholesteryl esters present in the sample to be investigated. The entirety of cholesteryl esters or the amount thereof is also sometimes referred to herein as biomarker. As described elsewhere herein, representative subfractions or derivatives of total cholesteryl esters may also serve as biomarkers indicative for the total amount of the cholesteryl esters.

A further cholesterol parameter in accordance with the present invention is the sum of the amount of total cholesterol and the amount of total cholesteryl esters, i.e. the sum parameter of total cholesterol and total cholesteryl esters. The sum parameter of total cholesterol and total cholesteryl esters is also sometimes referred to herein as biomarker.

In Examples 9, 10, and 11, the sum of the amount of total amount of cholesteryl esters and total cholesterol (herein also referred to the sum parameter of total cholesterol and total cholesteryl esters, see previous paragraph) was taken as approximation for the amount of cholesteryl esters. In an embodiment of the present invention, it is envisaged to determine the amount of total cholesteryl esters by determining the sum of the amount of total amount of cholesteryl esters and total cholesterol.

The term "total amount of triacylglycerols" as used herein refers to the amount of the entirety of triacylglycerols present in the sample to be investigated. The entirety of triacylglycerols or the amount thereof is also sometimes referred to herein as biomarker. As described elsewhere herein, representative subfractions or derivatives of total triacylglycerols may also serve as biomarkers indicative for the total amount of the triacylglycerols.

It will be understood that in accordance with the present invention, any combination of the aforementioned biomarkers can be used, i.e. determined and, preferably, evaluated together in the method of the present invention. Preferably, the at least one cholesterol parameter can be used together with total sphingomyelins. Likewise, one or more cholesterol parameters can be used together with total triacylglycerols. Furthermore, total sphingomyelins can be used together with total triacylglycerols. Even more preferably, all biomarkers can be used together, i.e. the at least one cholesterol parameter, total sphingomyelins and total triacylglycerols.

The term "determining the amount" as used herein refers to determining at least one characteristic feature of a biomarker, i.e. total sphingomyelins, at least one cholesterol parameter (for example total cholesterylesters) or total triacylglycerides, to be determined by the method of the present invention in the sample. Characteristic features in accordance with the present invention are features which characterize the physical and/or chemical properties including biochemical properties of a biomarker. Such properties include, e.g., molecular weight, viscosity, density, electrical charge, spin, optical activity, colour, fluorescence, chemoluminescence, elementary composition, chemical structure, capability to bind to a capturing agent (e.g. antibody, aptamers), capability to react with other compounds, capability to elicit a response in a biological read out system (e.g., induction of a reporter gene) and the like. Values for said properties may serve as characteristic features and can be determined by techniques well known in the art. Moreover, the characteristic feature may be any feature which is derived from the values of the physical and/or chemical properties of a biomarker by standard operations, e.g., mathematical calculations such as multiplication, division or logarithmic calculus. Most preferably, the at least one characteristic feature allows the determination and/or chemical identification of the said at least one biomarker and its amount. Accordingly, the characteristic value, preferably, also comprises information relating to the abundance of the biomarker from which the characteristic value is derived. For example, a characteristic value of a biomarker may be a peak in a mass spectrum. Such a peak contains characteristic information of the biomarker, i.e. the m/z information, as well as an intensity value being related to the abundance of the said biomarker (i.e. its amount) in the sample.

As discussed before, the biomarkers comprised by a sample may be, preferably, determined in accordance with the present invention quantitatively or semi-quantitatively. For quantitative determination, either the absolute or precise amount of the biomarkers will be determined or the relative amount of the biomarker will be determined based on the value determined for the characteristic feature(s) referred to herein above. The relative amount may be determined in a case were the precise amount of a biomarker can or shall not be determined. In said case, it can be determined whether the amount in which the biomarker is present is enlarged or diminished with respect to a second sample comprising said biomarker in a second amount. In a preferred embodiment said second sample comprising said biomarker shall be a calculated reference as specified elsewhere herein. Quantitatively analysing a biomarker, thus, also includes what is sometimes referred to as semi-quantitative analysis of a biomarker.

The aforementioned total amounts of triacylglycerols or sphingomyelins or the at least one cholesterol parameter (e.g. cholesteryl esters) can be determined by any suitable detection agent or detection device which allow for a specific determination of the total amounts. Suitable detection agents, preferably, include agents which specifically bind to the at least one cholesterol parameter (e.g. cholesteryl esters), the total sphingomyelins or the total triacylglycerides or derivatives thereof described elsewhere herein. Such agents may be antibodies or aptamers whose binding to the at least one cholesterol parameter (e.g. cholesteryl esters), the triacylglycerols or sphingomyelins can be determined by means well known in the art, such as secondary or higher order antibodies linked to a detectable label such as a fluorophore or chromophore. Specific antibodies, for instance, may be obtained using the biomarker as antigen by methods well known in the art. Antibodies as referred to herein include both polyclonal and monoclonal antibodies, as well as fragments thereof, such as Fv, Fab and F(ab)$_2$ fragments that are capable of binding the antigen or hapten. The present invention also includes humanized hybrid antibodies wherein amino acid sequences of a non-human donor antibody exhibiting a desired antigen-specificity are combined with sequences of a human acceptor antibody. Moreover, encompassed are single chain antibodies. The donor sequences will usually include at least the antigen-binding amino acid residues of the donor but may comprise other structurally and/or functionally relevant amino acid residues of the donor antibody as well. Such hybrids can be prepared by several methods well known in the art. Aptamers can be generated according to standard techniques. Aptameres as used herein are oligonucleic acid or peptide molecules that bind to a specific target molecule (Ellington 1990, Nature 346 (6287): 818-22). Bock 1992, Nature 355 (6360): 564-6). Oligonucleic acid aptamers are engineered through repeated rounds of selection or the so called systematic evolution of ligands by exponential enrichment (SELEX technology). Peptide aptamers are designed to interfere with molecular interactions inside cells. They usually comprise a variable peptide loop attached at both ends to a protein scaffold. This double structural constraint shall increase the binding affinity of the peptide aptamer into the nanomolar range. Said variable peptide loop length is, preferably, composed of ten to twenty amino acids, and the scaffold may be any protein having improved solubility and compact properties, such as thioredoxin-A. Suitable measurement methods based on the aforementioned specific detection agents include RIA (radioimmunoassay), ELISA (enzyme-linked immunosorbent assay), sandwich enzyme immune tests, electrochemiluminescence immunoassays such as electrochemiluminescence sandwich immunoassays (ECLIA), dissociation-enhanced lanthanide fluoro immuno assay (DELFIA), scintillation proximity assay (SPA), turbidimetry, nephelometry, latex-enhanced turbidimetry or nephelometry, solid phase immune tests or affimer technology based tests (Avacta, US). Further methods known in the art, such as gel electrophoresis, 2D gel electrophoresis, SDS polyacrylamid gel electrophoresis (SDS-PAGE), Western Blotting, surface plasmon resonance, IR spectroscopy, and mass spectrometry, can be used alone or in combination with labelling or other detection methods as described above.

The total amounts of triacylglycerols or sphingomyelins or the at least one cholesterol parameter (e.g. cholesteryl esters) can, however, also be determined by detection devices which allow for the specific detection of the said biomarkers. Determining of the biomarkers can, preferably, comprise mass spectrometry (MS). Mass spectrometry as used herein encompasses all techniques which allow for the determination of the molecular weight (i.e. the mass) or a mass variable corresponding to one or more compounds, i.e. a biomarker, to be determined in accordance with the present invention. Preferably, mass spectrometry as used herein relates to GC-MS, LC-MS, direct infusion mass spectrometry, FT-ICR-MS, CE-MS, HPLC-MS, quadrupole mass spectrometry, any sequentially coupled mass spectrometry such as MS-MS or MS-MS-MS, ICP-MS, Py-MS, TOF, MALDI-TOF or any combined approaches using the aforementioned techniques. How to apply these techniques is well known to the person skilled in the art. Typically, the compounds to be detected are ionized during MS and are brought into an electrical field. Moreover, suitable devices are commercially available. More preferably, mass spectrometry as used herein relates to LC-MS and/or GC-MS, i.e. to mass spectrometry being operatively linked to a prior chromatographic separation step. More preferably, mass spectrometry as used herein encompasses quadrupole MS. Most preferably, said quadrupole MS is carried out as follows: a) selection of a mass/charge quotient (m/z) of an ion created by ionisation in a first analytical quadrupole of the mass spectrometer, b) fragmentation of the ion selected in step a) by applying an acceleration voltage in an additional subsequent quadrupole which is filled with a collision gas and acts as a collision chamber, c) selection of a mass/charge quotient of an ion created by the fragmentation process in step b) in an additional subsequent quadrupole, whereby steps a) to c) of the method are carried out at least once and analysis of the mass/charge quotient of all the ions present in the mixture of substances as a result of the ionisation process, whereby the quadrupole is filled with collision gas but no acceleration voltage is applied during the analysis. The ionization technique in step a) preferably is electrospray ionization (ESI). Also preferably, the ionization technique in step a) is atmospheric pressure chemical ionization (AP-CI). Also preferably, the ionization technique in step a) is preferably matrix assisted laser desorption/ionization (MALDI). Details on said most preferred mass spectrometry to be used in accordance with the present invention can be found in WO2003/073464. Based on the mass spectrum generated by any of the aforementioned techniques, the amount of the entirety of a class of compounds, i.e. the total cholesteryl esters as at least one cholesterol parameter, the total triacylglycerols or the total sphingomyelins, can be determined, e.g. by summing up the amounts of the individual compounds belonging to said class determined in the mass spectrum.

More preferably, said mass spectrometry referred to herein is liquid chromatography (LC) MS and/or gas chromatography (GC) MS. Liquid chromatography as used herein refers to all techniques which allow for separation of compounds (i.e. metabolites) in liquid or supercritical phase. Liquid chromatography is characterized in that compounds in a mobile phase are passed through the stationary phase. When compounds pass through the stationary phase at different rates they become separated in time since each individual compound has its specific retention time (i.e. the time which is required by the compound to pass through the system). Liquid chromatography as used herein also includes HPLC. Devices for liquid chromatography are commercially available, e.g. from Agilent Technologies, USA. Gas chromatography as applied in accordance with the present invention, in principle, operates comparable to liquid chromatography. However, rather than having the compounds (i.e. metabolites) in a liquid mobile phase which is passed through the stationary phase, the compounds will be present in a gaseous volume. The compounds pass the column which may contain solid support materials as stationary phase or the walls of which may serve as or are coated with the stationary phase. Again, each compound has a specific time which is required for passing through the column. Moreover, in the case of gas chromatography it is preferably envisaged that the compounds are derivatised prior to gas chromatography. Suitable techniques for derivatisation are well known in the art. Preferably, derivatisation in accordance with the present invention relates to methoxymation and trimethylsilylation of, preferably, polar compounds and transmethylation, methoxymation and trimethylsilylation of, preferably, non-polar (i.e. lipophilic) compounds.

As an alternative or in addition to mass spectrometry techniques, the following techniques may be used for compound determination: nuclear magnetic resonance (NMR), magnetic resonance imaging (MRI), Fourier transform infrared analysis (FT-IR), ultraviolet (UV) spectroscopy, refraction index (RI), fluorescent detection, radiochemical detection, electrochemical detection, light scattering (LS), dispersive Raman spectroscopy or flame ionisation detection (FID). These techniques are well known to the person skilled in the art and can be applied without further ado. Based on the spectra generated by any of the aforementioned techniques, the amount of the entirety of a class of compounds, i.e. the total cholesteryl esters as at least one cholesterol parameter, the total triacylglycerols or the total sphingomyelins, can be determined, e.g. by summing up the amounts of the individual compounds belonging to said class determined in the mass spectrum.

The biomarkers according to the present invention, may also be determined by a biosensor adapted for determining them. Biosensors typically comprise a biological sensor element, such as a tissue, a cell or microorganism or a biological sensor molecule, e.g., an enzyme, antibody, aptamere, receptor, affimere, nucleic acid, etc. Moreover, they also comprise typically a detector element which produces a detectable signal, such as a physical, physicochemical, optical, electrochemical, or biological signal. The said signal can then be detected by a biosensor reader system. Usually, biosensors comprising the enzymes referred to elsewhere herein arranged in a way such that the detection reactions can be performed may be applied according to the invention for the determination of the biomarkers.

Yet as an alternative, the biomarkers of the invention may be determined by determining a representative subfraction or derivative of the said biomarkers. Preferred representative subfractions or derivatives are disclosed elsewhere herein. Typically, the molecular species present in the subfractions or the derivatives can be determined by mass spectroscopy techniques referred to above or by, e.g., nuclear magnetic resonance (NMR), magnetic resonance imaging (MRI), Fourier transform infrared analysis (FT-IR), ultraviolet (UV) spectroscopy, refraction index (RI), fluorescent detection, radiochemical detection, electrochemical detection, light scattering (LS), dispersive Raman spectroscopy or flame ionisation detection (FID). Based on the determined amounts of the said subfractions or derivatives, the amounts of the total sphingomyelins, the total triacylglycerols or the at least one cholesterol parameter can subsequently be calculated.

Preferably, the said total amount of cholesteryl esters, total amount of cholesterol, total amount of triacylglycerols and/or total amount of sphingomyelins are determined enzymatically. To this end, it is envisaged that enzymes are applied which specifically recognize cholesterylesters, cholesterol, triacylglycerols or sphingomyelins as substrates and which convert said substrates such that a detectable signal can be generated. Typically, the enzymatic conversion generates, e.g., redox equivalents e.g. in the form of $H_2O_2$. Said redox equivalents can be in turn detected in a further reaction by a peroxidase (such as a horseradish peroxidase) and a chromogenic substrate. The substrate is typically oxidized by the peroxidase using $H_2O_2$ as the oxidizing agent. The catalyzed reaction in the presence of $H_2O_2$, peroxidase, and the substrate typically results in a characteristic change that is detectable by spectrophotometric methods. E.g., the peroxidase catalyzes the conversion of chromogenic substrates into colored products, and produces light when acting on chemiluminescent substrates. For example, DAOS (N-ethyl-N-(2-hydroxy-3-sulfopropyl)-3,5-dimethoxyaniline) plus 4-aminoantipyrine in the presence of $H_2O_2$ and peroxidase results in the oxidative coupling of DAOS and 4-aminoantipyrine to form a blue chromogen. This chromogen can e.g. detected by measuring the absorbance of light at about 590 nm. Alternatively, 10-acetyl-3,7-dihydroxyphenoxazine can be used as substrate for peroxidase that enables detection of $H_2O_2$. This non-fluorescent reagent reacts with $H_2O_2$ to produce resorufin, a fluorescent compound. In another alternative 4-aminophenazon plus 4-chlorphenol in the presence of $H_2O_2$ and peroxidase result in the formation of 4-(p-benzochinonmonoimino)-phenazon, a red colored product, which can be determined by photometric means.

In a preferred embodiment, the enzymes used for the enzymatic determination of the amount(s) of at least one cholesterol parameter, total triacylglycerols and/or total sphingomyelins are non-human enzymes.

Preferably, the enzymatic determination of the amount(s) of at least one cholesterol parameter, total triacylglycerols and/or total sphingomyelins is based on artificial means, in particular on combinations which do not naturally occur in the sample to be tested.

For the enzymatic determination, sample pre-treatment may be required in order to allow the enzymes to get access to the analytes in a suitable way. Typically, the samples are physically and/or chemically treated such that the sphingomyelins, the cholesterol, the triacylglycerols or cholesteryl esters are released into solution. Physical treatments may include the application of heat or physical homogenization process. Chemical treatments may include solubilisation and extraction treatments by suitable buffers and agents, such as detergents, wetting agents and organic, preferably, apolar solvents. The analytes thus obtained, i.e. the total sphingomyelins or the total cholesteryl esters may than be diluted in an aqueous buffer system which allows for optimal enzyme activity. Such buffers are usually pH- and salt-optimized for the respective detection enzymes. Particular preferred sample pre-treatments are those described in the accompanying Examples, below, and in the manuals for the indicated detection assays.

The enzymatic determination of the amount of total cholesteryl esters, preferably, comprises the steps of:

a) contacting the sample with cholesterol esterase under conditions and for a time sufficient to allow conversion into cholesterol;
b) contacting the sample comprising the cholesterol with cholesterol oxidase under conditions and for a time sufficient to allow generation of $H_2O_2$; and
c) enzymatically or chemically determining the amount of generated $H_2O_2$.

More preferably, said method may comprise the further steps of:

- contacting the sample prior to step a) with cholesterol oxidase under conditions and for a time sufficient to allow generation of $H_2O_2$; and
- neutralizing the said $H_2O_2$.

In another preferred embodiment, the enzymatic determination of the amount of total cholesteryl esters comprises the steps of:

a) contacting the sample with cholesterol esterase under conditions and for a time sufficient to allow conversion into cholesterol;
b) contacting the sample comprising the cholesterol with cholesterol dehydrogenase under conditions and for a time sufficient to allow generation of redox equivalents and, preferably, NADH
c) enzymatically or chemically determining the amount of generated redox equivalents.

More preferably, said method may comprise the further steps of:

- contacting the sample prior to step a) with cholesterol dehydrogenase under conditions and for a time sufficient to allow generation of redox equivalents and, preferably, NADH and
- neutralizing the said redox equivalents.

Most preferably, the aforementioned determination method is carried out with plasma samples.

Said neutralizing the said redox equivalents and/or $H_2O_2$, in general, may comprise enzymatically or chemically determining the amount of generated redox equivalents and/or $H_2O_2$. The aforementioned additional step shall allow for a removal and/or determination of the total cholesterol present in a sample in addition to the total cholesteryl esters. Due to the treatment of the sample by cholesterol oxidase, the sample will be depleted of total cholesterol.

Even more preferably, the method of the invention further comprises the generation of the total cholesteryl esters to total cholesterol ratio based on the amounts of the cholesterol-derived redox equivalents and/or $H_2O_2$ determined prior to step a) and the cholesteryl ester-derived redox equivalents and/or $H_2O_2$ determined in step c). Most preferably, said method further comprises the step of comparing the ratio of total cholesteryl esters to total cholesterol to a reference, whereby it is diagnosed whether the subject suffers from heart failure, or not.

Also preferably, the method of the invention further comprises determining the amount of cholesterol (in particular total cholesterol) based on the amounts of the cholesterol-derived redox equivalents and/or $H_2O_2$ in step c) upon omission of step a). Most preferably, said method further comprises comparing the amount of cholesterol (in particular total cholesterol) to a reference, whereby it is diagnosed whether the subject suffers from heart failure, or not.

In another preferred embodiment, the enzymatic determination of the amount of total cholesteryl esters may be performed by determining in the sample i) the sum of the amount of total cholesteryl esters and the amount total cholesterol as described above and ii) the amount of total cholesterol as described above, and calculating the difference of the amounts (i.e. the amount of i) minus the amount of ii)). Preferably, the amounts of i) and ii) are determined separately.

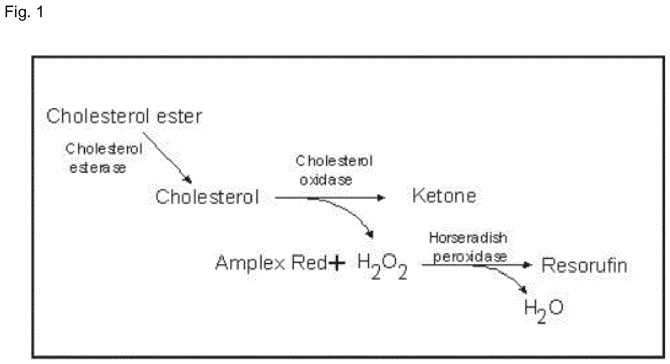

A particular preferred cholesterol/cholesteryl ester assay which can be applied in accordance with the present invention is disclosed in the accompanying Examples, below. The said assay is based on the conversion of cholesteryl esters into cholesterol by cholesterol esterase and the subsequent conversion of cholesterol into a ketone by a cholesterol oxidase whereby redox equivalents, e.g., in the form of $H_2O_2$ are generated. The latter one converts the dye amplex red (10-acetyl-3,7-dihydroxyphenoxazine) into resorufin which can be measured by its fluorescence. The assay is also described in detail in Held 2005, Biospektrum 2/05: 242-243 or Amudson 1999, Biochem Biophys Methods 38: 43-52 and can be commercially purchased from Cayman Chemical Company, Ann Arbor (US). Details on the reaction are also to be found in the Fig. 1, below. Furthermore, total cholesteryl esters and total cholesterol may be, preferably, determined by measuring the redox equivalents via conversion of 4-aminophenazone and phenol to 4-(p-benzoquinone-monoimino)-phenazone. Such an assay may be carried out on, e.g., Hitachi 704 Analyzer (Roche Diagnostics, US).

The enzymatic determination of the amount of total cholesterol, preferably, comprises the steps of:

a) contacting the sample comprising the cholesterol with cholesterol oxidase under conditions and for a time sufficient to allow generation of $H_2O_2$; and

b) enzymatically or chemically determining the amount of generated $H_2O_2$.

Alternatively, the enzymatic determination of the amount of total cholesterol, preferably, comprises the steps of:

a) contacting the sample comprising the cholesterol with cholesterol dehydrogenase under conditions and for a time sufficient to allow generation of redox equivalents and, preferably, NADH, and

b) enzymatically or chemically determining the amount of generated redox equivalents.

Alternatively, the amount of NADH can be determined. Thereby, the amount is total cholesterol is determined.

The enzymatic determination of the amount of total sphingomyelins, preferably, comprises the steps of:

a) contacting the sample with sphingomyelinase under conditions and for a time sufficient to allow conversion into phosphorylcholine;

b) contacting the sample comprising the phosphorylcholine with alkaline phosphatase and choline oxidase under conditions and for a time sufficient to allow conversion into choline and subsequent generation of $H_2O_2$; and

c) enzymatically or chemically determining the amount of generated $H_2O_2$.

More preferably, said method may comprise the further steps of:

- contacting the sample prior to step a) with alkaline phosphatase and choline oxidase under conditions and for a time sufficient to allow conversion into choline and subsequent generation of $H_2O_2$; and

- neutralizing the said $H_2O_2$.

Said neutralizing the said $H_2O_2$ may comprise enzymatically or chemically determining the amount of generated $H_2O_2$. The aforementioned additional step shall allow for a removal and/or determination of the total phosphorylcholine present in a sample in addition to sphingomyelins. Due to the treatment of the sample by alkaline phosphatase and choline oxidase, the sample will be depleted of total phosphorylcholine.

The determination of NADH can be carried out by well known in assays. Preferably, the amount of NADH is determined spectrophotometrically.

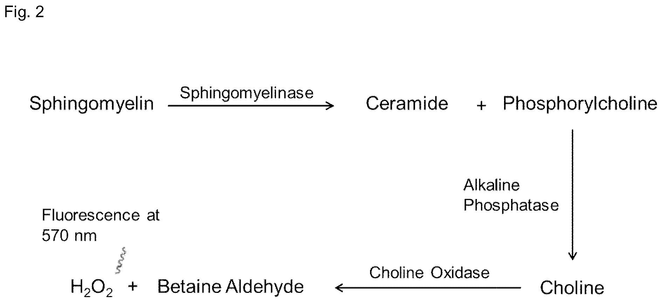

A particular preferred sphingomyelin assay which can be applied in accordance with the present invention is disclosed in the accompanying Examples, below. The said assay is based on the conversion of sphingomyelins into ceramide and phosphorylcholine by sphingomyelinase and the subsequent conversion of phosphorylcholine by alkaline phosphatase into choline followed by the conversion of choline into betaine aldehyde by choline oxidase, whereby $H_2O_2$ is generated. The latter results in conversion of a fluorogenic dye which can be measured by its fluorescence. The assay can be commercially purchased, e.g. from ImmunoWay Biotechnology Company, Newark (US) or BioVision Incorporated, Milpitas (US). Details on the reaction are also to be found in the Fig. 2, below. Similar assays are also available from other suppliers and readily available to the person skilled in the art; for example, other ways of determining redox equivalents and/or $H_2O_2$ are known to the person skilled in the art.

The enzymatic determination of the amount of total triacylglcerols, preferably, comprises the steps of:

a) contacting the sample with lipase under conditions and for a time sufficient to allow conversion into glycerol and free fatty acids;

b) contacting the sample comprising the glycerol with glycerokinase under conditions and for a time sufficient to allow conversion into glycerol-3-phosphate;

c) contacting the sample comprising the glycerol-3-phosphate with glycerophosphate oxidase under conditions and for a time sufficient to allow conversion into dihydroxyacetone phosphate and $H_2O_2$; and

d) enzymatically or chemically determining the amount of generated $H_2O_2$.

Preferably, total triacylglycerols may be, preferably, determined by measuring the redox equivalents via conversion of 4-aminophenazone and 4-chlorophenol to 4-(p-benzoquinonemonoimino)-phenazone. Such an assay may be carried out on, e.g., Hitachi 704 Analyzer (Roche Diagnostics, US).

The term "reference" preferably refers to values of characteristic features of each of the biomarkers, i.e. the at least one cholesterol parameter, the total triacylglycerols or the total sphingomyelins (and optionally NT-proBNP), which can be correlated to a medical condition, i.e. the presence or absence of the disease, diseases status or an effect referred to herein. Preferably, a reference is a threshold value (e.g., an amount, ratio or score of amounts) for a biomarker whereby, e.g., values found in a sample to be investigated which are lower than or essentially identical to the threshold are indicative for the presence of a medical condition while those being higher are indicative for the absence of the medical condition. This applies in particular for the at least one cholesterol parameter (e.g. total cholesteryl esters) and for total sphingomyelins. Also preferably, a reference is a threshold value (e.g., an amount, ratio or score of amounts) for a biomarker whereby, e.g., values found in a sample to be investigated which are higher than or essentially identical to the threshold are indicative for the presence of a medical condition while those being lower are indicative for the absence of the medical condition. This applies in particular for total triacylglycerols (and NT-proBNP).

In such cases, the threshold is typically at a value which separates the values associated with the medical condition from those which are not associated therewith. More typically, the threshold value may be the upper limit of the biomarkers found in diseased subjects. Moreover, vice versa it will be understood that values found in a sample to be investigated which are lower than the threshold are indicative for the presence of a medical condition while those being higher or essentially identical are indicative for the absence of the medical condition, if the threshold value may be the lower limit of the biomarkers found in healthy subjects.

Alternatively, the reference may be a range of values for each of the biomarkers.

Instead of comparing each biomarker separately against a reference, it is also possible to compare a real-valued mathematical function of the biomarkers (herein also referred to as a score) against a numerical threshold (in particular a reference score as described elsewhere herein).

In accordance with the aforementioned method of the present invention, a reference is, preferably, a reference obtained from a sample from a subject or group of subjects known to suffer from heart failure. In such a case, a value for the at least one cholesterol parameter and/or total sphingomyelins found in the test sample being essentially identical or decreased is indicative for the presence of the disease. In contrast, a value for the at least one cholesterol parameter (e.g the total cholesteryl esters) and/or total sphingomyelins found in the test sample being increased may be indicative for the absence of the disease. With regard to total triacylglycerols, a value for the total triacylglycerols found in the test sample being essentially identical or increased is indicative for the presence of the disease. In contrast, a value for the total triacylglycerols found in the test sample being decreased may be indicative for the absence of the disease.

In an embodiment, the subject(s) known to suffer from heart failure, is (are) known to suffer from HfrEF, in particular ICM or DCM, or from HFpEF. Depending on the type of heart failure to be diagnosed, the heart failure may be symptomatic or asymptomatic.

Moreover, the reference, also preferably, could be from a subject or group of subjects known not to suffer from heart failure, preferably, an apparently healthy subject. In such a case, a value for the at least one cholesterol parameter and/or total sphingomyelins found in the test sample being decreased with respect to the reference is indicative for the presence of the disease. In contrast, a value for the at least one cholesterol parameter (e.g. the total cholesteryl esters) and/or total sphingomyelins found in the test sample being essentially identical or increased may be indicative for the absence of the disease. With respect to total triacylglycerols, a value for the total triacylglycerols found in the test sample being increased with respect to the reference is indicative for the presence of the disease. In contrast, a value for the total triacylglycerols found in the test sample being essentially identical or decreased may be indicative for the absence of the disease. The same applies mutatis mutandis for a calculated reference, most preferably the average or median, for the relative or absolute value of the biomarker in a population of individuals comprising the subject to be investigated. The absolute or relative values of the biomarker of said individuals of the population or the groups of subjects can be determined as specified elsewhere herein. How to calculate a suitable reference value, preferably, the average or median, is well known in the art. The population or groups of subjects referred to before shall comprise a plurality of subjects, preferably, at least 5, 10, 50, 100, 1,000 or 10,000 subjects. It is to be understood that the subject to be diagnosed by the method of the present invention and the subjects of the said plurality of subjects are of the same species.

In an embodiment, the subject(s) known not to suffer from heart failure, is (are) known not to suffer from HfrEF, in particular ICM or DCM, or from HFpEF. Depending on the type of heart failure to be diagnosed, the subject(s) is (are) known not to suffer from symptomatic or asymptomatic heart failure.

As described above, artificially calculated values, such as cut off values or scores in a scoring system may be used, also preferably, as references. Preferably, the terms "cut off" and "threshold" are used herein interchangeably.

A cut off value (or reference score) which deliminates the group of subjects suffering from heart failure from those which do not suffer from heart failure can be calculated by algorithms well known in the art, e.g., on the basis of the amounts of the biomarkers found in either group. Typically, a cut-off value can be, preferably, determined based on sensitivity, specificity and expected, known (e.g., from literature) or estimated (e.g, based on a prospective cohort study) prevalence for the disease in a certain population of subjects to be investigated. Preferably, receiver-operating characteristics (ROC) can be used for determining cut-off values (Zweig 1993, Clin. Chem. 39:561-577). How to apply the ROC technique is well known to the skilled artisan and the cut off value preferably used in the method of the present invention is a cut-off value which allows discriminating between subjects suffering from the disease and those not suffering from the disease generated by establishing a ROC for either of said cohorts and deriving a cut-off value therefrom. Furthermore, each point of the ROC curve represents a sensitivity and specificity pair at a certain cut off value (or score). It will be understood that sensitivity and specificity are adjusted such that the group of false negatives is minimal in order to exclude a subject from being at increased risk efficiently (i.e. a rule-out) whereas sensitivity and specificity are adjusted such that the group of false positives is minimal in order for a subject to be assessed as being at an increased risk efficiently (i.e. a rule-in). Moreover, the area under the curve (AUC) values can be derived from the ROC plots giving an indication for the cut-off independent, overall performance of the biomarker.

In the context of step b) of the present invention, the amounts of the biomarkers as referred to in step a) of the methods of the present invention (and optionally NT-proBNP) shall be compared to a reference or references. Thereby, the presence or absence of a disease as referred to herein is diagnosed. In an embodiment references for the individual determined biomarkers, i.e. references for each of biomarkers as referred to in step a) are applied. However, it is also envisaged to calculate a score (in particular a single score) based on the amounts of the biomarkers as referred to in step a) of the method of the present invention (and optionally BNP or NT-proBNP), i.e. a single score, and to compare this score to a reference score. Preferably, the score is based on the amounts of the biomarkers in the sample from the test subject, and, if the amount on NT-proBNP or BNP is determined or provided, on the amounts of the biomarkers and the amount of NT-proBNP or BNP in the sample from the test subject. For example, if the amounts of total sphingomyelins and total triacylglycerols are determined, the calculated score is based on the amounts of total sphingomyelins and/or total triacylglycerols in the sample from the test subject. If additionally NT-proBNP is determined, the score is based on the amount of total sphingomyelins, total triacylglycerols and NT-proBNP of the test subject.

A score as a reference or test value in accordance with the present invention may be obtained by mathematical transformation of the amount(s) for the biomarker(s) to be determined. Upon transformation, a score value is obtained which can be compared in a scoring system to references (scores or score ranges) known to be associated with the presence or absence of a disease, in particular in a way equivalent to what is described for individual biomarkers elsewhere herein.

In an embodiment, the scoring algorithm is determined with an elastic net with the biomarkers referred to in step a) of the method of the present invention and optionally with BNP or NT-proBNP (see also Examples section).

In another embodiment, the scoring algorithm is determined with another suitable classification algorithm, such as e.g.random forest, neural nets, etc., with the biomarkers referred to in step a) of the method of the present invention and optionally with BNP or NT-proBNP.

Typically, a classification algorithm such as those implementing the elastic net method may be used for scoring (Zou 2005, Journal of the Royal Statistical Society, Series B: 301-320, Friedman 2010, J. Stat. Sotw. 33). Thus, the score for a subject can be, preferably, calculated with a logistic regression model fitted, e.g., by using the elastic net algorithm such as implemented in the R package glmnet. More specifically, the score may be calculated by the following formula

$$p = \frac{1}{1 + e^{-(w_0 + \sum_{i=1}^{n} w_i \hat{x}_i)}}$$

or a mathematically equivalent formula,
with the feature $\hat{x}_i$ being

$$\hat{x}_i = \frac{x_i - m_i}{s_i}$$

wherein $x_i$ are the log-transformed measurement values, e.g., absorption values and/or concentration values, $m_i$, $s_i$ are feature specific scaling factors, also taking into account the units of measurement, and $w_i$ are the coefficients of the model [$w_0$, intercept; $w_1$, coefficient for the first biomarker (feature); $w_2 ...w_n$, coefficients for the further features; $n$, number of feautures in the panel].

A score larger than the reference score is indicative for a subject who suffers from heart failure , whereas a score lower than (or equal to) the reference score is indicative for a subject who does not suffer from heart failure (such as HFrEF, in particular ICM or DCM, or HFpEF). The reference scores e.g. can be determined to maximize the Youden index or to find a desirable application specifc (e.g. rule-in) balance between specificity and sensitivy for the detection of heart failure (see above). A further preferred method to calculate the score and the reference score is described in Example 8 of the Examples section.

The calculated score combines information on the amounts of the biomarkers referred to above. Moreover, in the score, the biomarkers are, preferably, weighted in accordance with their significance or relevance for the establishment of the diagnosis. Based on the combination of biomarkers applied in the method of the invention, the weight of an individual biomarker may be different.

The score can be regarded as a classifier parameter for diagnosing heart failure. In particular, it enables the person to provide the diagnosis based on a single score based on the comparison with a reference score. The reference score is, preferably, a value, in particular a cut-off value which allows for differentiating between the presence of heart failure and the absence of heart failure in the subject to be tested. Preferably, the reference is a single value. Thus, the user, advantageously, does not have to interpret the entire information on the amounts of the individual biomarkers.

Thus, in a preferred embodiment of the method of the present invention, the comparison of the amounts of the biomarkers to a reference as set forth in step b) of the method of the present invention encompasses step (b1) of calculating a score based on the determined amounts of the biomarkers as referred to in step a), and step (b2) of comparing the, thus, calculated score to a reference score or reference score range. More preferably, a logistic regression method is used for calculating the score and, most preferably, said logistic regression method comprises elastic net regularization.

Alternatively, the amount of the biomarkers is compared to a reference, wherein the result of this comparison is used for the calculation of a score, in particular, a single score, and wherein said score is compared to a reference score or reference score range.

However, it is also envisaged to calculate a score, in particular, a single score, based on the amounts of the biomarkers referred to in step (a) of the method of the present invention, i.e. a single score, and to compare this score to a reference score or reference score range. Preferably, the score is based on the amounts of the biomarkers or biomarker combinations in the sample from the test subject, and, if a BNP, e.g., NT-proBNP or BNP (or ANP, NT-proANP) is determined, in addition on the the amount of the BNP (or ANP, NT-proANP) in the sample from the test subject.

When using a scoring system as described herein, advantageously, values of different dimensions or units for the biomarkers may be used since the values will be dimensionless after scaling. Accordingly, as described in Example 9, below, e.g., values for absolute concentrations may be combined in a score with absorption values.

The value for the biomarker of the test sample and the reference values are essentially identical, if the values for the characteristic features and, in the case of quantitative determination, the intensity values are essentially identical. Essentially identical means that the difference between two values is, preferably, not significant and shall be characterized in that the values for the intensity are within at least the interval between 1st and 99th percentile, 5th and 95th percentile, 10th and 90th percentile, 20th and 80th percentile, 30th and 70th percentile, 40th and 60th percentile of the reference value, preferably, the 50th, 60th, 70th, 80th, 90th or 95th percentile of the reference value. Statistical test for determining whether two amounts are essentially identical are well known in the art and are also described elsewhere herein.

An observed difference for two values, on the other hand, shall be statistically significant. A difference in the relative or absolute value is, preferably, significant outside of the interval between 45th and 55th percentile, 40th and 60th percentile, 30th and 70th percentile, 20th and 80th percentile, 10th and 90th percentile, 5th and 95th percentile, 1st and 99th percentile of the reference value. Preferably, the reference, i.e. values for at least one characteristic feature of the biomarker, will be stored in a suitable data storage medium such as a database and are, thus, also available for future assessments.

The term "comparing" refers to determining whether the determined value of a biomarker (or alternatively a score) is essentially identical to a reference or differs therefrom. Preferably, a value for a biomarker (or score) is deemed to differ from a reference if the observed difference is statistically significant, or relevant, which can be determined by e.g. statistical techniques referred to elsewhere in this description. If the difference is not statistically significant or relevant, the biomarker value and the reference are essentially identical. Based on the comparison referred to above, a subject can be assessed to suffer from the disease, or not.

Preferably, the values determined in a sample of a subject according to the present invention are adjusted for age, BMI, gender or other existing diseases before being compared to a reference. Alternatively, the references can be derived from values which have likewise been adjusted for age, BMI, gender or other diseases. Such an adjustment can be made by deriving the references and the underlying values from a group of subjects, the individual subjects of which are essentially identical with respect to these parameters, to the subject to be investigated. Alternatively, the adjustment may be done by statistical calculations.

Even more preferably, such adjustment is not carried out. Thus, a correction for confounders is not carried out.

If a scoring system is used for the diagnosis, it will be understood that the values obtained from the sample of the subject to be investigated shall be transformed into a score (preferably by a mathematical real-valued function), which subsequently will be compared to at least one reference score in the scoring system.

The comparison is, preferably, assisted by automation. For example, a suitable computer program comprising algorithms for the comparison of two different data sets (e.g., data sets comprising the values of the characteristic feature(s)) may be used. Such computer programs and algorithms are well known in the art. Notwithstanding the above, a comparison can also be carried out manually.

As set forth elsewhere herein, the method of the present invention may further comprise in step a) the determination of the amount of BNP or NT-proBNP. The amount of BNP or NT-proBNP may contribute to the score calculated in step b). Accordingly, the method comprises the following steps:

   a) determining in a sample of the said subject the amount of BNP or NT-proBNP (preferably NT-proBNP) and the amount(s) of at least one cholesterol parameter, total triacylglycerols and/or total sphingomyelins, and

b1) calculating a score based on the determined amounts as determined in step a), and

b2) comparing the, thus, calculated score to a reference score, whereby heart failure is to be diagnosed.

As set forth elsewhere herein, the amount of NT-proBNP or BNP can be also derived from the medical record of the subject to be tested. In this case, it is not required to the determine the amount of this marker in step a).

Alternatively, the amount of BNP or NT-proBNP may not contribute to the score calculated in step b1). Accordingly, the method comprises the following steps:

a) determining in a sample of the said subject the amount of BNP or NT-proBNP (preferably NT-proBNP) and the amount(s) of at least one cholesterol parameter, total triacylglycerols and/or total sphingomyelins, and

b1) calculating a score based amount(s) of at least one cholesterol parameter, total triacylglycerols and/or total sphingomyelins, and

b2) comparing the, thus, calculated score to a reference score, and comparing the amount of BNP or NT-proBNP to a reference, whereby heart failure is to be diagnosed.

As set forth elsewhere herein, the amount of NT-proBNP or BNP can be also derived from the medical record of the subject to be tested. In this case, it is not required to the determine the amount of this marker in step a).

Advantageously, it has been found in the study underlying the present invention that the amount(s) of total cholesteryl esters or total cholesterol or both, or the sum parameter of total cholesteryl esters and total cholesterol, and/or total sphingomyelins and/or total triacylglycerols (preferably in combination with the amount of NT-proBNP and BNP) are indicators for heart failure. It has been found that the amounts of the said at least one cholesterol parameter and/or total sphingomyelins are decreased in subjects suffering from or being at risk of developing heart failure in comparison to healthy volunteers, wherein the amount of said total triacylglycerols are increased in subjects suffering from or being at risk of developing heart failure in comparison to healthy volunteers. Accordingly, the said biomarkers in a sample can, in principle, be used for assessing whether a subject suffers from heart failure, or not. This is particularly helpful for an efficient diagnosis of the disease as well as for improving of the pre-clinical and clinical management of heart failure as well as an efficient monitoring of patients. Moreover, the findings underlying the present invention will also facilitate the development of efficient drug-based therapies or other interventions including nutritional diets against heart failure as set forth in detail below. Moreover, in accordance with the present invention, it was also found that uric acid and phosphatidylcholines also qualify as biomarkers for diagnosing heart failure. Changes in the amounts of said biomarkers could be found between patients suffering from heart failure as described herein and healthy volunteers.

The definitions and explanations of the terms made above apply mutatis mutandis to all embodiments characterized herein below.

In the following, particular preferred embodiments of the method of the invention are described.

In a preferred embodiment of the method of the invention, the at least one cholesterol parameter (e.g. total cholesteryl esters) and/or the amount of total triacylglycerols and/or total sphingomyelins in a sample of the said subject is enzymatically determined.

In yet an embodiment of the method of the present invention, said reference is derived from a subject or group of subjects known to suffer from heart failure. More preferably, an identical (in particular an essentially identical) or decreased amount for the at least one cholesterol parameter (e.g. total cholesteryl esters) and/or the total sphingomyelins in the test sample in comparison to the reference is indicative for a subject suffering from heart failure whereas an increased amount in the test sample in comparison to the reference is indicative for a subject not suffering from heart failure. With respect to total triacylglycerols, an identical or increased amount for this biomarker in the test sample and the reference is indicative for a subject suffering from heart failure, whereas an decreased amount in the test sample in comparison to the reference is indicative for a subject not suffering from heart failure.

In another preferred embodiment of the method of the invention, said reference is derived from a subject or group of subjects known not to suffer from heart failure. More preferably, an identical (in particular an essentially identical) or increased amount for the at least one cholesterol parameter (e.g. total cholesteryl esters) and/or the total sphingomyelins in the test sample and the reference is indicative for a subject not suffering from heart failure whereas a decreased amount in the test sample in comparison to the reference is indicative for a subject suffering from heart failure. With respect to total triacylglycerols, an identical or decreased amount for the biomarker in the test sample and the reference is indicative for a subject not suffering from heart failure whereas an increased amount in the test sample in comparison to the reference is indicative for a subject suffering from heart failure.

In a preferred embodiment of the method of the invention, said method further comprises the step of recommending a therapeutic or patient health management measure for the subject based on whether the subject is diagnosed as suffering from heart failure. if the subject is diagnosed to suffer from heart failure, it is in particular envisaged to initiate heart failure therapy. The term "heart failure therapy" is defined elsewhere. Preferably, said heart failure therapy is drug based therapy.

As a rule, patients are preferably treated with medication as recommended by the guidelines of the European Society of Cardiology (Ref: European Heart Journal (2012), 33:1787-1847).

In another embodiment the patients are treated as recommended by the 2013 ACCF/AHA guidelines (see Circulation. 2013; 128: e240-e327).

Preferably, the therapy comprises the administration of Mineralocorticoid / Aldosteron Antagonists and/or ACE Inhibitors, if the subject is diagnosed to suffer from HFrEF. Further envisaged is the treatment with a beta blocker.

Moreover, the subject may be treated with angiotensin receptor blockers (ARBs), ivabradine, digoxin and other digitalis glycosides, hydralazine and isosorbide dintrate (vasodilators) and omega-3 polyunsaturated fatty acids.

Preferably, the therapy comprises the administration of Diuretics, Aldosteron Antagonists and/or ACE Inhibitors, if the subject is diagnosed to suffer from DCMP.

Preferably, the therapy comprises the administration of Diuretics, Aldosteron Antagonists and/or ACE Inhibitors, if the subject is diagnosed to suffer from ICMP. Also preferred are Vitamin-K-antagonists and antiplatelet agents.

If the subject suffers from HFpEF, the therapy preferably comprises the administration of diuretics, ACE inhibitor, receptor blockers (ARBs) and/or beta blockers.

In yet another embodiment of the method of the invention, said sample is a blood, plasma or serum sample.

In a further embodiment of the method of the invention, said heart failure is asymptomatic heart failure.

In a preferred embodiment of the method of the invention, said heart failure is heart failure with reduced ejection fraction (HFrEF) and, preferably, heart failure with mildly reduced ejection fraction, in particular HFrEF with LVEF of larger than 35% but lower than 50%.

In a preferred embodiment of the method of the invention, said determining enzymatically the amount of total cholesteryl esters as the at least one cholesterol parameter comprises the steps of:

a) contacting the sample with cholesterol esterase under conditions and for a time sufficient to allow conversion into cholesterol;
b) contacting the sample comprising the cholesterol with cholesterol oxidase under conditions and for a time sufficient to allow generation of $H_2O_2$; and
c) enzymatically or chemically determining the amount of generated $H_2O_2$.

More preferably, said method comprises the further steps of:

- contacting the sample prior to step a) with cholesterol oxidase under conditions and for a time sufficient to allow generation of, $H_2O_2$; and
- neutralizing the said $H_2O_2$.

Furthermore preferably, said neutralizing the said $H_2O_2$ comprises enzymatically or chemically determining the amount of generated $H_2O_2$. Even more preferably, said method further comprises the generation of the total cholesteryl ester to total cholesterol ratio based on the amounts of the cholesterol-derived $H_2O_2$ determined prior to step a) and the cholesteryl ester-derived $H_2O_2$ determined in step c). Most preferably, said method further comprises the step of comparing the ratio of cholesteryl esters to cholesterol to a reference, whereby it is diagnosed whether the subject suffers from heart failure, or not.

In a preferred embodiment of the method of the invention, said determining enzymatically the amount of total cholesterol as at least one cholesterol parameter comprises the steps of:

a) contacting the sample comprising the cholesterol with cholesterol oxidase under conditions and for a time sufficient to allow generation of $H_2O_2$; and
b) enzymatically or chemically determining the amount of generated $H_2O_2$.

Alternatively, the enzymatic determination of the amount of total cholesterol as at least one cholesterol parameter preferably, comprises the steps of:

a) contacting the sample comprising the cholesterol with cholesterol dehydrogenase under conditions and for a time sufficient to allow generation of redox equivalents and, preferably, NADH, and
b) enzymatically or chemically determining the amount of generated redox equivalents.

Alternatively, the amount of NADH can be determined. Thereby, the amount of total cholesterol is determined.

In another preferred embodiment of the method of the present invention, said determining enzymatically the amount of total sphingomyelins comprises the steps of:

a) contacting the sample with sphingomyelinase under conditions and for a time sufficient to allow conversion into phosphorylcholine;

b) contacting the sample comprising the phosphorylcholine with alkaline phosphatase and choline oxidase under conditions and for a time sufficient to allow conversion into choline and subsequent generation of $H_2O_2$; and

c) enzymatically or chemically determining the amount of generated $H_2O_2$.

More preferably, said method comprises the further steps of:

- contacting the sample prior to step a) with alkaline phosphatase and choline oxidase under conditions and for a time sufficient to allow conversion into choline and subsequent generation of $H_2O_2$; and
- neutralizing the said $H_2O_2$.

In a preferred embodiment of the method of the invention, said determining enzymatically the amount of total triacylglcerols comprises the steps of:

a) contacting the sample with lipase under conditions and for a time sufficient to allow conversion into glycerol and free fatty acids;

b) contacting the sample comprising the glycerol with glycerokinase under conditions and for a time sufficient to allow conversion into glycerol-3-phosphate;

c) contacting the sample comprising the glycerol-3-phosphate with glycerophosphate oxidase under conditions and for a time sufficient to allow conversion into dihydroxyacetone phosphate and $H_2O_2$; and

d) enzymatically or chemically determining the amount of generated $H_2O_2$.

Brain natriuretic peptides, such as the N-terminal fragment of the propeptide of the brain natriuretic peptide(NT-proBNP) and the mature brain natriuretic peptide (BNP), are well known in the art as cardiac biomarkers and, in particular, as biomarkers for heart failure. BNP is a 32-amino acid polypeptide secreted by the ventricles of the heart in response to excessive stretching of cardiomyocytes. NT-proBNP is a 76 amino acid N-terminal inactive protein that is cleaved from proBNP to release brain natriuretic peptide. BNP is the active hormone and has a shorter half-life than the respective inactive counterpart NT-proBNP. The structure of the human BNP and NT-proBNP has been described already in detail in the prior art, see e.g., WO2002/089657, WO2002/083913.

Therefore, in yet a preferred embodiment of the method of the present invention, the said method further comprises the determination of the amount of BNP or NT-proBNP in a sample from the subject and, preferably, comparing the thus determined amount of BNP or NT-proBNP to a suitable reference. Based on the said comparison, heart failure can be diagnosed. Alternatively, the amount of NT-proBNP or BNP can be derived from the medical record of the subject to be tested. Thus, the method of the present invention comprises the step of providing and/or retrieving information on the amount of NT-proBNP or BNP (i.e. the value of this marker). Also preferably, said amount is compared to a reference. Further, preferably the value is used for calculating a score as described elsewhere herein. Preferably, said score is compared to a reference score.

Instead of NT-proBNP or BNP, or in addition to NT-proBNP or BNP, the present invention envisages the determination of the amount of ANP (atrial natriuretic peptide) or NT-proANP (N-terminus of the prohormone brain natriuretic peptide). Alternatively, the amount of C-type natriuretic peptide (CNP) or NT-proCNP (amino-terminal propeptide of C-type natriuretic peptide) can be determined.

The determination and diagnosis of heart failure based on a BNP as specified above can be carried out prior to the determination of the other biomarkers of the invention, simultaneously or afterwards. A determination prior to the determination of the other biomarkers may be carried out in order to get an initial diagnosis or first hint towards heart failure in a suspicious subject. The said initial diagnosis or first hint will than be further confirmed by determining the other biomarkers. A determination simultaneously with the other biomarkers may be carried out in order to get a more reliable diagnosis of heart failure when compared with a diagnosis solely based on a BNP or any of the other biomarkers. A determination afterwards shall further strengthen an initial diagnosis of heart failure or first hint towards it based on a diagnosis using the other biomarkers.

Preferably, the amount is determined by using at least one antibody which specifically binds to NT-proBNP or BNP (or to NT-proANP, ANP, NT-proCNP or CNP). The at least one antibody forms a complex with the marker to determined. Afterwards the amount of the formed complex is measured. The complex comprises the marker and the antibody (which might be labelled in order to allow for a detection of the complex).

Yet, the present invention also relates to a method for monitoring heart failure therapy in subject which undergoes such a therapy comprising:

(a) determining the amount of at least one cholesterol parameter and/or total sphingomyelins and/or total triacylglycerols, and optionally the amount of BNP or NT-proBNP, in a first and second sample of the said subject; and

(b) comparing the amount(s) determined in step (a) in the first sample to the amount(s) determined in step (a) in the second sample, whereby it is diagnosed whether the subject responds to heart failure therapy, or not.

In a preferred embodiment of the aforementioned method, the first sample has been taken prior and the second sample after the onset of the heart failure therapy. Alternatively, both samples, i.e. the first and the second sample, have been taken after the onset of the heart failure therapy. If both samples have been taken after the onset of therapy, the second sample is preferably obtained after the first sample. Preferably, the second sample is not obtained too early after the first sample (in order to observe a sufficiently significant change to allow for monitoring). More preferably, the second sample obtained at least one month, or at least three months after said first sample.

The term "monitoring heart failure therapy" as used herein in the context of the aforementioned method, preferably, relates to assessing whether a subject responds to said therapy, or not. Accordingly, it is assessed whether a subject benefits from said therapy, or not. Preferably, an increase of the amount(s) of the at least one cholesterol parameter (e.g. total cholesteryl esters) and/or total sphingomyelins in the second compared to the first sample shall be indicative for a subject who responds to heart failure therapy. In contrast, a decrease or equal amount(s), in particular a decrease or essentially equal amount(s) of the at least one cholesterol parameter (e.g. total cholesteryl esters) and/or total sphingomyelins in the second compared to the first sample shall be indicative for a subject who does not respond to heart failure therapy. Also preferably, a decrease of the amount(s) of the total triacylglycerols (and/or of NT-proBNP or BNP) in the second compared to the first sample shall be indicative for a subject who responds to heart failure therapy. In contrast, an increase or equal amount(s), in particular an increase or essentially equal amount(s), of the total triacylglycerols (and/or of NT-proBNP or BNP) in the second sample compared to the first sample shall be indicative for a subject who does not respond to heart failure therapy.

Preferably, by carrying out the aforementioned method, decisions can be made whether heart failure therapy in said subject shall be continued, stopped or amended. Thus, the method may comprise the further step of continuing, stopping or amending said therapy. Preferably, a subject responds to heart failure therapy, if said therapy improves the condition, clinical paramters such as LVEF or symptoms of the subject with respect to heart failure. Preferably, a subject does not respond to said therapy, if said therapy does not the improve the condition, clinical paramters such as LVEF or symptoms of the subject with respect to heart failure.

The term "heart failure therapy" as used herein, preferably, includes any therapy for the treatment of heart failure. Preferred therapies are drug-based therapies. Preferably, the therapy of heart failure is a therapy which comprises or consists of the administration of at least one drug selected from the group consisting of: ACE Inhibitors (ACEI), Beta Blockers, AT1-Inhibitors, Aldosteron Antagonists, Renin Antagonists, Diuretics, Ca-Sensitizer, Digitalis Glykosides, antiplatelet agents, and Vitamin-K-Antagonists. Alternatively, the therapy may be a therapy with assist devices such as ventricular assist devices.

An embodiment, not forming part of the invention, relates to a device for diagnosing whether a subject suffers from heart failure, or not, comprising:

a) an analysing unit comprising enzymatic detection agents for the amount of at least one cholesterol parameter, the amount of total triacylglycerols and/or the amount of total sphingomyelins, preferably, arranged with a detector such that the amount of the said biomarkers in a sample can be determined; and

b) an evaluation unit comprising a data processor and a database with stored references, preferably, as defined in above, wherein the evaluation unit has tangibly embedded an algorithm which carries out a comparison as defined above between the determined amount(s) of the biomarkers received from the analysing unit and the stored references.

Alternatively, said evaluation unit comprises a data processor and a database with a stored reference score (or stored reference scores), preferably, as defined in above, wherein the evaluation unit has tangibly embedded an algorithm which carries out a comparison as defined above between a calculated score based on the determined amount(s) of the biomarkers received from the analysing unit(s) and the stored references

In an embodiment, the device further comprises at least one further analysing unit comprising at least one detecting agent (such as an antibody) for NT-proBNP and/or BNP, wherein said further analyzing unit is adapted for determining the amounts BNP and/or NT-proBNP detected by the at least one detection agent.

The term "device" as used herein relates to a system comprising the aforementioned units operatively linked to each other as to allow the diagnosis or monitoring according to the methods of the invention. Preferred detection agents which can be used for the analysing unit are disclosed elsewhere herein. Preferably, said detection agents are agents required for the enzymatic determination of the total amount(s) of the biomarkers. The analysing unit, preferably, comprises said detection agents in immobilized form on a solid support which is to be contacted to the sample comprising the biomarkers the amount of which is to be determined. Alternatively, the detection agents may be stored in the analysing unit in a supply vial and may be admixed with the sample in a reaction vial. Moreover, the analysing unit can also comprise a detector which determines the amount of detection agent which is specifically bound to the biomarker(s). The determined amount can be transmitted to the evaluation unit.

Said evaluation unit comprises a data processing element, such as a computer, with an implemented algorithm for carrying out a comparison between the determined amount and a suitable reference. Suitable references are either derived from a subject or group of subjects as defined above in context with the method of the present invention. The results may be given as output of parametric diagnostic raw data, preferably, as absolute or relative amounts. It is to be understood that these data will need interpretation by the clinician. However, also envisaged are expert system devices wherein the output comprises processed diagnostic raw data the interpretation of which does not require a specialized clinician.

It follows from the above that according to some embodiments of the instant disclosure, portions of some steps of methods disclosed and described herein may be performed by a computing device. A computing device may be a general purpose computer or a portable computing device, for example. It should also be understood that multiple computing devices may be used together, such as over a network or other methods of transferring data, for performing one or more steps of the methods disclosed herein. Exemplary computing devices include desktop computers, laptop computers, personal data assistants, cellular devices, tablet computers, servers, and the like. In general, a computing device comprises a data processor capable of executing a plurality of instructions, such as a computer program. A computing device has access to a memory. A memory is a computer readable medium and may comprise a single storage device or multiple storage devices, located either locally with the computing device or accessible to the computing device across a network, for example. Computer-readable media may be any available media that can be accessed by the computing device and includes both volatile and non-volatile media. Further, computer readable-media may be one or both of removable and non-removable media. By way of example, and not limitation, computer-readable media may comprise computer storage media. Exemplary computer storage media includes, but is not limited to, RAM, ROM, EEPROM, flash memory or any other memory technology, CD-ROM, Digital Versatile Disk (DVD) or other optical disk storage, magnetic cassettes, magnetic tape, magnetic disk storage or other magnetic storage devices, or any other medium which can be used for storing a plurality of instructions capable of being accessed by the computing device and executed by the data processor of the computing device.

A computer program as referred to herein may include instructions which, when executed by a data processor of the computing device, may perform one or more steps of the methods disclosed herein. Some of the instructions may be adapted to produce signals that control operation of other machines and thus may operate through those control signals to transform materials far removed from the computer itself. The aforementioned instructions shall also comprise an algorithm which is generally conceived to be a self-consistent sequence of steps leading to a desired result, i.e. to carry out the method(s) of the invention or parts thereof. These steps are those requiring physical manipulations of physical quantities. Usually, though not necessarily, these quantities take the form of electrical or magnetic pulses or signals capable of being stored, transferred, transformed, combined, compared, and otherwise manipulated. It proves convenient at times, principally for reasons of common usage, to refer to these signals as values, characters, display data, numbers, or the like as a reference to the physical items or manifestations in which such signals are embodied or expressed.

The computing device may also have access to an output device. Exemplary output devices include fax machines, displays, printers, and files, for example. According to some embodiments of the present disclosure, a computing device may perform one or more steps of a method disclosed herein, and thereafter provide an output, via an output device, relating to a result, indication, ratio or other factor of the method.

Moreover, the invention, in general, provides for the use of enzymatic detection agents for at least one cholesterol parameter, total triacylglycerols and/or total sphingomyelins for diagnosing in a sample of a subject whether the said subject suffers from heart failure, or not.

Also, the invention, in general, provides for the use of enzymatic detection agents for at least one cholesterol parameter, total triacylglycerols and/or total sphingomyelins for the preparation of a diagnostic and/or pharmaceutical composition diagnosing in a sample of a subject whether the said subject suffers from heart failure, or not.

Finally, the present invention relates to a kit adapted or to be used for diagnosing in a sample of a subject whether the said subject suffers from heart failure, or not, comprising enzymatic detection agents for at least one cholesterol parameter, total triacylglycerols and/or total sphingomyelins.

The term "kit" as used herein refers to a collection of the aforementioned components, preferably, provided in separately or within a single container. The container also comprises instructions for carrying out the method of the present invention. These instructions may be in the form of a manual or may be provided by a computer program which is capable of carrying out the comparisons referred to in the methods of the present invention and to establish a diagnosis accordingly when implemented on a computer or a data processing device. The computer program may be provided on a data storage medium or device such as an optical storage medium (e.g., a Compact Disc) or directly on a computer or data processing device. Further, the kit may comprise at least one standard for a reference as defined herein above, i.e. a solution with a pre-defined amount of at least one cholesterol parameter, triacylglycerols and/or sphingomyelins representing a reference amount. Such a standard may represent, e.g., the amount of at least one cholesterol parameter, triacylglycerols and/or sphingomyelins from a subject or group of subjects suffering from heart failure or a clinically apparently healthy subject or group thereof or any other reference specified herein. Moreover, such a solution may be used for calibration purposes when carrying out the method of the present invention or in accordance with the aforementioned uses.

A container of the kits may be any container that is suitable for packaging and/or containing one or more components disclosed herein, including for example, detection agents such as enzymes, references, buffers, and reagents. Suitable materials include, but are not limited to, glass, plastic, cardboard or other paper product, wood, metal, and any alloy thereof.

FIGURES

Figure 1: The relevant enzymatic conversions of cholesteryl esters are shown.

Figure 2: The relevant enzymatic conversions of sphingomyelins are shown.

Figure 3: The amount of total sphingomyelin was measured in 4 CHF patients and 6 healthy controls in three technical replicates, signficant differences were observed with lower levels in the group of CHF patients (Ratio: 0.41 and $p<0.001$; ANOVA).

EXAMPLES

The following Examples shall illustrate the invention. They shall, however, not be construed as limiting the scope of the invention.

Example 1: Study design for the differentiation of CHF subtypes DCMP (dilated cardiomyopathy, herein also referred to as "DCM"), ICMP (ischemic cardiomyopathy, herein also referred to as "ICM") and HCMP (hypertrophic cardiomyopathy) from healthy controls

A multicentric study with three clinical centers and in total 843 subjects was conducted. The study comprised 194 male and female DCMP-, 183 male and female ICMP- and 210 male and female HCMP patients as well as 256 male and female healthy controls in an age range from 35-75 and a BMI rage from 20-35 kg/m2. NYHA (New York Heart Association) scores of the patients ranged from 1-3. Patients and controls were matched for age, gender and BMI. For all patients and controls, a blood sample was collected. Plasma was prepared by centrifugation, and samples were stored at -80 °C until measurements were performed.

Three subgroups of CHF (DCMP, ICMP and HCMP) were defined on the basis of echocardiography and hemodynamic criteria:

a) Subgroup DCMP: is hemodynamically defined as a systolic pump failure with cardiomegaly (echocardiographic enhancement of the left ventricular end diastolic diameter >55 mm and a restricted left ventricular ejection fraction - LVEF of <50%).
b) Subgroup ICMP: is hemodynamically defined as systolic pump failure due to a coronary insufficiency (>50% coronary stenosis and a stress inducible endocardium motion insufficiency as well as an LVEF of <50%)
c) Subgroup HCMP: concentric heart hypertrophy (echocardiography - septum >11 mm, posterior myocardial wall >11 mm) and with a diastolic CHF (non or mildly impaired pump function with LVEF of ≥50%).

NYHA IV patients were excluded as well as patients suffering from apoplex, patients who had myocardial infarction within the last 4 months before testing, patients with altered medications within the last 4 weeks before testing as well as patients who suffered from acute or chronic inflammatory diseases and malignant tumours.

Example 2: Determination of metabolites

Human plasma samples were prepared and subjected to LC-MS/MS and GC-MS analysis as described in the following:

Proteins were separated by precipitation from blood plasma. After addition of water and a mixture of ethanol and dichloromethane the remaining sample was fractioned into an aqueous, polar phase and an organic, lipophilic phase.

For the transmethanolysis of the lipid extracts a mixture of 140 $\mu$l of chloroform, 37 $\mu$l of hydrochloric acid (37% by weight HCl in water), 320 $\mu$l of methanol and 20 $\mu$l of toluene was added to the evaporated extract. The vessel was sealed tightly and heated for 2 hours at 100°C, with shaking. The solution was subsequently evaporated to dryness. The residue was dried completely.

The methoximation of the carbonyl groups was carried out by reaction with methoxyamine hydrochloride (20 mg/ml in pyridine, 100 l for 1.5 hours at 60°C) in a tightly sealed vessel. 20 $\mu$l of a solution of odd-numbered, straight-chain fatty acids (solution of each 0.3 mg/mL of fatty acids from 7 to 25 carbon atoms and each 0.6 mg/mL of fatty acids with 27, 29 and 31 carbon atoms in 3/7 (v/v) pyridine/toluene) were added as retention time standards. Finally, the derivatization with 100 $\mu$l of N-methyl-N-(trimethylsilyl)-2,2,2-trifluoroacetamide (MSTFA) was carried out for 30 minutes at 60°C, again in the tightly sealed vessel. The final volume before injection into the GC was 220 $\mu$l.

For the polar phase the derivatization was performed as follows: The methoximation of the carbonyl groups was carried out by reaction with methoxyamine hydrochloride (20 mg/ml in pyridine, 50 l for 1.5 hours at 60°C) in a tightly sealed vessel. 10 $\mu$l of a solution of odd-numbered, straight-chain fatty acids (solution of each 0.3 mg/mL of fatty acids from 7 to 25 carbon atoms and each 0.6 mg/mL of fatty acids with 27, 29 and 31 carbon atoms in 3/7 (v/v) pyridine/toluene) were added as retention time standards. Finally, the derivatization with 50 $\mu$l of N-methyl-N-(trimethylsilyl)-2,2,2-trifluoroacetamide (MSTFA) was carried out for 30 minutes at 60°C, again in the tightly sealed vessel. The final volume before injection into the GC was 110 $\mu$l.

The GC-MS systems consist of an Agilent 6890 GC coupled to an Agilent 5973 MSD. The autosamplers are CompiPal or GCPal from CTC.

For the analysis standard commercially available capillary separation columns (30 m x 0,25 mm x 0,25 $\mu$m) with different poly-methyl-sitoxane stationary phases containing 0% up to 35% of aromatic moieties, depending on the analysed sample materials and fractions from the phase separation step, were used (for example: DB-1ms, HP-5ms, DB-XLB, DB-35ms, Agilent Technologies). Up to 1 $\mu$L of the final volume was injected splitless and the oven temperature program was started at 70 °C and ended at 340 °C with different heating rates depending on the sample material and fraction from the phase separation step in order to achieve a sufficient chromatographic separation and number of scans within each analyte peak. Furthermore RTL (Retention Time Locking, Agilent Technologies) was used for the analysis and usual GC-MS standard conditions, for example constant flow with nominal 1 to 1.7 ml/min and helium as the mobile phase gas, ionisation was done by electron impact with 70 eV, scanning within a m/z range from 15 to 600 with scan rates from 2.5 to 3 scans/sec and standard tune conditions.

The HPLC-MS systems consisted of an Agilent 1100 LC system (Agilent Technologies, Wald-bronn, Germany) coupled with an API 4000 Mass spectrometer (Applied Biosystem/MDS SCI-EX, Toronto, Canada). HPLC analysis was performed on commercially available reversed phase separation columns with C18 stationary phases (for example: GROM ODS 7 pH, Thermo Betasil C18). Up to 10 $\mu$L of the final sample volume of evaporated and reconstituted polar and lipophilic phase was injected and separation was performed with gradient elution using methanol/water/formic acid or acetonitrile/water/formic acid gradients at a flowrate of 200 $\mu$L/min.

Mass spectrometry was carried out by electrospray ionisation in positive mode for the non-polar fraction and negative or positive mode for the polar fraction using multiple-reaction-monitoring-(MRM)-mode and fullscan from 100 - 1000 amu.

Analysis of complex lipids in plasma samples:

Total lipids were extracted from plasma by liquid/liquid extraction using chloroform/methanol. The lipid extracts were subsequently fractionated by normal phase liquid chromatography (NPLC) into eleven different lipid groups according to Christie (Journal of Lipid Research (26), 1985, 507-512).

The fractions were analyzed by LC-MS/MS using electrospray ionization (ESI) and atmospheric pressure chemical ionization (APCI) with detection of specific multiple reaction monitoring (MRM) transitions for cholesterol esters (CE) and sphingoymelins (SM). Metabolites in the Tables 1 through 13 below that are derived from one of these fractions include the respective abbreviation in their name.

Example 3: Data analysis and statistical evaluation

Plasma samples were analyzed in randomized analytical sequence design with pooled samples (so called "pool") generated from aliquots of each sample. Following comprehensive analytical validation steps, the raw peak data for each analyte were normalized to the median of pool per analytical sequence to account for process variability (so called "pool-normalized ratios"). In all cases, pool-normalized ratios were used. All data were log10-transformed to achieve normal distribution.

The data of the study described in Example 1 were utilized for the evaluation of the diagnostic . power for the classification of CHF subgroups compared with controls of (1) the sum paramter of all sphingomyelins compared to specific sphingomyelins and (2) the sum parameter of all cholesteryl esters compared to specific cholesteryl esters.

Metabolite data were corrected for confounding factors or uncorrected metabolite data were used. The ANOVA model for correction for confounders comprised the factors age, BMI, gender and CHF subgroup (ANOVA model: CHF_SUBGROUP + (GENDER+AGE+BMI)^2; correction factors: GENDER, AGE and BMI). CHF patients were subdivided based on a combination of NYHA class (I or II-III) and CHF subtype (DCMP, HCMP, ICMP or the joined DCMP+ICMP group named HFrEF (heart failure with reduced ejection fraction)). However, in the subsequent calculations, direct readouts without correction for confounders were used.

With regard to Tables 2 through 13 and 15 through 18, the sum parameter of all sphingomyelins was estimated with (1) 1-hydroxy-2-amino-(cis,trans)-3,5-octadecadiene and (2) with the calculated unweigthed sum of all measured sphingomyelins. 1-hydroxy-2-amino-(cis,trans)-3,5-octadecadiene was determined with GC-MS. This fragment derived from all classes of sphingolipids with a d18:1 base including sphingomyelins, sphingoid bases, sphingosine and the 1-phosphate thereof, ceramides, glycosylceramides (glucosylceramides, galactosylceramides, sulfatides, other hexosylceramides, lactosylceramides, globosides, gangliosides) and ceramide 1-phosphates. In plasma, sphingomyelins represent approximately 87.7% of total sphingolipids (Hammad 2010, J Lipid Res.51(10):3074-87) and d18:1 is the major sphingoid base with approximately 76% of all free bases (Scherer 2011, Biochimica et Biophysica Acta. 1811:68-75).

With regard to Tables 2 through 18, the sum parameter of all cholesteryl esters was estimated with the weighted sum of all measured cholesterylesters (n=21). The weight of the specific cholesteryl ester was determined by the plasma concentration (Table 1; concentrations from HMDB and Lindgren 1961, Am J Clin Nutr. 9:13-23).

The weight of the specific sphingomyelins can be determined by the plasma concentrations as provided in Quehenberger et al. (2010, J Lipid Res. 51: 3299-3305).

DCMP and ICMP defines heart failure with a systolic dysfunction and reduced ejection fraction of the left ventricle and therefore the combined group called heart failure with reduced ejection fraction (HFrEF) was included in the analysis. In contrast, HCMP defines heart failure with a diastolic dysfunction with preserved ejection fraction of the left ventricle and therefore this group can be called heart failure with preserved ejection fraction (HFpEF) as well.

To compare the levels of metabolites in the specific subgroups the study described in Example 1 was analyzed by an analysis of variance (ANOVA) model comprising factors age, BMI, gender (including all binary interactions) and diagnostic group. Levels for the factor diagnostic group were CHF subtype/grade (CHF NYHA I, CHF NYHA II-III, HFrEF NYHA I, HFrEF NYHA II-III, HFpEF NYHA I, HFpEF NYHA II-III) and control (set as reference). Estimates for the fold change derived from ANOVA were calculated with and without correction for the confounders age, BMI and gender. The corresponding results are listed in Tables 2 to 5.

In addition, the diagnostic power of the individual metabolites as well as the sum parameters was estimated with the area under the curve (AUC) of a receiver operating characteristic (ROC) analysis (Tables 6 to 13). The performance calculations were carried out with only metabolites and metabolites combined with NT-proBNP. To combine metabolite levels and NT-proBNP levels into a single classifier, the classification approach elastic net was used. In addition, performance calculations were carried out with and without prior correction for confounders age, BMI and gender.

In the following tables, AUC (area under the curve) values indicate classification performance estimated by cross validated ROC (receiver operating characteristic) analysis and ratio and p-value (from ANOVA) indicate the statistical significance of differences between groups. The higher the AUC value, the better is the classification performance. The lower the p-value the more significant is the observed difference between groups. Consequently, a higher AUC value indicates better separation of groups while a lower p-value indicates higher significance.

Calculations of AUC values shown in Tables 6 through 13 and 15 through 18 are based on the data set described in Example 1.

Example 4: Determining the total amount of sphingomyelin with an enzymatic assay

The total amount of sphingomyelin was measured with the Sphiomgomyelin Quantification Colorimetric Assay Kit from BioVision incorporated, Milpitas (US) (Catalog # K600-100). To determine the background level of choline in plasma, a dilution series was prepared on the one hand with choline and on the other hand with a sphingomyelin standard. Both dilution series were measured with the background control mix without sphingomyelinase. The measurement of plasma samples was carried out with the standard reaction kit including sphingomyelinase and the amount of total sphingomyelin was calculated with the sphingomyelin standard curve. The total amount of sphingomyelin was measured with the Sphiomgomyelin Quantification Colorimetric Assay Kit from BioVision incorporated, Milpitas (US) (Catalog # K600-100). For each sample 25 mg (sample fresh weight, equivalent to 25 µl plasma) were homogenized in 0.5 ml of SM Assay Buffer. After centrifugation at 4 °C for 5 min at 10.000 x g, the supernatant was transferred to a separate tube. 20 µl of SM Assay Buffer were added to an aliquot of 20 µl of the homogenate (supernatant). Two times, the mixture was incubated at 70 °C for 1-2 min and then cooled down to room temperature. Afterwards, the sample was centrifuged at room temperature for 2 min at 10.000 x g to remove the debris. 1-5 µl of the supernatant were transferred to a 96-well clear plate with flat bottom and the volume was adjusted to 50 µl with SM Assay Buffer. 50 µl of "Reaction Mix" (see table, below) was added to each well and mixed well. The plate was incubated at 37 °C for 1 hr and then the absorbance was measured at 570 nm. For quantification a standard curve was prepared with a dilution series of a sphingomyelin standard. In addition the concentration of choline in plasma was determined with a dilution series of choline and reactions were performed with background control mix without sphingomyelinase.

| Component | Reaction Mix | Background Control Mix |
|---|---|---|
| SM Assay Buffer | 34 $\mu$l | 36 $\mu$l |
| Sphingomyelinase | 2 $\mu$l | --- |
| ALP Enzyme* | 10 $\mu$l | 10 $\mu$l |
| SM Enzyme Mix | 2 $\mu$l | 2 $\mu$l |
| OxiRed Probe™ ** | 2 $\mu$l | 2 $\mu$l |

*ALP=Alkaline phosphatase

**10-acetyl-3,7-dihydroxyphenoxazine in DMSO

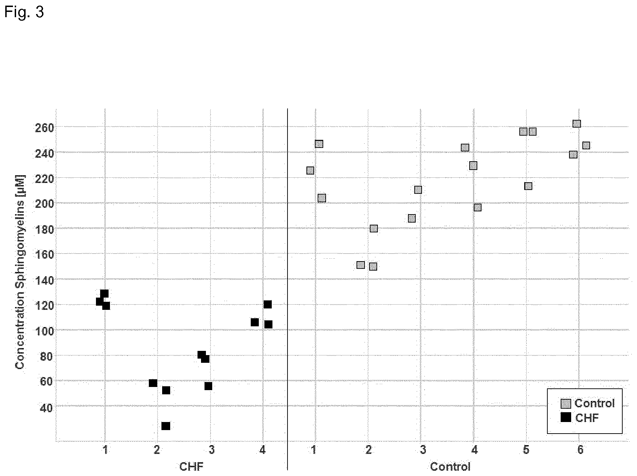

The amount of total sphingomyelin was measured in 4 CHF patients and 6 healthy controls in three technical replicates. Significant differences were observed with lower levels in the group of CHF patients (Ratio: 0.41 and $p<0.001$; ANOVA), as shown in Figure 3. The respective concentrations of total sphingomyelin are shown in Table 14.

For the measurements from which data is reported in Examples 9, 10 and 11, the total amount of sphingomyelin was determined as described above using final amount of 7.5 $\mu$l plasma in 50 $\mu$l reaction volume, except that the determination of the background level of choline in plasma was omitted.

For the performance calculations reported in Examples 9, 10 and 11, columns labelled 'Absorbance values', raw absorbance values at 570 nm were taken as total amount of sphingomyelin. For the performance calculations reported in the columns labelled 'Absolute concentrations', absolute concentrations in the samples were calculated using a linear regression model of the form $y = a_0 + a_1 * x$, where x was the absorbance value measured in the respective sample or standard, y was the absolute concentration in the same sample or standard, and $a_0$ and $a_1$ were contants fitted using the absorbance values and the known absolute concentrations in the standards.

Example 5: Determining the total amount of cholesteryl esters and the total amount of cholesterol with an enzymatic assay

The total amount of cholesteryl esters and the total amount of cholesterol was measured with the Cholesterol/Cholesteryl Ester Quantitation Colorimetric Kit II from BioVision, Inc., Milpitas (US) (Catalog # K623-100). The assay was carried out according to the manufactures instructions. Differences were observed with lower levels in the group of CHF patients.

In brief, the assay was carried out essentially as follows:
Standard curve was prepared by diluting cholesterol standards. Samples were prepared by diluting serum or plasma 10 times in Cholesterol Assay Buffer. 2 to 20 $\mu$l of the respective diluted sample were used for testing. In the measurements from which data is reported in Examples 9, 10, and 11, 10 $\mu$l of the pre-diluted samples were used in a final reaction volume of 50 $\mu$l. For detecting total cholesteryl esters, the sample may be pre-treated with cholesterol dehydrogenase in order to deplete the sample of free cholesterol for 30 min at 37°C. In the measurements from which data have been reported in Examples 9, 10, and 11, the pre-treatment with cholesterol dehydrogenase was omitted. Ester hydrolysis has been carried out for 30 min at 37°C out by adding 2 $\mu$l cholesterol esterase to each standard and to samples for which the total cholesterol value was desired, protected from light. Two measurements were carried out in parallel for the samples from which data is reported in Examples 9, 10, and 11: In one measurement, the treatment with cholesterol esterase was carried out as described, thus determining the sum of the total amount of cholesterol and the total amount of cholesteryl esters. In the other measurement, the treatment with esterase was omitted, thus determining the total amount of cholesterol. Absorbance at 450 nm was determined in a microplate reader.

For the performance calculations reported in Examples 9, 10, and 11, columns labelled 'Absorbance values', raw absorbance values at 450 nm were taken as total amount of sphingomyelin. For the performance calculations reported in the columns labelled 'Absolute concentrations', absolute concentrations in the samples were calculated using a linear regression model of the form $y = a_0 + a_1 * x$, where x was the absorbance value measured in the respective sample or standard, y was the absolute concentration in the same sample or standard, and $a_0$ and $a_1$ were contants fitted using the absorbance values and the known absolute concentrations in the standards.

Calculations of the absolute concentrations can also be done as follows: Subtract the 0 standard background reading from all readings. Plot the standard curve. Apply sample readings to the standard curve. Cholesterol concentration in samples can then be calculated: $C = A N * X * D$ ($\mu$g/$\mu$l), wherein A = amount of cholesterol determined from Standard Curve (in $\mu$g), V = volume of sample added into the reaction well (in $\mu$l) and D = Sample dilution factor.

The total amount of cholesteryl esters plus cholesterol was taken as approximation for the amount of cholesteryl esters in Examples 9, 10, and 11.

Table 1: Specific cholesteryl ester plasma concentrations as used for calculation of weighted sum of cholesteryl esters. Mean concentrations were calculated from all available HMDB entries for plasma of healthy subjects. Concentrations of metabolites marked with (*) were conservatively estimated from Lindgren loc cit.

| Cholesterylester | Plasma Concentration [μM] |
|---|---|
| Cholesterylester C12:0* | 10 |
| Cholesterylester C14:0 | 80 |
| Cholesterylester C14:1 | 17 |
| Cholesterylester C15:0 | 30 |
| Cholesterylester C16:0 | 208 |
| Cholesterylester C16:1 | 115 |
| Cholesterylester C16:2 | 31 |
| Cholesterylester C16:3* | 10 |
| Cholesterylester C18:0 | 48 |
| Cholesterylester C18:1 | 619 |
| Cholesterylester C18:2 | 1663 |
| Cholesterylester C18:3 | 85 |
| Cholesterylester C18:4* | 10 |
| Cholesterylester C20:1 | 15 |
| Cholesterylester C20:2 | 19 |
| Cholesterylester C20:3 | 17 |
| Cholesterylester C20:4 | 216 |
| Cholesterylester C20:5 | 39 |
| Cholesterylester C22:4* | 10 |
| Cholesterylester C22:5 | 3 |
| Cholesterylester C22:6 | 27 |

Table 2: Results of ANOVA for specific cholesteryl esters and weighted sum parameter of cholesteryl esters with correction for confounders age, BMI and gender

| CHF group | CHF | CHF | CHF | CHF | HFpEF | HFpEF | HFpEF | HFpEF | HFrEF | HFrEF | HFrEF | HFrEF |
|---|---|---|---|---|---|---|---|---|---|---|---|---|
| NYHA class | I | I | II-III | II-III | I | I | II-III | II-III | I | I | II-III | II-III |
| | | | | | | | | | | | | |
| Metabolite | Ratio | p.value | Ratio | p.value | Ratio | p.value | Ratio | p.value | Ratio | p.value | Ratio | p.value |
| Sum Cholesterylesters | 0.86 | 2.798E-09 | 0.80 | 1.69E-19 | 0.91 | 0.002345 | 0.89 | 0.001167 | 0.82 | 5.65E-12 | 0.77 | 1.23E-23 |
| Cholesterylester C12:0 | 0.88 | 0.0354468 | 0.73 | 3.49E-07 | 0.96 | 0.552784 | 0.81 | 0.013733 | 0.82 | 0.005249 | 0.72 | 1.76E-07 |
| Cholesterylester C14:0 | 0.91 | 0,0032367 | 0.84 | 5.61E-09 | 0.98 | 0.603738 | 0.92 | 0.04323 | 0.86 | 2.68E-05 | 0.82 | 3.02E-10 |
| Cholesterylester C14:1 | 0.96 | 0.3951464 | 0.87 | 0.008579 | 1.03 | 0.677673 | 1.02 | 0.837462 | 0.90 | 0.094173 | 0.84 | 0.001079 |
| Cholesterylester C15:0 | 0.87 | 3.327E-06 | 0.80 | 3.55E-13 | 0.92 | 0.020965 | 0.89 | 0.009025 | 0.83 | 1.21E-07 | 0.78 | 3.34E-15 |
| Cholesterylester C16:1 | 0.99 | 0.7370255 | 1.01 | 0.884781 | 1.03 | 0.500497 | 1.12 | 0.044705 | 0.95 | 0.277048 | 0.98 | 0.558058 |
| Cholesterylester C16:2 | 0.96 | 0.2297811 | 0.91 | 0.009124 | 1.03 | 0.511075 | 1.07 | 0.165879 | 0.90 | 0.014966 | 0.87 | 0.000201 |
| Cholesterylester C16:3 | 1.03 | 0.4381894 | 1.00 | 0.979695 | 1.11 | 0.01855 | 1.14 | 0.012994 | 0.96 | 0.402749 | 0.97 | 0.345649 |
| Cholesterylester C18:0 | 0.94 | 0.0427504 | 0.89 | 3.46E-05 | 1.02 | 0.654509 | 0.97 | 0.392355 | 0.89 | 0.000303 | 0.87 | 3.08E-06 |
| Cholesterylester C18:3 | 0.98 | 0.3766153 | 0.90 | 0.000243 | 1.04 | 0.260849 | 0.99 | 0.724242 | 0.93 | 0.017093 | 0.88 | 1.72E-05 |
| Cholesterylester C18:4 | 0.99 | 0.8808305 | 0.87 | 0.025194 | 1.13 | 0.104084 | 1.06 | 0.498071 | 0.89 | 0.096821 | 0.83 | 0.003218 |
| Cholesterylester C20:1 | 0.87 | 7.498E-05 | 0.84 | 3.42E-07 | 0.95 | 0.246302 | 0.93 | 0.148721 | 0.81 | 1.56E-07 | 0.82 | 1.9E-08 |
| Cholesterylester C20:2 | 0.97 | 0.1729355 | 0.95 | 0.027628 | 1.01 | 0.71819 | 1.01 | 0.842618 | 0.94 | 0.013692 | 0.94 | 0.006606 |
| Cholesterylester C20:3 | 1.01 | 0.7773566 | 0.99 | 0.822142 | 1.05 | 0.147663 | 1.03 | 0.368498 | 0.97 | 0.3969 | 0.99 | 0.572117 |
| Cholesterylester C20:5 | 0.92 | 0.0445879 | 0.86 | 0.000216 | 1.03 | 0.544867 | 1.03 | 0.5949 | 0.83 | 0.000211 | 0.81 | 2.58E-06 |
| Cholesterylester C22:4 | 0.98 | 0.4124077 | 0.98 | 0.412315 | 1.01 | 0.844029 | 1.07 | 0.091284 | 0,95 | 0,156637 | 0.95 | 0.095659 |
| Cholesterylester C22:5 | 0.92 | 0.0022797 | 0.90 | 2.35E-05 | 0.96 | 0.232478 | 1.01 | 0.888625 | 0.89 | 0.00016 | 0.87 | 1.27E-07 |
| Cholesterylester C22:6 | 0.93 | 0.027657 | 0.90 | 0.000905 | 0.99 | 0.769632 | 1.02 | 0.650228 | 0.88 | 0.001453 | 0.87 | 2.47E-05 |
| Cholesterylester C16:0 | 0.84 | 6.294E-05 | 0.80 | 2.05E-07 | 0.87 | 0.007459 | 0.91 | 0.128939 | 0.82 | 0.000101 | 0.77 | 7.19E-09 |
| Cholesterylester C18:1 | 0.79 | 2.331E-09 | 0.74 | 8.24E-15 | 0.86 | 0.001052 | 0.85 | 0.003816 | 0.74 | 6.31E-11 | 0.71 | 5.89E-17 |
| Cholesterylester C18:2 | 0.83 | 9.713E-12 | 0.76 | 1.08E-25 | 0.89 | 0.000167 | 0.85 | 1.69E-05 | 0.79 | 7.69E-14 | 0.73 | 3.11E-29 |
| Cholesterylester C20:4 | 0.94 | 0.0304506 | 0.95 | 0.045634 | 0.97 | 0.336072 | 1.01 | 0.88037 | 0.92 | 0.011814 | 0.93 | 0.012997 |

Table 3: Results of ANOVA for specific cholesteryl esters and weighted sum parameter of cholesteryl esters without correction for confounders age, BMI and gender

| CHF group | CHF | CHF | CHF | CHF | HFpEF | HFpEF | HFpEF | HFpEF | HFrEF | HFrEF | HFrEF | HFrEF |
|---|---|---|---|---|---|---|---|---|---|---|---|---|
| NYHA class | I | I | II-III | II-III | I | I | II-III | II-III | I | I | II-III | II-III |
| | | | | | | | | | | | | |
| Metabolite | Ratio | p.value | Ratio | p.value | Ratio | p.value | Ratio | p.value | Ratio | p.value | Ratio | p.value |
| Sum Cholesterylesters | 0.79 | 9.64E-19 | 0.74 | 1.84E-32 | 0.84 | 3.28E-08 | 0.85 | 7.92E-07 | 0.75 | 1.16E-20 | 0.70 | 4.82E-39 |
| Cholesterylester C12:0 | 0.86 | 0.008202 | 0.73 | 7.77E-09 | 0.94 | 0.351606 | 0.82 | 0.013929 | 0.79 | 0.000761 | 0.70 | 8.89E-10 |
| Cholesterylester C14:0 | 0.91 | 0.000557 | 0.84 | 6.84E-10 | 0.98 | 0.505008 | 0.94 | 0.122378 | 0.84 | 6.22E-07 | 0.81 | 7.45E-13 |
| Cholesterylester C14:1 | 0.92 | 0.111506 | 0.87 | 0.00234 | 1.00 | 0.958943 | 1.05 | 0.466423 | 0.86 | 0.010737 | 0.80 | 1.91E-05 |
| Cholesterylester C15:0 | 0.84 | 6.87E-09 | 0.80 | 1.96E-15 | 0.90 | 0.003817 | 0.93 | 0.062172 | 0.79 | 1.85E-11 | 0.76 | 1.26E-20 |
| Cholesterylester C16:1 | 0.99 | 0.752641 | 1.04 | 0.235247 | 1.05 | 0.29832 | 1.22 | 0.000175 | 0.93 | 0.123387 | 0.98 | 0.685727 |
| Cholesterylester C16:2 | 0.95 | 0.166695 | 0.93 | 0.030346 | 1.03 | 0.453043 | 1.13 | 0.008464 | 0.88 | 0.002436 | 0.87 | 3.2E-05 |
| Cholesterylester C16:3 | 1.05 | 0.121999 | 1.05 | 0.132881 | 1.14 | 0.00158 | 1.22 | 2.27E-05 | 0.98 | 0.563817 | 0.99 | 0.805261 |
| Cholesterylester C18:0 | 0.94 | 0.019977 | 0.89 | 8.3E-06 | 1.01 | 0.847645 | 0.97 | 0.45826 | 0.88 | 7.12E-05 | 0.87 | 8.12E-08 |
| Cholesterylester C18:3 | 0.96 | 0.092239 | 0.90 | 1.97E-05 | 1.02 | 0.559527 | 1.00 | 0.958817 | 0.90 | 0.000898 | 0.86 | 4.38E-08 |
| Cholesterylester C18:4 | 0.98 | 0.762124 | 0.89 | 0.034341 | 1.13 | 0.092746 | 1.11 | 0.173946 | 0.86 | 0.032479 | 0.82 | 0.000605 |
| Cholesterylester C20:1 | 0.88 | 5.65E-05 | 0.85 | 2.54E-08 | 0.95 | 0.200146 | 0.92 | 0.047013 | 0.82 | 1.34E-07 | 0.82 | 8.23E-10 |
| Cholesterylester C20:2 | 0.97 | 0.081445 | 0.96 | 0.01924 | 1.00 | 0.851478 | 1.01 | 0.635138 | 0.93 | 0.002623 | 0.94 | 0.001176 |
| Cholesterylester C20:3 | 1.02 | 0.373872 | 1.03 | 0.229353 | 1.07 | 0.033349 | 1.09 | 0.00927 | 0.98 | 0.52388 | 1.00 | 0.845825 |
| Cholesterylester C20:5 | 0.97 | 0.409276 | 0.93 | 0.066511 | 1.10 | 0.053925 | 1.15 | 0.010704 | 0.85 | 0.001174 | 0.86 | 0.000209 |
| Cholesterylester C22:4 | 0.97 | 0.203992 | 0.97 | 0.198815 | 0.99 | 0.704015 | 1.05 | 0.170593 | 0.95 | 0.089619 | 0.94 | 0.018482 |
| Cholesterylester C22:5 | 0.92 | 0.000993 | 0.91 | 1.28E-05 | 0.96 | 0.165515 | 1.02 | 0.608892 | 0.89 | 4.34E-05 | 0.87 | 3.26E-09 |
| Cholesterylester C22:6 | 0.97 | 0.314301 | 0.96 | 0.171376 | 1.04 | 0.279432 | 1.11 | 0.012992 | 0.90 | 0.006681 | 0.91 | 0.002314 |
| Cholesterylester C16:0 | 0.79 | 8.12E-09 | 0.76 | 5.49E-13 | 0.82 | 5.15E-05 | 0.88 | 0.015772 | 0.77 | 4.82E-08 | 0.72 | 1.08E-15 |
| Cholesterylester C18:1 | 0.71 | 2.46E-18 | 0.67 | 1.11E-26 | 0.78 | 4.5E-08 | 0.79 | 8.52E-06 | 0.66 | 2.27E-19 | 0.64 | 1.05E-30 |
| Cholesterylester C18:2 | 0.78 | 6.55E-21 | 0.70 | 5.55E-41 | 0.82 | 2.25E-09 | 0.79 | 3.31E-10 | 0.73 | 7.31E-22 | 0.67 | 1.67E-45 |
| Cholesterylester C20:4 | 0.89 | 4.46E-05 | 0.90 | 7.73E-05 | 0.92 | 0.015528 | 0.97 | 0.41097 | 0.87 | 2.17E-05 | 0.88 | 3.71E-06 |

Table 4: Results of ANOVA for specific sphingomyelins, sum parameter of sphingomyelins and 1-hydroxy-2-amino-(cis,trans)-3,5-octadecadiene with correction for confounders age, BMI and gender

| CHF group | CHF | CHF | CHF | CHF | HFpEF | HFpEF | HFpEF | HFpEF | HFrEF | HFrEF | HFrEF | HFrEF |
|---|---|---|---|---|---|---|---|---|---|---|---|---|
| NYHA class | I | I | II-III | II-III | I | I | II-III | II-III | I | I | II-III | II-III |
| | | | | | | | | | | | | |
| Metabolite | Ratio | p.value | Ratio | p.value | Ratio | p.value | Ratio | p.value | Ratio | p.value | Ratio | p.value |
| Sum Sphingomyelins | 0.883 | 1.26E-08 | 0.8443 | 8.32E-15 | 0.9265 | 0.003762 | 0.9063 | 0.001605 | 0.8483 | 1.29E-10 | 0.8291 | 1.61E-16 |
| 1-Hydroxy-2-amino-(cis, trans)-3,5-octadecadiene | 0.8143 | 1.08E-08 | 0.7427 | 1.15E-16 | 0.8797 | 0.003107 | 0.8141 | 6.09E-05 | 0.7628 | 1.36E-10 | 0.726 | 1.18E-17 |
| Sphingomyelin (d18:0,C16:0) | 0.9077 | 5.06E-06 | 0.8982 | 2.88E-07 | 0.9299 | 0.004806 | 0.9238 | 0.008934 | 0.8889 | 2.52E-06 | 0.892 | 1.85E-07 |
| Sphingomyelin (d18:0,C18:0) | 1.0379 | 0.341111 | 1.1038 | 0.010263 | 1.052 | 0.287788 | 1.0937 | 0.110693 | 1.0251 | 0.590967 | 1.1077 | 0.011305 |
| Sphingomyelin (d18:1,C14:0) | 0.8208 | 1.37E-09 | 0.7544 | 3.87E-18 | 0.8769 | 0.000787 | 0.8309 | 5.91E-05 | 0.7758 | 3.02E-11 | 0.7359 | 1.24E-19 |
| Sphingomyelin (d18:1,C16:0) | 0.9286 | 4.14E-06 | 0.9045 | 2.86E-10 | 0.9498 | 0.00802 | 0.9497 | 0.023844 | 0.9114 | 8.76E-07 | 0.8927 | 8.6E-12 |
| Sphingomyelin (d18:1,C18:0) | 0.9746 | 0.31174 | 0.9849 | 0.544018 | 1.0075 | 0.808745 | 1.0224 | 0.543947 | 0.9471 | 0.069568 | 0.976 | 0.35356 |
| Sphingomyelin (d18:1,C18:1) | 0.8936 | 2.02E-05 | 0.8827 | 1.62E-06 | 0.9185 | 0.008016 | 0.9145 | 0.017786 | 0.8727 | 1.21E-05 | 0.8749 | 9.48E-07 |
| Sphingomyelin (d18:1,C20:0) | 0.8784 | 8.7E-06 | 0.8579 | 9.85E-08 | 0.9156 | 0.01224 | 0.937 | 0.116129 | 0.8486 | 1.6E-06 | 0.8377 | 3.75E-09 |
| Sphingomyelin (d18:1,C20:1) | 0.9185 | 0.002233 | 0.9556 | 0.096184 | 0.9225 | 0.017358 | 0.9868 | 0.738407 | 0.9158 | 0.007302 | 0.9468 | 0.05622 |
| Sphingomyelin (d18:1,C21:0) | 0.8692 | 4.28E-06 | 0.8553 | 2E-07 | 0.8981 | 0.003643 | 0.9118 | 0.03373 | 0.8457 | 3E-06 | 0.8408 | 3.61E-08 |
| Sphingomyelin (d18:1,C22:0) | 0.8707 | 1.06E-07 | 0.8189 | 1.16E-14 | 0.9197 | 0.007402 | 0.8903 | 0.001613 | 0.8311 | 1.33E-09 | 0.8014 | 2.08E-16 |
| Sphingomyelin (d18:1,C22:1) | 0.8525 | 4.48E-07 | 0.8585 | 9.33E-07 | 0.8885 | 0.001954 | 0.9416 | 0.179875 | 0.8238 | 1.75E-07 | 0.8373 | 4.64E-08 |

(continued)

| CHF group | CHF | CHF | CHF | CHF | HFpEF | HFpEF | HFpEF | HFpEF | HFrEF | HFrEF | HFrEF | HFrEF |
|---|---|---|---|---|---|---|---|---|---|---|---|---|
| NYHA class | I | I | II-III | II-III | I | I | II-III | II-III | I | I | II-III | II-III |
| | | | | | | | | | | | | |
| Metabolite | Ratio | p.value | Ratio | p.value | Ratio | p.value | Ratio | p.value | Ratio | p.value | Ratio | p.value |
| Sphingomyelin (d18: 1,C23:0) | 0.8399 | 1.14E-08 | 0.7743 | 4.19E-17 | 0.9038 | 0.005796 | 0.8272 | 1.21E-05 | 0.7882 | 3.14E-11 | 0.7626 | 1.11E-17 |
| Sphingomyelin (d18: 1,C23:1) | 0.8878 | 9.84E-05 | 0.8796 | 2.02E-05 | 0.9226 | 0.029734 | 0.9217 | 0.061666 | 0.859 | 2.41E-05 | 0.8695 | 9.03E-06 |
| Sphingomyelin (d18: 1,C24:0) | 0.8354 | 1.5E-07 | 0.77 | 1.54E-14 | 0.9001 | 0.010564 | 0.8355 | 0.000217 | 0.7833 | 1.22E-09 | 0.7549 | 2.11E-15 |
| Sphingomyelin (d18: 1,C24:1) | 0.9506 | 0.019174 | 0.9406 | 0.004036 | 0.9752 | 0.337015 | 1.0084 | 0.786998 | 0.9311 | 0.004919 | 0.9227 | 0.000299 |
| Sphingomyelin (d18: 1,C24:2) | 0.8782 | 1.33E-05 | 0.8885 | 5.49E-05 | 0.898 | 0.002887 | 0.9703 | 0,476852 | 0.8633 | 2.64E-05 | 0.8667 | 3.06E-06 |
| Sphingomyelin (d18: 2,C14:0) | 0.8976 | 6.44E-05 | 0.8557 | 5.77E-09 | 0.945 | 0.082884 | 0.9273 | 0.049244 | 0.8592 | 1.7E-06 | 0.8381 | 2.42E-10 |
| Sphingomyelin (d18: 2,C16:0) | 0.9314 | 0.006619 | 0.9294 | 0.00449 | 0.9669 | 0.289627 | 0.9714 | 0.437566 | 0.9019 | 0,000805 | 0.9194 | 0.001806 |
| Sphingomyelin (d18: 2,C18:1) | 0.8821 | 1.59E-05 | 0.8719 | 1.68E-06 | 0.9038 | 0.004167 | 0.9088 | 0.021319 | 0.8643 | 2.04E-05 | 0.8625 | 8.21E-07 |
| Sphingomyelin (d18: 2,C20:0) | 0.9414 | 0.015914 | 0.9307 | 0.003595 | 0.971 | 0.334214 | 0.9719 | 0.427189 | 0.9168 | 0.003242 | 0.9205 | 0.001331 |
| Sphingomyelin (d18: 2,C20:1) | 0.9153 | 0.001785 | 0.9252 | 0.005294 | 0.935 | 0.050869 | 0.9816 | 0.647476 | 0.8996 | 0.001523 | 0.9103 | 0,001277 |
| Sphingomyelin (d18: 2,021:0) | 0.9156 | 0.000503 | 0.9013 | 3.17E-05 | 0.9426 | 0.05446 | 0.9466 | 0.129972 | 0.8934 | 0.000159 | 0.8898 | 7.95E-06 |
| Sphingomyelin (d18: 2.C22:0) | 0.8629 | 1.17E-06 | 0.8082 | 1.48E-12 | 0.9238 | 0.02954 | 0.8905 | 0.006903 | 0,8141 | 7.01E-09 | 0.7884 | 3.08E-14 |
| Sphingomyelin (d18: 2.C22;1) | 0.8955 | 2.16E-05 | 0.907 | 0.000132 | 0.9212 | 0.009175 | 0.9478 | 0.148281 | 0.8744 | 1.14E-05 | 0.8967 | 4.57E-05 |

EP 3 194 959 B1

(continued)

| CHF group | CHF | CHF | CHF | CHF | HFpEF | HFpEF | HFpEF | HFpEF | HFrEF | HFrEF | HFrEF | HFrEF |
|---|---|---|---|---|---|---|---|---|---|---|---|---|
| NYHA class | I | I | II-III | II-III | I | I | II-III | II-III | I | I | II-III | II-III |
| | | | | | | | | | | | | |
| Metabolite | Ratio | p.value | Ratio | p.value | Ratio | p.value | Ratio | p.value | Ratio | p.value | Ratio | p.value |
| Sphingomyelin (d18:2,C23:0) | 0.8626 | 8.04E-09 | 0.7913 | 6.38E-20 | 0.9257 | 0.011772 | 0.8351 | 6.96E-07 | 0.8111 | 3.16E-12 | 0.7821 | 2.35E-20 |
| Sphingomyelin (d18:2,C23:1) | 0.8619 | 1E-05 | 0.8239 | 5.73E-09 | 0.9107 | 0.021473 | 0.8823 | 0.008972 | 0.8221 | 7.41E-07 | 0.81 | 1.33E-09 |
| Sphingomyelin (d18:2,C24:0) | 0.833 | 1.13E-08 | 0.7604 | 8.53E-18 | 0.8997 | 0.005888 | 0.8211 | 1.38E-05 | 0.7793 | 3E-11 | 0.7466 | 1.26E-18 |
| Sphingomyelin (d182,C24:1) | 0.9112 | 0.001392 | 0.8948 | 0.000106 | 0.9515 | 0.156827 | 0.9924 | 0.853319 | 0.8794 | 0.000161 | 0.87 | 3.13E-06 |
| Sphingomyelin (d18:2,C24:2) | 0.9038 | 0.000107 | 0.8966 | 2.16E-05 | 0.9199 | 0.008323 | 0.96 | 0.272037 | 0.8916 | 0.000183 | 0.8795 | 1.82E-06 |
| Sphingomyelin (d16:1,C16:0) | 0.8552 | 5.32E-06 | 0.798 | 3.5E-11 | 0.9044 | 0.01564 | 0.8554 | 0.001436 | 0-8151 | 4.2E-07 | 0.7843 | 8.82E-12 |
| Sphingomyelin (d16:1,C18:0) | 0.943 | 0.05055 | 0.9049 | 0.00073 | 0.9862 | 0.702557 | 0.9594 | 0.333595 | 0.9076 | 0.006009 | 0.8917 | 0.00021 |
| Sphingomyelin (d16:1,C18:1) | 0.8534 | 4.8E-06 | 0.7997 | 7.49E-11 | 0.8967 | 0.009307 | 0.8649 | 0.003312 | 0.8183 | 8.7E-07 | 0.7836 | 1.09E-11 |
| Sphingomyelin (d16:1,C20:0) | 0.8784 | 5.97E-05 | 0.8222 | 9.11E-10 | 0.9368 | 0.093169 | 0.9036 | 0.027066 | 0.8317 | 1.12E-06 | 0.8025 | 3.97E-11 |
| Sphingomyelin (d16:1,C21:0) | 0.8542 | 2E-06 | 0.7993 | 9.36E-12 | 0.8844 | 0.002235 | 0.8551 | 0.000944 | 0.8297 | 1.71E-06 | 0.785 | 2.03E-12 |
| Sphingomyelin (d16:1,C22:0) | 0.7817 | 2.58E-10 | 0.6931 | 4.61E-21 | 0.8534 | 0.000659 | 0.797 | 3.57E-05 | 0.7257 | 1.88E-12 | 0.6685 | 2.38E-23 |
| Sphingomyelin (d16:1,C22:1 | 0.8295 | 2.98E-07 | 0.7918 | 9.56E-11 | 0.8727 | 0.00197 | 0.8802 | 0.013657 | 0.7953 | 8.67E-08 | 0.7697 | 3.63E-12 |
| Sphingomyelin (d16:1,C23:0) | 0.7785 | 1.04E-09 | 0.6701 | 2.02E-22 | 0.8582 | 0.001843 | 0.7546 | 1.24E-06 | 0.716 | 3.58E-12 | 0.6507 | 8.65E-24 |

| CHF group | CHF | CHF | CHF | CHF | HFpEF | HFpEF | HFpEF | HFpEF | HFrEF | HFrEF | HFrEF | HFrEF |
|---|---|---|---|---|---|---|---|---|---|---|---|---|
| NYHA class | I | I | II-III | II-III | I | I | II-III | II-III | I | I | II-III | II-III |
| | | | | | | | | | | | | |
| Metabolite | Ratio | p.value | Ratio | p.value | Ratio | p.value | Ratio | p.value | Ratio | p.value | Ratio | p.value |
| Sphingomvelin (d16: 1,C24:0) | 0.8253 | 1.21E-08 | 0.706 | 9.99E-25 | 0.9066 | 0.014387 | 0.8081 | 6.91E-06 | 0.7619 | 4.25E-12 | 0.682 | 1.15E-27 |
| Sphingomyelin (d16: 1,C24:1) | 0.8218 | 3.13E-08 | 0.7653 | 2.94E-14 | 0.8789 | 0.00239 | 0.8731 | 0.006683 | 0.777 | 1.16E-09 | 0.7389 | 1.44E-16 |
| Sphingomyelin (d17: 1,C16:0) | 0.8493 | 3.82E-08 | 0.7948 | 6.99E-15 | 0.8863 | 0.000749 | 0.8651 | 0.000589 | 0.8197 | 1.15E-08 | 0.777 | 2.71E-16 |
| Sphingomyelin (d17: 1,C18:0) | 0.928 | 0.007389 | 0.8951 | 5.63E-05 | 0.9604 | 0.23298 | 0.9392 | 0.116375 | 0.9012 | 0.001548 | 0.8841 | 1.93E-05 |
| Sphingomyelin (d17: 1,C20:0) | 0.8834 | 1.24E-05 | 0.832 | 6.11E-11 | 0.9125 | 0.007773 | 0.8843 | 0.002418 | 0.8596 | 5.86E-06 | 0.8186 | 1.05E-11 |
| Sphingomyelin (d17: 1,C22:0) | 0.7972 | 4.95E-10 | 0.7089 | 3.4E-21 | 0.8568 | 0.000408 | 0.7876 | 3.76E-06 | 0.7498 | 1.54E-11 | 0.69 | 1.39E-22 |
| Sphingomyelin (d17: 1,C23:0) | 0.8112 | 2.54E-09 | 0.7013 | 8.05E-24 | 0.885 | 0.003613 | 0.7789 | 4.99E-07 | 0.7528 | 4.88E-12 | 0.6833 | 2.67E-25 |
| Sphingomyelin (d17: 1,C24:0) | 0.7852 | 1.07E-11 | 0.6828 | 1.37E-26 | 0.8566 | 0.000268 | 0.7581 | 3.71E-08 | 0.7286 | 2.94E-14 | 0.6653 | 5.57E-28 |
| Sphingomyelin (d17: 1,C24:1) | 0.8522 | 9.8E-08 | 0.8011 | 9.15E-14 | 0.8885 | 0.001081 | 0.8784 | 0.002306 | 0.8233 | 3.26E-08 | 0.7814 | 2.1E-15 |
| Sphingomyelin (d18: 2,C18:0) | 1.0149 | 0.356941 | 1.036 | 0.026593 | 1.0156 | 0.432151 | 1.0169 | 0.471523 | 1.0137 | 0.473344 | 1.0418 | 0.014526 |

EP 3 194 959 B1

Table 5: Results of ANOVA for specific sphingomyelins, sum parameter of sphingomyelins and 1-hydroxy-2-amino-(cis,trans)-3,5-octadecadiene without correction for confounders age, BMI and gender

| CHF group | CHF | CHF | CHF | CHF | HFpEF | HFpEF | HFpEF | HFpEF | HFrEF | HFrEF | HFrEF | HFrEF |
|---|---|---|---|---|---|---|---|---|---|---|---|---|
| NYHA class | I | I | II-III | II-III | I | I | II-III | II-III | I | I | II-III | II-III |
| | | | | | | | | | | | | |
| Metabolite | Ratio | p.value | Ratio | p.value | Ratio | p.value | Ratio | p.value | Ratio | p.value | Ratio | p.value |
| Sum Sphingomyelins | 0.8459 | 1.43E-12 | 0.8386 | 4.15E-15 | 0.9051 | 0.00041 | 0.9578 | 0.176406 | 0.7931 | 1.42E-16 | 0.7978 | 2.01E-21 |
| 1-Hydroxy-2-amino-(cis,trans)-3,5-octadecadiene | 0.7838 | 4.2E-11 | 0.7473 | 1.09E-16 | 0.8681 | 0.001467 | 0.8862 | 0.016028 | 0.7117 | 1.2E-14 | 0.7009 | 2E-21 |
| Sphingomyelin (d18:0,C16:0) | 0.8822 | 1.5E-09 | 0.8858 | 5.77E-10 | 0.9133 | 0.000345 | 0.9315 | 0.012902 | 0.8535 | 2.47E-10 | 0.8695 | 2.71E-11 |
| Sphingomyelin (d18:0,C18:0) | 1.1076 | 0.008223 | 1.24 | 4.76E-09 | 1.1553 | 0.002512 | 1.3111 | 5.84E-07 | 1.064 | 0.184408 | 1.2146 | 8.1E-07 |
| Sphingomyelin (d18:1,C14:0) | 0.7521 | 1.71E-16 | 0.7194 | 1.41E-23 | 0.8221 | 2.09E-06 | 0.8531 | 0.00063 | 0.6907 | 2.62E-19 | 0.6754 | 3.3E-29 |
| Sphingomyelin (d18:1,C16:0) | 0.9017 | 1.41E-10 | 0.8855 | 1.98E-15 | 0.9267 | 0.000101 | 0.9421 | 0.006915 | 0.8784 | 2.14E-11 | 0.8654 | 1.17E-18 |
| Sphingomyelin (d18:1,C18:0) | 0.958 | 0.082239 | 0.9958 | 0.855589 | 1.0046 | 0.879375 | 1.0813 | 0.022258 | 0.9155 | 0.002966 | 0.9658 | 0.161611 |
| Sphingomyelin (d18:1,C18:1) | 0.8282 | 1.32E-11 | 0.8427 | 6.89E-11 | 0.8654 | 1.93E-05 | 0.9336 | 0.071044 | 0.7941 | 5.53E-12 | 0.8114 | 1.03E-13 |
| Sphingomyelin (d18:1,C20:0) | 0.8596 | 9.11E-08 | 0.8661 | 7.23E-08 | 0.908 | 0.004833 | 0.9892 | 0.778459 | 0.8156 | 1.72E-09 | 0.8246 | 1.2E-11 |
| Sphingomyelin (d18:1,C20:1) | 0.8581 | 9.85E-08 | 0.9227 | 0.002813 | 0.8797 | 0.000264 | 1.0184 | 0.643805 | 0.8376 | 2.93E-07 | 0.8898 | 5.26E-05 |
| Sphingomyelin (d18:1,C21:0) | 0.814 | 2.69E-10 | 0.8416 | 1.79E-08 | 0.8643 | 0.000211 | 0.9805 | 0.655976 | 0.7685 | 1.37E-11 | 0.7954 | 24E-12 |
| Sphingomyelin (d18:1,C22:0) | 0.8639 | 3.42E-09 | 0.8258 | 4.48E-16 | 0.9141 | 0.002712 | 0.9114 | 0.006057 | 0.8186 | 1.51E-11 | 0.7962 | 1.25E-19 |
| Sphingomyelin (d18:1,C22:1) | 0.8186 | 2.87E-10 | 0.8586 | 3.15E-07 | 0.8713 | 0.000317 | 1.0069 | 0.873184 | 0.7709 | 6.46E-12 | 0.8096 | 2.67E-11 |

EP 3 194 959 B1

| Metabolite | Ratio | p.value | Ratio | p.value | Ratio | p.value | Ratio | p.value | Ratio | p.value | Ratio | p.value |
|---|---|---|---|---|---|---|---|---|---|---|---|---|
| Sphingomyelin (d18:1,C23:0) | 0.8022 | 3.27E-13 | 0.7632 | 7.17E-21 | 0.8756 | 0.000258 | 0.8579 | 0.000189 | 0.738 | 5.14E-17 | 0.7308 | 1.54E-24 |
| Sphingomyelin (d18:1,C23:1) | 0.844 | 8.07E-08 | 0.8736 | 5.42E-06 | 0.8997 | 0.005827 | 0.9937 | 0.883096 | 0.794 | 1.15E-09 | 0.8329 | 8.2E-09 |
| Sphingomyelin (d18:1,C24:0) | 0.8261 | 1.63E-09 | 0.7676 | 2.08E-18 | 0.8875 | 0.001936 | 0.8352 | 3.61E-05 | 0.7717 | 1.12E-11 | 0.7438 | 5.61E-20 |
| Sphingomyelin (d18:1,C24:1) | 0.9404 | 0.00295 | 0.9442 | 0.003206 | 0.97 | 0.226562 | 1.0311 | 0.282109 | 0.9128 | 0.000233 | 0.9141 | 1.55E-05 |
| Sphingomyelin (d18:1,C24:2) | 0.8137 | 7.27E-12 | 0.8451 | 2.64E-09 | 0.8432 | 2.92E-06 | 0.9706 | 0.465826 | 0.786 | 2.2E-11 | 0.8031 | 3.99E-13 |
| Sphingomyelin (d18:2,C14:0) | 0.8275 | 2.94E-09 | 0.8374 | 3.62E-09 | 0,8999 | 0.00565 | 1.0077 | 0.857977 | 0.7635 | 9.9E-13 | 0.782 | 1.01E-14 |
| Sphingomyelin (d18:2,C16:0) | 0.8878 | 9.75E-06 | 0.9201 | 0.001003 | 0.9399 | 0.057669 | 1.0286 | 0.444011 | 0.8407 | 7.47E-08 | 0.8829 | 3.9E-06 |
| Sphingomyelin (d18:2,C18:1) | 0.8116 | 4.66E-11 | 0.8385 | 3.49E-09 | 0.8515 | 2.91E-05 | 0.9581 | 0.322197 | 0.775 | 2.01E-11 | 0.7982 | 1.59E-12 |
| Sphingomyelin (d18:2,C20:0) | 0.8839 | 1.92E-06 | 0.9018 | 2.31E-05 | 0.9269 | 0.016112 | 0.9995 | 0.989172 | 0.8445 | 5.61E-08 | 0.8682 | 5.96E-08 |
| Sphingomyelin (d18:2,C20:1) | 0.8366 | 1.11E-08 | 0.8825 | 2E-05 | 0.8756 | 0.00045 | 1.0214 | 0.618625 | 0.8005 | 2.61E-09 | 0.8361 | 1.06E-08 |
| Sphingomyelin (d18:2,C21:0) | 0.8412 | 1.62E-09 | 0.8644 | 6.51E-08 | 0.888 | 0.000608 | 0.9873 | 0.742635 | 0.7985 | 5.35E-11 | 0.8229 | 1.22E-11 |
| Sphingomyelin (d18:2,C22:0) | 0.7926 | 7.37E-14 | 0.7641 | 7.93E-20 | 0.8563 | 3.07E-05 | 0.8881 | 0.004624 | 0.7362 | 1.38E-16 | 0.7227 | 4.58E-25 |
| Sphingomyelin (d18:2,C22:1) | 0.84 | 6.1E-10 | 0.8875 | 6.23E-06 | 0.8852 | 0.00035 | 1.0056 | 0.884478 | 0.7988 | 2.71E-11 | 0.8474 | 4.21E-09 |
| Sphingomyelin (d18:2,C23:0) | 0.7836 | 6.72E-18 | 0.7411 | 1.78E-28 | 0.8538 | 2.84E-06 | 0.8346 | 2.11E-06 | 0.7221 | 5.61E-22 | 0.7091 | 6.04E-33 |
| Sphingomyelin (d18:2,C23:1) | 0.7899 | 1.52E-10 | 0.7981 | 8.44E-11 | 0.8612 | 0.000747 | 0.9518 | 0.32207 | 0.7272 | 4.33E-13 | 0.7477 | 3.61E-15 |

| Metabolite | Ratio | p.value | Ratio | p.value | Ratio | p.value | Ratio | p.value | Ratio | p.value | Ratio | p.value |
|---|---|---|---|---|---|---|---|---|---|---|---|---|
| Sphingomyelin (d18:2,C24:0) | 0.7634 | 1.79E-17 | 0.7078 | 6.97E-30 | 0.8272 | 6.68E-07 | 0.7908 | 5.41E-08 | 0.7072 | 9.08E-20 | 0.6792 | 1.02E-32 |
| Sphingomyelin (d18:2,C24:1) | 0.8473 | 2.37E-08 | 0.8575 | 3.9E-08 | 0.8959 | 0.002119 | 1.0058 | 0.886256 | 0.8029 | 5.38E-10 | 0.8085 | 8.69E-13 |
| Sphingomyelin (d18:2,C24:2) | 0.8259 | 7.33E-12 | 0.8445 | 1.23E-10 | 0.8547 | 3.39E-06 | 0.9623 | 0.310767 | 0.799 | 1.75E-11 | 0.8048 | 1.04E-14 |
| Sphingomyelin (d16:1,C16:0) | 0.8475 | 1.44E-06 | 0.8305 | 1.05E-08 | 0.9219 | 0.05018 | 0.9605 | 0.390234 | 0.7822 | 2.25E-09 | 0.787 | 4.1E-12 |
| Sphingomyelin (d16:1,C18:0) | 0.9658 | 0.259474 | 0.9786 | 0.457489 | 1.0417 | 0.276982 | 1.1194 | 0.007913 | 0.8985 | 0.003709 | 0.9311 | 0.020702 |
| Sphingomyelin (d16:1,C18:1) | 0.8071 | 5.91E-09 | 0.8017 | 2.09E-10 | 0.8761 | 0.002862 | 0.9634 | 0.455448 | 0.7461 | 2.59E-11 | 0.749 | 5.63E-15 |
| Sphingomyelin (d16:1,C20:0) | 0.8735 | 3.25E-05 | 0.8586 | 7.16E-07 | 0.9559 | 0.249915 | 1.0141 | 0.750837 | 0.8013 | 1.09E-08 | 0.8072 | 4.84E-11 |
| Sphingomyelin (d16:1,C21:0) | 0.8224 | 2.8E-08 | 0.8156 | 8.76E-10 | 0.8828 | 0.003382 | 0.9631 | 0.433102 | 0.7684 | 3.83E-10 | 0.7669 | 7E-14 |
| Sphingomyelin (d16:1,C22:0) | 0.7688 | 4.93E-12 | 0.7141 | 1.76E-20 | 0.8566 | 0.000653 | 0.8746 | 0.008921 | 0.6933 | 5.59E-16 | 0.6624 | 9.54E-27 |
| Sphingomyelin (d16:1,C22:1) | 0.8055 | 8.97E-09 | 0.8145 | 7.35E-09 | 0.876 | 0.003429 | 0.9941 | 0.907959 | 0.7431 | 3.45E-11 | 0.7567 | 1.34E-13 |
| Sphingomyelin (d16:1,C23:0) | 0.7372 | 3.8E-13 | 0.6706 | 2.4E-23 | 0.8376 | 0.000402 | 0.8254 | 0.000689 | 0.6526 | 1.33E-17 | 0.6209 | 3.93E-29 |
| Sphingomyelin (d16:1,C24:0) | 0.8166 | 4.24E-10 | 0.7183 | 3.54E-26 | 0.9056 | 0.010468 | 0.8472 | 0.000154 | 0.7398 | 5.58E-15 | 0.6756 | 2.23E-32 |
| Sphingomyelin (d16:1,C24:1) | 0.8101 | 2.68E-09 | 0.7937 | 5.21E-12 | 0.8886 | 0.005201 | 0.976 | 0.610411 | 0.7414 | 9.83E-13 | 0.7353 | 3.82E-18 |
| Sphingomyelin (d17:1,C16:0) | 0.8106 | 5.7E-12 | 0.7878 | 1.64E-16 | 0.8648 | 7.41E-05 | 0.9169 | 0.035427 | 0.7618 | 7.39E-14 | 0.7449 | 1.14E-21 |
| Sphingomyelin (d17:1,C18:0) | 0.9147 | 0.00194 | 0.9238 | 0.00344 | 0.9726 | 0.426229 | 1.0449 | 0.266118 | 0.8626 | 1.78E-05 | 0.8826 | 1.51E-05 |

EP 3 194 959 B1

(continued)

| Metabolite | Ratio | p.value | Ratio | p.value | Ratio | p.value | Ratio | p.value | Ratio | p.value | Ratio | p.value |
|---|---|---|---|---|---|---|---|---|---|---|---|---|
| Sphingomyelin (d17:1,C20:0) | 0.8595 | 1.32E-07 | 0.8412 | 1.9E-10 | 0.9079 | 0.005472 | 0.9534 | 0.22395 | 0.8155 | 2.97E-09 | 0.8031 | 3.91E-14 |
| Sphingomyelin (d17:1,C22:0) | 0.7599 | 5.52E-14 | 0.7067 | 2.36E-23 | 0.8343 | 3.38E-05 | 0.8454 | 0.000654 | 0.6951 | 6.33E-17 | 0.6614 | 6.83E-29 |
| Sphingomyelin (d17:1,C23:0) | 0.769 | 2.87E-13 | 0.6944 | 4.36E-26 | 0.8576 | 0.000344 | 0.8298 | 0.000119 | 0.693 | 1.09E-17 | 0.65 | 5.55E-32 |
| Sphingomyelin (d17:1,C24:0) | 0.7524 | 3.14E-16 | 0.6756 | 1.35E-31 | 0.8322 | 1E-05 | 0.7889 | 4.71E-07 | 0.6835 | 5.49E-20 | 0.6378 | 1.62E-36 |
| Sphingomyelin (d17:1,C24:1) | 0.8132 | 1.97E-11 | 0.7959 | 5.21E-15 | 0.8671 | 0.000116 | 0.9374 | 0.120524 | 0.7647 | 2.6E-13 | 0.7492 | 1.38E-20 |
| Sphingomyelin (d18:2,C18:0) | 0.9952 | 0.76838 | 1.0389 | 0.013561 | 1.011 | 0.587775 | 1.0638 | 0.006977 | 0.9805 | 0.318169 | 1.0296 | 0.079171 |

Table 6: Area under the curve (AUC) values of receiver operating characteristic (ROC) analysis for specific cholesteryl esters and weighted sum parameter of cholesteryl esters with correction for confounders age, BMI and gender

| CHF group | CHF | CHF | HFpEF | HFpEF | HFrEF | HFrEF |
|---|---|---|---|---|---|---|
| NYHA class | I | II or III | I | II or III | I | II or III |
| | | | | | | |
| NT-proBNP | 0.798 | 0.889 | 0.647 | 0.746 | 0.914 | 0.939 |
| Sum Cholesterylesters | 0.665 | 0.714 | 0.486 | 0.478 | 0.720 | 0.762 |
| CE_Cholesterylester C12:0 | 0.520 | 0.623 | 0.486 | 0.478 | 0.493 | 0.632 |
| CE_Cholesterylester C14:0 | 0.547 | 0.619 | 0.486 | 0.478 | 0.537 | 0.653 |
| CE_Cholesterylester C14:1 | 0.492 | 0.519 | 0.486 | 0.478 | 0.493 | 0.561 |
| CE_Cholesterylester C15:0 | 0.609 | 0.661 | 0.486 | 0.478 | 0.640 | 0.705 |
| CE_Cholesterylester C16:1 | 0.492 | 0.495 | 0.486 | 0.478 | 0.493 | 0.497 |
| CE_Cholesterylester C16:2 | 0.536 | 0.489 | 0.486 | 0.478 | 0.493 | 0.590 |
| CE_Cholesterylester C16:3 | 0.480 | 0.495 | 0.486 | 0.478 | 0.493 | 0.497 |
| CE_Cholesterylester C18:0 | 0.502 | 0.586 | 0.486 | 0.478 | 0.570 | 0.624 |
| CE_Cholesterylester C18:3 | 0.492 | 0.566 | 0.486 | 0.478 | 0.493 | 0.613 |
| CE_Cholesterylester C18:4 | 0.492 | 0.486 | 0.486 | 0.478 | 0.493 | 0.580 |
| CE_Cholesterylester C20:1 | 0.619 | 0.624 | 0.486 | 0.478 | 0.701 | 0.664 |
| CE_Cholesterylester C20:2 | 0.518 | 0.504 | 0.486 | 0.478 | 0.556 | 0.592 |
| CE_Cholesterylester C20:3 | 0.492 | 0.495 | 0.486 | 0.478 | 0.493 | 0.497 |
| CE_Cholesterylester C20:5 | 0.528 | 0.526 | 0.486 | 0.478 | 0.573 | 0.620 |
| CE_Cholesterylester C22:4 | 0.492 | 0.495 | 0.486 | 0.478 | 0.493 | 0.497 |
| CE_Cholesterylester C22:5 | 0.565 | 0.592 | 0.486 | 0.478 | 0.539 | 0.641 |
| CE_Cholesterylester C22:6 | 0.577 | 0.510 | 0.486 | 0.478 | 0.624 | 0.609 |
| Cholesterylester C16:0 | 0.603 | 0.629 | 0.486 | 0.478 | 0.537 | 0.661 |
| Cholesterylester C18:1 | 0.651 | 0.690 | 0.545 | 0.478 | 0.683 | 0.717 |

(continued)

| CHF group | CHF | CHF | HFpEF | HFpEF | HFrEF | HFrEF |
|---|---|---|---|---|---|---|
| NYHA class | I | II or III | I | II or III | I | II or III |
| Cholesterylester C18:2 | 0.684 | 0.748 | 0.530 | 0.558 | 0.746 | 0.801 |
| Cholesterylester C20:4 | 0.492 | 0.495 | 0.486 | 0.478 | 0.493 | 0.509 |

Table 7: Area under the curve (AUC) values of receiver operating characteristic (ROC) analysis for specific cholesteryl esters and weighted sum parameter of cholesteryl esters without correction for confounders age, BMI and gender

| CHF group | CHF | CHF | HFpEF | HFpEF | HFrEF | HFrEF |
|---|---|---|---|---|---|---|
| NYHA class | I | II or III | I | II or III | I | II or III |
| | | | | | | |
| NT-proBNP | 0.790 | 0.896 | 0.663 | 0.786 | 0.891 | 0.937 |
| Sum Cholesterylesters | 0.723 | 0.763 | 0.653 | 0.548 | 0.780 | 0.813 |
| CE_Cholesterylester C12:0 | 0.555 | 0.632 | 0.486 | 0.478 | 0.493 | 0.644 |
| CE_Cholesterylester C14:0 | 0.569 | 0.625 | 0.486 | 0.478 | 0.570 | 0.664 |
| CE_Cholesterylester C14:1 | 0.492 | 0.526 | 0.486 | 0.478 | 0.493 | 0.596 |
| CE_Cholesterylester C15:0 | 0.638 | 0.673 | 0.517 | 0.478 | 0.677 | 0.728 |
| CE_Cholesterylester C16:1 | 0.492 | 0.495 | 0.486 | 0.512 | 0.493 | 0.497 |
| CE_Cholesterylester C16:2 | 0.535 | 0.495 | 0.486 | 0.478 | 0.464 | 0.588 |
| CE_Cholesterylester C16:3 | 0.488 | 0.495 | 0.511 | 0.599 | 0.493 | 0.497 |
| CE_Cholesterylester C18:0 | 0.511 | 0.584 | 0.486 | 0.478 | 0.557 | 0.628 |
| CE_Cholesterylester C18:3 | 0.518 | 0.574 | 0.486 | 0.478 | 0.469 | 0.630 |
| CE_Cholesterylester C18:4 | 0.492 | 0.495 | 0.486 | 0.478 | 0.493 | 0.578 |
| CE_Cholesterylester C20:1 | 0.605 | 0.608 | 0.486 | 0.478 | 0.691 | 0.652 |
| CE_Cholesterylester C20:2 | 0.516 | 0.495 | 0.486 | 0.478 | 0.552 | 0.588 |
| CE_Cholesterylester C20:3 | 0.492 | 0.495 | 0.486 | 0.478 | 0.493 | 0.497 |
| CE_Cholesterylester C20:5 | 0.495 | 0.495 | 0.486 | 0.478 | 0.531 | 0.563 |

(continued)

| CHF group | CHF | CHF | HFpEF | HFpEF | HFrEF | HFrEF |
|---|---|---|---|---|---|---|
| NYHA class | I | II or III | I | II or III | I | II or III |
| CE_Cholesterylester C22:4 | 0.492 | 0.495 | 0.486 | 0.478 | 0.493 | 0.509 |
| CE_Cholesterylester C22:5 | 0.560 | 0.579 | 0.486 | 0.478 | 0.535 | 0.646 |
| CE_Cholesterylester C22:6 | 0.510 | 0.495 | 0.486 | 0.478 | 0.567 | 0.534 |
| Cholesterylester C16:0 | 0.637 | 0.661 | 0.578 | 0.478 | 0.626 | 0.706 |
| Cholesterylester C18:1 | 0.714 | 0.739 | 0.643 | 0.543 | 0.748 | 0.770 |
| Cholesterylester C18:2 | 0.739 | 0.798 | 0.655 | 0.684 | 0.800 | 0.844 |
| Cholesterylester C20:4 | 0.597 | 0.549 | 0.486 | 0.478 | 0.555 | 0.585 |

Table 8: Area under the curve (AUC) values of receiver operating characteristic (ROC) analysis for specific cholesteryl esters and weighted sum parameter of cholesteryl esters combined with NT-proBNP with correction for confounders age, BMI and gender

| CHF group | CHF | CHF | HFpEF | HFpEF | HFrEF | HFrEF |
|---|---|---|---|---|---|---|
| NYHA class | I | II or III | I | II or III | I | II or III |
| | | | | | | |
| NT-proBNP | 0.798 | 0,889 | 0.647 | 0.746 | 0.914 | 0.939 |
| Sum_ Cholesterylesters | 0.824 | 0.906 | 0.679 | 0.751 | 0.929 | 0.955 |
| CE_Cholesterylester C12:0 | 0.801 | 0.892 | 0.654 | 0.729 | 0.914 | 0.942 |
| CE_Cholesterylester C14:0 | 0.800 | 0.890 | 0.654 | 0.730 | 0.912 | 0.941 |
| CE_Cholesterylester C14:1 | 0.802 | 0.888 | 0.656 | 0.733 | 0.912 | 0.938 |
| CE_Cholesterylester C15:0 | 0.807 | 0.894 | 0.655 | 0.729 | 0.917 | 0.948 |
| CE_Cholesterylester C16:1 | 0.801 | 0.888 | 0.655 | 0.732 | 0.912 | 0.937 |
| CE_Cholesterylester C16:2 | 0.802 | 0.888 | 0.654 | 0.732 | 0.911 | 0.936 |
| CE_Cholesterylester C16:3 | 0.802 | 0.893 | 0.666 | 0.754 | 0.912 | 0.937 |
| CE_Cholesterylester C18:0 | 0.801 | 0.888 | 0.657 | 0.733 | 0.914 | 0.937 |
| CE_Cholesterylester C18:3 | 0.801 | 0.888 | 0.651 | 0.728 | 0.912 | 0.937 |

(continued)

| CHF group | CHF | CHF | HFpEF | HFpEF | HFrEF | HFrEF |
|---|---|---|---|---|---|---|
| NYHA class | I | II or III | I | II or III | I | II or III |
| CE_Cholesterylester C18:4 | 0.801 | 0.888 | 0.656 | 0.732 | 0.912 | 0.937 |
| CE_Cholesterylester C20:1 | 0.810 | 0.891 | 0.639 | 0.703 | 0.932 | 0.943 |
| CE_Cholesterylester C20:2 | 0.800 | 0.888 | 0.652 | 0.728 | 0.916 | 0.936 |
| CE_Cholesterylester C20:3 | 0.801 | 0.893 | 0.650 | 0.734 | 0.913 | 0.938 |
| CE_Cholesterylester C20:5 | 0.800 | 0.888 | 0.653 | 0.731 | 0.916 | 0.937 |
| CE_Cholesterylester C22:4 | 0.802 | 0.889 | 0.656 | 0.733 | 0.912 | 0.937 |
| CE_Cholesterylester C22:5 | 0.801 | 0.888 | 0.653 | 0.734 | 0.914 | 0.937 |
| CE_Cholesterylester C22:6 | 0.801 | 0.888 | 0.656 | 0.731 | 0.919 | 0.937 |
| Cholesterylester C16:0 | 0.812 | 0.896 | 0.670 | 0.733 | 0.915 | 0.946 |
| Cholesterylester C18:1 | 0.821 | 0.903 | 0.694 | 0.759 | 0.924 | 0.950 |
| Cholesterylester C18:2 | 0.827 | 0.913 | 0.681 | 0.765 | 0.934 | 0.960 |
| Cholesterylester C20:4 | 0.801 | 0.888 | 0.658 | 0.733 | 0.911 | 0.937 |

Table 9: Area under the curve (AUC) values of receiver operating characteristic (ROC) analysis for specific cholesteryl esters and weighted sum parameter of cholesteryl esters combined with NT-proBNP without correction for confounders age, BMI and gender

| CHF group | CHF | CHF | HFpEF | HFpEF | HFrEF | HFrEF |
|---|---|---|---|---|---|---|
| NYHA class | I | II or III | I | II or III | I | II or III |
| | | | | | | |
| NT-proBNP | 0.790 | 0.896 | 0.663 | 0.786 | 0.891 | 0.937 |
| Sum Cholesterylesters | 0.845 | 0.929 | 0.750 | 0.778 | 0.933 | 0.965 |
| CE_Cholesterylester C12:0 | 0.793 | 0.900 | 0.710 | 0.739 | 0.893 | 0.940 |
| CE_Cholesterylester C14:0 | 0.794 | 0.899 | 0.709 | 0.740 | 0.895 | 0.941 |
| CE_Cholesterylester C14:1 | 0.794 | 0.896 | 0.708 | 0.741 | 0.890 | 0.936 |
| CE_Cholesterylester C15:0 | 0.811 | 0.906 | 0.707 | 0.740 | 0.908 | 0.952 |

(continued)

| CHF group | CHF | CHF | HFpEF | HFpEF | HFrEF | HFrEF |
|---|---|---|---|---|---|---|
| NYHA class | I | II or III | I | II or III | I | II or III |
| | | | | | | |
| CE_Cholesterylester C16:1 | 0.794 | 0.897 | 0.708 | 0.764 | 0.890 | 0.935 |
| CE_Cholesterylester C16:2 | 0.794 | 0.896 | 0.711 | 0.752 | 0.889 | 0.935 |
| CE_Cholesterylester C16:3 | 0.793 | 0.905 | 0.718 | 0.776 | 0.891 | 0.937 |
| CE_Cholesterylester C18:0 | 0.794 | 0.896 | 0.710 | 0.741 | 0.892 | 0.935 |
| CE_Cholesterylester C18:3 | 0.794 | 0.896 | 0.710 | 0.739 | 0.889 | 0.936 |
| CE_Cholesterylester C18:4 | 0.795 | 0.896 | 0.710 | 0.739 | 0.890 | 0.936 |
| CE_Cholesterylester C20:1 | 0.802 | 0.899 | 0.706 | 0.719 | 0.913 | 0.941 |
| CE_Cholesterylester C20:2 | 0.793 | 0.896 | 0.710 | 0.738 | 0.897 | 0.935 |
| CE_Cholesterylester C20:3 | 0.794 | 0.906 | 0.709 | 0.755 | 0.891 | 0.939 |
| CE_Cholesterylester C20:5 | 0.793 | 0.896 | 0.710 | 0.740 | 0.897 | 0.934 |
| CE_Cholesterylester C22:4 | 0.794 | 0.895 | 0.711 | 0.741 | 0.891 | 0.935 |
| CE_Cholesterylester C22:5 | 0.795 | 0.896 | 0.709 | 0.741 | 0.893 | 0.935 |
| CE_Cholesterylester C22:6 | 0.793 | 0.896 | 0.709 | 0.741 | 0.901 | 0.935 |
| Cholesterylester C16:0 | 0.818 | 0.910 | 0.735 | 0.744 | 0.903 | 0.950 |
| Cholesterylester C18:1 | 0.843 | 0.923 | 0.754 | 0.783 | 0.924 | 0.959 |
| Cholesterylester C18:2 | 0.847 | 0.937 | 0.757 | 0.830 | 0.939 | 0.971 |
| Cholesterylester C20:4 | 0.804 | 0.899 | 0.709 | 0.743 | 0.897 | 0.937 |

Table 10: Area under the curve (AUC) values of receiver operating characteristic (ROC) analysis for specific sphingomyelins, sum parameter of sphingomyelins and 1-Hydroxy-2-amino-(cis,trans)-3,5-octadecadiene without correction for confounders age, BMI and gender

| CHF group | CHF | CHF | HFpEF | HFpEF | HFrEF | HFrEF |
|---|---|---|---|---|---|---|
| NYHA class | I | II or III | I | II or III | I | II or III |
| | | | | | | |

(continued)

| CHF group | CHF | CHF | HFpEF | HFpEF | HFrEF | HFrEF |
|---|---|---|---|---|---|---|
| NYHA class | I | II or III | I | II or III | I | II or III |
| NT-proBNP | 0.790 | 0.896 | 0.663 | 0.786 | 0.891 | 0.937 |
| 1-Hydroxy-2-amino-(cis,trans)-3,5-octadecadiene | 0.663 | 0.682 | 0.496 | 0.478 | 0.699 | 0.727 |
| Sum Sphingomyelins | 0.688 | 0.678 | 0.491 | 0.478 | 0.756 | 0.739 |
| SM_Sphingomyelin (d18:0,C16:0) | 0.659 | 0.639 | 0.479 | 0.478 | 0.722 | 0.650 |
| SM_Sphingomyelin (d18:0,C18:0) | 0.498 | 0.628 | 0.502 | 0.633 | 0.493 | 0.610 |
| SM_Sphingomyelin (d18:1,C14:0) | 0.711 | 0.725 | 0.576 | 0.478 | 0.772 | 0.774 |
| SM_Sphingomyelin (d18:1,C16;0) | 0.659 | 0.684 | 0.475 | 0.478 | 0.726 | 0.719 |
| SM_Sphingomyelin (d18:1,C18:0) | 0.514 | 0.495 | 0.486 | 0.478 | 0.543 | 0.497 |
| SM_Sphingomyelin (d18:1,C18:1) | 0.680 | 0.649 | 0.510 | 0.478 | 0.731 | 0.672 |
| SM_Sphingomyelin (d18:1,C20:0) | 0.651 | 0.617 | 0.473 | 0.478 | 0.705 | 0.669 |
| SM_Sphingomyelin (d18:1,C20:1) | 0.643 | 0.512 | 0.522 | 0.478 | 0.672 | 0.556 |
| SM_Sphingomyelin (d18:1,C21:0) | 0.673 | 0.620 | 0.534 | 0.478 | 0.707 | 0.678 |
| SM_Sphingomyelin (d18:1,C22:0) | 0.651 | 0.682 | 0.505 | 0.478 | 0.695 | 0.708 |
| SM_Sphingomyelin (d18:1,C22:1) | 0.673 | 0.612 | 0.486 | 0.478 | 0.741 | 0.661 |
| SM_Sphingomyelin (d18:1,C23:0) | 0.689 | 0.714 | 0.512 | 0.478 | 0.752 | 0.750 |
| SM_Sphingomyelin (d18:1,C23:1) | 0.640 | 0.578 | 0.486 | 0.478 | 0.698 | 0.636 |
| SM_Sphingomyelin (d18:1,C24:0) | 0.662 | 0.701 | 0.533 | 0.478 | 0.706 | 0.720 |
| SM_Sphingomyelin (d18:1,C24:1) | 0.559 | 0.539 | 0.486 | 0.478 | 0.627 | 0.603 |
| SM_Sphingomyelin (d18:1,C24:2) | 0.695 | 0.633 | 0.600 | 0.478 | 0.726 | 0.693 |
| SM_Sphingomyelin (d18:2,C14:0) | 0.651 | 0.622 | 0.486 | 0.478 | 0.716 | 0.687 |
| SM_Sphingomyelin (d18:2,C16:0) | 0.634 | 0.569 | 0.486 | 0.478 | 0.675 | 0.618 |
| SM_Sphingomyelin (d18:2,C18:1) | 0.672 | 0.632 | 0.521 | 0.478 | 0.713 | 0.667 |
| SM_Sphingomyelin (d18:2,C20:0) | 0.628 | 0.593 | 0.486 | 0.478 | 0.649 | 0.628 |
| SM_Sphingomyelin (d18:2,C20:1) | 0.646 | 0.578 | 0.506 | 0.478 | 0.693 | 0.628 |
| SM_Sphingomyelin (d18:2,C21:0) | 0.664 | 0.616 | 0.500 | 0.478 | 0.704 | 0.666 |
| SM_Sphingomyelin (d18:2,C22:0) | 0.697 | 0.709 | 0.566 | 0.478 | 0.751 | 0.763 |
| SM_Sphingomyelin (d18:2,C22:1) | 0.664 | 0.590 | 0.508 | 0.478 | 0.715 | 0.636 |
| SM_Sphingomyelin (d18:2,C23:0) | 0.712 | 0.751 | 0.612 | 0.571 | 0.766 | 0.792 |
| SM_Sphingomyelin (d18:2,C23:1) | 0.660 | 0.640 | 0.486 | 0.478 | 0.716 | 0.695 |
| SM_Sphingomyelin (d18:2,C24:0) | 0.718 | 0.763 | 0.653 | 0.652 | 0.763 | 0.794 |
| SM_Sphingomyelin (d18:2,C24:1) | 0.662 | 0.626 | 0.480 | 0.478 | 0.717 | 0.691 |
| SM_Sphingomyelin (d18:2,C24:2) | 0.683 | 0.640 | 0.607 | 0.478 | 0.712 | 0.692 |
| SM_Sphingomyelin (d16:1,C16:0) | 0.631 | 0.628 | 0.486 | 0.478 | 0.698 | 0.672 |
| SM_Sphingomyelin (d16:1,C18:0) | 0.492 | 0.495 | 0.486 | 0.478 | 0.493 | 0.510 |
| SM_Sphingomyelin (d16:1,C18:1) | 0.648 | 0.636 | 0.484 | 0.478 | 0.706 | 0.690 |
| SM_Sphingomyelin (d16:1,C20:0) | 0.607 | 0.609 | 0.486 | 0.478 | 0.683 | 0.663 |

(continued)

| CHF group | CHF | CHF | HFpEF | HFpEF | HFrEF | HFrEF |
|---|---|---|---|---|---|---|
| NYHA class | I | II or III | I | II or III | I | II or III |
| SM_Sphingomyelin (d16:1,C21:0) | 0.644 | 0.627 | 0.486 | 0.478 | 0.691 | 0.683 |
| SM_Sphingomyelin (d16:1,C22:0) | 0.684 | 0.714 | 0.492 | 0.478 | 0.752 | 0.765 |
| SM_Sphingomyelin (d16:1,C22:1) | 0.649 | 0.623 | 0.486 | 0.478 | 0.713 | 0.685 |
| SM_Sphingomyelin (d16:1,C23:0) | 0.688 | 0.730 | 0.495 | 0.532 | 0.749 | 0.776 |
| SM_Sphingomyelin (d16:1,C24:0) | 0.661 | 0.746 | 0.493 | 0.559 | 0.727 | 0.783 |
| SM_Sphingomyelin (d16:1,C24:1) | 0.667 | 0.654 | 0.479 | 0.478 | 0.738 | 0.717 |
| SM Sphingomyelin (d17:1,C16:0) | 0.681 | 0.687 | 0.510 | 0.478 | 0.727 | 0.740 |
| SM_Sphingomyelin (d17:1,C18:0) | 0.583 | 0.507 | 0.486 | 0.478 | 0.607 | 0.583 |
| SM_Sphingomyelin (d17:1,C20:0) | 0.642 | 0.637 | 0.486 | 0.478 | 0.672 | 0.681 |
| SM_Sphingomyelin (d17:1,C22:0) | 0.699 | 0.725 | 0.591 | 0.478 | 0.741 | 0.773 |
| SM_Sphingomyelin (d17:1,C23:0) | 0.684 | 0.735 | 0.504 | 0.551 | 0.742 | 0.779 |
| SM_Sphingomyelin (d17:1,C24:0) | 0.711 | 0.767 | 0.619 | 0.649 | 0.761 | 0.802 |
| SM_ Sphingomyelin (d17:1,C24:1) | 0.685 | 0.671 | 0.522 | 0.478 | 0.735 | 0.731 |
| Sphingomyelin (d18:2,C18:0) | 0.492 | 0.495 | 0.486 | 0.478 | 0.493 | 0.497 |

Table 11: Area under the curve (AUC) values of receiver operating characteristic (ROC) analysis for specific sphingomyelins, sum parameter of sphingomyelins and 1-Hydroxy-2-amino-(cis,trans)-3,5-octadecadiene with correction for confounders age, BMI and gender

| CHF group | CHF | CHF | HFpEF | HFpEF | HFrEF | HFrEF |
|---|---|---|---|---|---|---|
| NYHA class | I | II or III | I | II or III | I | II or III |
| | | | | | | |
| NT-proBNP | 0.798 | 0.889 | 0.647 | 0.746 | 0.914 | 0.939 |
| 1-Hydroxy-2-amino-(cis,trans)-3,5-octadecadiene | 0.642 | 0.697 | 0.503 | 0.545 | 0.672 | 0.717 |
| Sum Sphingomyelins | 0.662 | 0.705 | 0.505 | 0.513 | 0.721 | 0.734 |
| SM_Sphingomyelin (d18:0,C16:0) | 0.635 | 0.634 | 0.486 | 0.478 | 0.682 | 0.629 |
| SM_Sphingomyelin (d18:0,C18:0) | 0.492 | 0.510 | 0.486 | 0.478 | 0.493 | 0.541 |
| SM_Sphingomyelin (d18:1,C14:0) | 0.676 | 0.725 | 0.499 | 0.542 | 0.738 | 0.755 |
| SM_Sphingomyelin (d18:1,C16:0) | 0.617 | 0.665 | 0.486 | 0.478 | 0.683 | 0.687 |
| SM_Sphingomyelin (d18:1,C18:0) | 0.504 | 0.495 | 0.486 | 0.478 | 0.511 | 0.480 |
| SM_Sphingomyelin (d18:1,C18:1) | 0,628 | 0.628 | 0.486 | 0.478 | 0.677 | 0.624 |
| SM_Sphingomyelin (d18:1,C20:0) | 0.641 | 0.639 | 0.485 | 0.478 | 0.690 | 0.668 |

(continued)

| CHF group | CHF | CHF | HFpEF | HFpEF | HFrEF | HFrEF |
|---|---|---|---|---|---|---|
| NYHA class | I | II or III | I | II or III | I | II or III |
| SM_Sphingomyelin (d18:1,C20:1) | 0.580 | 0.495 | 0.486 | 0.478 | 0.583 | 0.497 |
| SM_Sphingomyelin (d18:1,C21:0) | 0.644 | 0.633 | 0.527 | 0.478 | 0.659 | 0.656 |
| SM_Sphingomyelin (d18:1,C22:0) | 0.643 | 0.686 | 0.513 | 0.478 | 0.693 | 0.702 |
| SM Sphingomyelin (d18:1,C22:1) | 0.657 | 0.635 | 0.554 | 0.478 | 0.715 | 0.658 |
| SM_Sphingomyelin (d18:1,C23:0) | 0.662 | 0.712 | 0.506 | 0.627 | 0.724 | 0.728 |
| SM_Sphingomyelin (d18:1,C23:1) | 0.608 | 0.594 | 0.486 | 0.478 | 0.656 | 0.620 |
| SM_Sphingomyelin (d18:1,C24:0) | 0.655 | 0.696 | 0.525 | 0.478 | 0.704 | 0.710 |
| SM_Sphingomyelin (d18:1,C24:1) | 0.552 | 0.557 | 0.486 | 0.478 | 0.601 | 0.597 |
| SM_Sphingomyelin (d18:1,C24:2) | 0.654 | 0.610 | 0.538 | 0.478 | 0.673 | 0.650 |
| SM_Sphingomyelin (d18:2,C14:0) | 0.616 | 0.647 | 0.486 | 0.478 | 0.662 | 0.680 |
| SM_Sphingomyelin (d18:2,C16:0) | 0.608 | 0.586 | 0.486 | 0.478 | 0.632 | 0.614 |
| SM_Sphingomyelin (d18:2,C18:1) | 0.620 | 0.626 | 0.486 | 0.478 | 0.648 | 0.633 |
| SM_Sphingomyelin (d18:2,C20:0) | 0.561 | 0.558 | 0.486 | 0.478 | 0.550 | 0.584 |
| SM_Sphingomyelin (d18:2,C20:1) | 0.582 | 0.546 | 0.486 | 0.478 | 0.588 | 0.570 |
| SM_Sphingomyelin (d18:2,C21:0) | 0.606 | 0.604 | 0.486 | 0.478 | 0.633 | 0.622 |
| SM_Sphingomyelin (d18:2,C22:0) | 0.652 | 0.689 | 0.488 | 0.478 | 0.709 | 0.724 |
| SM_Sphingomyelin (d18:2,C22:1) | 0.625 | 0.592 | 0.486 | 0.478 | 0.666 | 0.616 |
| SM_Sphingomyelin (d18:2,C23:0) | 0.648 | 0.733 | 0.486 | 0.648 | 0.712 | 0.750 |
| SM_Sphingomyelin (d18:2,C23:1) | 0.608 | 0.648 | 0.486 | 0.478 | 0.652 | 0.675 |
| SM_Sphingomyelin (d18:2,C24:0) | 0.668 | 0.726 | 0.517 | 0.610 | 0.721 | 0.747 |
| SM_Sphingomyelin (d18:2,C24:1) | 0.618 | 0.620 | 0.486 | 0.478 | 0.673 | 0.654 |

(continued)

| CHF group | CHF | CHF | HFpEF | HFpEF | HFrEF | HFrEF |
|---|---|---|---|---|---|---|
| NYHA class | I | II or III | I | II or III | I | II or III |
| SM_Sphingomyelin (d18:2,C24:2) | 0.618 | 0.612 | 0.486 | 0.478 | 0.632 | 0.636 |
| SM_Sphingomyelin (d16:1,C16:0) | 0.637 | 0.677 | 0.502 | 0.505 | 0.688 | 0.696 |
| SM_Sphingomyelin (d16:1,C18:0) | 0.511 | 0.550 | 0.486 | 0.478 | 0.493 | 0.590 |
| SM_Sphingomyelin (d16:1,C18:1) | 0.620 | 0.661 | 0.493 | 0.478 | 0.663 | 0.684 |
| SM_Sphingomyelin (d16:1,C20:0) | 0.615 | 0.659 | 0.486 | 0.478 | 0.670 | 0.683 |
| SM_Sphingomyelin (d16:1,C21:0) | 0.626 | 0.664 | 0.531 | 0.534 | 0.649 | 0.680 |
| SM_Sphingomyelin (d16:1,C22:0) | 0.684 | 0.743 | 0.532 | 0.578 | 0.742 | 0.769 |
| SM_Sphingomyelin (d16:1,C22:1) | 0.641 | 0.665 | 0.536 | 0.505 | 0.684 | 0.695 |
| SM_Sphingomyelin (d16:1,C23:0) | 0.669 | 0.747 | 0.508 | 0.622 | 0.721 | 0.770 |
| SM_Sphingomyelin (d16:1,C24:0) | 0.653 | 0.751 | 0.500 | 0.615 | 0.713 | 0.776 |
| SM_Sphingomyelin (d16:1,C24:1) | 0.674 | 0.699 | 0.516 | 0.516 | 0.729 | 0.734 |
| SM_Sphingomyelin (d17:1,C16:0) | 0.660 | 0.705 | 0.523 | 0.542 | 0.694 | 0.734 |
| SM_Sphingomyelin (d17:1,C18:0) | 0.546 | 0.574 | 0.486 | 0.478 | 0.532 | 0.606 |
| SM_Sphingomyelin (d17:1,C20:0) | 0.619 | 0.658 | 0.513 | 0.543 | 0.624 | 0.674 |
| SM_Sphingomyelin (d17:1,C22:0) | 0.677 | 0.740 | 0.568 | 0.629 | 0.712 | 0.761 |
| SM_Sphingomyelin (d17:1,C23:0) | 0.652 | 0.741 | 0.509 | 0.627 | 0.698 | 0.763 |
| SM_Sphingomyelin (d17:1,C24:0) | 0.690 | 0.767 | 0.592 | 0.682 | 0.736 | 0.784 |
| SM_Sphingomyelin (d17:1,C24:1) | 0.660 | 0.689 | 0.555 | 0.478 | 0.698 | 0.722 |
| Sphingomyelin (d18:2,C18:0) | 0.492 | 0.495 | 0.486 | 0.478 | 0.493 | 0.501 |

Table 12: Area under the curve (AUC) values of receiver operating characteristic (ROC) analysis for specific sphingomyelins, sum parameter of sphingomyelins and 1-Hydroxy-2-amino-(cis,trans)-3,5-octadecadiene combined with NT-proBNP with correction for confounders age, BMI and gender

| CHF group | CHF | CHF | HFpEF | HFpEF | HFrEF | HFrEF |
|---|---|---|---|---|---|---|
| NYHA class | I | II or III | I | II or III | I | II or III |
| | | | | | | |
| NT-proBNP | 0.798 | 0.889 | 0.647 | 0.746 | 0.914 | 0.939 |
| 1-Hydroxy-2-amino-(cis,trans)-3,5-octadecadiene | 0.813 | 0.901 | 0.666 | 0.755 | 0.920 | 0.948 |
| Sum Sphingomyelins | 0.817 | 0.897 | 0.665 | 0.748 | 0.925 | 0.945 |
| SM_Sphingomyelin (d18:0,C16:0) | 0.821 | 0.895 | 0.663 | 0.741 | 0.929 | 0.943 |
| SM_Sphingomyelin (d18:0,C18:0) | 0.801 | 0.892 | 0.657 | 0.729 | 0.913 | 0.938 |
| SM_Sphingomyelin (d18:1,C14:0) | 0.825 | 0.907 | 0.687 | 0,763 | 0.931 | 0.954 |
| SM_Sphingomyelin (d18:1,C16:0) | 0.818 | 0.897 | 0.651 | 0.735 | 0.932 | 0.947 |
| SM_Sphingomyelin (d18:1,C18:0) | 0.800 | 0.889 | 0.654 | 0.728 | 0.917 | 0.937 |
| SM_Sphingomyelin (d18:1,C18:1) | 0.817 | 0.895 | 0.664 | 0.750 | 0.928 | 0.942 |
| SM_Sphingomyelin (d18:1,C20:0) | 0.814 | 0.888 | 0.655 | 0.729 | 0.920 | 0.938 |
| SM_Sphingomyelin (d18:1,C20:1) | 0.813 | 0.888 | 0.662 | 0.734 | 0.919 | 0.939 |
| SM_Sphingomyelin (d18:1,C21:0) | 0.819 | 0.892 | 0.677 | 0.732 | 0.922 | 0.941 |
| SM_Sphingomyelin (d18:1,C22:0) | 0.810 | 0.891 | 0.648 | 0.730 | 0.919 | 0.937 |
| SM_Sphingomyelin (d18:1,C22:1) | 0.824 | 0.894 | 0.678 | 0.728 | 0.932 | 0.946 |
| SM_Sphingomyelin (d18:1,C23:0) | 0.812 | 0.895 | 0.659 | 0.760 | 0.920 | 0.941 |
| SM_Sphingomyelin (d18:1,C23:1) | 0.811 | 0.893 | 0.652 | 0.731 | 0.922 | 0.944 |
| SM_Sphingomyelin (d18:1,C24:0) | 0.809 | 0.892 | 0.659 | 0.739 | 0.918 | 0.938 |
| SM_Sphingomyelin (d18:1,C24:1) | 0.802 | 0.888 | 0.649 | 0.729 | 0.923 | 0.938 |
| SM_Sphingomyelin (d18:1,C24:2) | 0.829 | 0.891 | 0.686 | 0.728 | 0.932 | 0.941 |
| SM Sphingomyelin (d18:2,C14:0) | 0.808 | 0.893 | 0.656 | 0.731 | 0.920 | 0.942 |
| SM_Sphingomyelin (d18:2,C16:0) | 0.812 | 0.890 | 0.658 | 0.727 | 0.923 | 0.937 |

(continued)

| CHF group | CHF | CHF | HFpEF | HFpEF | HFrEF | HFrEF |
|---|---|---|---|---|---|---|
| NYHA class | I | II or III | I | II or III | I | II or III |
| SM_Sphingomyelin (d18:2,C18:1) | 0.815 | 0.893 | 0.666 | 0.745 | 0.923 | 0.940 |
| SM_(d18:2,C20:0) | 0.803 | 0.888 | 0.655 | 0.735 | 0.912 | 0.938 |
| SM_Sphingomyelin (d18:2,C20:1) | 0.809 | 0.890 | 0.653 | 0.730 | 0.920 | 0.939 |
| SM_Sphingomyelin (d18:2,C21:0) | 0.813 | 0.891 | 0.656 | 0.736 | 0.921 | 0.940 |
| SM_Sphingomyelin (d18:2,C22:0) | 0.815 | 0.892 | 0.656 | 0.734 | 0.923 | 0.941 |
| SM_Sphingomyelin (d18:2,C22:1) | 0.817 | 0.893 | 0.664 | 0.732 | 0.925 | 0.942 |
| SM_Sphingomyelin (d18:2,C23:0) | 0.813 | 0.900 | 0.662 | 0.776 | 0.923 | 0.945 |
| SM_Sphingomyelin (d18:2,C23:1) | 0.813 | 0.897 | 0.654 | 0.736 | 0.922 | 0.946 |
| SM_Sphingomyelin (d18:2,C24:0) | 0.817 | 0.895 | 0.679 | 0.764 | 0.922 | 0.942 |
| SM_Sphingomyelin (d18:2,C24:1) | 0.816 | 0.891 | 0.653 | 0.733 | 0.930 | 0.941 |
| SM_Sphingomyelin (d18:2,C24:2) | 0.821 | 0.891 | 0.676 | 0.733 | 0.926 | 0.940 |
| SM Sphingomyelin (d16:1,C16:0) | 0.811 | 0.898 | 0.662 | 0.746 | 0.919 | 0.945 |
| SM_Sphingomyelin (d16:1,C18:0) | 0.801 | 0.888 | 0.656 | 0.733 | 0.912 | 0.937 |
| SM_Sphingomyelin (d16:1,C18:1) | 0.809 | 0.896 | 0.656 | 0.742 | 0.918 | 0.942 |
| SM_Sphingomyelin (d16.:1,C20:0) | 0.804 | 0.890 | 0.654 | 0.727 | 0.916 | 0.939 |
| SM_Sphingomyelin (d16:1,C21 :0) | 0.810 | 0.895 | 0.661 | 0.735 | 0.915 | 0.942 |
| SM_Sphingomyelin (d16:1,C22:0) | 0.815 | 0.897 | 0.657 | 0.746 | 0.920 | 0.944 |
| SM_Sphingomyelin (d16:1,C22:1) | 0.813 | 0.895 | 0.664 | 0.730 | 0.919 | 0.944 |
| SM_Sphingomyelin (d16:1,C23:0) | 0.812 | 0.901 | 0.650 | 0.760 | 0.919 | 0.947 |
| SM_Sphingomyelin (d16:1,C24:0) | 0.805 | 0.895 | 0.653 | 0.754 | 0.917 | 0.942 |
| SM_Sphingomyelin (d16:1,C24:1) | 0.819 | 0.897 | 0.670 | 0.732 | 0.925 | 0.946 |
| SM_Sphingomyelin (d17:1,C16:0) | 0.821 | 0.903 | 0.684 | 0.753 | 0.924 | 0.952 |

(continued)

| CHF group | CHF | CHF | HFpEF | HFpEF | HFrEF | HFrEF |
|---|---|---|---|---|---|---|
| NYHA class | I | II or III | I | II or III | I | II or III |
| SM_Sphingomyelin (d17:1,C18:0) | 0.800 | 0.889 | 0.652 | 0.735 | 0.913 | 0.938 |
| SM_Sphingomyelin (d17;1,C20:0) | 0.807 | 0.890 | 0.658 | 0.735 | 0.913 | 0.938 |
| SM_Sphingomyelin (d17:1,C22:0) | 0.815 | 0.898 | 0.669 | 0.760 | 0.918 | 0.946 |
| SM_Sphingomyelin (d17:1,C23:0) | 0.810 | 0.902 | 0.657 | 0.771 | 0.919 | 0.948 |
| SM_Sphingomyelin (d17:1,C24:0) | 0.815 | 0.900 | 0.672 | 0.779 | 0.918 | 0.945 |
| SM_Sphingomyelin (d17:1,C24:1) | 0.821 | 0.896 | 0.682 | 0.739 | 0.924 | 0.947 |
| Sphingomyelin (d18:2,C18:0) | 0.801 | 0.888 | 0.655 | 0.733 | 0.911 | 0.937 |

Table 13: Area under the curve (AUC) values of receiver operating characteristic (ROC) analysis for specific sphingomyelins, sum parameter of sphingomyelins and 1-Hydroxy-2-amino-(cis,trans)-3,5-octadecadiene combined with NT-proBNP without correction for confounders age, BMI and gender

| CHF group | CHF | CHF | HFpEF | HFpEF | HFrEF | HFrEF |
|---|---|---|---|---|---|---|
| NYHA class | I | II or III | I | II or III | I | II or III |
| | | | | | | |
| NT-proBNP | 0.790 | 0.896 | 0.663 | 0.786 | 0.891 | 0.937 |
| 1-Hydroxy-2-amino-(cis,trans)-3,5-octadecadiene | 0.819 | 0.911 | 0.717 | 0.750 | 0.915 | 0.954 |
| Sum Sphingomyelins | 0.827 | 0.905 | 0.718 | 0.743 | 0.924 | 0.950 |
| SM_Sphingomyelin (d18:0,C16:0) | 0.829 | 0.906 | 0.722 | 0.750 | 0.927 | 0.947 |
| SM_Sphingomyelin (d18:0,C18:0) | 0.791 | 0.907 | 0.701 | 0.771 | 0.891 | 0.940 |
| SM_Sphingomyelin (d18:1,C14:0) | 0.841 | 0.919 | 0.730 | 0.756 | 0.935 | 0.962 |
| SM_Sphingomyelin (d18:1,C16:0) | 0.833 | 0.912 | 0.721 | 0.752 | 0.932 | 0.953 |
| SM_Sphingomyelin (d18:1,C18:0) | 0.795 | 0.896 | 0.710 | 0.741 | 0.902 | 0.935 |
| SM_Sphingomyelin (d18:1,C18:1) | 0.832 | 0.908 | 0.743 | 0.747 | 0.930 | 0.949 |
| SM_Sphingomyelin (d18:1,C20:0) | 0.814 | 0.896 | 0.721 | 0.740 | 0.908 | 0.938 |
| SM_Sphingomyelin (d18:1,C20:1) | 0.825 | 0.899 | 0.719 | 0.739 | 0.918 | 0.942 |
| SM_Sphingomyelin (d18:1,C21:0) | 0.828 | 0.901 | 0.730 | 0.741 | 0.921 | 0.946 |

(continued)

| CHF group | CHF | CHF | HFpEF | HFpEF | HFrEF | HFrEF |
|---|---|---|---|---|---|---|
| NYHA class | I | II or III | I | II or III | I | II or III |
| SM_Sphingomyelin (d18:1,C22:0) | 0.808 | 0.898 | 0.732 | 0.750 | 0.900 | 0.937 |
| SM_Sphingomyelin (d18:1,C22:1) | 0.829 | 0.902 | 0.722 | 0.741 | 0.929 | 0.949 |
| SM_Sphingomyelin (d18:1,C23:0) | 0.820 | 0.904 | 0.725 | 0.761 | 0.914 | 0.945 |
| SM_Sphingomyelin (d18:1,C23:1) | 0.819 | 0.902 | 0.711 | 0.742 | 0.919 | 0.948 |
| SM_Sphingomyelin (d18:1,C24:0) | 0.806 | 0.899 | 0.737 | 0.761 | 0.899 | 0.937 |
| SM_Sphingomyelin (d18:1,C24:1) | 0.801 | 0.896 | 0.708 | 0.739 | 0.910 | 0.938 |
| SM_Sphingomyelin (d18:1,C24:2) | 0.843 | 0.903 | 0.751 | 0.740 | 0.931 | 0.947 |
| SM Sphingomyelin (d18:2,C14:0) | 0.819 | 0.902 | 0.707 | 0.741 | 0.918 | 0.946 |
| SM_Sphingomyelin (d18:2,C16:0) | 0.816 | 0.898 | 0.709 | 0.739 | 0.916 | 0.940 |
| SM Sphingomyelin (d18:2,C18:1) | 0.828 | 0.903 | 0.723 | 0.742 | 0.921 | 0.945 |
| SM_Sphingomyelin (d18:2,C20:0) | 0.809 | 0.896 | 0.710 | 0.742 | 0.902 | 0.938 |
| SM_Sphingomyelin (d18:2,C20:1) | 0.824 | 0.901 | 0.712 | 0.742 | 0.921 | 0.944 |
| SM_Sphingomyelin (d18:2,C21:0) | 0.829 | 0.902 | 0.716 | 0.742 | 0.923 | 0.946 |
| SM_Sphingomyelin (d18:2,C22:0) | 0.828 | 0.904 | 0.747 | 0.743 | 0.918 | 0.947 |
| SM_Sphingomyelin (d18:2,C22:1) | 0.828 | 0.903 | 0.713 | 0.740 | 0.925 | 0.946 |
| SM_Sphingomyelin (d18:2,C23:0) | 0.835 | 0.916 | 0.725 | 0.770 | 0.925 | 0.955 |
| SM_Sphingomyelin (d18:2,C23:1) | 0.826 | 0.907 | 0.710 | 0.742 | 0.923 | 0.952 |
| SM_Sphingomyelin (d18:2,C24:0) | 0.832 | 0.911 | 0.758 | 0.785 | 0.916 | 0.948 |
| SM_Sphingomyelin (d18:2,C24:1) | 0.828 | 0.901 | 0.711 | 0.740 | 0.928 | 0.946 |
| SM_Sphingomyelin (d18:2,C24:2) | 0.842 | 0.905 | 0.741 | 0.744 | 0.927 | 0.947 |
| SM_Sphingomyelin (d16:1,C16:0) | 0.808 | 0.903 | 0.708 | 0.741 | 0.909 | 0.945 |

(continued)

| CHF group | CHF | CHF | HFpEF | HFpEF | HFrEF | HFrEF |
|---|---|---|---|---|---|---|
| NYHA class | I | II or III | I | II or III | I | II or III |
| SM_Sphingomyelin (d16:1,C18:0) | 0.795 | 0.896 | 0.708 | 0.740 | 0.892 | 0.935 |
| SM_Sphingomyelin (d16:1,C18:1) | 0.814 | 0.903 | 0.710 | 0.741 | 0.914 | 0.946 |
| SM_Sphingomyelin (d16:1,C20:0) | 0.801 | 0.896 | 0.710 | 0.740 | 0.903 | 0.939 |
| SM_Sphingomyelin (d16:1,C21:0) | 0.815 | 0.901 | 0.712 | 0.740 | 0.908 | 0.945 |
| SM_Sphingomyelin (d16:1,C22:0) | 0.815 | 0.902 | 0.721 | 0.745 | 0.909 | 0.945 |
| SM_Sphingomyelin (d16:1,C22:1) | 0.815 | 0.901 | 0.712 | 0.740 | 0.914 | 0.947 |
| SM_Sphingomyelin (d16:1,C23:0) | 0.820 | 0.909 | 0.717 | 0.752 | 0.915 | 0.951 |
| SM_Sphingomyelin (d16:1,C24:0) | 0.806 | 0.903 | 0.708 | 0.767 | 0.903 | 0.943 |
| SM_Sphingomyelin (d16:1,C24:1) | 0.818 | 0.901 | 0.712 | 0.743 | 0.918 | 0.947 |
| SM_Sphingomyelin (d17:1,C16:0) | 0.829 | 0.912 | 0.745 | 0.746 | 0.923 | 0.958 |
| SM_Sphingomyelin (d17:1,C18:0) | 0.799 | 0.896 | 0.710 | 0.741 | 0.900 | 0.938 |
| SM_Sphingomyelin (d17:1,C20:0) | 0.809 | 0.898 | 0.715 | 0.741 | 0.902 | 0.939 |
| SM_Sphingomyelin (d17:1,C22:0) | 0.823 | 0.907 | 0.752 | 0.755 | 0.912 | 0.950 |
| SM_Sphingomyelin (d17:1,C23:0) | 0.820 | 0.913 | 0.717 | 0.765 | 0.915 | 0.953 |
| SM_Sphingomyelin (d17:1,C24:0) | 0.823 | 0.910 | 0.742 | 0.782 | 0.911 | 0.950 |
| SM_Sphingomyelin (d17:1,C24:1) | 0.830 | 0.905 | 0.726 | 0.742 | 0.924 | 0.953 |
| Sphingomyelin (d18:2,C18:0) | 0.795 | 0.896 | 0.709 | 0.738 | 0.891 | 0.935 |

In essence, it has been shown that a weighted sum parameter for cholesteryl esters performs comparable to the best performing cholesteryl ester. Moreover, in combination with other heart failure biomarkers such as NT-proBNP an incremental value can be demonstrated for the best cholesteryl esters as well as the weighted sum parameter. The sum parameter for sphingomyelins shows relevant classification performance and highly significant group differences.

Table 14: Total amount of sphingomylein measured in three replicates in 4 CHF patients and 6 healthy controls.

| | Replicate 1 [$\mu$M] | Replicate 2 [$\mu$M] | Replicate 3 [$\mu$M] |
|---|---|---|---|
| CHF_1 | 122 | 128 | 119 |
| CHF_2 | 24 | 52 | 58 |
| CHF_3 | 56 | 80 | 77 |

(continued)

|  | Replicate 1 [μM] | Replicate 2 [μM] | Replicate 3 [μM] |
|---|---|---|---|
| CHF_4 | 106 | 120 | 105 |
| Control_1 | 247 | 225 | 204 |
| Control 2 | 151 | 150 | 180 |
| Control_3 | NA | 210 | 188 |
| Control_4 | 244 | 229 | 196 |
| Control_5 | 256 | 256 | 213 |
| Control_6 | 245 | 262 | 238 |

Table 15: Area under the curve (AUC) values of receiver operating characteristic (ROC) analysis for the sum parameter of sphingomyelins and sum parameter of cholesteryl esters without correction for confounders age, BMI and gender.

| CHF group | CHF | CHF | HFpEF | HFpEF | HFrEF | HFrEF |
|---|---|---|---|---|---|---|
| NYHA class | I | II or III | I | II or III | I | II or III |
|  |  |  |  |  |  |  |
| NT-proBNP | 0.790 | 0.896 | 0.663 | 0.786 | 0.891 | 0.937 |
| Sum Sphingomyelins & Sum Cholesterylesters | 0.739 | 0.772 | 0.640 | 0.519 | 0.818 | 0.829 |

Table 16: Area under the curve (AUC) values of receiver operating characteristic (ROC) analysis for the sum parameter of sphingomyelins and sum parameter of cholesteryl esters with correction for confounders age, BMI and gender.

| CHF group | CHF | CHF | HFpEF | HFpEF | HFrEF | HFrEF |
|---|---|---|---|---|---|---|
| NYHA class | I | II or III | I | II or III | I | II or III |
|  |  |  |  |  |  |  |
| NT-proBNP | 0.790 | 0.896 | 0.663 | 0.786 | 0.891 | 0.937 |
| Sum Sphingomyelins & Sum Cholesterylesters | 0.698 | 0.749 | 0.569 | 0.504 | 0.775 | 0.798 |

Table 17: Area under the curve (AUC) values of receiver operating characteristic (ROC) analysis for the sum parameter of sphingomyelins and sum parameter of cholesteryl esters with NT-proBNP without correction for confounders age, BMI and gender.

| CHF group | CHF | CHF | HFpEF | HFpEF | HFrEF | HFrEF |
|---|---|---|---|---|---|---|
| NYHA class | I | II or III | I | II or III | I | II or III |
|  |  |  |  |  |  |  |
| NT-proBNP | 0.790 | 0.896 | 0.663 | 0.786 | 0.891 | 0.937 |
| Sum Sphingomyelins & Sum Cholesterylesters & NT proBNP | 0.849 | 0.929 | 0.739 | 0.819 | 0.942 | 0.966 |

Table 18: Area under the curve (AUC) values of receiver operating characteristic (ROC) analysis for the sum parameter of sphingomyelins and sum parameter of cholesteryl esters with NT-proBNP with correction for confounders age, BMI and gender.

| CHF qroup | CHF | CHF | HFpEF | HFpEF | HFrEF | HFrEF |
|---|---|---|---|---|---|---|
| NYHA class | I | II or III | I | II or III | I | II or III |
|  |  |  |  |  |  |  |
| NT-proBNP | 0.790 | 0.896 | 0.663 | 0.786 | 0.891 | 0.937 |
| Sum Sphingomyelins & Sum Cholesterylesters & NT proBNP | 0.827 | 0.910 | 0.681 | 0.756 | 0.936 | 0.956 |

Example 6: Alternative determination of the total amounts of triacylglycerols and cholesteryl esters

The total amount of triacylglycerols and the total amount of cholesteryl esters plus cholesterol, were determined in human plasma samples by a colorimetric enzyme assay using the cobas® 8000 system (Roche Diagnostics Limited, Rotkreuz, Switzerland). The assay was based on a method published by Wahlefeld et al. (Wahlefeld AW, Bergmeyer HU, eds. Methods of Enzymatic Analysis. 2nd English ed. New York, NY: Academic Press Inc 1974; 1831). The values were reported as absolute concentrations (unit mg/dl). The total amount of cholesteryl esters plus cholesterol was taken as approximation for the total amount of cholesteryl esters in Example 9.

Example 7: Study design for the differentiation of CHF and its subtypes from healthy controls using metabolites determined with an enzymatic assay

The following changes were made to the study design described in Exampe 1: Twelve healthy controls and eight CHF patients were excluded, and two further CHF patients changed their classification from ICM to DCM. The resulting patient cohort thus included 823 subjects comprising 190 male and female DCM patients, 181 male and female ICM patients, and 208 male and female HCM patients as well as 244 healthy controls.

From the patient cohort, 534 randomly selected subjects (stratified by subgroup) were taken as training set. The remaining 289 subjects were taken as testing set.

The adapted study design was applied in Examples 9 and 10.

Example 8: Combination of one or more parameters and (optionally) NT-proBNP into a biomarker panel

One or more enzymatically determined parameters and (optionally) NT-proBNP were combined into a biomarker panel by employing a logistic regression model fitted using the elastic net algorithm as implemented in the R package glmnet (Zou, H. and Hastie, T., J. Roy. Stat. Soc. B Met. 67, 301-320, 2003; Friedman, J., Hastie, T., and Tibshirani, R, J. Stat. Softw. 33, 2010). Fitting was performed on subjects from the respective training set. The L1 and the L2 penalties were given equal weight. Log-transformed values for enzymatically determined parameters and/or NT-proBNP (absorption values or absolute concentrations) were centered and scaled to unit variance before the analysis. The prediction probalitity was calculated as

$$p = \frac{1}{1 + e^{-(w_0 + \sum_{i=1}^{n} w_i \hat{x}_i)}},$$

with the feature $\hat{x}_i$ being

$$\hat{x}_i = \frac{x_i - m_i}{s_i}$$

wherein $x_i$ are the log-transformed measurement values, e.g. absorption values and/or concentration values (i.e. enzymatically determined amounts and, optionally, the NT-proBNP amount), $m_i$, $s_i$ are feature specific scaling factors, also taking into account the units of measurement, and $w_i$ are the coefficients of the model [$w_0$, intercept; $w_1$, coefficient for the first feature(biomarker); $w_2$ ... $w_n$, coefficients for the further features; $n$, number of parameters in the panel including NT-proBNP, if taken into account].

The method for combining one or more parameters and (optionally) NT-proBNP into a biomarker panel was applied in Examples 3, 9, and 10.

Example 9: Differentiation of CHF and its subtypes from healthy controls using the enzymatically determined parameters and, optionally, NT-proBNP

The diagnostic power of the total amounts of cholesteryl esters, cholesterol, and sphingomyelin determined as described in Examples 4, 5, and 6 as well as the diagnostic power of their different combinations was estimated with the area under the curve (AUC) of a receiver operating characteristic (ROC) analysis (Table 19). Optionally, the total amount of cholesteryl esters, the total amount of cholesterol, and/or the total amount of sphingomyelin were additionally combined with NT-proBNP.

The study design is described in Example 7, with AUC values calculated separately for the training and the testing set. The total amounts of cholesteryl esters, cholesterol, and/or sphingomyelin were combined with each other and/or with NT-proBNP as described in Example 8.

It is also envisaged that absorption values for sphingomyelin determined as described in Example 4 be combined with absolute concentrations for cholesteryl esters and/or cholesterol determined as described in Example 5 (and optionally NT-proBNP), or the other way round, or that either absorption values or absolute concentrations for sphingomyelin determined as described in Example 4 be combined with absolute values for cholesteryl esters determinded as described in Example 6 (and optionally NT-proBNP).

Table 19: Area under the curve (AUC) of a receiver operating characteristic (ROC) analysis for the enzymatically determined total amounts of cholesteryl esters, cholesterol, and sphingomyelins and their different combinations (see Examples 4, 5 and 6), optionally in combination with NT-proBNP (determined via antibody reaction) , without correction for the confounders age, BMI, and gender. Panels 1, 2, 3, and 5 are combinations of two or three parameters, while panels 4, 6, and 7 contain only a single parameter. NT-proBNP alone is shown for comparison.

| Dataset | | | | | | | | | Training | | | | Testing | | | |
|---|---|---|---|---|---|---|---|---|---|---|---|---|---|---|---|---|
| Total amounts of sphingomyelins, cholesteryl esters and/or cholesterol determined as described in Example | | | | | | | | | 4 and/or 5 | 6 | | | 4 and/or 5 | 6 | | |
| Panel number | Number of parameters (without NT-proBNP) | Total amount of sphingomyelins | Total amount of cholesteryl esters | Total amount of cholesterol | NT-proBNP | CHF subgroup | Number of cases | Number of controls | Absorbance values | Absolute concentrations | Absolute concentrations | NT-proBNP alone | Absorbance values | Absolute concentrations | Absolute concentrations | NT-proBNP alone |
| 1 | 3 | yes | yes | yes | yes | CHF | 205 | 84 | 0.896 | 0.897 | | 0.858 | 0.869 | 0.869 | | 0.849 |
| 1 | 3 | yes | yes | yes | yes | HFpEF | 74 | 84 | 0.773 | 0.776 | | 0.739 | 0.748 | 0.746 | | 0.708 |
| 1 | 3 | yes | yes | yes | yes | HFrEF | 131 | 84 | 0.961 | 0.961 | | 0.924 | 0.941 | 0.941 | | 0.927 |
| 1 | 3 | yes | yes | yes | yes | ICM | 64 | 84 | 0.977 | 0.977 | | 0.935 | 0.979 | 0.978 | | 0.953 |
| 1 | 3 | yes | yes | yes | yes | DCM | 67 | 84 | 0.945 | 0.945 | | 0.912 | 0.900 | 0.901 | | 0.903 |
| 1 | 3 | yes | yes | yes | yes | CHF asymptomatic | 93 | 84 | 0.854 | 0.856 | | 0.798 | 0.840 | 0.838 | | 0.802 |
| 1 | 3 | yes | yes | yes | yes | HFpEF asymptomatic | 44 | 84 | 0.742 | 0.745 | | 0.690 | 0.752 | 0.744 | | 0.672 |
| 1 | 3 | yes | yes | yes | yes | HFrEF asymptomatic | 49 | 84 | 0.949 | 0.951 | | 0.888 | 0.924 | 0.927 | | 0.911 |

| | | | | | | | | | | | | | | | | |
|---|---|---|---|---|---|---|---|---|---|---|---|---|---|---|---|---|
| 1 | 3 | yes | yes | yes | yes | ICM asymptomatic | 24 | 84 | 0.975 | 0.977 | | 0.907 | 0.986 | 0.986 | | 0.970 |
| 1 | 3 | yes | yes | yes | yes | DCM asymptomatic | 25 | 84 | 0.927 | 0.930 | | 0.872 | 0.851 | 0.854 | | 0.846 |
| 1 | 3 | yes | yes | yes | yes | CHF symptomatic | 112 | 84 | 0.930 | 0.930 | | 0.908 | 0.897 | 0.897 | | 0.890 |
| 1 | 3 | yes | yes | yes | yes | HFpEF symptomatic | 30 | 84 | 0.816 | 0.818 | | 0.811 | 0.741 | 0.749 | | 0.755 |
| 1 | 3 | yes | yes | yes | yes | HFrEF symptomatic | 82 | 84 | 0.969 | 0.968 | | 0.944 | 0.953 | 0.951 | | 0.939 |
| 1 | 3 | yes | yes | yes | yes | ICM symptomatic | 40 | 84 | 0.978 | 0.977 | | 0.950 | 0.974 | 0.972 | | 0.941 |
| 1 | 3 | yes | yes | yes | yes | DCM symptomatic | 42 | 84 | 0.958 | 0.958 | | 0.937 | 0.931 | 0.929 | | 0.940 |
| 1 | 3 | yes | yes | yes | yes | HFrEF LVEF 35% to 50% | 71 | 84 | 0.948 | 0.949 | | 0.890 | 0.896 | 0.896 | | 0.874 |
| 1 | 3 | yes | yes | yes | yes | ICM LVEF 35% to 50% | 42 | 84 | 0.971 | 0.970 | | 0.905 | 0.962 | 0.960 | | 0.929 |
| 1 | 3 | yes | yes | yes | yes | DCM LVEF 35% to 50% | 29 | 84 | 0.919 | 0.923 | | 0.871 | 0.793 | 0.796 | | 0.794 |
| 1 | 3 | yes | yes | yes | yes | HFrEF LVEF < 35% | 60 | 84 | 0.980 | 0.980 | | 0.970 | 0.991 | 0.992 | | 0.987 |
| 1 | 3 | yes | yes | yes | yes | ICM LVEF < 35% | 22 | 84 | 0.990 | 0.991 | | 0.984 | 0.999 | 0.999 | | 0.989 |
| 1 | 3 | yes | yes | yes | yes | DCM LVEF < 35% | 38 | 84 | 0.973 | 0.971 | | 0.957 | 0.982 | 0.981 | | 0.986 |
| 1 | 3 | yes | yes | yes | no | CHF | 205 | 84 | 0.759 | 0.755 | | 0.858 | 0.743 | 0.742 | | 0.849 |
| 1 | 3 | yes | yes | yes | no | HFpEF | 74 | 84 | 0.650 | 0.649 | | 0.739 | 0.619 | 0.616 | | 0.708 |
| 1 | 3 | yes | yes | yes | no | HFrEF | 131 | 84 | 0.825 | 0.818 | | 0.924 | 0.814 | 0.814 | | 0.927 |
| 1 | 3 | yes | yes | yes | no | ICM | 64 | 84 | 0.880 | 0.874 | | 0.935 | 0.880 | 0.880 | | 0.953 |
| 1 | 3 | yes | yes | yes | no | DCM | 67 | 84 | 0.774 | 0.765 | | 0.912 | 0.753 | 0.751 | | 0.903 |
| 1 | 3 | yes | yes | yes | no | CHF asymptomatic | 93 | 84 | 0.741 | 0.741 | | 0.798 | 0.723 | 0.723 | | 0.802 |
| 1 | 3 | yes | yes | yes | no | HFpEF asymptomatic | 44 | 84 | 0.650 | 0.650 | | 0.690 | 0.669 | 0.658 | | 0.672 |
| 1 | 3 | yes | yes | yes | no | HFrEF asymptomatic | 49 | 84 | 0.826 | 0.825 | | 0.888 | 0.767 | 0.777 | | 0.911 |
| 1 | 3 | yes | yes | yes | no | ICM asymptomatic | 24 | 84 | 0.893 | 0.889 | | 0.907 | 0.826 | 0.838 | | 0.970 |
| 1 | 3 | yes | yes | yes | no | DCM asymptomatic | 25 | 84 | 0.764 | 0.765 | | 0.872 | 0.712 | 0.718 | | 0.846 |
| 1 | 3 | yes | yes | yes | no | CHF symptomatic | 112 | 84 | 0.774 | 0.766 | | 0.908 | 0.759 | 0.757 | | 0.890 |
| 1 | 3 | yes | yes | yes | no | HFpEF symptomatic | 30 | 84 | 0.650 | 0.648 | | 0.811 | 0.550 | 0.559 | | 0.755 |
| 1 | 3 | yes | yes | yes | no | HFrEF symptomatic | 82 | 84 | 0.825 | 0.814 | | 0.944 | 0.843 | 0.836 | | 0.939 |
| 1 | 3 | yes | yes | yes | no | ICM symptomatic | 40 | 84 | 0.871 | 0.865 | | 0.950 | 0.911 | 0.904 | | 0.941 |
| 1 | 3 | yes | yes | yes | no | DCM symptomatic | 42 | 84 | 0.779 | 0.765 | | 0.937 | 0.778 | 0.771 | | 0.940 |
| 1 | 3 | yes | yes | yes | no | HFrEF LVEF 35% to 50% | 71 | 84 | 0.822 | 0.817 | | 0.890 | 0.763 | 0.763 | | 0.874 |
| 1 | 3 | yes | yes | yes | no | ICM LVEF 35% to 50% | 42 | 84 | 0.883 | 0.876 | | 0.905 | 0.846 | 0.843 | | 0.929 |

EP 3 194 959 B1

| | | | | | | | | | | | | | | | | |
|---|---|---|---|---|---|---|---|---|---|---|---|---|---|---|---|---|
| 1 | 3 | yes | yes | yes | no | DCM LVEF 35% to 50% | 29 | 84 | 0.753 | 0.749 | | 0.871 | 0.649 | 0.653 | | 0.794 |
| 1 | 3 | yes | yes | yes | no | HFrEF LVEF < 35% | 60 | 84 | 0.829 | 0.819 | | 0.970 | 0.873 | 0.873 | | 0.987 |
| 1 | 3 | yes | yes | yes | no | ICM LVEF < 35% | 22 | 84 | 0.873 | 0.870 | | 0.984 | 0.940 | 0.949 | | 0.989 |
| 1 | 3 | yes | yes | yes | no | DCM LVEF < 35% | 38 | 84 | 0.796 | 0.782 | | 0.957 | 0.832 | 0.825 | | 0.986 |
| 2 | 2 | yes | yes | no | yes | CHF | 205 | 84 | 0.896 | 0.897 | | 0.858 | 0.869 | 0.869 | | 0.849 |
| 2 | 2 | yes | yes | no | yes | HFpEF | 74 | 84 | 0.773 | 0.776 | | 0.739 | 0.748 | 0.748 | | 0.708 |
| 2 | 2 | yes | yes | no | yes | HFrEF | 131 | 84 | 0.961 | 0.961 | | 0.924 | 0.941 | 0.942 | | 0.927 |
| 2 | 2 | yes | yes | no | yes | ICM | 64 | 84 | 0.977 | 0.977 | | 0.935 | 0.979 | 0.978 | | 0.953 |
| 2 | 2 | yes | yes | no | yes | DCM | 67 | 84 | 0.945 | 0.945 | | 0.912 | 0.900 | 0.901 | | 0.903 |
| 2 | 2 | yes | yes | no | yes | CHF asymptomatic | 93 | 84 | 0.854 | 0.856 | | 0.798 | 0.840 | 0.839 | | 0.802 |
| 2 | 2 | yes | yes | no | yes | HFpEF asymptomatic | 44 | 84 | 0.742 | 0.744 | | 0.690 | 0.752 | 0.746 | | 0.672 |
| 2 | 2 | yes | yes | no | yes | HFrEF asymptomatic | 49 | 84 | 0.949 | 0.951 | | 0.888 | 0.924 | 0.928 | | 0.911 |
| 2 | 2 | yes | yes | no | yes | ICM asymptomatic | 24 | 84 | 0.975 | 0.977 | | 0.907 | 0.986 | 0.987 | | 0.970 |
| 2 | 2 | yes | yes | no | yes | DCM asymptomatic | 25 | 84 | 0.927 | 0.930 | | 0.872 | 0.851 | 0.855 | | 0.846 |
| 2 | 2 | yes | yes | no | yes | CHF symptomatic | 112 | 84 | 0.930 | 0.930 | | 0.908 | 0.897 | 0.898 | | 0.890 |
| 2 | 2 | yes | yes | no | yes | HFpEF symptomatic | 30 | 84 | 0.816 | 0.820 | | 0.811 | 0.741 | 0.751 | | 0.755 |
| 2 | 2 | yes | yes | no | yes | HFrEF symptomatic | 82 | 84 | 0.969 | 0.968 | | 0.944 | 0.953 | 0.952 | | 0.939 |
| 2 | 2 | yes | yes | no | yes | ICM symptomatic | 40 | 84 | 0.978 | 0.977 | | 0.950 | 0.974 | 0.972 | | 0.941 |
| 2 | 2 | yes | yes | no | yes | DCM symptomatic | 42 | 84 | 0.958 | 0.958 | | 0.937 | 0.931 | 0.930 | | 0.940 |
| 2 | 2 | yes | yes | no | yes | HFrEF LVEF 35% to 50% | 71 | 84 | 0.948 | 0.949 | | 0.890 | 0.896 | 0.896 | | 0.874 |
| 2 | 2 | yes | yes | no | yes | ICM LVEF 35% to 50% | 42 | 84 | 0.971 | 0.970 | | 0.905 | 0.962 | 0.961 | | 0.929 |
| 2 | 2 | yes | yes | no | yes | DCM LVEF 35% to 50% | 29 | 84 | 0.919 | 0.922 | | 0.871 | 0.793 | 0.797 | | 0.794 |
| 2 | 2 | yes | yes | no | yes | HFrEF LVEF < 35% | 60 | 84 | 0.980 | 0.980 | | 0.970 | 0.991 | 0.992 | | 0.987 |
| 2 | 2 | yes | yes | no | yes | ICM LVEF < 35% | 22 | 84 | 0.990 | 0.991 | | 0.984 | 0.999 | 1.000 | | 0.989 |
| 2 | 2 | yes | yes | no | yes | DCM LVEF < 35% | 38 | 84 | 0.973 | 0.971 | | 0.957 | 0.982 | 0.982 | | 0.986 |
| 2 | 2 | yes | yes | no | no | CHF | 205 | 84 | 0.756 | 0.755 | | 0.858 | 0.737 | 0.742 | | 0.849 |
| 2 | 2 | yes | yes | no | no | HFpEF | 74 | 84 | 0.647 | 0.649 | | 0.739 | 0.611 | 0.616 | | 0.708 |
| 2 | 2 | yes | yes | no | no | HFrEF | 131 | 84 | 0.822 | 0.818 | | 0.924 | 0.809 | 0.814 | | 0.927 |
| 2 | 2 | yes | yes | no | no | ICM | 64 | 84 | 0.878 | 0.874 | | 0.935 | 0.877 | 0.880 | | 0.953 |
| 2 | 2 | yes | yes | no | no | DCM | 67 | 84 | 0.769 | 0.765 | | 0.912 | 0.746 | 0.751 | | 0.903 |

| | | | | | | | | | | | | | | | | |
|---|---|---|---|---|---|---|---|---|---|---|---|---|---|---|---|---|
| 2 | 2 | yes | yes | no | no | CHF asymptomatic | 93 | 84 | 0.740 | 0.741 | | 0.798 | 0.717 | 0.723 | | 0.802 |
| 2 | 2 | yes | yes | no | no | HFpEF asymptomatic | 44 | 84 | 0.650 | 0.650 | | 0.690 | 0.662 | 0.658 | | 0.672 |
| 2 | 2 | yes | yes | no | no | HFrEF asymptomatic | 49 | 84 | 0.824 | 0.824 | | 0.888 | 0.763 | 0.777 | | 0.911 |
| 2 | 2 | yes | yes | no | no | ICM asymptomatic | 24 | 84 | 0.893 | 0.889 | | 0.907 | 0.823 | 0.837 | | 0.970 |
| 2 | 2 | yes | yes | no | no | DCM asymptomatic | 25 | 84 | 0.760 | 0.764 | | 0.872 | 0.706 | 0.718 | | 0.846 |
| 2 | 2 | yes | yes | no | no | CHF symptomatic | 112 | 84 | 0.769 | 0.766 | | 0.908 | 0.752 | 0.757 | | 0.890 |
| 2 | 2 | yes | yes | no | no | HFpEF symptomatic | 30 | 84 | 0.644 | 0.647 | | 0.811 | 0.542 | 0.559 | | 0.755 |
| 2 | 2 | yes | yes | no | no | HFrEF symptomatic | 82 | 84 | 0.820 | 0.814 | | 0.944 | 0.838 | 0.836 | | 0.939 |
| 2 | 2 | yes | yes | no | no | ICM symptomatic | 40 | 84 | 0.868 | 0.865 | | 0.950 | 0.908 | 0.904 | | 0.941 |
| 2 | 2 | yes | yes | no | no | DCM symptomatic | 42 | 84 | 0.774 | 0.765 | | 0.937 | 0.771 | 0.771 | | 0.940 |
| 2 | 2 | yes | yes | no | no | HFrEF LVEF 35% to 50% | 71 | 84 | 0.820 | 0.817 | | 0.890 | 0.758 | 0.763 | | 0.874 |
| 2 | 2 | yes | yes | no | no | ICM LVEF 35% to 50% | 42 | 84 | 0.882 | 0.876 | | 0.905 | 0.843 | 0.842 | | 0.929 |
| 2 | 2 | yes | yes | no | no | DCM LVEF 35% to 50% | 29 | 84 | 0.750 | 0.749 | | 0.871 | 0.644 | 0.654 | | 0.794 |
| 2 | 2 | yes | yes | no | no | HFrEF LVEF < 35% | 60 | 84 | 0.823 | 0.819 | | 0.970 | 0.869 | 0.873 | | 0.987 |
| 2 | 2 | yes | yes | no | no | ICM LVEF < 35% | 22 | 84 | 0.869 | 0.870 | | 0.984 | 0.940 | 0.949 | | 0.989 |
| 2 | 2 | yes | yes | no | no | DCM LVEF < 35% | 38 | 84 | 0.790 | 0.783 | | 0.957 | 0.825 | 0.825 | | 0.986 |
| 3 | 2 | yes | no | yes | yes | CHF | 205 | 84 | 0.896 | 0.896 | | 0.858 | 0.872 | 0.868 | | 0.849 |
| 3 | 2 | yes | no | yes | yes | HFpEF | 74 | 84 | 0.775 | 0.774 | | 0.739 | 0.757 | 0.745 | | 0.708 |
| 3 | 2 | yes | no | yes | yes | HFrEF | 131 | 84 | 0.961 | 0.961 | | 0.924 | 0.943 | 0.941 | | 0.927 |
| 3 | 2 | yes | no | yes | yes | ICM | 64 | 84 | 0.976 | 0.977 | | 0.935 | 0.979 | 0.977 | | 0.953 |
| 3 | 2 | yes | no | yes | yes | DCM | 67 | 84 | 0.945 | 0.945 | | 0.912 | 0.904 | 0.901 | | 0.903 |
| 3 | 2 | yes | no | yes | yes | CHF asymptomatic | 93 | 84 | 0.855 | 0.856 | | 0.798 | 0.842 | 0.836 | | 0.802 |
| 3 | 2 | yes | no | yes | yes | HFpEF asymptomatic | 44 | 84 | 0.744 | 0.745 | | 0.690 | 0.758 | 0.742 | | 0.672 |
| 3 | 2 | yes | no | yes | yes | HFrEF asymptomatic | 49 | 84 | 0.949 | 0.949 | | 0.888 | 0.925 | 0.924 | | 0.911 |
| 3 | 2 | yes | no | yes | yes | ICM asymptomatic | 24 | 84 | 0.974 | 0.976 | | 0.907 | 0.984 | 0.984 | | 0.970 |
| 3 | 2 | yes | no | yes | yes | DCM asymptomatic | 25 | 84 | 0.927 | 0.928 | | 0.872 | 0.854 | 0.853 | | 0.846 |
| 3 | 2 | yes | no | yes | yes | CHF symptomatic | 112 | 84 | 0.931 | 0.929 | | 0.908 | 0.901 | 0.897 | | 0.890 |
| 3 | 2 | yes | no | yes | yes | HFpEF symptomatic | 30 | 84 | 0.818 | 0.813 | | 0.811 | 0.754 | 0.749 | | 0.755 |
| 3 | 2 | yes | no | yes | yes | HFrEF symptomatic | 82 | 84 | 0.969 | 0.968 | | 0.944 | 0.955 | 0.952 | | 0.939 |
| 3 | 2 | yes | no | yes | yes | ICM symptomatic | 40 | 84 | 0.977 | 0.977 | | 0.950 | 0.975 | 0.972 | | 0.941 |

| | | | | | | | | | | | | | | | | |
|---|---|---|---|---|---|---|---|---|---|---|---|---|---|---|---|---|
| 3 | 2 | yes | no | yes | yes | DCM symptomatic | 42 | 84 | 0.959 | 0.958 | | 0.937 | 0.934 | 0.930 | | 0.940 |
| 3 | 2 | yes | no | yes | yes | HFrEF LVEF 35% to 50% | 71 | 84 | 0.948 | 0.948 | | 0.890 | 0.899 | 0.895 | | 0.874 |
| 3 | 2 | yes | no | yes | yes | ICM LVEF 35% to 50% | 42 | 84 | 0.970 | 0.970 | | 0.905 | 0.963 | 0.959 | | 0.929 |
| 3 | 2 | yes | no | yes | yes | DCM LVEF 35% to 50% | 29 | 84 | 0.918 | 0.922 | | 0.871 | 0.798 | 0.795 | | 0.794 |
| 3 | 2 | yes | no | yes | yes | HFrEF LVEF < 35% | 60 | 84 | 0.981 | 0.980 | | 0.970 | 0.992 | 0.991 | | 0.987 |
| 3 | 2 | yes | no | yes | yes | ICM LVEF < 35% | 22 | 84 | 0.990 | 0.990 | | 0.984 | 0.999 | 0.999 | | 0.989 |
| 3 | 2 | yes | no | yes | yes | DCM LVEF < 35% | 38 | 84 | 0.974 | 0.971 | | 0.957 | 0.984 | 0.982 | | 0.986 |
| 3 | 2 | yes | no | yes | no | CHF | 205 | 84 | 0.756 | 0.750 | | 0.858 | 0.742 | 0.741 | | 0.849 |
| 3 | 2 | yes | no | yes | no | HFpEF | 74 | 84 | 0.647 | 0.643 | | 0.739 | 0.621 | 0.619 | | 0.708 |
| 3 | 2 | yes | no | yes | no | HFrEF | 131 | 84 | 0.822 | 0.814 | | 0.924 | 0.812 | 0.811 | | 0.927 |
| 3 | 2 | yes | no | yes | no | ICM | 64 | 84 | 0.877 | 0.870 | | 0.935 | 0.875 | 0.872 | | 0.953 |
| 3 | 2 | yes | no | yes | no | DCM | 67 | 84 | 0.771 | 0.761 | | 0.912 | 0.753 | 0.752 | | 0.903 |
| 3 | 2 | yes | no | yes | no | CHF asymptomatic | 93 | 84 | 0.739 | 0.735 | | 0.798 | 0.721 | 0.720 | | 0.802 |
| 3 | 2 | yes | no | yes | no | HFpEF asymptomatic | 44 | 84 | 0.649 | 0.646 | | 0.690 | 0.669 | 0.661 | | 0.672 |
| 3 | 2 | yes | no | yes | no | HFrEF asymptomatic | 49 | 84 | 0.823 | 0.816 | | 0.888 | 0.765 | 0.768 | | 0.911 |
| 3 | 2 | yes | no | yes | no | ICM asymptomatic | 24 | 84 | 0.891 | 0.882 | | 0.907 | 0.818 | 0.822 | | 0.970 |
| 3 | 2 | yes | no | yes | no | DCM asymptomatic | 25 | 84 | 0.760 | 0.754 | | 0.872 | 0.714 | 0.716 | | 0.846 |
| 3 | 2 | yes | no | yes | no | CHF symptomatic | 112 | 84 | 0.769 | 0.762 | | 0.908 | 0.759 | 0.758 | | 0.890 |
| 3 | 2 | yes | no | yes | no | HFpEF symptomatic | 30 | 84 | 0.643 | 0.640 | | 0.811 | 0.555 | 0.561 | | 0.755 |
| 3 | 2 | yes | no | yes | no | HFrEF symptomatic | 82 | 84 | 0.822 | 0.813 | | 0.944 | 0.841 | 0.836 | | 0.939 |
| 3 | 2 | yes | no | yes | no | ICM symptomatic | 40 | 84 | 0.867 | 0.862 | | 0.950 | 0.909 | 0.901 | | 0.941 |
| 3 | 2 | yes | no | yes | no | DCM symptomatic | 42 | 84 | 0.777 | 0.765 | | 0.937 | 0.777 | 0.774 | | 0.940 |
| 3 | 2 | yes | no | yes | no | HFrEF LVEF 35% to 50% | 71 | 84 | 0.820 | 0.811 | | 0.890 | 0.762 | 0.761 | | 0.874 |
| 3 | 2 | yes | no | yes | no | ICM LVEF 35% to 50% | 42 | 84 | 0.881 | 0.873 | | 0.905 | 0.844 | 0.837 | | 0.929 |
| 3 | 2 | yes | no | yes | no | DCM LVEF 35% to 50% | 29 | 84 | 0.750 | 0.740 | | 0.871 | 0.653 | 0.656 | | 0.794 |
| 3 | 2 | yes | no | yes | no | HFrEF LVEF < 35% | 60 | 84 | 0.825 | 0.818 | | 0.970 | 0.869 | 0.868 | | 0.987 |
| 3 | 2 | yes | no | yes | no | ICM LVEF < 35% | 22 | 84 | 0.867 | 0.863 | | 0.984 | 0.934 | 0.937 | | 0.989 |
| 3 | 2 | yes | no | yes | no | DCM LVEF < 35% | 38 | 84 | 0.795 | 0.785 | | 0.957 | 0.830 | 0.825 | | 0.986 |
| 4 | 1 | yes | no | no | yes | CHF | 205 | 84 | 0.896 | 0.894 | | 0.858 | 0.873 | 0.872 | | 0.849 |
| 4 | 1 | yes | no | no | yes | HFpEF | 74 | 84 | 0.776 | 0.773 | | 0.739 | 0.758 | 0.758 | | 0.708 |

| | | | | | | | | | | | | | | | | |
|---|---|---|---|---|---|---|---|---|---|---|---|---|---|---|---|---|
| 4 | 1 | yes | no | no | yes | HFrEF | 131 | 84 | 0.961 | 0.959 | | 0.924 | 0.943 | 0.942 | | 0.927 |
| 4 | 1 | yes | no | no | yes | ICM | 64 | 84 | 0.976 | 0.975 | | 0.935 | 0.979 | 0.976 | | 0.953 |
| 4 | 1 | yes | no | no | yes | DCM | 67 | 84 | 0.945 | 0.943 | | 0.912 | 0.904 | 0.905 | | 0.903 |
| 4 | 1 | yes | no | no | yes | CHF asymptomatic | 93 | 84 | 0.855 | 0.850 | | 0.798 | 0.843 | 0.842 | | 0.802 |
| 4 | 1 | yes | no | no | yes | HFpEF asymptomatic | 44 | 84 | 0.744 | 0.739 | | 0.690 | 0.760 | 0.755 | | 0.672 |
| 4 | 1 | yes | no | no | yes | HFrEF asymptomatic | 49 | 84 | 0.949 | 0.944 | | 0.888 | 0.925 | 0.925 | | 0.911 |
| 4 | 1 | yes | no | no | yes | ICM asymptomatic | 24 | 84 | 0.974 | 0.971 | | 0.907 | 0.984 | 0.983 | | 0.970 |
| 4 | 1 | yes | no | no | yes | DCM asymptomatic | 25 | 84 | 0.927 | 0.921 | | 0.872 | 0.854 | 0.857 | | 0.846 |
| 4 | 1 | yes | no | no | yes | CHF symptomatic | 112 | 84 | 0.931 | 0.930 | | 0.908 | 0.902 | 0.901 | | 0.890 |
| 4 | 1 | yes | no | no | yes | HFpEF symptomatic | 30 | 84 | 0.819 | 0.819 | | 0.811 | 0.756 | 0.762 | | 0.755 |
| 4 | 1 | yes | no | no | yes | HFrEF symptomatic | 82 | 84 | 0.969 | 0.968 | | 0.944 | 0.955 | 0.954 | | 0.939 |
| 4 | 1 | yes | no | no | yes | ICM symptomatic | 40 | 84 | 0.977 | 0.977 | | 0.950 | 0.975 | 0.972 | | 0.941 |
| 4 | 1 | yes | no | no | yes | DCM symptomatic | 42 | 84 | 0.959 | 0.959 | | 0.937 | 0.935 | 0.935 | | 0.940 |
| 4 | 1 | yes | no | no | yes | HFrEF LVEF 35% to 50% | 71 | 84 | 0.947 | 0.944 | | 0.890 | 0.899 | 0.898 | | 0.874 |
| 4 | 1 | yes | no | no | yes | ICM LVEF 35% to 50% | 42 | 84 | 0.970 | 0.968 | | 0.905 | 0.963 | 0.959 | | 0.929 |
| 4 | 1 | yes | no | no | yes | DCM LVEF 35% to 50% | 29 | 84 | 0.918 | 0.914 | | 0.871 | 0.800 | 0.805 | | 0.794 |
| 4 | 1 | yes | no | no | yes | HFrEF LVEF < 35% | 60 | 84 | 0.981 | 0.981 | | 0.970 | 0.992 | 0.991 | | 0.987 |
| 4 | 1 | yes | no | no | yes | ICM LVEF < 35% | 22 | 84 | 0.990 | 0.990 | | 0.984 | 0.999 | 0.999 | | 0.989 |
| 4 | 1 | yes | no | no | yes | DCM LVEF < 35% | 38 | 84 | 0.974 | 0.974 | | 0.957 | 0.984 | 0.983 | | 0.986 |
| 4 | 1 | yes | no | no | no | CHF | 205 | 84 | 0.756 | 0.749 | | 0.858 | 0.742 | 0.743 | | 0.849 |
| 4 | 1 | yes | no | no | no | HFpEF | 74 | 84 | 0.647 | 0.642 | | 0.739 | 0.621 | 0.624 | | 0.708 |
| 4 | 1 | yes | no | no | no | HFrEF | 131 | 84 | 0.822 | 0.813 | | 0.924 | 0.812 | 0.811 | | 0.927 |
| 4 | 1 | yes | no | no | no | ICM | 64 | 84 | 0.877 | 0.868 | | 0.935 | 0.875 | 0.868 | | 0.953 |
| 4 | 1 | yes | no | no | no | DCM | 67 | 84 | 0.771 | 0.761 | | 0.912 | 0.753 | 0.756 | | 0.903 |
| 4 | 1 | yes | no | no | no | CHF asymptomatic | 93 | 84 | 0.739 | 0.731 | | 0.798 | 0.721 | 0.720 | | 0.802 |
| 4 | 1 | yes | no | no | no | HFpEF asymptomatic | 44 | 84 | 0.649 | 0.642 | | 0.690 | 0.669 | 0.666 | | 0.672 |
| 4 | 1 | yes | no | no | no | HFrEF asymptomatic | 49 | 84 | 0.823 | 0.811 | | 0.888 | 0.765 | 0.766 | | 0.911 |
| 4 | 1 | yes | no | no | no | ICM asymptomatic | 24 | 84 | 0.891 | 0.877 | | 0.907 | 0.818 | 0.815 | | 0.970 |
| 4 | 1 | yes | no | no | no | DCM asymptomatic | 25 | 84 | 0.760 | 0.750 | | 0.872 | 0.714 | 0.717 | | 0.846 |
| 4 | 1 | yes | no | no | no | CHF symptomatic | 112 | 84 | 0.769 | 0.763 | | 0.908 | 0.759 | 0.761 | | 0.890 |

| | | | | | | | | | | | | | | | | |
|---|---|---|---|---|---|---|---|---|---|---|---|---|---|---|---|---|
| 4 | 1 | yes | no | no | no | HFpEF symptomatic | 30 | 84 | 0.643 | 0.642 | | 0.811 | 0.555 | 0.565 | | 0.755 |
| 4 | 1 | yes | no | no | no | HFrEF symptomatic | 82 | 84 | 0.822 | 0.813 | | 0.944 | 0.841 | 0.838 | | 0.939 |
| 4 | 1 | yes | no | no | no | ICM symptomatic | 40 | 84 | 0.867 | 0.862 | | 0.950 | 0.909 | 0.900 | | 0.941 |
| 4 | 1 | yes | no | no | no | DCM symptomatic | 42 | 84 | 0.777 | 0.767 | | 0.937 | 0.777 | 0.779 | | 0.940 |
| 4 | 1 | yes | no | no | no | HFrEF LVEF 35% to 50% | 71 | 84 | 0.820 | 0.807 | | 0.890 | 0.762 | 0.761 | | 0.874 |
| 4 | 1 | yes | no | no | no | ICM LVEF 35% to 50% | 42 | 84 | 0.881 | 0.870 | | 0.905 | 0.844 | 0.835 | | 0.929 |
| 4 | 1 | yes | no | no | no | DCM LVEF 35% to 50% | 29 | 84 | 0.750 | 0.736 | | 0.871 | 0.653 | 0.659 | | 0.794 |
| 4 | 1 | yes | no | no | no | HFrEF LVEF < 35% | 60 | 84 | 0.825 | 0.820 | | 0.970 | 0.869 | 0.867 | | 0.987 |
| 4 | 1 | yes | no | no | no | ICM LVEF < 35% | 22 | 84 | 0.867 | 0.862 | | 0.984 | 0.934 | 0.930 | | 0.989 |
| 4 | 1 | yes | no | no | no | DCM LVEF < 35% | 38 | 84 | 0.795 | 0.789 | | 0.957 | 0.830 | 0.829 | | 0.986 |
| 5 | 2 | no | yes | yes | yes | CHF | 205 | 84 | 0.878 | 0.876 | | 0.858 | 0.844 | 0.850 | | 0.849 |
| 5 | 2 | no | yes | yes | yes | HFpEF | 74 | 84 | 0.753 | 0.756 | | 0.739 | 0.691 | 0.703 | | 0.708 |
| 5 | 2 | no | yes | yes | yes | HFrEF | 131 | 84 | 0.945 | 0.941 | | 0.924 | 0.927 | 0.931 | | 0.927 |
| 5 | 2 | no | yes | yes | yes | ICM | 64 | 84 | 0.962 | 0.957 | | 0.935 | 0.967 | 0.965 | | 0.953 |
| 5 | 2 | no | yes | yes | yes | DCM | 67 | 84 | 0.929 | 0.926 | | 0.912 | 0.887 | 0.896 | | 0.903 |
| 5 | 2 | no | yes | yes | yes | CHF asymptomatic | 93 | 84 | 0.827 | 0.828 | | 0.798 | 0.808 | 0.807 | | 0.802 |
| 5 | 2 | no | yes | yes | yes | HFpEF asymptomatic | 44 | 84 | 0.714 | 0.720 | | 0.690 | 0.680 | 0.674 | | 0.672 |
| 5 | 2 | no | yes | yes | yes | HFrEF asymptomatic | 49 | 84 | 0.924 | 0.921 | | 0.888 | 0.917 | 0.920 | | 0.911 |
| 5 | 2 | no | yes | yes | yes | ICM asymptomatic | 24 | 84 | 0.951 | 0.948 | | 0.907 | 0.985 | 0.983 | | 0.970 |
| 5 | 2 | no | yes | yes | yes | DCM asymptomatic | 25 | 84 | 0.903 | 0.899 | | 0.872 | 0.838 | 0.846 | | 0.846 |
| 5 | 2 | no | yes | yes | yes | CHF symptomatic | 112 | 84 | 0.918 | 0.914 | | 0.908 | 0.877 | 0.886 | | 0.890 |
| 5 | 2 | no | yes | yes | yes | HFpEF symptomatic | 30 | 84 | 0.806 | 0.806 | | 0.811 | 0.704 | 0.740 | | 0.755 |
| 5 | 2 | no | yes | yes | yes | HFrEF symptomatic | 82 | 84 | 0.958 | 0.953 | | 0.944 | 0.938 | 0.939 | | 0.939 |
| 5 | 2 | no | yes | yes | yes | ICM symptomatic | 40 | 84 | 0.967 | 0.962 | | 0.950 | 0.955 | 0.951 | | 0.941 |
| 5 | 2 | no | yes | yes | yes | DCM symptomatic | 42 | 84 | 0.948 | 0.943 | | 0.937 | 0.921 | 0.927 | | 0.940 |
| 5 | 2 | no | yes | yes | yes | HFrEF LVEF 35% to 50% | 71 | 84 | 0.926 | 0.921 | | 0.890 | 0.874 | 0.876 | | 0.874 |
| 5 | 2 | no | yes | yes | yes | ICM LVEF 35% to 50% | 42 | 84 | 0.943 | 0.937 | | 0.905 | 0.943 | 0.941 | | 0.929 |
| 5 | 2 | no | yes | yes | yes | DCM LVEF 35% to 50% | 29 | 84 | 0.906 | 0.902 | | 0.871 | 0.769 | 0.778 | | 0.794 |
| 5 | 2 | no | yes | yes | yes | HFrEF LVEF < 35% | 60 | 84 | 0.973 | 0.969 | | 0.970 | 0.988 | 0.991 | | 0.987 |
| 5 | 2 | no | yes | yes | yes | ICM LVEF < 35% | 22 | 84 | 0.990 | 0.989 | | 0.984 | 0.998 | 0.998 | | 0.989 |

| | | | | | | | | | | | | | | | | |
|---|---|---|---|---|---|---|---|---|---|---|---|---|---|---|---|---|
| 5 | 2 | no | yes | yes | yes | DCM LVEF < 35% | 38 | 84 | 0.957 | 0.953 | | 0.957 | 0.977 | 0.983 | | 0.986 |
| 5 | 2 | no | yes | yes | no | CHF | 205 | 84 | 0.690 | 0.661 | | 0.858 | 0.641 | 0.639 | | 0.849 |
| 5 | 2 | no | yes | yes | no | HFpEF | 74 | 84 | 0.616 | 0.605 | | 0.739 | 0.501 | 0.528 | | 0.708 |
| 5 | 2 | no | yes | yes | no | HFrEF | 131 | 84 | 0.731 | 0.692 | | 0.924 | 0.718 | 0.702 | | 0.927 |
| 5 | 2 | no | yes | yes | no | ICM | 64 | 84 | 0.789 | 0.744 | | 0.935 | 0.801 | 0.776 | | 0.953 |
| 5 | 2 | no | yes | yes | no | DCM | 67 | 84 | 0.674 | 0.640 | | 0.912 | 0.640 | 0.628 | | 0.903 |
| 5 | 2 | no | yes | yes | no | CHF asymptomatic | 93 | 84 | 0.680 | 0.670 | | 0.798 | 0.623 | 0.637 | | 0.802 |
| 5 | 2 | no | yes | yes | no | HFpEF asymptomatic | 44 | 84 | 0.614 | 0.612 | | 0.690 | 0.552 | 0.541 | | 0.672 |
| 5 | 2 | no | yes | yes | no | HFrEF asymptomatic | 49 | 84 | 0.741 | 0.719 | | 0.888 | 0.679 | 0.712 | | 0.911 |
| 5 | 2 | no | yes | yes | no | ICM asymptomatic | 24 | 84 | 0.807 | 0.772 | | 0.907 | 0.771 | 0.790 | | 0.970 |
| 5 | 2 | no | yes | yes | no | DCM asymptomatic | 25 | 84 | 0.676 | 0.664 | | 0.872 | 0.587 | 0.631 | | 0.846 |
| 5 | 2 | no | yes | yes | no | CHF symptomatic | 112 | 84 | 0.698 | 0.653 | | 0.908 | 0.655 | 0.641 | | 0.890 |
| 5 | 2 | no | yes | yes | no | HFpEF symptomatic | 30 | 84 | 0.618 | 0.594 | | 0.811 | 0.439 | 0.511 | | 0.755 |
| 5 | 2 | no | yes | yes | no | HFrEF symptomatic | 82 | 84 | 0.726 | 0.675 | | 0.944 | 0.741 | 0.696 | | 0.939 |
| 5 | 2 | no | yes | yes | no | ICM symptomatic | 40 | 84 | 0.778 | 0.727 | | 0.950 | 0.817 | 0.767 | | 0.941 |
| 5 | 2 | no | yes | yes | no | DCM symptomatic | 42 | 84 | 0.674 | 0.625 | | 0.937 | 0.670 | 0.625 | | 0.940 |
| 5 | 2 | no | yes | yes | no | HFrEF LVEF 35% to 50% | 71 | 84 | 0.740 | 0.714 | | 0.890 | 0.661 | 0.652 | | 0.874 |
| 5 | 2 | no | yes | yes | no | ICM LVEF 35% to 50% | 42 | 84 | 0.785 | 0.749 | | 0.905 | 0.748 | 0.718 | | 0.929 |
| 5 | 2 | no | yes | yes | no | DCM LVEF 35% to 50% | 29 | 84 | 0.685 | 0.672 | | 0.871 | 0.540 | 0.560 | | 0.794 |
| 5 | 2 | no | yes | yes | no | HFrEF LVEF < 35% | 60 | 84 | 0.718 | 0.657 | | 0.970 | 0.784 | 0.758 | | 0.987 |
| 5 | 2 | no | yes | yes | no | ICM LVEF < 35% | 22 | 84 | 0.796 | 0.736 | | 0.984 | 0.897 | 0.881 | | 0.989 |
| 5 | 2 | no | yes | yes | no | DCM LVEF < 35% | 38 | 84 | 0.661 | 0.598 | | 0.957 | 0.716 | 0.681 | | 0.986 |
| 6 | 1 | no | yes | no | yes | CHF | 205 | 84 | 0.878 | 0.876 | 0.871 | 0.858 | 0.845 | 0.852 | 0.845 | 0.849 |
| 6 | 1 | no | yes | no | yes | HFpEF | 74 | 84 | 0.754 | 0.757 | 0.742 | 0.739 | 0.694 | 0.707 | 0.698 | 0.708 |
| 6 | 1 | no | yes | no | yes | HFrEF | 131 | 84 | 0.945 | 0.941 | 0.941 | 0.924 | 0.928 | 0.933 | 0.926 | 0.927 |
| 6 | 1 | no | yes | no | yes | ICM | 64 | 84 | 0.962 | 0.957 | 0.959 | 0.935 | 0.967 | 0.966 | 0.966 | 0.953 |
| 6 | 1 | no | yes | no | yes | DCM | 67 | 84 | 0.929 | 0.926 | 0.922 | 0.912 | 0.888 | 0.898 | 0.886 | 0.903 |
| 6 | 1 | no | yes | no | yes | CHF asymptomatic | 93 | 84 | 0.827 | 0.828 | 0.816 | 0.798 | 0.810 | 0.811 | 0.811 | 0.802 |
| 6 | 1 | no | yes | no | yes | HFpEF asymptomatic | 44 | 84 | 0.714 | 0.719 | 0.696 | 0.690 | 0.683 | 0.679 | 0.688 | 0.672 |
| 6 | 1 | no | yes | no | yes | HFrEF asymptomatic | 49 | 84 | 0.924 | 0.921 | 0.917 | 0.888 | 0.918 | 0.923 | 0.916 | 0.911 |

| | | | | | | | | | | | | | | | | |
|---|---|---|---|---|---|---|---|---|---|---|---|---|---|---|---|---|
| 6 | 1 | no | yes | no | yes | ICM asymptomatic | 24 | 84 | 0.951 | 0.948 | 0.948 | 0.907 | 0.985 | 0.985 | 0.987 | 0.970 |
| 6 | 1 | no | yes | no | yes | DCM asymptomatic | 25 | 84 | 0.902 | 0.899 | 0.892 | 0.872 | 0.839 | 0.849 | 0.833 | 0.846 |
| 6 | 1 | no | yes | no | yes | CHF symptomatic | 112 | 84 | 0.919 | 0.915 | 0.915 | 0.908 | 0.878 | 0.888 | 0.878 | 0.890 |
| 6 | 1 | no | yes | no | yes | HFpEF symptomatic | 30 | 84 | 0.809 | 0.810 | 0.805 | 0.811 | 0.707 | 0.744 | 0.712 | 0.755 |
| 6 | 1 | no | yes | no | yes | HFrEF symptomatic | 82 | 84 | 0.958 | 0.953 | 0.954 | 0.944 | 0.938 | 0.940 | 0.937 | 0.939 |
| 6 | 1 | no | yes | no | yes | ICM symptomatic | 40 | 84 | 0.967 | 0.962 | 0.965 | 0.950 | 0.955 | 0.952 | 0.953 | 0.941 |
| 6 | 1 | no | yes | no | yes | DCM symptomatic | 42 | 84 | 0.948 | 0.944 | 0.942 | 0.937 | 0.922 | 0.929 | 0.922 | 0.940 |
| 6 | 1 | no | yes | no | yes | HFrEF LVEF 35% to 50% | 71 | 84 | 0.926 | 0.921 | 0.921 | 0.890 | 0.875 | 0.878 | 0.874 | 0.874 |
| 6 | 1 | no | yes | no | yes | ICM LVEF 35% to 50% | 42 | 84 | 0.943 | 0.936 | 0.940 | 0.905 | 0.944 | 0.943 | 0.945 | 0.929 |
| 6 | 1 | no | yes | no | yes | DCM LVEF 35% to 50% | 29 | 84 | 0.906 | 0.902 | 0.898 | 0.871 | 0.772 | 0.782 | 0.768 | 0.794 |
| 6 | 1 | no | yes | no | yes | HFrEF LVEF < 35% | 60 | 84 | 0.973 | 0.970 | 0.969 | 0.970 | 0.988 | 0.991 | 0.987 | 0.987 |
| 6 | 1 | no | yes | no | yes | ICM LVEF < 35% | 22 | 84 | 0.990 | 0.989 | 0.989 | 0.984 | 0.998 | 0.998 | 0.997 | 0.989 |
| 6 | 1 | no | yes | no | yes | DCM LVEF < 35% | 38 | 84 | 0.957 | 0.954 | 0.950 | 0.957 | 0.978 | 0.984 | 0.977 | 0.986 |
| 6 | 1 | no | yes | no | no | CHF | 205 | 84 | 0.690 | 0.661 | 0.663 | 0.858 | 0.641 | 0.639 | 0.638 | 0.849 |
| 6 | 1 | no | yes | no | no | HFpEF | 74 | 84 | 0.616 | 0.605 | 0.574 | 0.739 | 0.501 | 0.528 | 0.498 | 0.708 |
| 6 | 1 | no | yes | no | no | HFrEF | 131 | 84 | 0.731 | 0.692 | 0.710 | 0.924 | 0.718 | 0.702 | 0.715 | 0.927 |
| 6 | 1 | no | yes | no | no | ICM | 64 | 84 | 0.789 | 0.744 | 0.773 | 0.935 | 0.801 | 0.776 | 0.801 | 0.953 |
| 6 | 1 | no | yes | no | no | DCM | 67 | 84 | 0.674 | 0.640 | 0.647 | 0.912 | 0.640 | 0.628 | 0.634 | 0.903 |
| 6 | 1 | no | yes | no | no | CHF asymptomatic | 93 | 84 | 0.680 | 0.670 | 0.650 | 0.798 | 0.623 | 0.637 | 0.618 | 0.802 |
| 6 | 1 | no | yes | no | no | HFpEF asymptomatic | 44 | 84 | 0.614 | 0.612 | 0.562 | 0.690 | 0.552 | 0.541 | 0.549 | 0.672 |
| 6 | 1 | no | yes | no | no | HFrEF asymptomatic | 49 | 84 | 0.741 | 0.719 | 0.726 | 0.888 | 0.679 | 0.712 | 0.671 | 0.911 |
| 6 | 1 | no | yes | no | no | ICM asymptomatic | 24 | 84 | 0.807 | 0.772 | 0.802 | 0.907 | 0.771 | 0.790 | 0.781 | 0.970 |
| 6 | 1 | no | yes | no | no | DCM asymptomatic | 25 | 84 | 0.676 | 0.664 | 0.648 | 0.872 | 0.587 | 0.631 | 0.564 | 0.846 |
| 6 | 1 | no | yes | no | no | CHF symptomatic | 112 | 84 | 0.698 | 0.653 | 0.672 | 0.908 | 0.655 | 0.641 | 0.653 | 0.890 |
| 6 | 1 | no | yes | no | no | HFpEF symptomatic | 30 | 84 | 0.618 | 0.594 | 0.591 | 0.811 | 0.439 | 0.511 | 0.439 | 0.755 |
| 6 | 1 | no | yes | no | no | HFrEF symptomatic | 82 | 84 | 0.726 | 0.675 | 0.701 | 0.944 | 0.741 | 0.696 | 0.741 | 0.939 |
| 6 | 1 | no | yes | no | no | ICM symptomatic | 40 | 84 | 0.778 | 0.727 | 0.755 | 0.950 | 0.817 | 0.767 | 0.812 | 0.941 |
| 6 | 1 | no | yes | no | no | DCM symptomatic | 42 | 84 | 0.674 | 0.625 | 0.647 | 0.937 | 0.670 | 0.625 | 0.674 | 0.940 |
| 6 | 1 | no | yes | no | no | HFrEF LVEF 35% to 50% | 71 | 84 | 0.740 | 0.714 | 0.730 | 0.890 | 0.661 | 0.652 | 0.653 | 0.874 |
| 6 | 1 | no | yes | no | no | ICM LVEF 35% to 50% | 42 | 84 | 0.785 | 0.749 | 0.775 | 0.905 | 0.748 | 0.718 | 0.748 | 0.929 |

| | | | | | | | n | | | | | | | | | |
|---|---|---|---|---|---|---|---|---|---|---|---|---|---|---|---|---|
| 6 | 1 | no | yes | no | no | DCM LVEF 35% to 50% | 29 | 84 | 0.685 | 0.672 | 0.674 | 0.871 | 0.540 | 0.560 | 0.520 | 0.794 |
| 6 | 1 | no | yes | no | no | HFrEF LVEF < 35% | 60 | 84 | 0.718 | 0.657 | 0.681 | 0.970 | 0.784 | 0.758 | 0.786 | 0.987 |
| 6 | 1 | no | yes | no | no | ICM LVEF < 35% | 22 | 84 | 0.796 | 0.736 | 0.769 | 0.984 | 0.897 | 0.881 | 0.899 | 0.989 |
| 6 | 1 | no | yes | no | no | DCM LVEF < 35% | 38 | 84 | 0.661 | 0.598 | 0.618 | 0.957 | 0.716 | 0.681 | 0.718 | 0.986 |
| 7 | 1 | no | no | yes | yes | CHF | 205 | 84 | 0.871 | 0.873 | | 0.858 | 0.839 | 0.846 | | 0.849 |
| 7 | 1 | no | no | yes | yes | HFpEF | 74 | 84 | 0.743 | 0.752 | | 0.739 | 0.680 | 0.696 | | 0.708 |
| 7 | 1 | no | no | yes | yes | HFrEF | 131 | 84 | 0.940 | 0.939 | | 0.924 | 0.924 | 0.928 | | 0.927 |
| 7 | 1 | no | no | yes | yes | ICM | 64 | 84 | 0.956 | 0.955 | | 0.935 | 0.962 | 0.961 | | 0.953 |
| 7 | 1 | no | no | yes | yes | DCM | 67 | 84 | 0.924 | 0.923 | | 0.912 | 0.887 | 0.895 | | 0.903 |
| 7 | 1 | no | no | yes | yes | CHF asymptomatic | 93 | 84 | 0.821 | 0.826 | | 0.798 | 0.795 | 0.799 | | 0.802 |
| 7 | 1 | no | no | yes | yes | HFpEF asymptomatic | 44 | 84 | 0.711 | 0.719 | | 0.690 | 0.657 | 0.662 | | 0.672 |
| 7 | 1 | no | no | yes | yes | HFrEF asymptomatic | 49 | 84 | 0.915 | 0.916 | | 0.888 | 0.909 | 0.913 | | 0.911 |
| 7 | 1 | no | no | yes | yes | ICM asymptomatic | 24 | 84 | 0.941 | 0.944 | | 0.907 | 0.977 | 0.977 | | 0.970 |
| 7 | 1 | no | no | yes | yes | DCM asymptomatic | 25 | 84 | 0.896 | 0.895 | | 0.872 | 0.832 | 0.841 | | 0.846 |
| 7 | 1 | no | no | yes | yes | CHF symptomatic | 112 | 84 | 0.911 | 0.911 | | 0.908 | 0.878 | 0.886 | | 0.890 |
| 7 | 1 | no | no | yes | yes | HFpEF symptomatic | 30 | 84 | 0.788 | 0.797 | | 0.811 | 0.710 | 0.740 | | 0.755 |
| 7 | 1 | no | no | yes | yes | HFrEF symptomatic | 82 | 84 | 0.954 | 0.951 | | 0.944 | 0.937 | 0.938 | | 0.939 |
| 7 | 1 | no | no | yes | yes | ICM symptomatic | 40 | 84 | 0.963 | 0.960 | | 0.950 | 0.952 | 0.948 | | 0.941 |
| 7 | 1 | no | no | yes | yes | DCM symptomatic | 42 | 84 | 0.944 | 0.942 | | 0.937 | 0.923 | 0.928 | | 0.940 |
| 7 | 1 | no | no | yes | yes | HFrEF LVEF 35% to 50% | 71 | 84 | 0.919 | 0.918 | | 0.890 | 0.868 | 0.871 | | 0.874 |
| 7 | 1 | no | no | yes | yes | ICM LVEF 35% to 50% | 42 | 84 | 0.937 | 0.935 | | 0.905 | 0.937 | 0.937 | | 0.929 |
| 7 | 1 | no | no | yes | yes | DCM LVEF 35% to 50% | 29 | 84 | 0.899 | 0.898 | | 0.871 | 0.763 | 0.773 | | 0.794 |
| 7 | 1 | no | no | yes | yes | HFrEF LVEF < 35% | 60 | 84 | 0.969 | 0.968 | | 0.970 | 0.987 | 0.990 | | 0.987 |
| 7 | 1 | no | no | yes | yes | ICM LVEF < 35% | 22 | 84 | 0.986 | 0.986 | | 0.984 | 0.996 | 0.996 | | 0.989 |
| 7 | 1 | no | no | yes | yes | DCM LVEF < 35% | 38 | 84 | 0.955 | 0.952 | | 0.957 | 0.979 | 0.984 | | 0.986 |
| 7 | 1 | no | no | yes | no | CHF | 205 | 84 | 0.632 | 0.627 | | 0.858 | 0.569 | 0.591 | | 0.849 |
| 7 | 1 | no | no | yes | no | HFpEF | 74 | 84 | 0.572 | 0.583 | | 0.739 | 0.441 | 0.490 | | 0.708 |
| 7 | 1 | no | no | yes | no | HFrEF | 131 | 84 | 0.667 | 0.652 | | 0.924 | 0.643 | 0.647 | | 0.927 |
| 7 | 1 | no | no | yes | no | ICM | 64 | 84 | 0.735 | 0.711 | | 0.935 | 0.731 | 0.722 | | 0.953 |
| 7 | 1 | no | no | yes | no | DCM | 67 | 84 | 0.600 | 0.591 | | 0.912 | 0.562 | 0.568 | | 0.903 |

| 7 | 1 | no | no | yes | no | CHF asymptomatic | 93 | 84 | 0.654 | 0.658 | | 0.798 | 0.548 | 0.590 | | 0.802 |
|---|---|----|----|-----|----|------------------|-----|----|-------|-------|--|-------|-------|-------|--|-------|
| 7 | 1 | no | no | yes | no | HFpEF asymptomatic | 44 | 84 | 0.603 | 0.615 | | 0.690 | 0.474 | 0.494 | | 0.672 |
| 7 | 1 | no | no | yes | no | HFrEF asymptomatic | 49 | 84 | 0.700 | 0.693 | | 0.888 | 0.606 | 0.663 | | 0.911 |
| 7 | 1 | no | no | yes | no | ICM asymptomatic | 24 | 84 | 0.792 | 0.764 | | 0.907 | 0.683 | 0.736 | | 0.970 |
| 7 | 1 | no | no | yes | no | DCM asymptomatic | 25 | 84 | 0.614 | 0.621 | | 0.872 | 0.529 | 0.583 | | 0.846 |
| 7 | 1 | no | no | yes | no | CHF symptomatic | 112 | 84 | 0.617 | 0.601 | | 0.908 | 0.586 | 0.591 | | 0.890 |
| 7 | 1 | no | no | yes | no | HFpEF symptomatic | 30 | 84 | 0.532 | 0.534 | | 0.811 | 0.405 | 0.486 | | 0.755 |
| 7 | 1 | no | no | yes | no | HFrEF symptomatic | 82 | 84 | 0.650 | 0.625 | | 0.944 | 0.666 | 0.637 | | 0.939 |
| 7 | 1 | no | no | yes | no | ICM symptomatic | 40 | 84 | 0.706 | 0.678 | | 0.950 | 0.761 | 0.713 | | 0.941 |
| 7 | 1 | no | no | yes | no | DCM symptomatic | 42 | 84 | 0.592 | 0.573 | | 0.937 | 0.582 | 0.558 | | 0.940 |
| 7 | 1 | no | no | yes | no | HFrEF LVEF 35% to 50% | 71 | 84 | 0.693 | 0.687 | | 0.890 | 0.600 | 0.606 | | 0.874 |
| 7 | 1 | no | no | yes | no | ICM LVEF 35% to 50% | 42 | 84 | 0.757 | 0.733 | | 0.905 | 0.676 | 0.662 | | 0.929 |
| 7 | 1 | no | no | yes | no | DCM LVEF 35% to 50% | 29 | 84 | 0.617 | 0.631 | | 0.871 | 0.496 | 0.525 | | 0.794 |
| 7 | 1 | no | no | yes | no | HFrEF LVEF < 35% | 60 | 84 | 0.628 | 0.594 | | 0.970 | 0.692 | 0.692 | | 0.987 |
| 7 | 1 | no | no | yes | no | ICM LVEF < 35% | 22 | 84 | 0.693 | 0.666 | | 0.984 | 0.833 | 0.826 | | 0.989 |
| 7 | 1 | no | no | yes | no | DCM LVEF < 35% | 38 | 84 | 0.579 | 0.541 | | 0.957 | 0.613 | 0.602 | | 0.986 |

Example 10: Differentiation of CHF and its subtypes from healthy controls using combinations of the total amounts of cholesteryl esters, cholesterol, and sphingomyelins with the total amount of triacylglycerols

The diagnostic power of the total amounts of cholesteryl esters, cholesterol, and/or sphingomyelins determined as described in Examples 4 and 5 in combination with the total amount of triacylglycerols was estimated with the area under the curve (AUC) of a receiver operating characteristic (ROC) analysis (Table 20). The total amount of triacylglycerols was determined as described in Example 6. Optionally, the resulting parameter combinations were additionally combined with NT-proBNP.

The study design is described in Example 7, with AUC values calculated separately for the training and the testing set. The parameters were combined with each other and/or with NT-proBNP as described in Example 8.

Alternatively, it is envisaged that the total amount of cholesteryl esters be determined as described in Example 6.

Table 20: Area under the curve (AUC) of a receiver operating characteristic (ROC) analysis for the enzymatically determined total amounts of cholesteryl esters, cholesterol, and/or sphingomyelins in combination with the total amount of triacylglycerols (see Examples 4, 5 and 6) , optionally also in combination with NT-proBNP (determined via antibody reaction), without correction for the confounders age, BMI, and gender. Panels 8, 9 and 10 are combinations of three or four parameters. NT-proBNP alone is shown for comparison.

| | | | | | | | | | | Dataset | Training | | | Testing | | |
|---|---|---|---|---|---|---|---|---|---|---|---|---|---|---|---|---|
| | | Total amounts of sphingomyelins, cholesteryl esters and/or cholesterol determined as described in Example | | | | | | | | | 4 and/or 5 | | | 4 and/or 5 | | |
| Panel number | Number of parameters (without NT-proBNP) | Total amount of sphingomyelins | Total amount of cholesteryl esters | Total amount of cholesterol | Total amount of triacylglycerols | NT-proBNP | CHF subgroup | | | Number of cases | Number of controls | Absorbance values | Absolute concentrations | NT-proBNP alone | Absorbance values | Absolute concentrations | NT-proBNP alone |
| 8 | 4 | yes | yes | yes | yes | yes | CHF | | | 205 | 84 | 0.915 | 0.917 | 0.858 | 0.894 | 0.897 | 0.849 |
| 8 | 4 | yes | yes | yes | yes | yes | HFpEF | | | 74 | 84 | 0.809 | 0.813 | 0.739 | 0.793 | 0.798 | 0.708 |
| 8 | 4 | yes | yes | yes | yes | yes | HFrEF | | | 131 | 84 | 0.971 | 0.972 | 0.924 | 0.956 | 0.958 | 0.927 |
| 8 | 4 | yes | yes | yes | yes | yes | ICM | | | 64 | 84 | 0.985 | 0.985 | 0.935 | 0.986 | 0.986 | 0.953 |
| 8 | 4 | yes | yes | yes | yes | yes | DCM | | | 67 | 84 | 0.956 | 0.958 | 0.912 | 0.921 | 0.924 | 0.903 |
| 8 | 4 | yes | yes | yes | yes | yes | CHF asymptomatic | | | 93 | 84 | 0.877 | 0.880 | 0.798 | 0.863 | 0.864 | 0.802 |
| 8 | 4 | yes | yes | yes | yes | yes | HFpEF asymptomatic | | | 44 | 84 | 0.778 | 0.782 | 0.690 | 0.785 | 0.785 | 0.672 |
| 8 | 4 | yes | yes | yes | yes | yes | HFrEF asymptomatic | | | 49 | 84 | 0.961 | 0.963 | 0.888 | 0.938 | 0.942 | 0.911 |
| 8 | 4 | yes | yes | yes | yes | yes | ICM asymptomatic | | | 24 | 84 | 0.985 | 0.985 | 0.907 | 0.989 | 0.989 | 0.970 |
| 8 | 4 | yes | yes | yes | yes | yes | DCM asymptomatic | | | 25 | 84 | 0.939 | 0.943 | 0.872 | 0.875 | 0.881 | 0.846 |
| 8 | 4 | yes | yes | yes | yes | yes | CHF symptomatic | | | 112 | 84 | 0.946 | 0.946 | 0.908 | 0.922 | 0.926 | 0.890 |

| 8 | 4 | yes | yes | yes | yes | yes | HFpEF symptomatic | 30 | 84 | 0.852 | 0.855 | 0.811 | 0.803 | 0.815 | 0.755 |
| 8 | 4 | yes | yes | yes | yes | yes | HFrEF symptomatic | 82 | 84 | 0.977 | 0.978 | 0.944 | 0.967 | 0.968 | 0.939 |
| 8 | 4 | yes | yes | yes | yes | yes | ICM symptomatic | 40 | 84 | 0.985 | 0.984 | 0.950 | 0.984 | 0.984 | 0.941 |
| 8 | 4 | yes | yes | yes | yes | yes | DCM symptomatic | 42 | 84 | 0.969 | 0.970 | 0.937 | 0.948 | 0.949 | 0.940 |
| 8 | 4 | yes | yes | yes | yes | yes | HFrEF LVEF 35% to 50% | 71 | 84 | 0.958 | 0.959 | 0.890 | 0.920 | 0.923 | 0.874 |
| 8 | 4 | yes | yes | yes | yes | yes | ICM LVEF 35% to 50% | 42 | 84 | 0.980 | 0.979 | 0.905 | 0.975 | 0.975 | 0.929 |
| 8 | 4 | yes | yes | yes | yes | yes | DCM LVEF 35% to 50% | 29 | 84 | 0.929 | 0.934 | 0.871 | 0.832 | 0.840 | 0.794 |
| 8 | 4 | yes | yes | yes | yes | yes | HFrEF LVEF < 35% | 60 | 84 | 0.989 | 0.989 | 0.970 | 0.995 | 0.995 | 0.987 |
| 8 | 4 | yes | yes | yes | yes | yes | ICM LVEF < 35% | 22 | 84 | 0.996 | 0.996 | 0.984 | 1.000 | 1.000 | 0.989 |
| 8 | 4 | yes | yes | yes | yes | yes | DCM LVEF < 35% | 38 | 84 | 0.984 | 0.984 | 0.957 | 0.988 | 0.989 | 0.986 |
| 8 | 4 | yes | yes | yes | yes | no | CHF | 205 | 84 | 0.810 | 0.809 | 0.858 | 0.792 | 0.801 | 0.849 |
| 8 | 4 | yes | yes | yes | yes | no | HFpEF | 74 | 84 | 0.709 | 0.709 | 0.739 | 0.697 | 0.709 | 0.708 |
| 8 | 4 | yes | yes | yes | yes | no | HFrEF | 131 | 84 | 0.869 | 0.866 | 0.924 | 0.847 | 0.854 | 0.927 |
| 8 | 4 | yes | yes | yes | yes | no | ICM | 64 | 84 | 0.917 | 0.911 | 0.935 | 0.892 | 0.897 | 0.953 |
| 8 | 4 | yes | yes | yes | yes | no | DCM | 67 | 84 | 0.822 | 0.822 | 0.912 | 0.803 | 0.812 | 0.903 |
| 8 | 4 | yes | yes | yes | yes | no | CHF asymptomatic | 93 | 84 | 0.787 | 0.787 | 0.798 | 0.769 | 0.780 | 0.802 |
| 8 | 4 | yes | yes | yes | yes | no | HFpEF asymptomatic | 44 | 84 | 0.702 | 0.701 | 0.690 | 0.725 | 0.729 | 0.672 |
| 8 | 4 | yes | yes | yes | yes | no | HFrEF asymptomatic | 49 | 84 | 0.864 | 0.864 | 0.888 | 0.807 | 0.824 | 0.911 |
| 8 | 4 | yes | yes | yes | yes | no | ICM asymptomatic | 24 | 84 | 0.925 | 0.919 | 0.907 | 0.851 | 0.865 | 0.970 |
| 8 | 4 | yes | yes | yes | yes | no | DCM asymptomatic | 25 | 84 | 0.807 | 0.812 | 0.872 | 0.764 | 0.783 | 0.846 |
| 8 | 4 | yes | yes | yes | yes | no | CHF symptomatic | 112 | 84 | 0.829 | 0.826 | 0.908 | 0.811 | 0.819 | 0.890 |
| 8 | 4 | yes | yes | yes | yes | no | HFpEF symptomatic | 30 | 84 | 0.718 | 0.719 | 0.811 | 0.655 | 0.680 | 0.755 |
| 8 | 4 | yes | yes | yes | yes | no | HFrEF symptomatic | 82 | 84 | 0.872 | 0.867 | 0.944 | 0.871 | 0.872 | 0.939 |
| 8 | 4 | yes | yes | yes | yes | no | ICM symptomatic | 40 | 84 | 0.913 | 0.907 | 0.950 | 0.915 | 0.915 | 0.941 |
| 8 | 4 | yes | yes | yes | yes | no | DCM symptomatic | 42 | 84 | 0.831 | 0.828 | 0.937 | 0.828 | 0.831 | 0.940 |
| 8 | 4 | yes | yes | yes | yes | no | HFrEF LVEF 35% to 50% | 71 | 84 | 0.853 | 0.850 | 0.890 | 0.807 | 0.817 | 0.874 |
| 8 | 4 | yes | yes | yes | yes | no | ICM LVEF 35% to 50% | 42 | 84 | 0.909 | 0.901 | 0.905 | 0.864 | 0.867 | 0.929 |
| 8 | 4 | yes | yes | yes | yes | no | DCM LVEF 35% to 50% | 29 | 84 | 0.788 | 0.790 | 0.871 | 0.724 | 0.742 | 0.794 |
| 8 | 4 | yes | yes | yes | yes | no | HFrEF LVEF < 35% | 60 | 84 | 0.892 | 0.889 | 0.970 | 0.893 | 0.897 | 0.987 |
| 8 | 4 | yes | yes | yes | yes | no | ICM LVEF < 35% | 22 | 84 | 0.932 | 0.929 | 0.984 | 0.943 | 0.952 | 0.989 |

EP 3 194 959 B1

| | | | | | | | | | | | | | | |
|---|---|---|---|---|---|---|---|---|---|---|---|---|---|---|
| 8 | 4 | yes | yes | yes | yes | no | DCM LVEF < 35% | 38 | 84 | 0.861 | 0.860 | 0.957 | 0.863 | 0.864 | 0.986 |
| 9 | 3 | yes | yes | no | yes | yes | CHF | 205 | 84 | 0.915 | 0.916 | 0.858 | 0.895 | 0.898 | 0.849 |
| 9 | 3 | yes | yes | no | yes | yes | HFpEF | 74 | 84 | 0.809 | 0.812 | 0.739 | 0.795 | 0.801 | 0.708 |
| 9 | 3 | yes | yes | no | yes | yes | HFrEF | 131 | 84 | 0.971 | 0.971 | 0.924 | 0.957 | 0.959 | 0.927 |
| 9 | 3 | yes | yes | no | yes | yes | ICM | 64 | 84 | 0.985 | 0.984 | 0.935 | 0.986 | 0.986 | 0.953 |
| 9 | 3 | yes | yes | no | yes | yes | DCM | 67 | 84 | 0.956 | 0.957 | 0.912 | 0.921 | 0.925 | 0.903 |
| 9 | 3 | yes | yes | no | yes | yes | CHF asymptomatic | 93 | 84 | 0.877 | 0.878 | 0.798 | 0.865 | 0.866 | 0.802 |
| 9 | 3 | yes | yes | no | yes | yes | HFpEF asymptomatic | 44 | 84 | 0.776 | 0.779 | 0.690 | 0.788 | 0.788 | 0.672 |
| 9 | 3 | yes | yes | no | yes | yes | HFrEF asymptomatic | 49 | 84 | 0.961 | 0.962 | 0.888 | 0.939 | 0.943 | 0.911 |
| 9 | 3 | yes | yes | no | yes | yes | ICM asymptomatic | 24 | 84 | 0.985 | 0.985 | 0.907 | 0.989 | 0.990 | 0.970 |
| 9 | 3 | yes | yes | no | yes | yes | DCM asymptomatic | 25 | 84 | 0.939 | 0.941 | 0.872 | 0.875 | 0.882 | 0.846 |
| 9 | 3 | yes | yes | no | yes | yes | CHF symptomatic | 112 | 84 | 0.946 | 0.946 | 0.908 | 0.923 | 0.926 | 0.890 |
| 9 | 3 | yes | yes | no | yes | yes | HFpEF symptomatic | 30 | 84 | 0.854 | 0.858 | 0.811 | 0.804 | 0.817 | 0.755 |
| 9 | 3 | yes | yes | no | yes | yes | HFrEF symptomatic | 82 | 84 | 0.977 | 0.977 | 0.944 | 0.968 | 0.968 | 0.939 |
| 9 | 3 | yes | yes | no | yes | yes | ICM symptomatic | 40 | 84 | 0.985 | 0.984 | 0.950 | 0.984 | 0.984 | 0.941 |
| 9 | 3 | yes | yes | no | yes | yes | DCM symptomatic | 42 | 84 | 0.969 | 0.969 | 0.937 | 0.948 | 0.950 | 0.940 |
| 9 | 3 | yes | yes | no | yes | yes | HFrEF LVEF 35% to 50% | 71 | 84 | 0.958 | 0.958 | 0.890 | 0.921 | 0.924 | 0.874 |
| 9 | 3 | yes | yes | no | yes | yes | ICM LVEF 35% to 50% | 42 | 84 | 0.980 | 0.979 | 0.905 | 0.975 | 0.975 | 0.929 |
| 9 | 3 | yes | yes | no | yes | yes | DCM LVEF 35% to 50% | 29 | 84 | 0.928 | 0.932 | 0.871 | 0.833 | 0.842 | 0.794 |
| 9 | 3 | yes | yes | no | yes | yes | HFrEF LVEF < 35% | 60 | 84 | 0.989 | 0.989 | 0.970 | 0.995 | 0.996 | 0.987 |
| 9 | 3 | yes | yes | no | yes | yes | ICM LVEF < 35% | 22 | 84 | 0.996 | 0.996 | 0.984 | 1.000 | 1.000 | 0.989 |
| 9 | 3 | yes | yes | no | yes | yes | DCM LVEF < 35% | 38 | 84 | 0.984 | 0.984 | 0.957 | 0.988 | 0.989 | 0.986 |
| 9 | 3 | yes | yes | no | yes | no | CHF | 205 | 84 | 0.810 | 0.809 | 0.858 | 0.792 | 0.801 | 0.849 |
| 9 | 3 | yes | yes | no | yes | no | HFpEF | 74 | 84 | 0.709 | 0.710 | 0.739 | 0.697 | 0.710 | 0.708 |
| 9 | 3 | yes | yes | no | yes | no | HFrEF | 131 | 84 | 0.869 | 0.866 | 0.924 | 0.847 | 0.854 | 0.927 |
| 9 | 3 | yes | yes | no | yes | no | ICM | 64 | 84 | 0.917 | 0.911 | 0.935 | 0.892 | 0.897 | 0.953 |
| 9 | 3 | yes | yes | no | yes | no | DCM | 67 | 84 | 0.822 | 0.823 | 0.912 | 0.803 | 0.812 | 0.903 |
| 9 | 3 | yes | yes | no | yes | no | CHF asymptomatic | 93 | 84 | 0.787 | 0.787 | 0.798 | 0.769 | 0.780 | 0.802 |
| 9 | 3 | yes | yes | no | yes | no | HFpEF asymptomatic | 44 | 84 | 0.702 | 0.702 | 0.690 | 0.725 | 0.729 | 0.672 |
| 9 | 3 | yes | yes | no | yes | no | HFrEF asymptomatic | 49 | 84 | 0.864 | 0.864 | 0.888 | 0.807 | 0.825 | 0.911 |

| | | | | | | | | | | | | | | |
|---|---|---|---|---|---|---|---|---|---|---|---|---|---|---|
| 9 | 3 | yes | yes | no | yes | no | ICM asymptomatic | 24 | 84 | 0.925 | 0.919 | 0.907 | 0.851 | 0.865 | 0.970 |
| 9 | 3 | yes | yes | no | yes | no | DCM asymptomatic | 25 | 84 | 0.807 | 0.813 | 0.872 | 0.764 | 0.783 | 0.846 |
| 9 | 3 | yes | yes | no | yes | no | CHF symptomatic | 112 | 84 | 0.829 | 0.827 | 0.908 | 0.811 | 0.819 | 0.890 |
| 9 | 3 | yes | yes | no | yes | no | HFpEF symptomatic | 30 | 84 | 0.718 | 0.720 | 0.811 | 0.655 | 0.680 | 0.755 |
| 9 | 3 | yes | yes | no | yes | no | HFrEF symptomatic | 82 | 84 | 0.872 | 0.867 | 0.944 | 0.871 | 0.872 | 0.939 |
| 9 | 3 | yes | yes | no | yes | no | ICM symptomatic | 40 | 84 | 0.913 | 0.906 | 0.950 | 0.915 | 0.915 | 0.941 |
| 9 | 3 | yes | yes | no | yes | no | DCM symptomatic | 42 | 84 | 0.831 | 0.828 | 0.937 | 0.828 | 0.831 | 0.940 |
| 9 | 3 | yes | yes | no | yes | no | HFrEF LVEF 35% to 50% | 71 | 84 | 0.853 | 0.850 | 0.890 | 0.807 | 0.817 | 0.874 |
| 9 | 3 | yes | yes | no | yes | no | ICM LVEF 35% to 50% | 42 | 84 | 0.909 | 0.901 | 0.905 | 0.864 | 0.867 | 0.929 |
| 9 | 3 | yes | yes | no | yes | no | DCM LVEF 35% to 50% | 29 | 84 | 0.788 | 0.791 | 0.871 | 0.724 | 0.742 | 0.794 |
| 9 | 3 | yes | yes | no | yes | no | HFrEF LVEF < 35% | 60 | 84 | 0.892 | 0.889 | 0.970 | 0.893 | 0.897 | 0.987 |
| 9 | 3 | yes | yes | no | yes | no | ICM LVEF < 35% | 22 | 84 | 0.932 | 0.929 | 0.984 | 0.943 | 0.952 | 0.989 |
| 9 | 3 | yes | yes | no | yes | no | DCM LVEF < 35% | 38 | 84 | 0.861 | 0.860 | 0.957 | 0.863 | 0.864 | 0.986 |
| 10 | 3 | yes | no | yes | yes | yes | CHF | 205 | 84 | 0.914 | 0.917 | 0.858 | 0.896 | 0.897 | 0.849 |
| 10 | 3 | yes | no | yes | yes | yes | HFpEF | 74 | 84 | 0.808 | 0.813 | 0.739 | 0.797 | 0.799 | 0.708 |
| 10 | 3 | yes | no | yes | yes | yes | HFrEF | 131 | 84 | 0.971 | 0.972 | 0.924 | 0.957 | 0.958 | 0.927 |
| 10 | 3 | yes | no | yes | yes | yes | ICM | 64 | 84 | 0.985 | 0.985 | 0.935 | 0.986 | 0.986 | 0.953 |
| 10 | 3 | yes | no | yes | yes | yes | DCM | 67 | 84 | 0.956 | 0.959 | 0.912 | 0.922 | 0.925 | 0.903 |
| 10 | 3 | yes | no | yes | yes | yes | CHF asymptomatic | 93 | 84 | 0.877 | 0.880 | 0.798 | 0.863 | 0.864 | 0.802 |
| 10 | 3 | yes | no | yes | yes | yes | HFpEF asymptomatic | 44 | 84 | 0.780 | 0.784 | 0.690 | 0.786 | 0.785 | 0.672 |
| 10 | 3 | yes | no | yes | yes | yes | HFrEF asymptomatic | 49 | 84 | 0.961 | 0.963 | 0.888 | 0.938 | 0.941 | 0.911 |
| 10 | 3 | yes | no | yes | yes | yes | ICM asymptomatic | 24 | 84 | 0.984 | 0.985 | 0.907 | 0.988 | 0.988 | 0.970 |
| 10 | 3 | yes | no | yes | yes | yes | DCM asymptomatic | 25 | 84 | 0.940 | 0.943 | 0.872 | 0.876 | 0.881 | 0.846 |
| 10 | 3 | yes | no | yes | yes | yes | CHF symptomatic | 112 | 84 | 0.945 | 0.946 | 0.908 | 0.924 | 0.926 | 0.890 |
| 10 | 3 | yes | no | yes | yes | yes | HFpEF symptomatic | 30 | 84 | 0.848 | 0.853 | 0.811 | 0.809 | 0.817 | 0.755 |
| 10 | 3 | yes | no | yes | yes | yes | HFrEF symptomatic | 82 | 84 | 0.978 | 0.978 | 0.944 | 0.968 | 0.968 | 0.939 |
| 10 | 3 | yes | no | yes | yes | yes | ICM symptomatic | 40 | 84 | 0.985 | 0.985 | 0.950 | 0.985 | 0.984 | 0.941 |
| 10 | 3 | yes | no | yes | yes | yes | DCM symptomatic | 42 | 84 | 0.969 | 0.971 | 0.937 | 0.949 | 0.950 | 0.940 |
| 10 | 3 | yes | no | yes | yes | yes | HFrEF LVEF 35% to 50% | 71 | 84 | 0.958 | 0.960 | 0.890 | 0.921 | 0.923 | 0.874 |
| 10 | 3 | yes | no | yes | yes | yes | ICM LVEF 35% to 50% | 42 | 84 | 0.980 | 0.980 | 0.905 | 0.975 | 0.975 | 0.929 |

| | | | | | | | | | | | | | | | |
|---|---|---|---|---|---|---|---|---|---|---|---|---|---|---|---|
| 10 | 3 | yes | no | yes | yes | yes | DCM LVEF 35% to 50% | 29 | 84 | 0.929 | 0.934 | 0.871 | 0.834 | 0.840 | 0.794 |
| 10 | 3 | yes | no | yes | yes | yes | HFrEF LVEF < 35% | 60 | 84 | 0.989 | 0.989 | 0.970 | 0.995 | 0.995 | 0.987 |
| 10 | 3 | yes | no | yes | yes | yes | ICM LVEF < 35% | 22 | 84 | 0.995 | 0.996 | 0.984 | 0.999 | 1.000 | 0.989 |
| 10 | 3 | yes | no | yes | yes | yes | DCM LVEF < 35% | 38 | 84 | 0.984 | 0.984 | 0.957 | 0.989 | 0.989 | 0.986 |
| 10 | 3 | yes | no | yes | yes | no | CHF | 205 | 84 | 0.805 | 0.807 | 0.858 | 0.793 | 0.799 | 0.849 |
| 10 | 3 | yes | no | yes | yes | no | HFpEF | 74 | 84 | 0.704 | 0.708 | 0.739 | 0.704 | 0.710 | 0.708 |
| 10 | 3 | yes | no | yes | yes | no | HFrEF | 131 | 84 | 0.865 | 0.865 | 0.924 | 0.845 | 0.850 | 0.927 |
| 10 | 3 | yes | no | yes | yes | no | ICM | 64 | 84 | 0.912 | 0.911 | 0.935 | 0.887 | 0.892 | 0.953 |
| 10 | 3 | yes | no | yes | yes | no | DCM | 67 | 84 | 0.819 | 0.820 | 0.912 | 0.804 | 0.809 | 0.903 |
| 10 | 3 | yes | no | yes | yes | no | CHF asymptomatic | 93 | 84 | 0.784 | 0.787 | 0.798 | 0.769 | 0.777 | 0.802 |
| 10 | 3 | yes | no | yes | yes | no | HFpEF asymptomatic | 44 | 84 | 0.701 | 0.704 | 0.690 | 0.728 | 0.728 | 0.672 |
| 10 | 3 | yes | no | yes | yes | no | HFrEF asymptomatic | 49 | 84 | 0.860 | 0.862 | 0.888 | 0.805 | 0.819 | 0.911 |
| 10 | 3 | yes | no | yes | yes | no | ICM asymptomatic | 24 | 84 | 0.920 | 0.918 | 0.907 | 0.839 | 0.855 | 0.970 |
| 10 | 3 | yes | no | yes | yes | no | DCM asymptomatic | 25 | 84 | 0.805 | 0.808 | 0.872 | 0.770 | 0.782 | 0.846 |
| 10 | 3 | yes | no | yes | yes | no | CHF symptomatic | 112 | 84 | 0.823 | 0.823 | 0.908 | 0.813 | 0.817 | 0.890 |
| 10 | 3 | yes | no | yes | yes | no | HFpEF symptomatic | 30 | 84 | 0.708 | 0.712 | 0.811 | 0.667 | 0.682 | 0.755 |
| 10 | 3 | yes | no | yes | yes | no | HFrEF symptomatic | 82 | 84 | 0.868 | 0.867 | 0.944 | 0.870 | 0.869 | 0.939 |
| 10 | 3 | yes | no | yes | yes | no | ICM symptomatic | 40 | 84 | 0.907 | 0.906 | 0.950 | 0.913 | 0.912 | 0.941 |
| 10 | 3 | yes | no | yes | yes | no | DCM symptomatic | 42 | 84 | 0.828 | 0.827 | 0.937 | 0.827 | 0.828 | 0.940 |
| 10 | 3 | yes | no | yes | yes | no | HFrEF LVEF 35% to 50% | 71 | 84 | 0.848 | 0.848 | 0.890 | 0.808 | 0.815 | 0.874 |
| 10 | 3 | yes | no | yes | yes | no | ICM LVEF 35% to 50% | 42 | 84 | 0.902 | 0.900 | 0.905 | 0.861 | 0.863 | 0.929 |
| 10 | 3 | yes | no | yes | yes | no | DCM LVEF 35% to 50% | 29 | 84 | 0.785 | 0.786 | 0.871 | 0.732 | 0.743 | 0.794 |
| 10 | 3 | yes | no | yes | yes | no | HFrEF LVEF < 35% | 60 | 84 | 0.889 | 0.890 | 0.970 | 0.887 | 0.891 | 0.987 |
| 10 | 3 | yes | no | yes | yes | no | ICM LVEF < 35% | 22 | 84 | 0.928 | 0.930 | 0.984 | 0.934 | 0.945 | 0.989 |
| 10 | 3 | yes | no | yes | yes | no | DCM LVEF < 35% | 38 | 84 | 0.859 | 0.859 | 0.957 | 0.858 | 0.858 | 0.986 |

Example 11: Differentiation of CHF and its subtypes from healthy controls using a combination of the total amount of sphingomyelins with the total amount of triacylglycerols

The diagnostic power of the total amount of sphingomyelins determined as described in Example 4 in combination with total amount of triacylglycerols was estimated with the area under the curve (AUC) of a receiver operating characteristic (ROC) analysis (Table 21). The total amount of triacylglycerols was determined as described in Example 6. Optionally, NT-proBNP was also taken into account.

The study design is described in Example 7, with AUC values calculated separately for the training and the testing set. The parameters were combined with each other and/or with NT-proBNP as described in Example 8.

Table 21: Area under the curve (AUC) of a receiver operating characteristic (ROC) analysis for the enzymatically determined total amount of sphingomyelins in combination with the enzymatically determined total amount of triacylglycerols, optionally also in combination with NT-proBNP (determined via antibody reaction), without correction for the confounders age, BMI, and gender. Panel 11 is a combinations of two parameters. NT-proBNP alone is shown for comparison.

| Dataset | | | | | | | | | | Training | | | Testing | | |
|---|---|---|---|---|---|---|---|---|---|---|---|---|---|---|---|
| Total amount of sphingomyelins determined as described in Example | | | | | | | | | | 4 | | | 4 | | |
| Panel number | Number of parameters (without NT-proBNP) | Total amount of sphingomyelins | Total amount of cholesteryl esters | Total amount of cholesterol | Total amount of triacylglycerols | NT-proBNP | CHF subgroup | Number of cases | Number of controls | Absorbance values | Absolute concentrations | NT-proBNP alone | Absorbance values | Absolute concentrations | NT-proBNP alone |
| 11 | 2 | yes | no | no | yes | yes | CHF | 205 | 84 | 0.913 | 0.911 | 0.858 | 0.899 | 0.899 | 0.849 |
| 11 | 2 | yes | no | no | yes | yes | HFpEF | 74 | 84 | 0.809 | 0.807 | 0.739 | 0.806 | 0.807 | 0.708 |
| 11 | 2 | yes | no | no | yes | yes | HFrEF | 131 | 84 | 0.969 | 0.968 | 0.924 | 0.958 | 0.957 | 0.927 |
| 11 | 2 | yes | no | no | yes | yes | ICM | 64 | 84 | 0.982 | 0.981 | 0.935 | 0.985 | 0.984 | 0.953 |
| 11 | 2 | yes | no | no | yes | yes | DCM | 67 | 84 | 0.955 | 0.953 | 0.912 | 0.925 | 0.926 | 0.903 |
| 11 | 2 | yes | no | no | yes | yes | CHF asymptomatic | 93 | 84 | 0.875 | 0.871 | 0.798 | 0.866 | 0.866 | 0.802 |
| 11 | 2 | yes | no | no | yes | yes | HFpEF asymptomatic | 44 | 84 | 0.776 | 0.773 | 0.690 | 0.795 | 0.792 | 0.672 |
| 11 | 2 | yes | no | no | yes | yes | HFrEF asymptomatic | 49 | 84 | 0.959 | 0.956 | 0.888 | 0.938 | 0.938 | 0.911 |

EP 3 194 959 B1

| 11 | 2 | yes | no | no | yes | yes | ICM asymptomatic | 24 | 84 | 0.982 | 0.980 | 0.907 | 0.986 | 0.985 | 0.970 |
| 11 | 2 | yes | no | no | yes | yes | DCM asymptomatic | 25 | 84 | 0.937 | 0.933 | 0.872 | 0.878 | 0.880 | 0.846 |
| 11 | 2 | yes | no | no | yes | yes | CHF symptomatic | 112 | 84 | 0.945 | 0.944 | 0.908 | 0.928 | 0.928 | 0.890 |
| 11 | 2 | yes | no | no | yes | yes | HFpEF symptomatic | 30 | 84 | 0.854 | 0.856 | 0.811 | 0.820 | 0.827 | 0.755 |
| 11 | 2 | yes | no | no | yes | yes | HFrEF symptomatic | 82 | 84 | 0.976 | 0.975 | 0.944 | 0.970 | 0.968 | 0.939 |
| 11 | 2 | yes | no | no | yes | yes | ICM symptomatic | 40 | 84 | 0.983 | 0.982 | 0.950 | 0.985 | 0.983 | 0.941 |
| 11 | 2 | yes | no | no | yes | yes | DCM symptomatic | 42 | 84 | 0.968 | 0.967 | 0.937 | 0.952 | 0.952 | 0.940 |
| 11 | 2 | yes | no | no | yes | yes | HFrEF LVEF 35% to 50% | 71 | 84 | 0.955 | 0.952 | 0.890 | 0.923 | 0.923 | 0.874 |
| 11 | 2 | yes | no | no | yes | yes | ICM LVEF 35% to 50% | 42 | 84 | 0.977 | 0.975 | 0.905 | 0.975 | 0.972 | 0.929 |
| 11 | 2 | yes | no | no | yes | yes | DCM LVEF 35% to 50% | 29 | 84 | 0.925 | 0.921 | 0.871 | 0.840 | 0.845 | 0.794 |
| 11 | 2 | yes | no | no | yes | yes | HFrEF LVEF < 35% | 60 | 84 | 0.989 | 0.989 | 0.970 | 0.995 | 0.994 | 0.987 |
| 11 | 2 | yes | no | no | yes | yes | ICM LVEF < 35% | 22 | 84 | 0.995 | 0.995 | 0.984 | 0.999 | 0.999 | 0.989 |
| 11 | 2 | yes | no | no | yes | yes | DCM LVEF < 35% | 38 | 84 | 0.984 | 0.984 | 0.957 | 0.990 | 0.989 | 0.986 |
| 11 | 2 | yes | no | no | yes | no | CHF | 205 | 84 | 0.804 | 0.799 | 0.858 | 0.796 | 0.798 | 0.849 |
| 11 | 2 | yes | no | no | yes | no | HFpEF | 74 | 84 | 0.702 | 0.701 | 0.739 | 0.710 | 0.716 | 0.708 |
| 11 | 2 | yes | no | no | yes | no | HFrEF | 131 | 84 | 0.863 | 0.856 | 0.924 | 0.846 | 0.845 | 0.927 |
| 11 | 2 | yes | no | no | yes | no | ICM | 64 | 84 | 0.908 | 0.902 | 0.935 | 0.885 | 0.880 | 0.953 |
| 11 | 2 | yes | no | no | yes | no | DCM | 67 | 84 | 0.820 | 0.813 | 0.912 | 0.807 | 0.811 | 0.903 |
| 11 | 2 | yes | no | no | yes | no | CHF asymptomatic | 93 | 84 | 0.782 | 0.776 | 0.798 | 0.771 | 0.772 | 0.802 |
| 11 | 2 | yes | no | no | yes | no | HFpEF asymptomatic | 44 | 84 | 0.697 | 0.693 | 0.690 | 0.732 | 0.732 | 0.672 |
| 11 | 2 | yes | no | no | yes | no | HFrEF asymptomatic | 49 | 84 | 0.859 | 0.850 | 0.888 | 0.805 | 0.806 | 0.911 |
| 11 | 2 | yes | no | no | yes | no | ICM asymptomatic | 24 | 84 | 0.916 | 0.907 | 0.907 | 0.835 | 0.833 | 0.970 |
| 11 | 2 | yes | no | no | yes | no | DCM asymptomatic | 25 | 84 | 0.805 | 0.797 | 0.872 | 0.773 | 0.778 | 0.846 |
| 11 | 2 | yes | no | no | yes | no | CHF symptomatic | 112 | 84 | 0.822 | 0.818 | 0.908 | 0.818 | 0.820 | 0.890 |

| 11 | 2 | yes | no | no | yes | no | HFpEF symptomatic | 30 | 84 | 0.709 | 0.711 | 0.811 | 0.676 | 0.690 | 0.755 |
| 11 | 2 | yes | no | no | yes | no | HFrEF symptomatic | 82 | 84 | 0.866 | 0.860 | 0.944 | 0.872 | 0.869 | 0.939 |
| 11 | 2 | yes | no | no | yes | no | ICM symptomatic | 40 | 84 | 0.903 | 0.899 | 0.950 | 0.914 | 0.906 | 0.941 |
| 11 | 2 | yes | no | no | yes | no | DCM symptomatic | 42 | 84 | 0.829 | 0.822 | 0.937 | 0.831 | 0.833 | 0.940 |
| 11 | 2 | yes | no | no | yes | no | HFrEF LVEF 35% to 50% | 71 | 84 | 0.845 | 0.835 | 0.890 | 0.810 | 0.810 | 0.874 |
| 11 | 2 | yes | no | no | yes | no | ICM LVEF 35% to 50% | 42 | 84 | 0.897 | 0.889 | 0.905 | 0.860 | 0.855 | 0.929 |
| 11 | 2 | yes | no | no | yes | no | DCM LVEF 35% to 50% | 29 | 84 | 0.785 | 0.774 | 0.871 | 0.736 | 0.743 | 0.794 |
| 11 | 2 | yes | no | no | yes | no | HFrEF LVEF < 35% | 60 | 84 | 0.888 | 0.886 | 0.970 | 0.887 | 0.885 | 0.987 |
| 11 | 2 | yes | no | no | yes | no | ICM LVEF < 35% | 22 | 84 | 0.925 | 0.924 | 0.984 | 0.930 | 0.925 | 0.989 |
| 11 | 2 | yes | no | no | yes | no | DCM LVEF < 35% | 38 | 84 | 0.861 | 0.858 | 0.957 | 0.861 | 0.861 | 0.986 |

Example 12: Sensitivity and specificity at different cutoff values

Cut-off values for the prediction probability *p*, calculated as described in Example 8, were defined in order classifiy a subject as suffering from hear failure or not. A subject with a prediction probability *p* equal to or greater than the cut-off value was given the diagnosis to be suffering from heart failure. A subject with a prediction probability *p* smaller than the cut-off value was given the diagnosis not to be suffering from heart failure.

Three methods were used for cut-off value selection for each of panels 8 + NT-proBNP, 9 + NT-proBNP, and 10 + NT-proBNP (listed in Table 20), and for panel 11 + NT-proBNP (listed in Table 21).. Absolute concentrations were used for all parameters. Instead of absolute concentrations, absorbance values may also be used. Panels 8, 9, 10, and 11 may also be used without NT-proBNP.

A fixed cut-off value of 125 pg/ml was used for NT-proBNP alone. Three cut-off values for each of panels 8 + NT-proBNP, 9 + NT-proBNP, 10 + NT-proBNP, and 11 + NT-proBNP were chosen according to the following three different methods: 1) The cut-off value for each panel "Panel + NT-proBNP" was chosen so that each panel had the same sensitivity on the subgroup 'HFrEF' in the training set (Example 7) as NT-proBNP alone. 2) The cut-off value for each panel "Panel + NT-proBNP" was chosen so that each panel had a 5% higher sensitivity on the subgroup 'HFrEF' in the training set (Example 7) as NT-proBNP alone. 3) The cut-off value for each panel "Panel + NT-proBNP" was chosen to maximize Youden's index for the subgroup 'HFrEF' in the training set (Example 7).

How to calculate sensitivity and specificity is well known to the person skilled in the art. Sensitivity is defined as the fraction of subjects suffering from heart failure that are diagnosed as suffering from heart failure. Specificity is defined as the fraction of subjects not suffering from heart failure that are diagnosed as not suffering from heart failure. Youden's index is well known to the person skilled in the art and is defined as sensitivity + specificity - 1.

Cut-off values chosen according to methods 1), 2), and 3) as well as the respective sensitivity and specificity in each subgroup are given in Table 22 for panels 8 + NT-proBNP, 9 + NT-proBNP, 10 + NT-proBNP, and 11 + NT-proBNP as well as for NT-proBNP alone. Method 1) is referred to in Table 22 as 'Match sensitivity', method 2) as 'Match sensitivity + 5%', and method 3) as 'Maximize Youden's index'. Sensitivities and specificities shown in Table 22 were calculated on the testing set.

Table 22: Sensitivity and specificity in the testing set at different cutoff values for panels 8 + NT-proBNP, 9 + NT-proBNP, 10 + NT-proBNP, and 11 + NT-proBNP, without correction for the confounders age, BMI, and gender. Panel composition, number of parameters in the panel, number of cases and controls, and AUC values are given in Table 20 for panels 8 + NT-proBNP, 9 + NT-proBNP, and 10 + NT-proBNP and in Table 21 for panel 11 + NT-proBNP (rows with "yes" in column "NT-proBNP"). Absolute concentrations were used for all parameters (the concentrations have been determined via antibody reaction for NT-proBNP and enzymatically for all others). Sensitivity and specificity for NT-proBNP alone is shown for comparison.

| Panel number | CHF subgroup | Biomarker panel incl. NT-proBNP | | | | | | | | | NT-proBNP alone | |
| | | Match sensitivity | | | Match sensitivity + 5% | | | Maximize Youden's index | | | | |
| | | Cutoff | Sens. | Spec. | Cutoff | Sens. | Spec. | Cutoff | Sens. | Spec. | Sens. | Spec. |
|---|---|---|---|---|---|---|---|---|---|---|---|---|
| 8 | CHF | 0.710 | 0.595 | 0.976 | 0.633 | 0.659 | 0.952 | 0.535 | 0.722 | 0.905 | 0.649 | 0.881 |
| 8 | HFpEF | 0.710 | 0.297 | 0.976 | 0.633 | 0.351 | 0.952 | 0.535 | 0.459 | 0.905 | 0.351 | 0.881 |
| 8 | HFrEF | 0.710 | 0.763 | 0.976 | 0.633 | 0.832 | 0.952 | 0.535 | 0.870 | 0.905 | 0.817 | 0.881 |
| 8 | ICM | 0.710 | 0.844 | 0.976 | 0.633 | 0.906 | 0.952 | 0.535 | 0.969 | 0.905 | 0.891 | 0.881 |
| 8 | DCM | 0.710 | 0.687 | 0.976 | 0.633 | 0.761 | 0.952 | 0.535 | 0.776 | 0.905 | 0.746 | 0.881 |
| 8 | CHF asymptomatic | 0.710 | 0.473 | 0.976 | 0.633 | 0.538 | 0.952 | 0.535 | 0.613 | 0.905 | 0.548 | 0.881 |
| 8 | HFpEF asymptomatic | 0.710 | 0.250 | 0.976 | 0.633 | 0.318 | 0.952 | 0.535 | 0.455 | 0.905 | 0.273 | 0.881 |
| 8 | HFrEF asymptomatic | 0.710 | 0.673 | 0.976 | 0.633 | 0.735 | 0.952 | 0.535 | 0.755 | 0.905 | 0.796 | 0.881 |
| 8 | ICM asymptomatic | 0.710 | 0.875 | 0.976 | 0.633 | 0.917 | 0.952 | 0.535 | 0.917 | 0.905 | 0.958 | 0.881 |
| 8 | DCM asymptomatic | 0.710 | 0.480 | 0.976 | 0.633 | 0.560 | 0.952 | 0.535 | 0.600 | 0.905 | 0.640 | 0.881 |
| 8 | CHF symptomatic | 0.710 | 0.696 | 0.976 | 0.633 | 0.759 | 0.952 | 0.535 | 0.813 | 0.905 | 0.732 | 0.881 |
| 8 | HFpEF symptomatic | 0.710 | 0.367 | 0.976 | 0.633 | 0.400 | 0.952 | 0.535 | 0.467 | 0.905 | 0.467 | 0.881 |
| 8 | HFrEF symptomatic | 0.710 | 0.817 | 0.976 | 0.633 | 0.890 | 0.952 | 0.535 | 0.939 | 0.905 | 0.829 | 0.881 |
| 8 | ICM symptomatic | 0.710 | 0.825 | 0.976 | 0.633 | 0.900 | 0.952 | 0.535 | 1.000 | 0.905 | 0.850 | 0.881 |
| 8 | DCM symptomatic | 0.710 | 0.810 | 0.976 | 0.633 | 0.881 | 0.952 | 0.535 | 0.881 | 0.905 | 0.810 | 0.881 |

EP 3 194 959 B1

| | | | | | | | | | | | |
|---|---|---|---|---|---|---|---|---|---|---|---|
| 8 | HFrEF LVEF 35% to 50% | 0.710 | 0.620 | 0.976 | 0.633 | 0.718 | 0.952 | 0.535 | 0.775 | 0.905 | 0.676 | 0.881 |
| 8 | ICM LVEF 35% to 50% | 0.710 | 0.762 | 0.976 | 0.633 | 0.857 | 0.952 | 0.535 | 0.952 | 0.905 | 0.833 | 0.881 |
| 8 | DCM LVEF 35% to 50% | 0.710 | 0.414 | 0.976 | 0.633 | 0.517 | 0.952 | 0.535 | 0.517 | 0.905 | 0.448 | 0.881 |
| 8 | HFrEF LVEF < 35% | 0.710 | 0.933 | 0.976 | 0.633 | 0.967 | 0.952 | 0.535 | 0.983 | 0.905 | 0.983 | 0.881 |
| 8 | ICM LVEF < 35% | 0.710 | 1.000 | 0.976 | 0.633 | 1.000 | 0.952 | 0.535 | 1.000 | 0.905 | 1.000 | 0.881 |
| 8 | DCM LVEF < 35% | 0.710 | 0.895 | 0.976 | 0.633 | 0.947 | 0.952 | 0.535 | 0.974 | 0.905 | 0.974 | 0.881 |
| 9 | CHF | 0.702 | 0.595 | 0.988 | 0.625 | 0.659 | 0.952 | 0.494 | 0.741 | 0.893 | 0.649 | 0.881 |
| 9 | HFpEF | 0.702 | 0.297 | 0.988 | 0.625 | 0.351 | 0.952 | 0.494 | 0.500 | 0.893 | 0.351 | 0.881 |
| 9 | HFrEF | 0.702 | 0.763 | 0.988 | 0.625 | 0.832 | 0.952 | 0.494 | 0.878 | 0.893 | 0.817 | 0.881 |
| 9 | ICM | 0.702 | 0.844 | 0.988 | 0.625 | 0.906 | 0.952 | 0.494 | 0.969 | 0.893 | 0.891 | 0.881 |
| 9 | DCM | 0.702 | 0.687 | 0.988 | 0.625 | 0.761 | 0.952 | 0.494 | 0.791 | 0.893 | 0.746 | 0.881 |
| 9 | CHF asymptomatic | 0.702 | 0.484 | 0.988 | 0.625 | 0.538 | 0.952 | 0.494 | 0.634 | 0.893 | 0.548 | 0.881 |
| 9 | HFpEF asymptomatic | 0.702 | 0.250 | 0.988 | 0.625 | 0.318 | 0.952 | 0.494 | 0.477 | 0.893 | 0.273 | 0.881 |
| 9 | HFrEF asymptomatic | 0.702 | 0.694 | 0.988 | 0.625 | 0.735 | 0.952 | 0.494 | 0.776 | 0.893 | 0.796 | 0.881 |
| 9 | ICM asymptomatic | 0.702 | 0.875 | 0.988 | 0.625 | 0.917 | 0.952 | 0.494 | 0.917 | 0.893 | 0.958 | 0.881 |
| 9 | DCM asymptomatic | 0.702 | 0.520 | 0.988 | 0.625 | 0.560 | 0.952 | 0.494 | 0.640 | 0.893 | 0.640 | 0.881 |
| 9 | CHF symptomatic | 0.702 | 0.688 | 0.988 | 0.625 | 0.759 | 0.952 | 0.494 | 0.830 | 0.893 | 0.732 | 0.881 |
| 9 | HFpEF symptomatic | 0.702 | 0.367 | 0.988 | 0.625 | 0.400 | 0.952 | 0.494 | 0.533 | 0.893 | 0.467 | 0.881 |
| 9 | HFrEF symptomatic | 0.702 | 0.805 | 0.988 | 0.625 | 0.890 | 0.952 | 0.494 | 0.939 | 0.893 | 0.829 | 0.881 |
| 9 | ICM symptomatic | 0.702 | 0.825 | 0.988 | 0.625 | 0.900 | 0.952 | 0.494 | 1.000 | 0.893 | 0.850 | 0.881 |
| 9 | DCM symptomatic | 0.702 | 0.786 | 0.988 | 0.625 | 0.881 | 0.952 | 0.494 | 0.881 | 0.893 | 0.810 | 0.881 |
| 9 | HFrEF LVEF 35% to 50% | 0.702 | 0.620 | 0.988 | 0.625 | 0.718 | 0.952 | 0.494 | 0.789 | 0.893 | 0.676 | 0.881 |
| 9 | ICM LVEF 35% to 50% | 0.702 | 0.762 | 0.988 | 0.625 | 0.857 | 0.952 | 0.494 | 0.952 | 0.893 | 0.833 | 0.881 |
| 9 | DCM LVEF 35% to 50% | 0.702 | 0.414 | 0.988 | 0.625 | 0.517 | 0.952 | 0.494 | 0.552 | 0.893 | 0.448 | 0.881 |
| 9 | HFrEF LVEF < 35% | 0.702 | 0.933 | 0.988 | 0.625 | 0.967 | 0.952 | 0.494 | 0.983 | 0.893 | 0.983 | 0.881 |
| 9 | ICM LVEF < 35% | 0.702 | 1.000 | 0.988 | 0.625 | 1.000 | 0.952 | 0.494 | 1.000 | 0.893 | 1.000 | 0.881 |
| 9 | DCM LVEF < 35% | 0.702 | 0.895 | 0.988 | 0.625 | 0.947 | 0.952 | 0.494 | 0.974 | 0.893 | 0.974 | 0.881 |
| 10 | CHF | 0.715 | 0.595 | 0.976 | 0.641 | 0.639 | 0.952 | 0.505 | 0.737 | 0.893 | 0.649 | 0.881 |
| 10 | HFpEF | 0.715 | 0.297 | 0.976 | 0.641 | 0.324 | 0.952 | 0.505 | 0.473 | 0.893 | 0.351 | 0.881 |
| 10 | HFrEF | 0.715 | 0.763 | 0.976 | 0.641 | 0.817 | 0.952 | 0.505 | 0.885 | 0.893 | 0.817 | 0.881 |

| | | | | | | | | | | | |
|---|---|---|---|---|---|---|---|---|---|---|---|
| 10 | ICM | 0.715 | 0.844 | 0.976 | 0.641 | 0.891 | 0.952 | 0.505 | 0.969 | 0.893 | 0.891 | 0.881 |
| 10 | DCM | 0.715 | 0.687 | 0.976 | 0.641 | 0.746 | 0.952 | 0.505 | 0.806 | 0.893 | 0.746 | 0.881 |
| 10 | CHF asymptomatic | 0.715 | 0.473 | 0.976 | 0.641 | 0.505 | 0.952 | 0.505 | 0.634 | 0.893 | 0.548 | 0.881 |
| 10 | HFpEF asymptomatic | 0.715 | 0.250 | 0.976 | 0.641 | 0.273 | 0.952 | 0.505 | 0.455 | 0.893 | 0.273 | 0.881 |
| 10 | HFrEF asymptomatic | 0.715 | 0.673 | 0.976 | 0.641 | 0.714 | 0.952 | 0.505 | 0.796 | 0.893 | 0.796 | 0.881 |
| 10 | ICM asymptomatic | 0.715 | 0.875 | 0.976 | 0.641 | 0.917 | 0.952 | 0.505 | 0.917 | 0.893 | 0.958 | 0.881 |
| 10 | DCM asymptomatic | 0.715 | 0.480 | 0.976 | 0.641 | 0.520 | 0.952 | 0.505 | 0.680 | 0.893 | 0.640 | 0.881 |
| 10 | CHF symptomatic | 0.715 | 0.696 | 0.976 | 0.641 | 0.750 | 0.952 | 0.505 | 0.821 | 0.893 | 0.732 | 0.881 |
| 10 | HFpEF symptomatic | 0.715 | 0.367 | 0.976 | 0.641 | 0.400 | 0.952 | 0.505 | 0.500 | 0.893 | 0.467 | 0.881 |
| 10 | HFrEF symptomatic | 0.715 | 0.817 | 0.976 | 0.641 | 0.878 | 0.952 | 0.505 | 0.939 | 0.893 | 0.829 | 0.881 |
| 10 | ICM symptomatic | 0.715 | 0.825 | 0.976 | 0.641 | 0.875 | 0.952 | 0.505 | 1.000 | 0.893 | 0.850 | 0.881 |
| 10 | DCM symptomatic | 0.715 | 0.810 | 0.976 | 0.641 | 0.881 | 0.952 | 0.505 | 0.881 | 0.893 | 0.810 | 0.881 |
| 10 | HFrEF LVEF 35% to 50% | 0.715 | 0.620 | 0.976 | 0.641 | 0.690 | 0.952 | 0.505 | 0.803 | 0.893 | 0.676 | 0.881 |
| 10 | ICM LVEF 35% to 50% | 0.715 | 0.762 | 0.976 | 0.641 | 0.833 | 0.952 | 0.505 | 0.952 | 0.893 | 0.833 | 0.881 |
| 10 | DCM LVEF 35% to 50% | 0.715 | 0.414 | 0.976 | 0.641 | 0.483 | 0.952 | 0.505 | 0.586 | 0.893 | 0.448 | 0.881 |
| 10 | HFrEF LVEF < 35% | 0.715 | 0.933 | 0.976 | 0.641 | 0.967 | 0.952 | 0.505 | 0.983 | 0.893 | 0.983 | 0.881 |
| 10 | ICM LVEF < 35% | 0.715 | 1.000 | 0.976 | 0.641 | 1.000 | 0.952 | 0.505 | 1.000 | 0.893 | 1.000 | 0.881 |
| 10 | DCM LVEF < 35% | 0.715 | 0.895 | 0.976 | 0.641 | 0.947 | 0.952 | 0.505 | 0.974 | 0.893 | 0.974 | 0.881 |
| 11 | CHF | 0.708 | 0.566 | 0.988 | 0.612 | 0.644 | 0.964 | 0.548 | 0.698 | 0.893 | 0.649 | 0.881 |
| 11 | HFpEF | 0.708 | 0.257 | 0.988 | 0.612 | 0.324 | 0.964 | 0.548 | 0.432 | 0.893 | 0.351 | 0.881 |
| 11 | HFrEF | 0.708 | 0.740 | 0.988 | 0.612 | 0.824 | 0.964 | 0.548 | 0.847 | 0.893 | 0.817 | 0.881 |
| 11 | ICM | 0.708 | 0.813 | 0.988 | 0.612 | 0.906 | 0.964 | 0.548 | 0.953 | 0.893 | 0.891 | 0.881 |
| 11 | DCM | 0.708 | 0.672 | 0.988 | 0.612 | 0.746 | 0.964 | 0.548 | 0.746 | 0.893 | 0.746 | 0.881 |
| 11 | CHF asymptomatic | 0.708 | 0.484 | 0.988 | 0.612 | 0.505 | 0.964 | 0.548 | 0.570 | 0.893 | 0.548 | 0.881 |
| 11 | HFpEF asymptomatic | 0.708 | 0.273 | 0.988 | 0.612 | 0.273 | 0.964 | 0.548 | 0.409 | 0.893 | 0.273 | 0.881 |
| 11 | HFrEF asymptomatic | 0.708 | 0.673 | 0.988 | 0.612 | 0.714 | 0.964 | 0.548 | 0.714 | 0.893 | 0.796 | 0.881 |
| 11 | ICM asymptomatic | 0.708 | 0.875 | 0.988 | 0.612 | 0.875 | 0.964 | 0.548 | 0.875 | 0.893 | 0.958 | 0.881 |
| 11 | DCM asymptomatic | 0.708 | 0.480 | 0.988 | 0.612 | 0.560 | 0.964 | 0.548 | 0.560 | 0.893 | 0.640 | 0.881 |
| 11 | CHF symptomatic | 0.708 | 0.634 | 0.988 | 0.612 | 0.759 | 0.964 | 0.548 | 0.804 | 0.893 | 0.732 | 0.881 |
| 11 | HFpEF symptomatic | 0.708 | 0.233 | 0.988 | 0.612 | 0.400 | 0.964 | 0.548 | 0.467 | 0.893 | 0.467 | 0.881 |

| 11 | HFrEF symptomatic | 0.708 | 0.780 | 0.988 | 0.612 | 0.890 | 0.964 | 0.548 | 0.927 | 0.893 | 0.829 | 0.881 |
| 11 | ICM symptomatic | 0.708 | 0.775 | 0.988 | 0.612 | 0.925 | 0.964 | 0.548 | 1.000 | 0.893 | 0.850 | 0.881 |
| 11 | DCM symptomatic | 0.708 | 0.786 | 0.988 | 0.612 | 0.857 | 0.964 | 0.548 | 0.857 | 0.893 | 0.810 | 0.881 |
| 11 | HFrEF LVEF 35% to 50% | 0.708 | 0.563 | 0.988 | 0.612 | 0.718 | 0.964 | 0.548 | 0.761 | 0.893 | 0.676 | 0.881 |
| 11 | ICM LVEF 35% to 50% | 0.708 | 0.714 | 0.988 | 0.612 | 0.857 | 0.964 | 0.548 | 0.929 | 0.893 | 0.833 | 0.881 |
| 11 | DCM LVEF 35% to 50% | 0.708 | 0.345 | 0.988 | 0.612 | 0.517 | 0.964 | 0.548 | 0.517 | 0.893 | 0.448 | 0.881 |
| 11 | HFrEF LVEF < 35% | 0.708 | 0.950 | 0.988 | 0.612 | 0.950 | 0.964 | 0.548 | 0.950 | 0.893 | 0.983 | 0.881 |
| 11 | ICM LVEF < 35% | 0.708 | 1.000 | 0.988 | 0.612 | 1.000 | 0.964 | 0.548 | 1.000 | 0.893 | 1.000 | 0.881 |
| 11 | DCM LVEF < 35% | 0.708 | 0.921 | 0.988 | 0.612 | 0.921 | 0.964 | 0.548 | 0.921 | 0.893 | 0.974 | 0.881 |

Example 13: Additional performance statistics

Additional performance statistics were calculated for panels 8 + NT-proBNP, 9 + NT-proBNP, 10 + NT-proBNP, and 11 + NT-proBNP on the testing set. The following additional performance statistics are given in Table 23: positive likelihood ratio (LR+), negative likelihood ratio (LR-), positive predictive value (PPV), and negative predictive value (NPV).

How to calculate LR+ and LR- is well known to the person skilled in the art. LR+ is a measure comparing the likelihood of obtaining a true positive versus a false positive result, while LR- is a measure comparing the likelihood of obtaining a false negative versus a true negative result. LR+ and LR- for a particular panel combined with a particular cut-off value in a particular subgroup were calculated as:

$$LR+ \; = \; \frac{\text{sensitivity}}{1 - \text{specificity}}$$

$$LR- \; = \; \frac{1 - \text{sensitivity}}{\text{specificity}}$$

Highest LR+ were observed for CHF subgroups with severely reduced LVEF (subgroup 'ICM LVEF < 35%', followed by 'HFrEF LVEF < 35%'), using any panel selected from panels 8 + NT-proBNP, 9 + NT-proBNP, 10 + NT-proBNP, and 11 + NT-proBNP, combined with any of the cut-off values determined by one of the methods 'Match sensitivity' or 'Match sensitivity + 5%'.

With respect to mild or asymptomatic forms of CHF, highest LR+ were observed for mild or asymptomatic forms of ICM (subgroups 'ICM asymptomatic' and 'ICM LVEF 35% to 50%'), followed by mild or asymptomatic forms of HFrEF (subgroups 'HFrEF asymptomatic' and 'HFrEF LVEF 35% to 50%'), using any panel selected from panels 8 + NT-proBNP, 9 + NT-proBNP, 10 + NT-proBNP, and 11 + NT-proBNP, combined with a cut-off value determined by one of the methods 'Match sensitivity' or 'Match sensitivity + 5%'.

How to calculate PPV and NPV is well known to the person skilled in the art. PPV and NPV indicate the probability that a positive or negative diagnosis, respectively, is correct in a particular subgroup using a particular biomarker panel combined with a particular cut-off value. PPV and NPV depend on the prevalence of the disease in the population. For the performance statistics shown in Table 23, the prevalence of subgroup 'CHF' was assumed to be 10%, and the prevalence of subgroup 'HFrEF' was assumed to be 5%. These estimations are based on the prevalence of heart failure in the Medicare-eligible population in the United States of America, which was in the range between 9% and 12% in the years between 1994 and 2003 (Curtis et al. 2008, Arch. Intern, Med 168, 418-424), and on the assumption that about half of the heart failure cases are HFrEF (Yancy et al. 2013., J. Am. Coll. Cardiol. 62, e147-e239). PPV and NPV were calculated as:

$$PPV = \frac{\text{sensitivity} \cdot \text{prevalence}}{\text{sensitivity} \cdot \text{prevalence} + (1 - \text{specificity}) \cdot (1 - \text{prevalence})}$$

$$NPV = \frac{\text{specificity} \cdot (1 - \text{prevalence})}{\text{specificity} \cdot (1 - \text{prevalence}) + (1 - \text{sensitivity}) \cdot \text{prevalence}}$$

Highest PPV were observed for CHF, using any panel selected from panels 8 + NT-proBNP, 9 + NT-proBNP, 10 + NT-proBNP, and 11 + NT-proBNP, combined with any of the cut-off values determined by one of the methods 'Match sensitivity' or 'Match sensitivity + 5%'.

Table 23: Additional performance statistics calculated on the testing set for NT-proBNP alone and for panels 8 + NT-proBNP, 9 + NT-proBNP, 10 + NT-proBNP, and 11 + NT-proBNP based on the sensitivities and specificities at different cutoff values shown in Table 22.

| Panel number | CHF subgroup | Prevalence | Method | Cutoff | Biomarker panel incl. NT-proBNP | | | | | NT-proBNP alone | | | |
| | | | | | LR+ | LR− | PPV | NPV | | LR+ | LR− | PPV | NPV |
|---|---|---|---|---|---|---|---|---|---|---|---|---|---|
| 8 | CHF | 10% | Match sensitivity | 0.710 | 25.0 | 0.415 | 0.735 | 0.956 | | 5.4 | 0.399 | 0.377 | 0.958 |
| 8 | HFpEF | | Match sensitivity | 0.710 | 12.5 | 0.720 | N/A | N/A | | 3.0 | 0.736 | N/A | N/A |
| 8 | HFrEF | 5% | Match sensitivity | 0.710 | 32.1 | 0.242 | 0.628 | 0.987 | | 6.9 | 0.208 | 0.265 | 0.989 |
| 8 | ICM | | Match sensitivity | 0.710 | 35.4 | 0.160 | N/A | N/A | | 7.5 | 0.124 | N/A | N/A |
| 8 | DCM | | Match sensitivity | 0.710 | 28.8 | 0.321 | N/A | N/A | | 6.3 | 0.288 | N/A | N/A |
| 8 | CHF asymptomatic | | Match sensitivity | 0.710 | 19.9 | 0.540 | N/A | N/A | | 4.6 | 0.513 | N/A | N/A |
| 8 | HFpEF asymptomatic | | Match sensitivity | 0.710 | 10.5 | 0.768 | N/A | N/A | | 2.3 | 0.826 | N/A | N/A |
| 8 | HFrEF asymptomatic | | Match sensitivity | 0.710 | 28.3 | 0.334 | N/A | N/A | | 6.7 | 0.232 | N/A | N/A |
| 8 | ICM asymptomatic | | Match sensitivity | 0.710 | 36.7 | 0.128 | N/A | N/A | | 8.1 | 0.047 | N/A | N/A |
| 8 | DCM asymptomatic | | Match sensitivity | 0.710 | 20.2 | 0.533 | N/A | N/A | | 5.4 | 0.409 | N/A | N/A |
| 8 | CHF symptomatic | | Match sensitivity | 0.710 | 29.2 | 0.311 | N/A | N/A | | 6.2 | 0.304 | N/A | N/A |
| 8 | HFpEF symptomatic | | Match sensitivity | 0.710 | 15.4 | 0.649 | N/A | N/A | | 3.9 | 0.605 | N/A | N/A |
| 8 | HFrEF symptomatic | | Match sensitivity | 0.710 | 34.3 | 0.187 | N/A | N/A | | 7.0 | 0.194 | N/A | N/A |
| 8 | ICM symptomatic | | Match sensitivity | 0.710 | 34.6 | 0.179 | N/A | N/A | | 7.1 | 0.170 | N/A | N/A |
| 8 | DCM symptomatic | | Match sensitivity | 0.710 | 34.0 | 0.195 | N/A | N/A | | 6.8 | 0.216 | N/A | N/A |
| 8 | HFrEF LVEF 35% to 50% | | Match sensitivity | 0.710 | 26.0 | 0.390 | N/A | N/A | | 5.7 | 0.368 | N/A | N/A |
| 8 | ICM LVEF 35% to 50% | | Match sensitivity | 0.710 | 32.0 | 0.244 | N/A | N/A | | 7.0 | 0.189 | N/A | N/A |

| | | | | | | | | | | | | |
|---|---|---|---|---|---|---|---|---|---|---|---|---|
| 8 | DCM LVEF 35% to 50% | | Match sensitivity | 0.710 | 17.4 | 0.601 | N/A | N/A | 3.8 | 0.626 | N/A | N/A |
| 8 | HFrEF LVEF < 35% | | Match sensitivity | 0.710 | 39.2 | 0.068 | N/A | N/A | 8.3 | 0.019 | N/A | N/A |
| 8 | ICM LVEF < 35% | | Match sensitivity | 0.710 | 42.0 | 0.000 | N/A | N/A | 8.4 | 0.000 | N/A | N/A |
| 8 | DCM LVEF < 35% | | Match sensitivity | 0.710 | 37.6 | 0.108 | N/A | N/A | 8.2 | 0.030 | N/A | N/A |
| 9 | CHF | 10% | Match sensitivity | 0.702 | 50.0 | 0.410 | 0.847 | 0.956 | 5.4 | 0.399 | 0.377 | 0.958 |
| 9 | HFpEF | | Match sensitivity | 0.702 | 25.0 | 0.711 | N/A | N/A | 3.0 | 0.736 | N/A | N/A |
| 9 | HFrEF | 5% | Match sensitivity | 0.702 | 64.1 | 0.239 | 0.771 | 0.988 | 6.9 | 0.208 | 0.265 | 0.989 |
| 9 | ICM | | Match sensitivity | 0.702 | 70.9 | 0.158 | N/A | N/A | 7.5 | 0.124 | N/A | N/A |
| 9 | DCM | | Match sensitivity | 0.702 | 57.7 | 0.317 | N/A | N/A | 6.3 | 0.288 | N/A | N/A |
| 9 | CHF asymptomatic | | Match sensitivity | 0.702 | 40.6 | 0.522 | N/A | N/A | 4.6 | 0.513 | N/A | N/A |
| 9 | HFpEF asymptomatic | | Match sensitivity | 0.702 | 21.0 | 0.759 | N/A | N/A | 2.3 | 0.826 | N/A | N/A |
| 9 | HFrEF asymptomatic | | Match sensitivity | 0.702 | 58.3 | 0.310 | N/A | N/A | 6.7 | 0.232 | N/A | N/A |
| 9 | ICM asymptomatic | | Match sensitivity | 0.702 | 73.5 | 0.127 | N/A | N/A | 8.1 | 0.047 | N/A | N/A |
| 9 | DCM asymptomatic | | Match sensitivity | 0.702 | 43.7 | 0.486 | N/A | N/A | 5.4 | 0.409 | N/A | N/A |
| 9 | CHF symptomatic | | Match sensitivity | 0.702 | 57.7 | 0.316 | N/A | N/A | 6.2 | 0.304 | N/A | N/A |
| 9 | HFpEF symptomatic | | Match sensitivity | 0.702 | 30.8 | 0.641 | N/A | N/A | 3.9 | 0.605 | N/A | N/A |
| 9 | HFrEF symptomatic | | Match sensitivity | 0.702 | 67.6 | 0.197 | N/A | N/A | 7.0 | 0.194 | N/A | N/A |
| 9 | ICM symptomatic | | Match sensitivity | 0.702 | 69.3 | 0.177 | N/A | N/A | 7.1 | 0.170 | N/A | N/A |
| 9 | DCM symptomatic | | Match sensitivity | 0.702 | 66.0 | 0.217 | N/A | N/A | 6.8 | 0.216 | N/A | N/A |
| 9 | HFrEF LVEF 35% to 50% | | Match sensitivity | 0.702 | 52.1 | 0.385 | N/A | N/A | 5.7 | 0.368 | N/A | N/A |
| 9 | ICM LVEF 35% to 50% | | Match sensitivity | 0.702 | 64.0 | 0.241 | N/A | N/A | 7.0 | 0.189 | N/A | N/A |
| 9 | DCM LVEF 35% to 50% | | Match sensitivity | 0.702 | 34.8 | 0.593 | N/A | N/A | 3.8 | 0.626 | N/A | N/A |
| 9 | HFrEF LVEF < 35% | | Match sensitivity | 0.702 | 78.4 | 0.067 | N/A | N/A | 8.3 | 0.019 | N/A | N/A |
| 9 | ICM LVEF < 35% | | Match sensitivity | 0.702 | 84.0 | 0.000 | N/A | N/A | 8.4 | 0.000 | N/A | N/A |
| 9 | DCM LVEF < 35% | | Match sensitivity | 0.702 | 75.2 | 0.107 | N/A | N/A | 8.2 | 0.030 | N/A | N/A |

| | | | | | | | | | | | | |
|---|---|---|---|---|---|---|---|---|---|---|---|---|
| 10 | CHF | 10% | Match sensitivity | 0.715 | 25.0 | 0.415 | 0.735 | 0.956 | 5.4 | 0.399 | 0.377 | 0.958 |
| 10 | HFpEF | | Match sensitivity | 0.715 | 12.5 | 0.720 | N/A | N/A | 3.0 | 0.736 | N/A | N/A |
| 10 | HFrEF | 5% | Match sensitivity | 0.715 | 32.1 | 0.242 | 0.628 | 0.987 | 6.9 | 0.208 | 0.265 | 0.989 |
| 10 | ICM | | Match sensitivity | 0.715 | 35.4 | 0.160 | N/A | N/A | 7.5 | 0.124 | N/A | N/A |
| 10 | DCM | | Match sensitivity | 0.715 | 28.8 | 0.321 | N/A | N/A | 6.3 | 0.288 | N/A | N/A |
| 10 | CHF asymptomatic | | Match sensitivity | 0.715 | 19.9 | 0.540 | N/A | N/A | 4.6 | 0.513 | N/A | N/A |
| 10 | HFpEF asymptomatic | | Match sensitivity | 0.715 | 10.5 | 0.768 | N/A | N/A | 2.3 | 0.826 | N/A | N/A |
| 10 | HFrEF asymptomatic | | Match sensitivity | 0.715 | 28.3 | 0.334 | N/A | N/A | 6.7 | 0.232 | N/A | N/A |
| 10 | ICM asymptomatic | | Match sensitivity | 0.715 | 36.7 | 0.128 | N/A | N/A | 8.1 | 0.047 | N/A | N/A |
| 10 | DCM asymptomatic | | Match sensitivity | 0.715 | 20.2 | 0.533 | N/A | N/A | 5.4 | 0.409 | N/A | N/A |
| 10 | CHF symptomatic | | Match sensitivity | 0.715 | 29.2 | 0.311 | N/A | N/A | 6.2 | 0.304 | N/A | N/A |
| 10 | HFpEF symptomatic | | Match sensitivity | 0.715 | 15.4 | 0.649 | N/A | N/A | 3.9 | 0.605 | N/A | N/A |
| 10 | HFrEF symptomatic | | Match sensitivity | 0.715 | 34.3 | 0.187 | N/A | N/A | 7.0 | 0.194 | N/A | N/A |
| 10 | ICM symptomatic | | Match sensitivity | 0.715 | 34.6 | 0.179 | N/A | N/A | 7.1 | 0.170 | N/A | N/A |
| 10 | DCM symptomatic | | Match sensitivity | 0.715 | 34.0 | 0.195 | N/A | N/A | 6.8 | 0.216 | N/A | N/A |
| 10 | HFrEF LVEF 35% to 50% | | Match sensitivity | 0.715 | 26.0 | 0.390 | N/A | N/A | 5.7 | 0.368 | N/A | N/A |
| 10 | ICM LVEF 35% to 50% | | Match sensitivity | 0.715 | 32.0 | 0.244 | N/A | N/A | 7.0 | 0.189 | N/A | N/A |
| 10 | DCM LVEF 35% to 50% | | Match sensitivity | 0.715 | 17.4 | 0.601 | N/A | N/A | 3.8 | 0.626 | N/A | N/A |
| 10 | HFrEF LVEF < 35% | | Match sensitivity | 0.715 | 39.2 | 0.068 | N/A | N/A | 8.3 | 0.019 | N/A | N/A |
| 10 | ICM LVEF < 35% | | Match sensitivity | 0.715 | 42.0 | 0.000 | N/A | N/A | 8.4 | 0.000 | N/A | N/A |
| 10 | DCM LVEF < 35% | | Match sensitivity | 0.715 | 37.6 | 0.108 | N/A | N/A | 8.2 | 0.030 | N/A | N/A |
| 11 | CHF | 10% | Match sensitivity | 0.708 | 47.5 | 0.439 | 0.841 | 0.953 | 5.4 | 0.399 | 0.377 | 0.958 |
| 11 | HFpEF | | Match sensitivity | 0.708 | 21.6 | 0.752 | N/A | N/A | 3.0 | 0.736 | N/A | N/A |
| 11 | HFrEF | 5% | Match sensitivity | 0.708 | 62.2 | 0.263 | 0.766 | 0.986 | 6.9 | 0.208 | 0.265 | 0.989 |
| 11 | ICM | | Match sensitivity | 0.708 | 68.2 | 0.190 | N/A | N/A | 7.5 | 0.124 | N/A | N/A |

EP 3 194 959 B1

84

| | | | | | | | | | | | | |
|---|---|---|---|---|---|---|---|---|---|---|---|---|
| 11 | DCM | | Match sensitivity | 0.708 | 56.4 | 0.332 | N/A | N/A | 6.3 | 0.288 | N/A | N/A |
| 11 | CHF asymptomatic | | Match sensitivity | 0.708 | 40.6 | 0.522 | N/A | N/A | 4.6 | 0.513 | N/A | N/A |
| 11 | HFpEF asymptomatic | | Match sensitivity | 0.708 | 22.9 | 0.736 | N/A | N/A | 2.3 | 0.826 | N/A | N/A |
| 11 | HFrEF asymptomatic | | Match sensitivity | 0.708 | 56.6 | 0.330 | N/A | N/A | 6.7 | 0.232 | N/A | N/A |
| 11 | ICM asymptomatic | | Match sensitivity | 0.708 | 73.5 | 0.127 | N/A | N/A | 8.1 | 0.047 | N/A | N/A |
| 11 | DCM asymptomatic | | Match sensitivity | 0.708 | 40.3 | 0.526 | N/A | N/A | 5.4 | 0.409 | N/A | N/A |
| 11 | CHF symptomatic | | Match sensitivity | 0.708 | 53.2 | 0.370 | N/A | N/A | 6.2 | 0.304 | N/A | N/A |
| 11 | HFpEF symptomatic | | Match sensitivity | 0.708 | 19.6 | 0.776 | N/A | N/A | 3.9 | 0.605 | N/A | N/A |
| 11 | HFrEF symptomatic | | Match sensitivity | 0.708 | 65.6 | 0.222 | N/A | N/A | 7.0 | 0.194 | N/A | N/A |
| 11 | ICM symptomatic | | Match sensitivity | 0.708 | 65.1 | 0.228 | N/A | N/A | 7.1 | 0.170 | N/A | N/A |
| 11 | DCM symptomatic | | Match sensitivity | 0.708 | 66.0 | 0.217 | N/A | N/A | 6.8 | 0.216 | N/A | N/A |
| 11 | HFrEF LVEF 35% to 50% | | Match sensitivity | 0.708 | 47.3 | 0.442 | N/A | N/A | 5.7 | 0.368 | N/A | N/A |
| 11 | ICM LVEF 35% to 50% | | Match sensitivity | 0.708 | 60.0 | 0.289 | N/A | N/A | 7.0 | 0.189 | N/A | N/A |
| 11 | DCM LVEF 35% to 50% | | Match sensitivity | 0.708 | 29.0 | 0.663 | N/A | N/A | 3.8 | 0.626 | N/A | N/A |
| 11 | HFrEF LVEF < 35% | | Match sensitivity | 0.708 | 79.8 | 0.051 | N/A | N/A | 8.3 | 0.019 | N/A | N/A |
| 11 | ICM LVEF < 35% | | Match sensitivity | 0.708 | 84.0 | 0.000 | N/A | N/A | 8.4 | 0.000 | N/A | N/A |
| 11 | DCM LVEF < 35% | | Match sensitivity | 0.708 | 77.4 | 0.080 | N/A | N/A | 8.2 | 0.030 | N/A | N/A |
| 8 | CHF | 10% | Match sensitivity + 5% | 0.633 | 13.8 | 0.359 | 0.606 | 0.962 | 5.4 | 0.399 | 0.377 | 0.958 |
| 8 | HFpEF | | Match sensitivity + 5% | 0.633 | 7.4 | 0.681 | N/A | N/A | 3.0 | 0.736 | N/A | N/A |
| 8 | HFrEF | 5% | Match sensitivity + 5% | 0.633 | 17.5 | 0.176 | 0.479 | 0.991 | 6.9 | 0.208 | 0.265 | 0.989 |
| 8 | ICM | | Match sensitivity + 5% | 0.633 | 19.0 | 0.098 | N/A | N/A | 7.5 | 0.124 | N/A | N/A |
| 8 | DCM | | Match sensitivity + 5% | 0.633 | 16.0 | 0.251 | N/A | N/A | 6.3 | 0.288 | N/A | N/A |
| 8 | CHF asymptomatic | | Match sensitivity + 5% | 0.633 | 11.3 | 0.485 | N/A | N/A | 4.6 | 0.513 | N/A | N/A |
| 8 | HFpEF asymptomatic | | Match sensitivity + 5% | 0.633 | 6.7 | 0.716 | N/A | N/A | 2.3 | 0.826 | N/A | N/A |
| 8 | HFrEF asymptomatic | | Match sensitivity + 5% | 0.633 | 15.4 | 0.279 | N/A | N/A | 6.7 | 0.232 | N/A | N/A |

| | | | | | | | | | | | | |
|---|---|---|---|---|---|---|---|---|---|---|---|---|
| 8 | ICM asymptomatic | | Match sensitivity + 5% | 0.633 | 19.2 | 0.087 | N/A | N/A | 8.1 | 0.047 | N/A | N/A |
| 8 | DCM asymptomatic | | Match sensitivity + 5% | 0.633 | 11.8 | 0.462 | N/A | N/A | 5.4 | 0.409 | N/A | N/A |
| 8 | CHF symptomatic | | Match sensitivity + 5% | 0.633 | 15.9 | 0.253 | N/A | N/A | 6.2 | 0.304 | N/A | N/A |
| 8 | HFpEF symptomatic | | Match sensitivity + 5% | 0.633 | 8.4 | 0.630 | N/A | N/A | 3.9 | 0.605 | N/A | N/A |
| 8 | HFrEF symptomatic | | Match sensitivity + 5% | 0.633 | 18.7 | 0.115 | N/A | N/A | 7.0 | 0.194 | N/A | N/A |
| 8 | ICM symptomatic | | Match sensitivity + 5% | 0.633 | 18.9 | 0.105 | N/A | N/A | 7.1 | 0.170 | N/A | N/A |
| 8 | DCM symptomatic | | Match sensitivity + 5% | 0.633 | 18.5 | 0.125 | N/A | N/A | 6.8 | 0.216 | N/A | N/A |
| 8 | HFrEF LVEF 35% to 50% | | Match sensitivity + 5% | 0.633 | 15.1 | 0.296 | N/A | N/A | 5.7 | 0.368 | N/A | N/A |
| 8 | ICM LVEF 35% to 50% | | Match sensitivity + 5% | 0.633 | 18.0 | 0.150 | N/A | N/A | 7.0 | 0.189 | N/A | N/A |
| 8 | DCM LVEF 35% to 50% | | Match sensitivity + 5% | 0.633 | 10.9 | 0.507 | N/A | N/A | 3.8 | 0.626 | N/A | N/A |
| 8 | HFrEF LVEF < 35% | | Match sensitivity + 5% | 0.633 | 20.3 | 0.035 | N/A | N/A | 8.3 | 0.019 | N/A | N/A |
| 8 | ICM LVEF < 35% | | Match sensitivity + 5% | 0.633 | 21.0 | 0.000 | N/A | N/A | 8.4 | 0.000 | N/A | N/A |
| 8 | DCM LVEF < 35% | | Match sensitivity + 5% | 0.633 | 19.9 | 0.055 | N/A | N/A | 8.2 | 0.030 | N/A | N/A |
| 9 | CHF | 10% | Match sensitivity + 5% | 0.625 | 13.8 | 0.359 | 0.606 | 0.962 | 5.4 | 0.399 | 0.377 | 0.958 |
| 9 | HFpEF | | Match sensitivity + 5% | 0.625 | 7.4 | 0.681 | N/A | N/A | 3.0 | 0.736 | N/A | N/A |
| 9 | HFrEF | 5% | Match sensitivity + 5% | 0.625 | 17.5 | 0.176 | 0.479 | 0.991 | 6.9 | 0.208 | 0.265 | 0.989 |
| 9 | ICM | | Match sensitivity + 5% | 0.625 | 19.0 | 0.098 | N/A | N/A | 7.5 | 0.124 | N/A | N/A |
| 9 | DCM | | Match sensitivity + 5% | 0.625 | 16.0 | 0.251 | N/A | N/A | 6.3 | 0.288 | N/A | N/A |
| 9 | CHF asymptomatic | | Match sensitivity + 5% | 0.625 | 11.3 | 0.485 | N/A | N/A | 4.6 | 0.513 | N/A | N/A |
| 9 | HFpEF asymptomatic | | Match sensitivity + 5% | 0.625 | 6.7 | 0.716 | N/A | N/A | 2.3 | 0.826 | N/A | N/A |
| 9 | HFrEF asymptomatic | | Match sensitivity + 5% | 0.625 | 15.4 | 0.279 | N/A | N/A | 6.7 | 0.232 | N/A | N/A |
| 9 | ICM asymptomatic | | Match sensitivity + 5% | 0.625 | 19.2 | 0.087 | N/A | N/A | 8.1 | 0.047 | N/A | N/A |
| 9 | DCM asymptomatic | | Match sensitivity + 5% | 0.625 | 11.8 | 0.462 | N/A | N/A | 5.4 | 0.409 | N/A | N/A |
| 9 | CHF symptomatic | | Match sensitivity + 5% | 0.625 | 15.9 | 0.253 | N/A | N/A | 6.2 | 0.304 | N/A | N/A |
| 9 | HFpEF symptomatic | | Match sensitivity + 5% | 0.625 | 8.4 | 0.630 | N/A | N/A | 3.9 | 0.605 | N/A | N/A |

| | | | | 0.625 | 18.7 | 0.115 | N/A | N/A | 7.0 | 0.194 | N/A | N/A |
|---|---|---|---|---|---|---|---|---|---|---|---|---|
| 9 | HFrEF symptomatic | | Match sensitivity + 5% | 0.625 | 18.7 | 0.115 | N/A | N/A | 7.0 | 0.194 | N/A | N/A |
| 9 | ICM symptomatic | | Match sensitivity + 5% | 0.625 | 18.9 | 0.105 | N/A | N/A | 7.1 | 0.170 | N/A | N/A |
| 9 | DCM symptomatic | | Match sensitivity + 5% | 0.625 | 18.5 | 0.125 | N/A | N/A | 6.8 | 0.216 | N/A | N/A |
| 9 | HFrEF LVEF 35% to 50% | | Match sensitivity + 5% | 0.625 | 15.1 | 0.296 | N/A | N/A | 5.7 | 0.368 | N/A | N/A |
| 9 | ICM LVEF 35% to 50% | | Match sensitivity + 5% | 0.625 | 18.0 | 0.150 | N/A | N/A | 7.0 | 0.189 | N/A | N/A |
| 9 | DCM LVEF 35% to 50% | | Match sensitivity + 5% | 0.625 | 10.9 | 0.507 | N/A | N/A | 3.8 | 0.626 | N/A | N/A |
| 9 | HFrEF LVEF < 35% | | Match sensitivity + 5% | 0.625 | 20.3 | 0.035 | N/A | N/A | 8.3 | 0.019 | N/A | N/A |
| 9 | ICM LVEF < 35% | | Match sensitivity + 5% | 0.625 | 21.0 | 0.000 | N/A | N/A | 8.4 | 0.000 | N/A | N/A |
| 9 | DCM LVEF < 35% | | Match sensitivity + 5% | 0.625 | 19.9 | 0.055 | N/A | N/A | 8.2 | 0.030 | N/A | N/A |
| 10 | CHF | 10% | Match sensitivity + 5% | 0.641 | 13.4 | 0.379 | 0.599 | 0.960 | 5.4 | 0.399 | 0.377 | 0.958 |
| 10 | HFpEF | | Match sensitivity + 5% | 0.641 | 6.8 | 0.709 | N/A | N/A | 3.0 | 0.736 | N/A | N/A |
| 10 | HFrEF | 5% | Match sensitivity + 5% | 0.641 | 17.2 | 0.192 | 0.474 | 0.990 | 6.9 | 0.208 | 0.265 | 0.989 |
| 10 | ICM | | Match sensitivity + 5% | 0.641 | 18.7 | 0.115 | N/A | N/A | 7.5 | 0.124 | N/A | N/A |
| 10 | DCM | | Match sensitivity + 5% | 0.641 | 15.7 | 0.266 | N/A | N/A | 6.3 | 0.288 | N/A | N/A |
| 10 | CHF asymptomatic | | Match sensitivity + 5% | 0.641 | 10.6 | 0.519 | N/A | N/A | 4.6 | 0.513 | N/A | N/A |
| 10 | HFpEF asymptomatic | | Match sensitivity + 5% | 0.641 | 5.7 | 0.764 | N/A | N/A | 2.3 | 0.826 | N/A | N/A |
| 10 | HFrEF asymptomatic | | Match sensitivity + 5% | 0.641 | 15.0 | 0.300 | N/A | N/A | 6.7 | 0.232 | N/A | N/A |
| 10 | ICM asymptomatic | | Match sensitivity + 5% | 0.641 | 19.2 | 0.087 | N/A | N/A | 8.1 | 0.047 | N/A | N/A |
| 10 | DCM asymptomatic | | Match sensitivity + 5% | 0.641 | 10.9 | 0.504 | N/A | N/A | 5.4 | 0.409 | N/A | N/A |
| 10 | CHF symptomatic | | Match sensitivity + 5% | 0.641 | 15.7 | 0.263 | N/A | N/A | 6.2 | 0.304 | N/A | N/A |
| 10 | HFpEF symptomatic | | Match sensitivity + 5% | 0.641 | 8.4 | 0.630 | N/A | N/A | 3.9 | 0.605 | N/A | N/A |
| 10 | HFrEF symptomatic | | Match sensitivity + 5% | 0.641 | 18.4 | 0.128 | N/A | N/A | 7.0 | 0.194 | N/A | N/A |
| 10 | ICM symptomatic | | Match sensitivity + 5% | 0.641 | 18.4 | 0.131 | N/A | N/A | 7.1 | 0.170 | N/A | N/A |
| 10 | DCM symptomatic | | Match sensitivity + 5% | 0.641 | 18.5 | 0.125 | N/A | N/A | 6.8 | 0.216 | N/A | N/A |
| 10 | HFrEF LVEF 35% to 50% | | Match sensitivity + 5% | 0.641 | 14.5 | 0.325 | N/A | N/A | 5.7 | 0.368 | N/A | N/A |

| 10 | ICM LVEF 35% to 50% | | Match sensitivity + 5% | 0.641 | 17.5 | 0.175 | N/A | N/A | 7.0 | 0.189 | N/A | N/A |
|---|---|---|---|---|---|---|---|---|---|---|---|---|
| 10 | DCM LVEF 35% to 50% | | Match sensitivity + 5% | 0.641 | 10.1 | 0.543 | N/A | N/A | 3.8 | 0.626 | N/A | N/A |
| 10 | HFrEF LVEF < 35% | | Match sensitivity + 5% | 0.641 | 20.3 | 0.035 | N/A | N/A | 8.3 | 0.019 | N/A | N/A |
| 10 | ICM LVEF < 35% | | Match sensitivity + 5% | 0.641 | 21.0 | 0.000 | N/A | N/A | 8.4 | 0.000 | N/A | N/A |
| 10 | DCM LVEF < 35% | | Match sensitivity + 5% | 0.641 | 19.9 | 0.055 | N/A | N/A | 8.2 | 0.030 | N/A | N/A |
| 11 | CHF | 10% | Match sensitivity + 5% | 0.612 | 18.0 | 0.369 | 0.667 | 0.961 | 5.4 | 0.399 | 0.377 | 0.958 |
| 11 | HFpEF | | Match sensitivity + 5% | 0.612 | 9.1 | 0.701 | N/A | N/A | 3.0 | 0.736 | N/A | N/A |
| 11 | HFrEF | 5% | Match sensitivity + 5% | 0.612 | 23.1 | 0.182 | 0.549 | 0.991 | 6.9 | 0.208 | 0.265 | 0.989 |
| 11 | ICM | | Match sensitivity + 5% | 0.612 | 25.4 | 0.097 | N/A | N/A | 7.5 | 0.124 | N/A | N/A |
| 11 | DCM | | Match sensitivity + 5% | 0.612 | 20.9 | 0.263 | N/A | N/A | 6.3 | 0.288 | N/A | N/A |
| 11 | CHF asymptomatic | | Match sensitivity + 5% | 0.612 | 14.2 | 0.513 | N/A | N/A | 4.6 | 0.513 | N/A | N/A |
| 11 | HFpEF asymptomatic | | Match sensitivity + 5% | 0.612 | 7.6 | 0.754 | N/A | N/A | 2.3 | 0.826 | N/A | N/A |
| 11 | HFrEF asymptomatic | | Match sensitivity + 5% | 0.612 | 20.0 | 0.296 | N/A | N/A | 6.7 | 0.232 | N/A | N/A |
| 11 | ICM asymptomatic | | Match sensitivity + 5% | 0.612 | 24.5 | 0.130 | N/A | N/A | 8.1 | 0.047 | N/A | N/A |
| 11 | DCM asymptomatic | | Match sensitivity + 5% | 0.612 | 15.7 | 0.456 | N/A | N/A | 5.4 | 0.409 | N/A | N/A |
| 11 | CHF symptomatic | | Match sensitivity + 5% | 0.612 | 21.2 | 0.250 | N/A | N/A | 6.2 | 0.304 | N/A | N/A |
| 11 | HFpEF symptomatic | | Match sensitivity + 5% | 0.612 | 11.2 | 0.622 | N/A | N/A | 3.9 | 0.605 | N/A | N/A |
| 11 | HFrEF symptomatic | | Match sensitivity + 5% | 0.612 | 24.9 | 0.114 | N/A | N/A | 7.0 | 0.194 | N/A | N/A |
| 11 | ICM symptomatic | | Match sensitivity + 5% | 0.612 | 25.9 | 0.078 | N/A | N/A | 7.1 | 0.170 | N/A | N/A |
| 11 | DCM symptomatic | | Match sensitivity + 5% | 0.612 | 24.0 | 0.148 | N/A | N/A | 6.8 | 0.216 | N/A | N/A |
| 11 | HFrEF LVEF 35% to 50% | | Match sensitivity + 5% | 0.612 | 20.1 | 0.292 | N/A | N/A | 5.7 | 0.368 | N/A | N/A |
| 11 | ICM LVEF 35% to 50% | | Match sensitivity + 5% | 0.612 | 24.0 | 0.148 | N/A | N/A | 7.0 | 0.189 | N/A | N/A |
| 11 | DCM LVEF 35% to 50% | | Match sensitivity + 5% | 0.612 | 14.5 | 0.501 | N/A | N/A | 3.8 | 0.626 | N/A | N/A |
| 11 | HFrEF LVEF < 35% | | Match sensitivity + 5% | 0.612 | 26.6 | 0.052 | N/A | N/A | 8.3 | 0.019 | N/A | N/A |
| 11 | ICM LVEF < 35% | | Match sensitivity + 5% | 0.612 | 28.0 | 0.000 | N/A | N/A | 8.4 | 0.000 | N/A | N/A |

| | | | | 0.612 | 25.8 | 0.082 | N/A | N/A | 8.2 | 0.030 | N/A | N/A |
|---|---|---|---|---|---|---|---|---|---|---|---|---|
| 11 | DCM LVEF < 35% | | Match sensitivity + 5% | 0.612 | 25.8 | 0.082 | N/A | N/A | 8.2 | 0.030 | N/A | N/A |
| 8 | CHF | 10% | Maximize Youden's index | 0.535 | 7.6 | 0.307 | 0.457 | 0.967 | 5.4 | 0.399 | 0.377 | 0.958 |
| 8 | HFpEF | | Maximize Youden's index | 0.535 | 4.8 | 0.597 | N/A | N/A | 3.0 | 0.736 | N/A | N/A |
| 8 | HFrEF | 5% | Maximize Youden's index | 0.535 | 9.1 | 0.143 | 0.325 | 0.993 | 6.9 | 0.208 | 0.265 | 0.989 |
| 8 | ICM | | Maximize Youden's index | 0.535 | 10.2 | 0.035 | N/A | N/A | 7.5 | 0.124 | N/A | N/A |
| 8 | DCM | | Maximize Youden's index | 0.535 | 8.1 | 0.247 | N/A | N/A | 6.3 | 0.288 | N/A | N/A |
| 8 | CHF asymptomatic | | Maximize Youden's index | 0.535 | 6.4 | 0.428 | N/A | N/A | 4.6 | 0.513 | N/A | N/A |
| 8 | HFpEF asymptomatic | | Maximize Youden's index | 0.535 | 4.8 | 0.603 | N/A | N/A | 2.3 | 0.826 | N/A | N/A |
| 8 | HFrEF asymptomatic | | Maximize Youden's index | 0.535 | 7.9 | 0.271 | N/A | N/A | 6.7 | 0.232 | N/A | N/A |
| 8 | ICM asymptomatic | | Maximize Youden's index | 0.535 | 9.6 | 0.092 | N/A | N/A | 8.1 | 0.047 | N/A | N/A |
| 8 | DCM asymptomatic | | Maximize Youden's index | 0.535 | 6.3 | 0.442 | N/A | N/A | 5.4 | 0.409 | N/A | N/A |
| 8 | CHF symptomatic | | Maximize Youden's index | 0.535 | 8.5 | 0.207 | N/A | N/A | 6.2 | 0.304 | N/A | N/A |
| 8 | HFpEF symptomatic | | Maximize Youden's index | 0.535 | 4.9 | 0.589 | N/A | N/A | 3.9 | 0.605 | N/A | N/A |
| 8 | HFrEF symptomatic | | Maximize Youden's index | 0.535 | 9.9 | 0.067 | N/A | N/A | 7.0 | 0.194 | N/A | N/A |
| 8 | ICM symptomatic | | Maximize Youden's index | 0.535 | 10.5 | 0.000 | N/A | N/A | 7.1 | 0.170 | N/A | N/A |
| 8 | DCM symptomatic | | Maximize Youden's index | 0.535 | 9.3 | 0.132 | N/A | N/A | 6.8 | 0.216 | N/A | N/A |
| 8 | HFrEF LVEF 35% to 50% | | Maximize Youden's index | 0.535 | 8.1 | 0.249 | N/A | N/A | 5.7 | 0.368 | N/A | N/A |
| 8 | ICM LVEF 35% to 50% | | Maximize Youden's index | 0.535 | 10.0 | 0.053 | N/A | N/A | 7.0 | 0.189 | N/A | N/A |
| 8 | DCM LVEF 35% to 50% | | Maximize Youden's index | 0.535 | 5.4 | 0.534 | N/A | N/A | 3.8 | 0.626 | N/A | N/A |
| 8 | HFrEF LVEF < 35% | | Maximize Youden's index | 0.535 | 10.3 | 0.018 | N/A | N/A | 8.3 | 0.019 | N/A | N/A |
| 8 | ICM LVEF < 35% | | Maximize Youden's index | 0.535 | 10.5 | 0.000 | N/A | N/A | 8.4 | 0.000 | N/A | N/A |
| 8 | DCM LVEF < 35% | | Maximize Youden's index | 0.535 | 10.2 | 0.029 | N/A | N/A | 8.2 | 0.030 | N/A | N/A |
| 9 | CHF | 10% | Maximize Youden's index | 0.494 | 6.9 | 0.290 | 0.435 | 0.969 | 5.4 | 0.399 | 0.377 | 0.958 |
| 9 | HFpEF | | Maximize Youden's index | 0.494 | 4.7 | 0.560 | N/A | N/A | 3.0 | 0.736 | N/A | N/A |
| 9 | HFrEF | 5% | Maximize Youden's index | 0.494 | 8.2 | 0.137 | 0.301 | 0.993 | 6.9 | 0.208 | 0.265 | 0.989 |

| 9 | ICM | | Maximize Youden's index | 0.494 | 9.0 | 0.035 | N/A | N/A | 7.5 | 0.124 | N/A | N/A |
|---|---|---|---|---|---|---|---|---|---|---|---|---|
| 9 | DCM | | Maximize Youden's index | 0.494 | 7.4 | 0.234 | N/A | N/A | 6.3 | 0.288 | N/A | N/A |
| 9 | CHF asymptomatic | | Maximize Youden's index | 0.494 | 5.9 | 0.409 | N/A | N/A | 4.6 | 0.513 | N/A | N/A |
| 9 | HFpEF asymptomatic | | Maximize Youden's index | 0.494 | 4.5 | 0.585 | N/A | N/A | 2.3 | 0.826 | N/A | N/A |
| 9 | HFrEF asymptomatic | | Maximize Youden's index | 0.494 | 7.2 | 0.251 | N/A | N/A | 6.7 | 0.232 | N/A | N/A |
| 9 | ICM asymptomatic | | Maximize Youden's index | 0.494 | 8.6 | 0.093 | N/A | N/A | 8.1 | 0.047 | N/A | N/A |
| 9 | DCM asymptomatic | | Maximize Youden's index | 0.494 | 6.0 | 0.403 | N/A | N/A | 5.4 | 0.409 | N/A | N/A |
| 9 | CHF symptomatic | | Maximize Youden's index | 0.494 | 7.8 | 0.190 | N/A | N/A | 6.2 | 0.304 | N/A | N/A |
| 9 | HFpEF symptomatic | | Maximize Youden's index | 0.494 | 5.0 | 0.523 | N/A | N/A | 3.9 | 0.605 | N/A | N/A |
| 9 | HFrEF symptomatic | | Maximize Youden's index | 0.494 | 8.8 | 0.068 | N/A | N/A | 7.0 | 0.194 | N/A | N/A |
| 9 | ICM symptomatic | | Maximize Youden's index | 0.494 | 9.3 | 0.000 | N/A | N/A | 7.1 | 0.170 | N/A | N/A |
| 9 | DCM symptomatic | | Maximize Youden's index | 0.494 | 8.2 | 0.133 | N/A | N/A | 6.8 | 0.216 | N/A | N/A |
| 9 | HFrEF LVEF 35% to 50% | | Maximize Youden's index | 0.494 | 7.4 | 0.237 | N/A | N/A | 5.7 | 0.368 | N/A | N/A |
| 9 | ICM LVEF 35% to 50% | | Maximize Youden's index | 0.494 | 8.9 | 0.053 | N/A | N/A | 7.0 | 0.189 | N/A | N/A |
| 9 | DCM LVEF 35% to 50% | | Maximize Youden's index | 0.494 | 5.1 | 0.502 | N/A | N/A | 3.8 | 0.626 | N/A | N/A |
| 9 | HFrEF LVEF < 35% | | Maximize Youden's index | 0.494 | 9.2 | 0.019 | N/A | N/A | 8.3 | 0.019 | N/A | N/A |
| 9 | ICM LVEF < 35% | | Maximize Youden's index | 0.494 | 9.3 | 0.000 | N/A | N/A | 8.4 | 0.000 | N/A | N/A |
| 9 | DCM LVEF < 35% | | Maximize Youden's index | 0.494 | 9.1 | 0.029 | N/A | N/A | 8.2 | 0.030 | N/A | N/A |
| 10 | CHF | 10% | Maximize Youden's index | 0.505 | 6.9 | 0.295 | 0.433 | 0.968 | 5.4 | 0.399 | 0.377 | 0.958 |
| 10 | HFpEF | | Maximize Youden's index | 0.505 | 4.4 | 0.590 | N/A | N/A | 3.0 | 0.736 | N/A | N/A |
| 10 | HFrEF | 5% | Maximize Youden's index | 0.505 | 8.3 | 0.128 | 0.303 | 0.993 | 6.9 | 0.208 | 0.265 | 0.989 |
| 10 | ICM | | Maximize Youden's index | 0.505 | 9.0 | 0.035 | N/A | N/A | 7.5 | 0.124 | N/A | N/A |
| 10 | DCM | | Maximize Youden's index | 0.505 | 7.5 | 0.217 | N/A | N/A | 6.3 | 0.288 | N/A | N/A |
| 10 | CHF asymptomatic | | Maximize Youden's index | 0.505 | 5.9 | 0.409 | N/A | N/A | 4.6 | 0.513 | N/A | N/A |
| 10 | HFpEF asymptomatic | | Maximize Youden's index | 0.505 | 4.2 | 0.611 | N/A | N/A | 2.3 | 0.826 | N/A | N/A |

| 10 | HFrEF asymptomatic | | Maximize Youden's index | 0.505 | 7.4 | 0.229 | N/A | N/A | 6.7 | 0.232 | N/A | N/A |
|---|---|---|---|---|---|---|---|---|---|---|---|---|
| 10 | ICM asymptomatic | | Maximize Youden's index | 0.505 | 8.6 | 0.093 | N/A | N/A | 8.1 | 0.047 | N/A | N/A |
| 10 | DCM asymptomatic | | Maximize Youden's index | 0.505 | 6.3 | 0.358 | N/A | N/A | 5.4 | 0.409 | N/A | N/A |
| 10 | CHF symptomatic | | Maximize Youden's index | 0.505 | 7.7 | 0.200 | N/A | N/A | 6.2 | 0.304 | N/A | N/A |
| 10 | HFpEF symptomatic | | Maximize Youden's index | 0.505 | 4.7 | 0.560 | N/A | N/A | 3.9 | 0.605 | N/A | N/A |
| 10 | HFrEF symptomatic | | Maximize Youden's index | 0.505 | 8.8 | 0.068 | N/A | N/A | 7.0 | 0.194 | N/A | N/A |
| 10 | ICM symptomatic | | Maximize Youden's index | 0.505 | 9.3 | 0.000 | N/A | N/A | 7.1 | 0.170 | N/A | N/A |
| 10 | DCM symptomatic | | Maximize Youden's index | 0.505 | 8.2 | 0.133 | N/A | N/A | 6.8 | 0.216 | N/A | N/A |
| 10 | HFrEF LVEF 35% to 50% | | Maximize Youden's index | 0.505 | 7.5 | 0.221 | N/A | N/A | 5.7 | 0.368 | N/A | N/A |
| 10 | ICM LVEF 35% to 50% | | Maximize Youden's index | 0.505 | 8.9 | 0.053 | N/A | N/A | 7.0 | 0.189 | N/A | N/A |
| 10 | DCM LVEF 35% to 50% | | Maximize Youden's index | 0.505 | 5.5 | 0.463 | N/A | N/A | 3.8 | 0.626 | N/A | N/A |
| 10 | HFrEF LVEF < 35% | | Maximize Youden's index | 0.505 | 9.2 | 0.019 | N/A | N/A | 8.3 | 0.019 | N/A | N/A |
| 10 | ICM LVEF < 35% | | Maximize Youden's index | 0.505 | 9.3 | 0.000 | N/A | N/A | 8.4 | 0.000 | N/A | N/A |
| 10 | DCM LVEF < 35% | | Maximize Youden's index | 0.505 | 9.1 | 0.029 | N/A | N/A | 8.2 | 0.030 | N/A | N/A |
| 11 | CHF | 10% | Maximize Youden's index | 0.548 | 6.5 | 0.339 | 0.420 | 0.964 | 5.4 | 0.399 | 0.377 | 0.958 |
| 11 | HFpEF | | Maximize Youden's index | 0.548 | 4.0 | 0.636 | N/A | N/A | 3.0 | 0.736 | N/A | N/A |
| 11 | HFrEF | 5% | Maximize Youden's index | 0.548 | 7.9 | 0.171 | 0.294 | 0.991 | 6.9 | 0.208 | 0.265 | 0.989 |
| 11 | ICM | | Maximize Youden's index | 0.548 | 8.9 | 0.053 | N/A | N/A | 7.5 | 0.124 | N/A | N/A |
| 11 | DCM | | Maximize Youden's index | 0.548 | 7.0 | 0.284 | N/A | N/A | 6.3 | 0.288 | N/A | N/A |
| 11 | CHF asymptomatic | | Maximize Youden's index | 0.548 | 5.3 | 0.482 | N/A | N/A | 4.6 | 0.513 | N/A | N/A |
| 11 | HFpEF asymptomatic | | Maximize Youden's index | 0.548 | 3.8 | 0.662 | N/A | N/A | 2.3 | 0.826 | N/A | N/A |
| 11 | HFrEF asymptomatic | | Maximize Youden's index | 0.548 | 6.7 | 0.320 | N/A | N/A | 6.7 | 0.232 | N/A | N/A |
| 11 | ICM asymptomatic | | Maximize Youden's index | 0.548 | 8.2 | 0.140 | N/A | N/A | 8.1 | 0.047 | N/A | N/A |
| 11 | DCM asymptomatic | | Maximize Youden's index | 0.548 | 5.2 | 0.493 | N/A | N/A | 5.4 | 0.409 | N/A | N/A |
| 11 | CHF symptomatic | | Maximize Youden's index | 0.548 | 7.5 | 0.220 | N/A | N/A | 6.2 | 0.304 | N/A | N/A |

| 11 | HFpEF symptomatic | | Maximize Youden's index | 0.548 | 4.4 | 0.597 | N/A | N/A | 3.9 | 0.605 | N/A | N/A |
| 11 | HFrEF symptomatic | | Maximize Youden's index | 0.548 | 8.7 | 0.082 | N/A | N/A | 7.0 | 0.194 | N/A | N/A |
| 11 | ICM symptomatic | | Maximize Youden's index | 0.548 | 9.3 | 0.000 | N/A | N/A | 7.1 | 0.170 | N/A | N/A |
| 11 | DCM symptomatic | | Maximize Youden's index | 0.548 | 8.0 | 0.160 | N/A | N/A | 6.8 | 0.216 | N/A | N/A |
| 11 | HFrEF LVEF 35% to 50% | | Maximize Youden's index | 0.548 | 7.1 | 0.268 | N/A | N/A | 5.7 | 0.368 | N/A | N/A |
| 11 | ICM LVEF 35% to 50% | | Maximize Youden's index | 0.548 | 8.7 | 0.080 | N/A | N/A | 7.0 | 0.189 | N/A | N/A |
| 11 | DCM LVEF 35% to 50% | | Maximize Youden's index | 0.548 | 4.8 | 0.541 | N/A | N/A | 3.8 | 0.626 | N/A | N/A |
| 11 | HFrEF LVEF < 35% | | Maximize Youden's index | 0.548 | 8.9 | 0.056 | N/A | N/A | 8.3 | 0.019 | N/A | N/A |
| 11 | ICM LVEF < 35% | | Maximize Youden's index | 0.548 | 9.3 | 0.000 | N/A | N/A | 8.4 | 0.000 | N/A | N/A |
| 11 | DCM LVEF < 35% | | Maximize Youden's index | 0.548 | 8.6 | 0.088 | N/A | N/A | 8.2 | 0.030 | N/A | N/A |

Example 14: Performance of panels 8 + NT-proBNP, 9 + NT-proBNP, 10 + NT-proBNP, and 11 + NT-proBNP as compared to NT-proBNP alone

The performance of panels 8 + NT-proBNP, 9 + NT-proBNP, 10 + NT-proBNP, and 11 + NT-proBNP was compared to the performance of NT-proBNP alone based on ratio $LR+_{panel}/LR+_{NT\text{-}proBNP\ alone}$, on the difference $PPV_{panel} - PPV_{NT\text{-}proBNP\ alone}$, and on the NRI. The comparison of performance statistics based on the testing set is shown in Table 24.

How to calculate the NRI is well known to the person skilled in the art. The NRI is a measure comparing the sensitivity and specificity of the biomarker panel to the sensitivity and specificity of NT-proBNP alone in a particular subgroup. The NRI was calculated as:

$$NRI = \left(sensitivity_{panel} - sensitivity_{NT\text{-}proBNP\ alone}\right) + \left(specificity_{panel} - specificity_{NT\text{-}proBNP\ alone}\right)$$

Highest NRI was observed in subgroup 'HFpEF asymptomatic' followed by 'ICM symptomatic', using any panel selected from the panels 8 + NT-proBNP, 9 + NT-proBNP, and 10 + NT-proBNP, combined with a cut-off value determined according the method 'Maximize Youden's index'.

With respect to HFpEF, highest NRI was observed for the subgroup 'HFpEF asymptomatic' followed by 'HFpEF', using any panel selected from panels 8 + NT-proBNP, 9 + NT-proBNP, 10 + NT-proBNP, and 11 + NT-proBNP, combined with a cut-off value determined according the method 'Maximize Youden's index'.

With respect to the HFpEF subgroups 'HFpEF', 'HFpEF asymptomatic', and 'HFpEF symptomatic', highest NRI was observed with panel 9 + NT-proBNP combined with a cut-off value determined according the method 'Maximize Youden's index'.

Highest improvements in LR+ were observed in the subgroup 'ICM LVEF < 35%' using any panel selected from panels 8 + NT-proBNP, 9 + NT-proBNP, 10 + NT-proBNP, and 11 + NT-proBNP combined with a cut-off value determined according to the method 'Match sensitivity'. Also, subgroup 'HFrEF LVEF < 35%' as well as subgroups showing symptoms of heart failure ('CHF symptomatic', 'HFrEF symptomatic', 'ICM symptomatic', and 'DCM symptomatic') show high improvements in LR+ using any of these panels combined with a cut-off value determined according to the method 'Match sensitivity'.

With respect to asymptomatic forms of HFrEF, highest improvements in LR+ were observed in the subgroup 'HFpEF asymptomatic', followed by the subgroups 'CHF asymptomatic', 'HFrEF asymptomatic', and 'ICM asymptomatic', using any panel selected from panels 8 + NT-proBNP, 9 + NT-proBNP, 10 + NT-proBNP, and 11 + NT-proBNP combined with a cut-off value determined according to one the methods 'Match sensitivity' or 'Match sensitivity + 5%'.

Highest improvements in LR+ were observed with Panel 9 + NT-proBNP or Panel 11 + NT-proBNP in combination with a cut-off value determined according to the method 'Match sensitivity' in most CHF subgroups.

Highest improvements in LR+ in combination with a cut-off value determined according to the method 'Match sensitivity + 5%' were observed with Panel 11 + NT-proBNP in most CHF subgroups.

Highest improvements in PPV were observed for subgroup 'HFrEF', using any panel selected from panels 8 + NT-proBNP, 9 + NT-proBNP, 10 + NT-proBNP, and 11 + NT-proBNP, combined with a cut-off value determined according to one of the methods 'Match sensitivity' or 'Match sensitivity + 5%".

Table 24: Comparision of selected performance statistics calculated on the testing set for Panels 8 + NT-proBNP, 9 + NT-proBNP, 10 + NT-proBNP, and 11 + NT-proBNP to NT-proBNP alone based on sensitivities shown in Table 22 and LR+ and PPV shown in Table 23. Comparisons were calculated as described in Example 14.

| Panel number | CHF subgroup | Prevalence | Method | Cutoff | NRI | Ratio LR+ | Diff. PPV |
|---|---|---|---|---|---|---|---|
| 8 | CHF | 10% | Match sensitivity | 0.710 | 0.042 | 4.59 | 0.358 |
| 8 | HFpEF | | Match sensitivity | 0.710 | 0.041 | 4.23 | N/A |
| 8 | HFrEF | 5% | Match sensitivity | 0.710 | 0.042 | 4.67 | 0.363 |
| 8 | ICM | | Match sensitivity | 0.710 | 0.048 | 4.74 | N/A |
| 8 | DCM | | Match sensitivity | 0.710 | 0.036 | 4.60 | N/A |
| 8 | CHF asymptomatic | | Match sensitivity | 0.710 | 0.020 | 4.31 | N/A |
| 8 | HFpEF asymptomatic | | Match sensitivity | 0.710 | 0.073 | 4.58 | N/A |
| 8 | HFrEF asymptomatic | | Match sensitivity | 0.710 | -0.027 | 4.23 | N/A |
| 8 | ICM asymptomatic | | Match sensitivity | 0.710 | 0.012 | 4.57 | N/A |
| 8 | DCM asymptomatic | | Match sensitivity | 0.710 | -0.065 | 3.75 | N/A |
| 8 | CHF symptomatic | | Match sensitivity | 0.710 | 0.060 | 4.76 | N/A |
| 8 | HFpEF symptomatic | | Match sensitivity | 0.710 | -0.005 | 3.93 | N/A |
| 8 | HFrEF symptomatic | | Match sensitivity | 0.710 | 0.083 | 4.93 | N/A |
| 8 | ICM symptomatic | | Match sensitivity | 0.710 | 0.070 | 4.85 | N/A |
| 8 | DCM symptomatic | | Match sensitivity | 0.710 | 0.095 | 5.00 | N/A |
| 8 | HFrEF LVEF 35% to 50% | | Match sensitivity | 0.710 | 0.039 | 4.58 | N/A |
| 8 | ICM LVEF 35% to 50% | | Match sensitivity | 0.710 | 0.024 | 4.57 | N/A |
| 8 | DCM LVEF 35% to 50% | | Match sensitivity | 0.710 | 0.061 | 4.62 | N/A |
| 8 | HFrEF LVEF < 35% | | Match sensitivity | 0.710 | 0.045 | 4.75 | N/A |
| 8 | ICM LVEF < 35% | | Match sensitivity | 0.710 | 0.095 | 5.00 | N/A |
| 8 | DCM LVEF < 35% | | Match sensitivity | 0.710 | 0.016 | 4.59 | N/A |
| 9 | CHF | 10% | Match sensitivity | 0.702 | 0.053 | 9.17 | 0.470 |
| 9 | HFpEF | | Match sensitivity | 0.702 | 0.053 | 8.46 | N/A |
| 9 | HFrEF | 5% | Match sensitivity | 0.702 | 0.054 | 9.35 | 0.506 |
| 9 | ICM | | Match sensitivity | 0.702 | 0.060 | 9.47 | N/A |
| 9 | DCM | | Match sensitivity | 0.702 | 0.047 | 9.20 | N/A |
| 9 | CHF asymptomatic | | Match sensitivity | 0.702 | 0.043 | 8.82 | N/A |
| 9 | HFpEF asymptomatic | | Match sensitivity | 0.702 | 0.084 | 9.17 | N/A |
| 9 | HFrEF asymptomatic | | Match sensitivity | 0.702 | 0.005 | 8.72 | N/A |
| 9 | ICM asymptomatic | | Match sensitivity | 0.702 | 0.024 | 9.13 | N/A |

| | | | | | | | |
|---|---|---|---|---|---|---|---|
| 9 | DCM asymptomatic | | Match sensitivity | 0.702 | -0.013 | 8.12 | N/A |
| 9 | CHF symptomatic | | Match sensitivity | 0.702 | 0.063 | 9.39 | N/A |
| 9 | HFpEF symptomatic | | Match sensitivity | 0.702 | 0.007 | 7.86 | N/A |
| 9 | HFrEF symptomatic | | Match sensitivity | 0.702 | 0.083 | 9.71 | N/A |
| 9 | ICM symptomatic | | Match sensitivity | 0.702 | 0.082 | 9.71 | N/A |
| 9 | DCM symptomatic | | Match sensitivity | 0.702 | 0.083 | 9.71 | N/A |
| 9 | HFrEF LVEF 35% to 50% | | Match sensitivity | 0.702 | 0.051 | 9.17 | N/A |
| 9 | ICM LVEF 35% to 50% | | Match sensitivity | 0.702 | 0.036 | 9.14 | N/A |
| 9 | DCM LVEF 35% to 50% | | Match sensitivity | 0.702 | 0.073 | 9.23 | N/A |
| 9 | HFrEF LVEF < 35% | | Match sensitivity | 0.702 | 0.057 | 9.49 | N/A |
| 9 | ICM LVEF < 35% | | Match sensitivity | 0.702 | 0.107 | 10.00 | N/A |
| 9 | DCM LVEF < 35% | | Match sensitivity | 0.702 | 0.028 | 9.19 | N/A |
| 10 | CHF | 10% | Match sensitivity | 0.715 | 0.042 | 4.59 | 0.358 |
| 10 | HFpEF | | Match sensitivity | 0.715 | 0.041 | 4.23 | N/A |
| 10 | HFrEF | 5% | Match sensitivity | 0.715 | 0.042 | 4.67 | 0.363 |
| 10 | ICM | | Match sensitivity | 0.715 | 0.048 | 4.74 | N/A |
| 10 | DCM | | Match sensitivity | 0.715 | 0.036 | 4.60 | N/A |
| 10 | CHF asymptomatic | | Match sensitivity | 0.715 | 0.020 | 4.31 | N/A |
| 10 | HFpEF asymptomatic | | Match sensitivity | 0.715 | 0.073 | 4.58 | N/A |
| 10 | HFrEF asymptomatic | | Match sensitivity | 0.715 | -0.027 | 4.23 | N/A |
| 10 | ICM asymptomatic | | Match sensitivity | 0.715 | 0.012 | 4.57 | N/A |
| 10 | DCM asymptomatic | | Match sensitivity | 0.715 | -0.065 | 3.75 | N/A |
| 10 | CHF symptomatic | | Match sensitivity | 0.715 | 0.060 | 4.76 | N/A |
| 10 | HFpEF symptomatic | | Match sensitivity | 0.715 | -0.005 | 3.93 | N/A |
| 10 | HFrEF symptomatic | | Match sensitivity | 0.715 | 0.083 | 4.93 | N/A |
| 10 | ICM symptomatic | | Match sensitivity | 0.715 | 0.070 | 4.85 | N/A |
| 10 | DCM symptomatic | | Match sensitivity | 0.715 | 0.095 | 5.00 | N/A |
| 10 | HFrEF LVEF 35% to 50% | | Match sensitivity | 0.715 | 0.039 | 4.58 | N/A |
| 10 | ICM LVEF 35% to 50% | | Match sensitivity | 0.715 | 0.024 | 4.57 | N/A |
| 10 | DCM LVEF 35% to 50% | | Match sensitivity | 0.715 | 0.061 | 4.62 | N/A |
| 10 | HFrEF LVEF < 35% | | Match sensitivity | 0.715 | 0.045 | 4.75 | N/A |
| 10 | ICM LVEF < 35% | | Match sensitivity | 0.715 | 0.095 | 5.00 | N/A |
| 10 | DCM LVEF < 35% | | Match sensitivity | 0.715 | 0.016 | 4.59 | N/A |
| 11 | CHF | 10% | Match sensitivity | 0.708 | 0.024 | 8.72 | 0.464 |
| 11 | HFpEF | | Match sensitivity | 0.708 | 0.013 | 7.31 | N/A |
| 11 | HFrEF | 5% | Match sensitivity | 0.708 | 0.031 | 9.07 | 0.501 |
| 11 | ICM | | Match sensitivity | 0.708 | 0.029 | 9.12 | N/A |
| 11 | DCM | | Match sensitivity | 0.708 | 0.033 | 9.00 | N/A |

| | | | | | | | |
|---|---|---|---|---|---|---|---|
| 11 | CHF asymptomatic | | Match sensitivity | 0.708 | 0.043 | 8.82 | N/A |
| 11 | HFpEF asymptomatic | | Match sensitivity | 0.708 | 0.107 | 10.00 | N/A |
| 11 | HFrEF asymptomatic | | Match sensitivity | 0.708 | -0.015 | 8.46 | N/A |
| 11 | ICM asymptomatic | | Match sensitivity | 0.708 | 0.024 | 9.13 | N/A |
| 11 | DCM asymptomatic | | Match sensitivity | 0.708 | -0.053 | 7.50 | N/A |
| 11 | CHF symptomatic | | Match sensitivity | 0.708 | 0.009 | 8.66 | N/A |
| 11 | HFpEF symptomatic | | Match sensitivity | 0.708 | -0.126 | 5.00 | N/A |
| 11 | HFrEF symptomatic | | Match sensitivity | 0.708 | 0.058 | 9.41 | N/A |
| 11 | ICM symptomatic | | Match sensitivity | 0.708 | 0.032 | 9.12 | N/A |
| 11 | DCM symptomatic | | Match sensitivity | 0.708 | 0.083 | 9.71 | N/A |
| 11 | HFrEF LVEF 35% to 50% | | Match sensitivity | 0.708 | -0.006 | 8.33 | N/A |
| 11 | ICM LVEF 35% to 50% | | Match sensitivity | 0.708 | -0.012 | 8.57 | N/A |
| 11 | DCM LVEF 35% to 50% | | Match sensitivity | 0.708 | 0.004 | 7.69 | N/A |
| 11 | HFrEF LVEF < 35% | | Match sensitivity | 0.708 | 0.074 | 9.66 | N/A |
| 11 | ICM LVEF < 35% | | Match sensitivity | 0.708 | 0.107 | 10.00 | N/A |
| 11 | DCM LVEF < 35% | | Match sensitivity | 0.708 | 0.055 | 9.46 | N/A |
| 8 | CHF | 10% | Match sensitivity + 5% | 0.633 | 0.081 | 2.54 | 0.229 |
| 8 | HFpEF | | Match sensitivity + 5% | 0.633 | 0.071 | 2.50 | N/A |
| 8 | HFrEF | 5% | Match sensitivity + 5% | 0.633 | 0.087 | 2.55 | 0.214 |
| 8 | ICM | | Match sensitivity + 5% | 0.633 | 0.087 | 2.54 | N/A |
| 8 | DCM | | Match sensitivity + 5% | 0.633 | 0.086 | 2.55 | N/A |
| 8 | CHF asymptomatic | | Match sensitivity + 5% | 0.633 | 0.061 | 2.45 | N/A |
| 8 | HFpEF asymptomatic | | Match sensitivity + 5% | 0.633 | 0.117 | 2.92 | N/A |
| 8 | HFrEF asymptomatic | | Match sensitivity + 5% | 0.633 | 0.010 | 2.31 | N/A |
| 8 | ICM asymptomatic | | Match sensitivity + 5% | 0.633 | 0.030 | 2.39 | N/A |
| 8 | DCM asymptomatic | | Match sensitivity + 5% | 0.633 | -0.009 | 2.19 | N/A |
| 8 | CHF symptomatic | | Match sensitivity + 5% | 0.633 | 0.098 | 2.59 | N/A |
| 8 | HFpEF symptomatic | | Match sensitivity + 5% | 0.633 | 0.005 | 2.14 | N/A |
| 8 | HFrEF symptomatic | | Match sensitivity + 5% | 0.633 | 0.132 | 2.68 | N/A |
| 8 | ICM symptomatic | | Match sensitivity + 5% | 0.633 | 0.121 | 2.65 | N/A |
| 8 | DCM symptomatic | | Match sensitivity + 5% | 0.633 | 0.143 | 2.72 | N/A |
| 8 | HFrEF LVEF 35% to 50% | | Match sensitivity + 5% | 0.633 | 0.114 | 2.66 | N/A |
| 8 | ICM LVEF 35% to 50% | | Match sensitivity + 5% | 0.633 | 0.095 | 2.57 | N/A |
| 8 | DCM LVEF 35% to 50% | | Match sensitivity + 5% | 0.633 | 0.140 | 2.88 | N/A |
| 8 | HFrEF LVEF < 35% | | Match sensitivity + 5% | 0.633 | 0.055 | 2.46 | N/A |
| 8 | ICM LVEF < 35% | | Match sensitivity + 5% | 0.633 | 0.071 | 2.50 | N/A |
| 8 | DCM LVEF < 35% | | Match sensitivity + 5% | 0.633 | 0.045 | 2.43 | N/A |
| 9 | CHF | 10% | Match sensitivity + 5% | 0.625 | 0.081 | 2.54 | 0.229 |

| | | | | | | | |
|---|---|---|---|---|---|---|---|
| 9 | HFpEF | | Match sensitivity + 5% | 0.625 | 0.071 | 2.50 | N/A |
| 9 | HFrEF | 5% | Match sensitivity + 5% | 0.625 | 0.087 | 2.55 | 0.214 |
| 9 | ICM | | Match sensitivity + 5% | 0.625 | 0.087 | 2.54 | N/A |
| 9 | DCM | | Match sensitivity + 5% | 0.625 | 0.086 | 2.55 | N/A |
| 9 | CHF asymptomatic | | Match sensitivity + 5% | 0.625 | 0.061 | 2.45 | N/A |
| 9 | HFpEF asymptomatic | | Match sensitivity + 5% | 0.625 | 0.117 | 2.92 | N/A |
| 9 | HFrEF asymptomatic | | Match sensitivity + 5% | 0.625 | 0.010 | 2.31 | N/A |
| 9 | ICM asymptomatic | | Match sensitivity + 5% | 0.625 | 0.030 | 2.39 | N/A |
| 9 | DCM asymptomatic | | Match sensitivity + 5% | 0.625 | -0.009 | 2.19 | N/A |
| 9 | CHF symptomatic | | Match sensitivity + 5% | 0.625 | 0.098 | 2.59 | N/A |
| 9 | HFpEF symptomatic | | Match sensitivity + 5% | 0.625 | 0.005 | 2.14 | N/A |
| 9 | HFrEF symptomatic | | Match sensitivity + 5% | 0.625 | 0.132 | 2.68 | N/A |
| 9 | ICM symptomatic | | Match sensitivity + 5% | 0.625 | 0.121 | 2.65 | N/A |
| 9 | DCM symptomatic | | Match sensitivity + 5% | 0.625 | 0.143 | 2.72 | N/A |
| 9 | HFrEF LVEF 35% to 50% | | Match sensitivity + 5% | 0.625 | 0.114 | 2.66 | N/A |
| 9 | ICM LVEF 35% to 50% | | Match sensitivity + 5% | 0.625 | 0.095 | 2.57 | N/A |
| 9 | DCM LVEF 35% to 50% | | Match sensitivity + 5% | 0.625 | 0.140 | 2.88 | N/A |
| 9 | HFrEF LVEF < 35% | | Match sensitivity + 5% | 0.625 | 0.055 | 2.46 | N/A |
| 9 | ICM LVEF < 35% | | Match sensitivity + 5% | 0.625 | 0.071 | 2.50 | N/A |
| 9 | DCM LVEF < 35% | | Match sensitivity + 5% | 0.625 | 0.045 | 2.43 | N/A |
| 10 | CHF | 10% | Match sensitivity + 5% | 0.641 | 0.062 | 2.46 | 0.221 |
| 10 | HFpEF | | Match sensitivity + 5% | 0.641 | 0.044 | 2.31 | N/A |
| 10 | HFrEF | 5% | Match sensitivity + 5% | 0.641 | 0.071 | 2.50 | 0.209 |
| 10 | ICM | | Match sensitivity + 5% | 0.641 | 0.071 | 2.50 | N/A |
| 10 | DCM | | Match sensitivity + 5% | 0.641 | 0.071 | 2.50 | N/A |
| 10 | CHF asymptomatic | | Match sensitivity + 5% | 0.641 | 0.028 | 2.30 | N/A |
| 10 | HFpEF asymptomatic | | Match sensitivity + 5% | 0.641 | 0.071 | 2.50 | N/A |
| 10 | HFrEF asymptomatic | | Match sensitivity + 5% | 0.641 | -0.010 | 2.24 | N/A |
| 10 | ICM asymptomatic | | Match sensitivity + 5% | 0.641 | 0.030 | 2.39 | N/A |
| 10 | DCM asymptomatic | | Match sensitivity + 5% | 0.641 | -0.049 | 2.03 | N/A |
| 10 | CHF symptomatic | | Match sensitivity + 5% | 0.641 | 0.089 | 2.56 | N/A |
| 10 | HFpEF symptomatic | | Match sensitivity + 5% | 0.641 | 0.005 | 2.14 | N/A |
| 10 | HFrEF symptomatic | | Match sensitivity + 5% | 0.641 | 0.120 | 2.65 | N/A |
| 10 | ICM symptomatic | | Match sensitivity + 5% | 0.641 | 0.096 | 2.57 | N/A |
| 10 | DCM symptomatic | | Match sensitivity + 5% | 0.641 | 0.143 | 2.72 | N/A |
| 10 | HFrEF LVEF 35% to 50% | | Match sensitivity + 5% | 0.641 | 0.086 | 2.55 | N/A |
| 10 | ICM LVEF 35% to 50% | | Match sensitivity + 5% | 0.641 | 0.071 | 2.50 | N/A |
| 10 | DCM LVEF 35% to 50% | | Match sensitivity + 5% | 0.641 | 0.106 | 2.69 | N/A |

| 10 | HFrEF LVEF < 35% | | Match sensitivity + 5% | 0.641 | 0.055 | 2.46 | N/A |
|---|---|---|---|---|---|---|---|
| 10 | ICM LVEF < 35% | | Match sensitivity + 5% | 0.641 | 0.071 | 2.50 | N/A |
| 10 | DCM LVEF < 35% | | Match sensitivity + 5% | 0.641 | 0.045 | 2.43 | N/A |
| 11 | CHF | 10% | Match sensitivity + 5% | 0.612 | 0.078 | 3.31 | 0.290 |
| 11 | HFpEF | | Match sensitivity + 5% | 0.612 | 0.056 | 3.08 | N/A |
| 11 | HFrEF | 5% | Match sensitivity + 5% | 0.612 | 0.091 | 3.36 | 0.283 |
| 11 | ICM | | Match sensitivity + 5% | 0.612 | 0.099 | 3.39 | N/A |
| 11 | DCM | | Match sensitivity + 5% | 0.612 | 0.083 | 3.33 | N/A |
| 11 | CHF asymptomatic | | Match sensitivity + 5% | 0.612 | 0.040 | 3.07 | N/A |
| 11 | HFpEF asymptomatic | | Match sensitivity + 5% | 0.612 | 0.083 | 3.33 | N/A |
| 11 | HFrEF asymptomatic | | Match sensitivity + 5% | 0.612 | 0.002 | 2.99 | N/A |
| 11 | ICM asymptomatic | | Match sensitivity + 5% | 0.612 | 0.000 | 3.04 | N/A |
| 11 | DCM asymptomatic | | Match sensitivity + 5% | 0.612 | 0.003 | 2.92 | N/A |
| 11 | CHF symptomatic | | Match sensitivity + 5% | 0.612 | 0.110 | 3.46 | N/A |
| 11 | HFpEF symptomatic | | Match sensitivity + 5% | 0.612 | 0.017 | 2.86 | N/A |
| 11 | HFrEF symptomatic | | Match sensitivity + 5% | 0.612 | 0.144 | 3.58 | N/A |
| 11 | ICM symptomatic | | Match sensitivity + 5% | 0.612 | 0.158 | 3.63 | N/A |
| 11 | DCM symptomatic | | Match sensitivity + 5% | 0.612 | 0.131 | 3.53 | N/A |
| 11 | HFrEF LVEF 35% to 50% | | Match sensitivity + 5% | 0.612 | 0.126 | 3.54 | N/A |
| 11 | ICM LVEF 35% to 50% | | Match sensitivity + 5% | 0.612 | 0.107 | 3.43 | N/A |
| 11 | DCM LVEF 35% to 50% | | Match sensitivity + 5% | 0.612 | 0.152 | 3.85 | N/A |
| 11 | HFrEF LVEF < 35% | | Match sensitivity + 5% | 0.612 | 0.050 | 3.22 | N/A |
| 11 | ICM LVEF < 35% | | Match sensitivity + 5% | 0.612 | 0.083 | 3.33 | N/A |
| 11 | DCM LVEF < 35% | | Match sensitivity + 5% | 0.612 | 0.031 | 3.15 | N/A |
| 8 | CHF | 10% | Maximize Youden's index | 0.535 | 0.097 | 1.39 | 0.080 |
| 8 | HFpEF | | Maximize Youden's index | 0.535 | 0.132 | 1.63 | N/A |
| 8 | HFrEF | 5% | Maximize Youden's index | 0.535 | 0.077 | 1.33 | 0.059 |
| 8 | ICM | | Maximize Youden's index | 0.535 | 0.102 | 1.36 | N/A |
| 8 | DCM | | Maximize Youden's index | 0.535 | 0.054 | 1.30 | N/A |
| 8 | CHF asymptomatic | | Maximize Youden's index | 0.535 | 0.088 | 1.40 | N/A |
| 8 | HFpEF asymptomatic | | Maximize Youden's index | 0.535 | 0.206 | 2.08 | N/A |
| 8 | HFrEF asymptomatic | | Maximize Youden's index | 0.535 | -0.017 | 1.19 | N/A |
| 8 | ICM asymptomatic | | Maximize Youden's index | 0.535 | -0.018 | 1.20 | N/A |
| 8 | DCM asymptomatic | | Maximize Youden's index | 0.535 | -0.016 | 1.17 | N/A |
| 8 | CHF symptomatic | | Maximize Youden's index | 0.535 | 0.104 | 1.39 | N/A |
| 8 | HFpEF symptomatic | | Maximize Youden's index | 0.535 | 0.024 | 1.25 | N/A |
| 8 | HFrEF symptomatic | | Maximize Youden's index | 0.535 | 0.134 | 1.42 | N/A |
| 8 | ICM symptomatic | | Maximize Youden's index | 0.535 | 0.174 | 1.47 | N/A |

| 8 | DCM symptomatic | | Maximize Youden's index | 0.535 | 0.095 | 1.36 | N/A |
| 8 | HFrEF LVEF 35% to 50% | | Maximize Youden's index | 0.535 | 0.122 | 1.43 | N/A |
| 8 | ICM LVEF 35% to 50% | | Maximize Youden's index | 0.535 | 0.143 | 1.43 | N/A |
| 8 | DCM LVEF 35% to 50% | | Maximize Youden's index | 0.535 | 0.093 | 1.44 | N/A |
| 8 | HFrEF LVEF < 35% | | Maximize Youden's index | 0.535 | 0.024 | 1.25 | N/A |
| 8 | ICM LVEF < 35% | | Maximize Youden's index | 0.535 | 0.024 | 1.25 | N/A |
| 8 | DCM LVEF < 35% | | Maximize Youden's index | 0.535 | 0.024 | 1.25 | N/A |
| 9 | CHF | 10% | Maximize Youden's index | 0.494 | 0.105 | 1.27 | 0.058 |
| 9 | HFpEF | | Maximize Youden's index | 0.494 | 0.161 | 1.58 | N/A |
| 9 | HFrEF | 5% | Maximize Youden's index | 0.494 | 0.073 | 1.19 | 0.036 |
| 9 | ICM | | Maximize Youden's index | 0.494 | 0.090 | 1.21 | N/A |
| 9 | DCM | | Maximize Youden's index | 0.494 | 0.057 | 1.18 | N/A |
| 9 | CHF asymptomatic | | Maximize Youden's index | 0.494 | 0.098 | 1.29 | N/A |
| 9 | HFpEF asymptomatic | | Maximize Youden's index | 0.494 | 0.216 | 1.94 | N/A |
| 9 | HFrEF asymptomatic | | Maximize Youden's index | 0.494 | -0.009 | 1.08 | N/A |
| 9 | ICM asymptomatic | | Maximize Youden's index | 0.494 | -0.030 | 1.06 | N/A |
| 9 | DCM asymptomatic | | Maximize Youden's index | 0.494 | 0.012 | 1.11 | N/A |
| 9 | CHF symptomatic | | Maximize Youden's index | 0.494 | 0.110 | 1.26 | N/A |
| 9 | HFpEF symptomatic | | Maximize Youden's index | 0.494 | 0.079 | 1.27 | N/A |
| 9 | HFrEF symptomatic | | Maximize Youden's index | 0.494 | 0.122 | 1.26 | N/A |
| 9 | ICM symptomatic | | Maximize Youden's index | 0.494 | 0.162 | 1.31 | N/A |
| 9 | DCM symptomatic | | Maximize Youden's index | 0.494 | 0.083 | 1.21 | N/A |
| 9 | HFrEF LVEF 35% to 50% | | Maximize Youden's index | 0.494 | 0.125 | 1.30 | N/A |
| 9 | ICM LVEF 35% to 50% | | Maximize Youden's index | 0.494 | 0.131 | 1.27 | N/A |
| 9 | DCM LVEF 35% to 50% | | Maximize Youden's index | 0.494 | 0.115 | 1.37 | N/A |
| 9 | HFrEF LVEF < 35% | | Maximize Youden's index | 0.494 | 0.012 | 1.11 | N/A |
| 9 | ICM LVEF < 35% | | Maximize Youden's index | 0.494 | 0.012 | 1.11 | N/A |
| 9 | DCM LVEF < 35% | | Maximize Youden's index | 0.494 | 0.012 | 1.11 | N/A |
| 10 | CHF | 10% | Maximize Youden's index | 0.505 | 0.100 | 1.26 | 0.056 |
| 10 | HFpEF | | Maximize Youden's index | 0.505 | 0.134 | 1.50 | N/A |
| 10 | HFrEF | 5% | Maximize Youden's index | 0.505 | 0.081 | 1.20 | 0.038 |
| 10 | ICM | | Maximize Youden's index | 0.505 | 0.090 | 1.21 | N/A |
| 10 | DCM | | Maximize Youden's index | 0.505 | 0.072 | 1.20 | N/A |
| 10 | CHF asymptomatic | | Maximize Youden's index | 0.505 | 0.098 | 1.29 | N/A |
| 10 | HFpEF asymptomatic | | Maximize Youden's index | 0.505 | 0.194 | 1.85 | N/A |
| 10 | HFrEF asymptomatic | | Maximize Youden's index | 0.505 | 0.012 | 1.11 | N/A |
| 10 | ICM asymptomatic | | Maximize Youden's index | 0.505 | -0.030 | 1.06 | N/A |
| 10 | DCM asymptomatic | | Maximize Youden's index | 0.505 | 0.052 | 1.18 | N/A |

| | | | | | | | |
|---|---|---|---|---|---|---|---|
| 10 | CHF symptomatic | | Maximize Youden's index | 0.505 | 0.101 | 1.25 | N/A |
| 10 | HFpEF symptomatic | | Maximize Youden's index | 0.505 | 0.045 | 1.19 | N/A |
| 10 | HFrEF symptomatic | | Maximize Youden's index | 0.505 | 0.122 | 1.26 | N/A |
| 10 | ICM symptomatic | | Maximize Youden's index | 0.505 | 0.162 | 1.31 | N/A |
| 10 | DCM symptomatic | | Maximize Youden's index | 0.505 | 0.083 | 1.21 | N/A |
| 10 | HFrEF LVEF 35% to 50% | | Maximize Youden's index | 0.505 | 0.139 | 1.32 | N/A |
| 10 | ICM LVEF 35% to 50% | | Maximize Youden's index | 0.505 | 0.131 | 1.27 | N/A |
| 10 | DCM LVEF 35% to 50% | | Maximize Youden's index | 0.505 | 0.150 | 1.45 | N/A |
| 10 | HFrEF LVEF < 35% | | Maximize Youden's index | 0.505 | 0.012 | 1.11 | N/A |
| 10 | ICM LVEF < 35% | | Maximize Youden's index | 0.505 | 0.012 | 1.11 | N/A |
| 10 | DCM LVEF < 35% | | Maximize Youden's index | 0.505 | 0.012 | 1.11 | N/A |
| 11 | CHF | 10% | Maximize Youden's index | 0.548 | 0.061 | 1.19 | 0.043 |
| 11 | HFpEF | | Maximize Youden's index | 0.548 | 0.093 | 1.37 | N/A |
| 11 | HFrEF | 5% | Maximize Youden's index | 0.548 | 0.042 | 1.15 | 0.029 |
| 11 | ICM | | Maximize Youden's index | 0.548 | 0.074 | 1.19 | N/A |
| 11 | DCM | | Maximize Youden's index | 0.548 | 0.012 | 1.11 | N/A |
| 11 | CHF asymptomatic | | Maximize Youden's index | 0.548 | 0.033 | 1.15 | N/A |
| 11 | HFpEF asymptomatic | | Maximize Youden's index | 0.548 | 0.148 | 1.67 | N/A |
| 11 | HFrEF asymptomatic | | Maximize Youden's index | 0.548 | -0.070 | 1.00 | N/A |
| 11 | ICM asymptomatic | | Maximize Youden's index | 0.548 | -0.071 | 1.01 | N/A |
| 11 | DCM asymptomatic | | Maximize Youden's index | 0.548 | -0.068 | 0.97 | N/A |
| 11 | CHF symptomatic | | Maximize Youden's index | 0.548 | 0.083 | 1.22 | N/A |
| 11 | HFpEF symptomatic | | Maximize Youden's index | 0.548 | 0.012 | 1.11 | N/A |
| 11 | HFrEF symptomatic | | Maximize Youden's index | 0.548 | 0.109 | 1.24 | N/A |
| 11 | ICM symptomatic | | Maximize Youden's index | 0.548 | 0.162 | 1.31 | N/A |
| 11 | DCM symptomatic | | Maximize Youden's index | 0.548 | 0.060 | 1.18 | N/A |
| 11 | HFrEF LVEF 35% to 50% | | Maximize Youden's index | 0.548 | 0.096 | 1.25 | N/A |
| 11 | ICM LVEF 35% to 50% | | Maximize Youden's index | 0.548 | 0.107 | 1.24 | N/A |
| 11 | DCM LVEF 35% to 50% | | Maximize Youden's index | 0.548 | 0.081 | 1.28 | N/A |
| 11 | HFrEF LVEF < 35% | | Maximize Youden's index | 0.548 | -0.021 | 1.07 | N/A |
| 11 | ICM LVEF < 35% | | Maximize Youden's index | 0.548 | 0.012 | 1.11 | N/A |
| 11 | DCM LVEF < 35% | | Maximize Youden's index | 0.548 | -0.041 | 1.05 | N/A |

Example 15: Single case assessment using Panel 8 + NT-proBNP in groups of subjects characterized by gender and/or BMI

For certain groups of subjects characterized by gender and/or BMI (selected from the entire patient cohort described in Example 7), the diagnostic outcome using Panel 8 + NT-proBNP was compared to the diagnostic outcome using NT-proBNP alone.

Using Panel 8 + NT-proBNP with a cut-off value determined according to the method 'Match sensitivity' (see Example 12), classification of subjects with BMI > 28 was improved compared to NT-proBNP alone in 4 subjects, of which one was not suffering from heart failure and 3 were suffering from HFrEF. Of these latter 3 subjects, two were male and one was female.

Using Panel 8 + NT-proBNP with a cut-off value determined according to the method 'Match sensitivity' (see Example 12), classification of 9 female subjects was improved compared to NT-proBNP alone, of which one was suffering from HFrEF and 8 were not suffering from heart failure. Of these latter 8 subjects, 7 had BMI < 28.

Example 16: Weights and scaling factors used for the calculation of the biomarker prediction score

For panels 8 + NT-proBNP, 9 + NT-proBNP, 10 + NT-proBNP, and 11 + NT-proBNP, the coefficients $w_i$ and the scaling factors $m_i$ and $s_i$ shown in Table 25 were used for the calculation of prediction probabilities as described in Example 8.

Table 25: Coefficients $w_i$ and the scaling factors $m_i$ and $s_i$ for panels 8 + NT-proBNP, 9 + NT-proBNP, 10 + NT-proBNP, and 11 + NT-proBNP using absolute concentrations for all parameters (the concentrations for NT-proBNP have been determined via antibody reaction and for all others via enzymatic methods; for the determination of cholesteryl esters, the sum of the total amount of cholesterol contained in cholesteryl esters plus cholesterol was taken as approximation for the amount of cholesteryl esters, as described above, e.g. in Example 5).

| | | | | Scaling Factors | | Coefficients |
|---|---|---|---|---|---|---|
| Panel | Parameter | Index $i$ | Units | $m_i$ | $s_i$ | $w_i$ |
| 8* | Intercept | 0 | N/A | N/A | N/A | 0.7425838 |
| 8* | NT-proBNP | 1 | pg/ml | 2.20815226 | 0.63538927 | 1.5498776 |
| 8* | Total amount of triacylglycerols | 3 | mg/dl | 2.04087703 | 0.21052875 | 0.5105663 |
| 8* | Total amount of sphingomyelins | 2 | nmol/μl | -0.84133005 | 0.14546292 | -0.6437630 |
| 8* | Total amount of cholesteryl esters | 4 | μg/μl | 0.59850929 | 0.12017917 | -0.1575292 |
| 8* | Total amount of cholesterol | 5 | μg/μl | 0.03227242 | 0.14279049 | -0.2374175 |
| 9* | Intercept | 0 | N/A | N/A | N/A | 0.7194159 |
| 9* | NT-proBNP | 1 | pg/ml | 2.20815226 | 0.63538927 | 1.4823448 |
| 9* | Total amount of triacylglycerols | 2 | mg/dl | 2.04087703 | 0.21052875 | 0.4670435 |
| 9* | Total amount of sphingomyelins | 3 | nmol/μl | -0.84133005 | 0.14546292 | -0.6428591 |
| 9* | Total amount of cholesteryl esters | 4 | μg/μl | 0.59850929 | 0.12017917 | -0.3294130 |
| 10* | Intercept | 0 | N/A | N/A | N/A | 0.7382621 |
| 10* | NT-proBNP | 1 | pg/ml | 2.20815226 | 0.63538927 | 1.5551770 |
| 10* | Total amount of triacylglycerols | 2 | mg/dl | 2.04087703 | 0.21052875 | 0.5173727 |
| 10* | Total amount of sphingomyelins | 3 | nmol/μl | -0.84133005 | 0.14546292 | -0.6577244 |
| 10* | Total amount of cholesterol | 4 | μg/μl | 0.03227242 | 0.14279049 | -0.3582858 |
| 11* | Intercept | 0 | N/A | N/A | N/A | 0.6830203 |
| 11* | NT-proBNP | 1 | pg/ml | 2.20815226 | 0.63538927 | 1.4350546 |
| 11* | Total amount of triacylglycerols | 2 | mg/dl | 2.04087703 | 0.21052875 | 0.4169432 |

(continued)

| Panel | Parameter | Index $i$ | Units | Scaling Factors | | Coefficients |
|---|---|---|---|---|---|---|
| | | | | $m_i$ | $s_i$ | $w_i$ |
| 11* | Total amount of sphingomyelins | 3 | nmol/μl | -0.84133005 | 0.14546292 | -0.7453965 |
| * + NT-proBNP | | | | | | |

**Claims**

1. A method for diagnosing heart failure in a subject suspected to suffer therefrom comprising:

   (a) determining the amount of total triacylglycerols and the amount of total sphingomyelins in a sample of the said subject; and
   (b) comparing the amount(s) determined in step (a) to a reference, whereby it is diagnosed whether the subject suffers from heart failure, or not.

2. The method of claim 1, further comprising in step a) the determination of at least one cholesterol parameter selected from the group consisting of: total cholesterol, total cholesteryl esters and the sum parameter of total cholesterol and total cholesteryl esters in a sample from the subject.

3. The method of claim 1 or 2, wherein the amount of at least one cholesterol parameter, total triacylglycerols and/or total sphingomyelins in a sample of the said subject is enzymatically determined.

4. The method of any one of claims 1 to 3, wherein

   a) said reference is derived from a subject or group of subjects known to suffer from heart failure, in particular wherein an identical or decreased amount of at least one cholesterol parameter and/or total sphingomyelins in the test sample and the reference is indicative for a subject suffering from heart failure whereas an increased amount in the test sample in comparison to the reference is indicative for a subject not suffering from heart failure, and/or wherein an identical or increased amount of total triacylglycerols in the test sample and the reference is indicative for a subject suffering from heart failure, whereas a decreased amount in the test sample in comparison to the reference is indicative for a subject not suffering from heart failure, or
   b) said reference is derived from a subject or group of subjects known not to suffer from heart failure, wherein an identical or increased amount of at least one cholesterol parameter and/or total sphingomyelins in the test sample and the reference is indicative for a subject not suffering from heart failure whereas an decreased amount in the test sample in comparison to the reference is indicative for a subject suffering from heart failure, and/or wherein an identical or decreased amount of total triacylglycerols in the test sample and the reference is indicative for a subject not suffering from heart failure whereas an increased amount in the test sample in comparison to the reference differs is indicative for a subject suffering from heart failure.

5. The method of any one of claims 1 to 4, wherein said method further comprises the step of recommending a therapeutic or patient health management measure for the subject based on whether the subject is diagnosed as suffering from heart failure.

6. The method of any one of claims 1 to 5, wherein said sample is a blood, plasma or serum sample.

7. The method of any one of claims 1 to 6, wherein said heart failure is asymptomatic heart failure.

8. The method of any one of claims 3 to 7, wherein said determining enzymatically the amount of total cholesteryl esters as at least one cholesterol parameter comprises the steps of:

   a) contacting the sample with cholesterol esterase under conditions and for a time sufficient to allow conversion into cholesterol;
   b) contacting the sample comprising the cholesterol with cholesterol oxidase under conditions and for a time sufficient to allow generation of, $H_2O_2$; and

c) enzymatically or chemically determining the amount of generated $H_2O_2$ and optionally wherein said method comprise the further steps of:

- contacting the sample prior to step a) with cholesterol oxidase under conditions and for a time sufficient to allow generation of redox equivalents and, preferably, $H_2O_2$; and
- neutralizing the said redox equivalents, and optionally wherein said neutralizing the said $H_2O_2$ comprises enzymatically or chemically

determining the amount of generated $H_2O_2$.

9. The method of claim 8, wherein said method further comprises the generation of the total cholesteryl ester to total cholesterol ratio based on the amounts of the cholesterol-derived $H_2O_2$ determined prior to step a) and the cholesteryl ester-derived $H_2O_2$ determined in step c), and optionally wherein said method further comprises the step of comparing the ratio of cholesteryl esters to cholesterol to a reference, whereby it is diagnosed whether the subject suffers from heart failure, or not.

10. The method of any one of claims 3 to 7, wherein said determining enzymatically the amount of total cholesterol as at least one cholesterol parameter comprises the steps of:

a) contacting the sample comprising the cholesterol with cholesterol oxidase under conditions and for a time sufficient to allow generation of $H_2O_2$; and
b) enzymatically or chemically determining the amount of generated $H_2O_2$.

11. The method of any one of claims 3 to 7, wherein said determining enzymatically the amount of total sphingomyelins comprises the steps of:

a) contacting the sample with sphingomyelinase under conditions and for a time sufficient to allow conversion into phosphorylcholine;
b) contacting the sample comprising the phosphorylcholine with alkaline phosphatase and choline oxidase under conditions and for a time sufficient to allow conversion into choline and subsequent generation of $H_2O_2$; and
c) enzymatically or chemically determining the amount of generated $H_2O_2$, and optionally wherein said method comprises the further steps of:

- contacting the sample prior to step a) with alkaline phosphatase and choline oxidase under conditions and for a time sufficient to allow conversion into choline and subsequent generation of $H_2O_2$ and, preferably, $H_2O_2$; and
- neutralizing the said $H_2O_2$.

12. The method of any one of claims 3 to 7, wherein said determining enzymatically the amount of total triacylglcerols comprises the steps of:

a) contacting the sample with lipase under conditions and for a time sufficient to allow conversion into glycerol and free fatty acids;
b) contacting the sample comprising the glycerol with glycerokinase under conditions and for a time sufficient to allow conversion into glycerol-3-phosphate;
c) contacting the sample comprising the glycerol-3-phosphate with glycerophosphate oxidase under conditions and for a time sufficient to allow conversion into dihydroxyacetone phosphate and $H_2O_2$; and
d) enzymatically or chemically determining the amount of generated $H_2O_2$.

13. Use of enzymatic detection agents for total triacylglycerols and for total sphingomyelins and optionally of detection agents for at least one cholesterol parameter selected from the group consisting of: total cholesterol, total cholesteryl esters and the sum parameter of total cholesterol and total cholesteryl esters for diagnosing in a sample of a subject whether the said subject suffers from heart failure, or not,

wherein the detection agents for the amount of total triacylglycerols are lipase, glycerokinase and glycerophosphate oxidase,

wherein the detection agents for the amount of total sphingomyelins are sphingomyelinase, choline oxidase and alkaline phosphatase, and

wherein the detection agents for the amount of at least one cholesterol parameter are i) cholesterol oxidase, ii) cholesterol esterase and cholesterol oxidase, iii) cholesterol dehydrogenase, or iv) cholesterol esterase and cholesterol dehydrogenase.

14. A kit adapted for diagnosing in a sample of a subject whether the said subject suffers from heart failure, or not, comprising enzymatic detection agents for total triacylglycerols and for total sphingomyelins, and wherein optionally said kit further comprises enzymatic detection agents for at least one cholesterol parameter selected from the group consisting of: total cholesterol, total cholesteryl esters and the sum parameter of total cholesterol and total cholesteryl esters,

wherein the detection agents for the amount of total triacylglycerols are lipase, glycerokinase and glycerophosphate oxidase,

wherein the detection agents for the amount of total sphingomyelins are sphingomyelinase, choline oxidase and alkaline phosphatase, and

wherein the detection agents for the amount of at least one cholesterol parameter are i) cholesterol oxidase, ii) cholesterol esterase and cholesterol oxidase, iii) cholesterol dehydrogenase, or iv) cholesterol esterase and cholesterol dehydrogenase.

**Patentansprüche**

1. Verfahren zum Diagnostizieren von Herzinsuffizienz bei einem Probanden, bei dem ein Verdacht besteht, dass er darunter leidet, umfassend:

   (a) Bestimmen der Menge der Gesamt-Triacylglycerine und der Gesamt-Sphingomyeline in einer Probe des Probanden; und
   (b) Vergleichen der in Schritt (a) bestimmten Menge(n) mit einer Referenz, wodurch diagnostiziert wird, ob der Proband an Herzinsuffizienz leidet oder nicht.

2. Verfahren nach Anspruch 1, zudem umfassend in Schritt a) die Bestimmung von mindestens einem Cholesterinparameter, ausgewählt aus der Gruppe, bestehend aus Gesamt-Cholesterin, Gesamt-Cholesterylestern und dem Summenparameter von Gesamt-Cholesterin und Gesamt-Cholesterylestern in einer Probe aus dem Probanden.

3. Verfahren nach Anspruch 1 oder 2, wobei die Menge mindestens eines Cholesterinparameters, der Gesamt-Triacylglycerine und/oder Gesamt-Sphingomyeline in einer Probe des Probanden enzymatisch bestimmt wird.

4. Verfahren nach einem der Ansprüche 1 bis 3, wobei

   a) die Referenz von einem Probanden oder einer Gruppe von Probanden stammt, von dem/denen bekannt ist, dass er/sie an Herzinsuffizienz leidet/leiden, insbesondere wobei eine identische oder verringerte Menge von mindestens einem Cholesterinparameter und/oder Gesamt-Sphingomyelinen in der Testprobe und der Referenz auf einen an Herzinsuffizienz leidenden Probanden deutet, während eine erhöhte Menge in der Testprobe im Vergleich zur Referenz auf einen Probanden deutet, der nicht an Herzinsuffizienz leidet, und/oder wobei eine identische oder erhöhte Menge an Gesamt-Triacylglycerinen in der Testprobe und der Referenz auf einen an Herzinsuffizienz leidenden Probanden deutet, während eine verringerte Menge in der Testprobe im Vergleich zur Referenz auf einen Probanden deutet, der nicht an Herzinsuffizienz leidet, oder
   b) die Referenz von einem Probanden oder einer Gruppe von Probanden stammt, von dem/denen bekannt ist, dass er/sie nicht an Herzinsuffizienz leidet/leiden, wobei eine identische oder erhöhte Menge von mindestens einem Cholesterinparameter und/oder Gesamt-Sphingomyelinen in der Testprobe und in der Referenz auf einen Probanden deutet, der nicht an Herzinsuffizienz leidet, während eine verringerte Menge in der Testprobe im Vergleich zu der Referenz auf einen Probanden deutet, der an Herzinsuffizienz leidet, und/oder wobei eine identische oder verringerte Menge an Gesamt-Triacylglycerinen in der Testprobe und in der Referenz auf einen Probanden deutet, der nicht an Herzinsuffizienz leidet, während eine erhöhte Menge in der Testprobe im Vergleich zu der Referenz abweicht, auf einen Probanden deutet, der an Herzinsuffizienz leidet.

5. Verfahren nach einem der Ansprüche 1 bis 4, wobei das Verfahren zudem den Schritt umfasst, bei dem eine therapeutische oder eine Maßnahme für das Patientengesundheitsmanagement für den Probanden, basierend darauf, ob bei dem Probanden eine Herzinsuffizienz diagnostiziert wird, empfohlen wird.

**6.** Verfahren nach einem der Ansprüche 1 bis 5, wobei die Probe eine Blut-, Plasma oder Serumprobe ist.

**7.** Verfahren nach einem der Ansprüche 1 bis 6, wobei die Herzinsuffizienz asymptomatische Herzinsuffizienz ist.

**8.** Verfahren nach einem der Ansprüche 3 bis 7, wobei das enzymatische Bestimmen der Menge an Gesamt-Cholestylestern als mindestens ein Cholesterinparameter die Schritte umfasst:

a) Inkontaktbringen der Probe mit Cholesterinesterase unter Bedingungen und für eine ausreichende Zeit, dass die Umwandlung in Cholesterin ermöglicht wird;
b) Inkontaktbringen der das Cholesterin umfassenden Probe mit Cholesterinoxidase unter Bedingungen und für eine ausreichende Zeit, dass die Erzeugung von $H_2O_2$ ermöglicht wird; und
c) enzymatisches oder chemisches Bestimmen der Menge an erzeugtem $H_2O_2$, und wobei das Verfahren gegebenenfalls die weiteren Schritte umfasst:

- Inkontaktbringen der Probe vor Schritt a) mit Cholesterinoxidase unter Bedingungen und für eine ausreichende Zeit, dass die Erzeugung von Redoxäquivalenten und vorzugsweise $H_2O_2$ ermöglicht wird; und
- Neutralisieren der Redoxäquivalente, und wobei gegebenenfalls das Neutralisieren des $H_2O_2$ das enzymatische oder chemische Bestimmen der erzeugten $H_2O_2$-Menge umfasst.

**9.** Verfahren nach Anspruch 8, wobei das Verfahren zudem die Erzeugung des Verhältnisses von Gesamt-Cholesterylestern zu Gesamt-Cholesterin basierend auf den vor Schritt a) bestimmten Mengen an von Cholesterin abgeleitetem $H_2O_2$ und dem in Schritt c) bestimmten von Cholesterylester abgeleitetem $H_2O_2$ umfasst und wobei das Verfahren gegebenenfalls zudem den Schritt umfasst, bei dem das Verhältnis von Cholesterylestern zu Cholesterin mit einer Referenz verglichen wird, wodurch diagnostiziert wird, ob der Proband an Herzinsuffizienz leidet oder nicht.

**10.** Verfahren nach einem der Ansprüche 3 bis 7, wobei das enzymatische Bestimmen der Menge an Gesamt-Cholesterin als mindestens ein Cholesterinparameter die Schritte umfasst:

a) Inkontaktbringen der das Cholesterin umfassenden Probe mit Cholesterinoxidase unter Bedingungen und für eine ausreichende Zeit, dass die Erzeugung von $H_2O_2$ ermöglicht wird; und
b) enzymatisches oder chemisches Bestimmen der Menge an erzeugtem $H_2O_2$.

**11.** Verfahren nach einem der Ansprüche 3 bis 7, wobei das enzymatische Bestimmen der Menge der Gesamt-Sphingomyeline die Schritte umfasst:

a) Inkontaktbringen der Probe mit Sphingomyelinase unter Bedingungen und für eine ausreichende Zeit, dass die Umwandlung in Phosphorylcholin ermöglicht wird;
b) Inkontaktbringen der das Phosphorylcholin umfassenden Probe mit alkalischer Phosphatase und Cholinoxidase unter Bedingungen und für eine ausreichende Zeit, dass die Umwandlung in Cholin und die nachfolgende Erzeugung von $H_2O_2$ ermöglicht wird; und
c) enzymatisches oder chemisches Bestimmen der Menge an erzeugtem $H_2O_2$, und wobei das Verfahren gegebenenfalls die weiteren Schritte umfasst:

- Inkontaktbringen der Probe vor Schritt a) mit alkalischer Phosphatase und Cholinoxidase unter Bedingungen und für eine ausreichende Zeit, dass die Umwandlung in Cholin und die nachfolgende Erzeugung von $H_2O_2$ und vorzugsweise $H_2O_2$ ermöglicht wird; und
- Neutralisieren des $H_2O_2$.

**12.** Verfahren nach einem der Ansprüche 3 bis 7, wobei das enzymatische Bestimmen der Menge an Gesamt-Triacylglcerinen die Schritte umfasst:

a) Inkontaktbringen der Probe mit Lipase unter Bedingungen und für eine ausreichende Zeit, dass die Umwandlung in Glycerin und freie Fettsäuren ermöglicht wird;
b) Inkontaktbringen der das Glycerin umfassenden Probe mit Glycerokinase unter Bedingungen und für eine ausreichende Zeit, dass die Umwandlung in Glycerin-3-phosphat ermöglicht wird;
c) Inkontaktbringen der das Glycerin-3-phosphat umfassenden Probe mit Glycerophosphatoxidase unter Bedingungen und für eine ausreichende Zeit, dass die Umwandlung in Dihydroxyacetonphosphat und $H_2O_2$ ermöglicht wird; und

d) enzymatisches oder chemisches Bestimmen der Menge an erzeugtem $H_2O_2$.

**13.** Verwendung enzymatischer Nachweismittel für Gesamt-Triacylglycerine und für Gesamt-Sphingomyeline und gegebenenfalls von Nachweismitteln für mindestens einen Cholesterinparameter, ausgewählt aus der Gruppe, bestehend aus Gesamt-Cholesterin, Gesamt-Cholesterylestern und dem Summenparameter von Gesamt-Cholesterin und Gesamt-Cholesterylestern zur Diagnose in einer Probe eines Probanden, ob der Proband an Herzinsuffizienz leidet oder nicht,
wobei die Nachweismittel für die Menge der Gesamt-Triacylglycerine Lipase, Glycerokinase und Glycerophosphatoxidase sind,
wobei die Nachweismittel für die Menge der Gesamt-Sphingomyeline Sphingomyelinase, Cholinoxidase und alkalische Phosphatase sind, und
wobei die Nachweismittel für die Menge mindestens eines Cholesterinparameters i) Cholesterinoxidase, ii) Cholesterinesterase und Cholesterinoxidase, iii) Cholesterindehydrogenase oder iv) Cholesterinesterase und Cholesterindehydrogenase sind.

**14.** Kit, eingerichtet zur Diagnose in einer Probe eines Probanden, ob der Proband an Herzinsuffizienz leidet oder nicht, umfassend enzymatische Nachweismittel für Gesamt-Triacylglycerine und für Gesamt-Sphingomyeline, und wobei der Kit gegebenenfalls zudem enzymatische Nachweismittel für mindestens einen Cholesterinparameter, ausgewählt aus der Gruppe, bestehend aus Gesamt-Cholesterin, Gesamt-Cholesterylestern und dem Summenparameter von Gesamt-Cholesterin und Gesamt-Cholesterylestern umfasst,
wobei die Nachweismittel für die Menge der Gesamt-Triacylglycerine Lipase, Glycerokinase und Glycerophosphatoxidase sind,
wobei die Nachweismittel für die Menge der Gesamt-Sphingomyeline Sphingomyelinase, Cholinoxidase und alkalische Phosphatase sind, und
wobei die Nachweismittel für die Menge mindestens eines Cholesterinparameters i) Cholesterinoxidase, ii) Cholesterinesterase und Cholesterinoxidase, iii) Cholesterindehydrogenase oder iv) Cholesterinesterase und Cholesterindehydrogenase sind.

**Revendications**

**1.** Procédé de diagnostic d'une insuffisance cardiaque chez un sujet suspecté de souffrir de celle-ci comprenant :

(a) la détermination de la quantité de triacylglycérols totaux et la quantité de sphingomyélines totales dans un échantillon dudit sujet ; et
(b) la comparaison de la/des quantité(s) déterminée(s) dans l'étape (a) à une référence, de sorte qu'il soit diagnostiqué si le sujet souffre d'insuffisance cardiaque, ou non.

**2.** Procédé selon la revendication 1, comprenant en outre, dans l'étape a) la détermination d'au moins un paramètre de cholestérol choisi dans le groupe constitué des : cholestérol total, esters de cholestéryle totaux et le paramètre de somme du cholestérol total et des esters de cholestéryle totaux dans un échantillon du sujet.

**3.** Procédé selon la revendication 1 ou 2, dans lequel la quantité d'au moins un paramètre de cholestérol, des triacylglycérols totaux et/ou des sphingomyélines totales dans un échantillon dudit sujet est déterminée de façon enzymatique.

**4.** Procédé selon l'une quelconque des revendications 1 à 3, dans lequel

a) ladite référence est dérivée d'un sujet ou groupe de sujets connu comme souffrant d'insuffisance cardiaque, en particulier dans lequel une quantité identique ou réduite d'au moins un paramètre de cholestérol et/ou des sphingomyélines totales dans l'échantillon d'essai et la référence est indicative d'un sujet souffrant d'insuffisance cardiaque tandis qu'une quantité augmentée dans l'échantillon d'essai par rapport à la référence est indicative d'un sujet ne souffrant pas d'insuffisance cardiaque, et/ou dans lequel une quantité identique ou augmentée des triacylglycérols totaux dans l'échantillon d'essai et la référence est indicative d'un sujet souffrant d'insuffisance cardiaque, tandis qu'une quantité réduite dans l'échantillon d'essai par rapport à la référence est indicative d'un sujet ne souffrant pas d'insuffisance cardiaque, ou
b) ladite référence est dérivée d'un sujet ou groupe de sujets connu comme ne souffrant pas d'insuffisance cardiaque, dans lequel une quantité identique ou augmentée d'au moins un paramètre de cholestérol et/ou de sphingomyélines totales dans l'échantillon d'essai et la référence est indicative d'un sujet ne souffrant pas d'insuffisance cardiaque tandis qu'une quantité réduite dans l'échantillon d'essai par rapport à la référence est indicative d'un sujet souffrant d'insuffisance cardiaque, et/ou dans lequel une quantité identique ou réduite de triacylglycérols totaux dans l'échantillon d'essai et la référence est indicative d'un sujet ne souffrant pas d'insuffisance cardiaque tandis qu'une quantité augmentée dans l'échantillon d'essai par rapport à la référence diffère est indicative d'un sujet souffrant d'insuffisance cardiaque.

5. Procédé selon l'une quelconque des revendications 1 à 4, dans lequel ledit procédé comprend en outre l'étape de recommandation d'une mesure thérapeutique ou de prise en charge de la santé du patient pour le sujet sur la base du fait que le sujet est ou non diagnostiqué comme souffrant d'insuffisance cardiaque.

6. Procédé selon l'une quelconque des revendications 1 à 5, dans lequel ledit échantillon est un échantillon de sang, de plasma ou de sérum.

7. Procédé selon l'une quelconque des revendications 1 à 6, dans lequel ladite insuffisance cardiaque est une insuffisance cardiaque asymptomatique.

8. Procédé selon l'une quelconque des revendications 3 à 7, dans lequel ladite détermination enzymatique de la quantité d'esters de cholestéryle totaux en tant qu'au moins un paramètre de cholestérol comprend les étapes de :

   a) mise en contact de l'échantillon avec une cholestérol estérase dans des conditions et pendant un temps suffisant pour permettre la conversion en cholestérol ;
   b) mise en contact de l'échantillon comprenant le cholestérol avec une cholestérol oxydase dans des conditions et pendant un temps suffisant pour permettre la génération de $H_2O_2$ ; et
   c) détermination enzymatique ou chimique de la quantité de $H_2O_2$ généré et ledit procédé comprenant facultativement les étapes supplémentaires de :

   - mise en contact de l'échantillon avant l'étape a) avec une cholestérol oxydase dans des conditions et pendant un temps suffisant pour permettre la génération d'équivalents redox et, de préférence, $H_2O_2$ ; et
   - neutralisation desdits équivalents redox, et facultativement, dans lequel ladite neutralisation dudit $H_2O_2$ comprend la détermination enzymatique ou chimique de la quantité de $H_2O_2$ généré.

9. Procédé selon la revendication 8, ledit procédé comprenant en outre la génération du rapport de l'ester de cholestéryle total au cholestérol total sur la base des quantités du $H_2O_2$ dérivé de cholestérol déterminées avant l'étape a) et du $H_2O_2$ dérivé d'ester de cholestéryle déterminé dans l'étape c), et ledit procédé comprenant facultativement en outre l'étape de comparaison du rapport des esters de cholestéryle au cholestérol à une référence, de sorte qu'il soit diagnostiqué si le sujet souffre d'insuffisance cardiaque, ou non.

10. Procédé selon l'une quelconque des revendications 3 à 7, dans lequel ladite détermination enzymatique de la quantité de cholestérol total en tant qu'au moins un paramètre de cholestérol comprend les étapes de :

   a) mise en contact de l'échantillon comprenant le cholestérol avec une cholestérol oxydase dans des conditions et pendant un temps suffisant pour permettre la génération de $H_2O_2$ ; et
   b) détermination enzymatique ou chimique de la quantité de $H_2O_2$ généré.

11. Procédé selon l'une quelconque des revendications 3 à 7, dans lequel ladite détermination enzymatique de la quantité de sphingomyélines totales comprend les étapes de :

   a) mise en contact de l'échantillon avec une sphingomyélinase dans des conditions et pendant un temps suffisant pour permettre la conversion en phosphorylcholine ;
   b) mise en contact de l'échantillon comprenant la phosphorylcholine avec une phosphatase alcaline et une choline oxydase dans des conditions et pendant un temps suffisant pour permettre la conversion en choline et la génération consécutive de $H_2O_2$ ; et
   c) détermination enzymatique ou chimique de la quantité de $H_2O_2$ généré, et ledit procédé comprenant facultativement les étapes supplémentaires de :

   - mise en contact de l'échantillon avant l'étape a) avec une phosphatase alcaline et une choline oxydase dans des conditions et pendant un temps suffisant pour permettre la conversion en choline et la génération

consécutive de $H_2O_2$ et, de préférence, $H_2O_2$ ; et
- neutralisation dudit $H_2O_2$.

12. Procédé selon l'une quelconque des revendications 3 à 7, dans lequel ladite détermination enzymatique de la quantité de triacylglycérols totaux comprend les étapes de :

a) mise en contact de l'échantillon avec une lipase dans des conditions et pendant un temps suffisant pour permettre la conversion en glycérol et en acides gras libres ;
b) mise en contact de l'échantillon comprenant le glycérol avec une glycérokinase dans des conditions et pendant un temps suffisant pour permettre la conversion en glycérol-3-phosphate ;
c) mise en contact de l'échantillon comprenant le glycérol-3-phosphate avec une glycérophosphate oxydase dans des conditions et pendant un temps suffisant pour permettre la conversion en dihydroxyacétone phosphate et en $H_2O_2$ ; et
d) détermination enzymatique ou chimique de la quantité de $H_2O_2$ généré.

13. Utilisation d'agents de détection enzymatique pour les triacylglycérols totaux et pour les sphingomyélines totales et facultativement d'agents de détection pour au moins un paramètre de cholestérol choisi dans le groupe constitué des : cholestérol total, esters de cholestéryle totaux et le paramètre de somme du cholestérol total et des esters de cholestéryle totaux dans un échantillon du sujet pour diagnostiquer dans un échantillon d'un sujet si ledit sujet souffre d'insuffisance cardiaque, ou non,
dans laquelle les agents de détection de la quantité de triacylglycérols totaux sont une lipase, une glycérokinase et une glycérophosphate oxydase,
dans laquelle les agents de détection de la quantité de sphingomyélines totales sont une sphingomyélinase, une choline oxydase et une phosphatase alcaline, et
dans laquelle les agents de détection de la quantité d'au moins un paramètre de cholestérol sont i) une cholestérol oxydase, ii) une cholestérol estérase et une cholestérol oxydase, iii) une cholestérol déshydrogénase, ou iv) une cholestérol estérase et une cholestérol déshydrogénase.

14. Trousse adaptée pour diagnostiquer dans un échantillon d'un sujet si ledit sujet souffre d'insuffisance cardiaque, ou non, comprenant des agents de détection enzymatique pour les triacylglycérols totaux et pour les sphingomyélines totales, et dans laquelle, facultativement, ladite trousse comprend en outre des agents de détection enzymatique pour au moins un paramètre de cholestérol choisi dans le groupe constitué des : cholestérol total, esters de cholestéryle totaux et le paramètre de somme du cholestérol total et des esters de cholestéryle totaux, dans laquelle les agents de détection de la quantité de triacylglycérols totaux sont une lipase, une glycérokinase et une glycérophosphate oxydase, dans laquelle les agents de détection de la quantité de sphingomyélines totales sont une sphingomyélinase, une choline oxydase et une phosphatase alcaline, et
dans laquelle les agents de détection de la quantité d'au moins un paramètre de cholestérol sont i) une cholestérol oxydase, ii) une cholestérol estérase et une cholestérol oxydase, iii) une cholestérol déshydrogénase, ou iv) une cholestérol estérase et une cholestérol déshydrogénase.

Fig. 1

Fig. 2

$$\text{Sphingomyelin} \xrightarrow{\text{Sphingomyelinase}} \text{Ceramide} \quad + \quad \text{Phosphorylcholine}$$

Alkaline
Phosphatase

Fluorescence at
570 nm

$$H_2O_2 \quad + \quad \text{Betaine Aldehyde} \xleftarrow{\text{Choline Oxidase}} \text{Choline}$$

Fig. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2013014286 A
- WO 2003073464 A
- WO 2002089657 A
- WO 2002083913 A

### Non-patent literature cited in the description

- New York Heart Association. Diseases of the heart and blood vessels. Nomenclature and criteria for diagnosis. Little, Brown and co, 1964, 114
- **FOX C.S. et al.** *Circulation,* 2004, vol. 110, 522-527
- **ROGER VL ; WESTON SA ; REDFIELD MM et al.** *JAMA-J. Am. Med. Assoc.,* 2004, vol. 292, 344-350
- The SOLVD Investigators. *NEJM,* 1991, vol. 325, 293-302
- The SOLVD Investigators. *New Engl. J. Med.,* 1992, vol. 327, 685-691
- The Consensus Trial Study Group. *New Engl. J. Med.,* 1987, vol. 316, 1429-1435
- **ZORDOKY et al.** Metabolomic Fingerprint of Heart Failure with Preserved Ejection Fraction. *PLoS ONE,* 2015, vol. 10 (5), e0124844
- **RODEHEFFER, R.J.** *J. Am. Coll. Cardiol.,* 2004, vol. 44, 740-749
- **BALION C. et al.** AHRQ Comparative Effectiveness Reviews. Agency for Healthcare Research and Quality (US), 2013
- **EWALD.** *Intern Med J,* 2008, vol. 38 (2), 101-13
- **DOWDY ; WEARDEN.** Statistics for Research. John Wiley & Sons, 1983
- **ELLINGTON.** *Nature,* 1990, vol. 346 (6287), 818-22
- **BOCK.** *Nature,* 1992, vol. 355 (6360), 564-6
- **HELD.** *Biospektrum 2/05,* 2005, 242-243
- **AMUDSON.** *Biochem Biophys Methods,* 1999, vol. 38, 43-52
- **ZWEIG.** *Clin. Chem.,* 1993, vol. 39, 561-577
- **ZOU.** *Journal of the Royal Statistical Society,* 2005, 301-320
- **FRIEDMAN.** *J. Stat. Sotw.,* 2010, 33
- *European Heart Journal,* 2012, vol. 33, 1787-1847
- *Circulation,* 2013, vol. 128, e240-e327
- **CHRISTIE.** *Journal of Lipid Research,* 1985, vol. 26, 507-512
- **HAMMAD.** *J Lipid Res.,* 2010, vol. 51 (10), 3074-87
- **SCHERER.** *Biochimica et Biophysica Acta,* 2011, vol. 1811, 68-75
- **LINDGREN.** *Am J Clin Nutr.,* 1961, vol. 9, 13-23
- **QUEHENBERGER et al.** *J Lipid Res.,* 2010, vol. 51, 3299-3305
- Methods of Enzymatic Analysis. Academic Press Inc, 1974, 1831
- **ZOU, H. ; HASTIE, T.** *J. Roy. Stat. Soc. B Met.,* 2003, vol. 67, 301-320
- **FRIEDMAN, J. ; HASTIE, T. ; TIBSHIRANI, R.** *J. Stat. Softw.,* 2010, vol. 33
- **CURTIS et al.** *Arch. Intern, Med,* 2008, vol. 168, 418-424
- **YANCY et al.** *J. Am. Coll. Cardiol.,* 2013, vol. 62, e147-e239